(19) Europäisches Patentamt
European Patent Office
Office européen des brevets

(11) Numéro de publication : **0 084 999**
**B1**

# (12) FASCICULE DE BREVET EUROPÉEN

(45) Date de publication du fascicule du brevet : **13.04.88**

(21) Numéro de dépôt : **83400110.9**

(22) Date de dépôt : **17.01.83**

(51) Int. Cl.[4] : **C 07 H 3/04**, C 07 H 3/06, C 07 H 11/00, A 61 K 31/70, A 61 K 31/725, C 08 B 37/10, G 01 N 33/48, C 07 H 7/02

## (54) Procédé de synthèse organique d'oligosaccharides, correspondant à des fragments de muco-polysaccharides naturels, nouveaux oligosaccharides obtenus et leurs applications biologiques.

(30) Priorité : **15.01.82 FR 8200621
01.02.82 FR 8201575
16.02.82 FR 8202526
28.05.82 FR 8209392
22.06.82 FR 8210891
22.06.82 FR 8210892
02.07.82 FR 8211679
06.08.82 FR 8213804
20.09.82 FR 8215804
20.09.82 FR 8215803
27.10.82 FR 8218003**

(43) Date de publication de la demande :
**03.08.83 Bulletin 83/31**

(45) Mention de la délivrance du brevet :
**13.04.88 Bulletin 88/15**

(84) Etats contractants désignés :
**AT BE CH DE FR GB IT LI LU NL SE**

(56) Documents cités :
**EP-A- 0 014 184
EP-A- 0 027 089
EP-A- 0 048 231
EP-A- 0 064 012
CHEMICAL ABSTRACTS, vol. 55, no. 26, 25 décembre 1961, colonne 27533e, Columbus, Ohio, USA; S.A. BARKER: "Some enzyme transfer reactions involving 2-acetamido-2-deoxy-D-glucose"
CARBOHYDRATE RESEARCH, vol. 78, 1980, pages 249-256, Elsevier Scientific Publishing Company, Amsterdam, NL; U. KLEIN et al.:"A 3H-labelled trisaccharide from heparin as substrate for acetyl-CoA: 2-amino-2-deoxy-alpha-D-glucoside N-acetyltransferasé"**

(73) Titulaire : **D.R.O.P.I.C. (Société Civile)
10, Avenue Matignon
F-75008 Paris (FR)**

(72) Inventeur : **Petitou, Maurice
27, rue du Javelot
F-75645 Paris Cedex 13 (FR)**
Inventeur : **Jacquinet, Jean-Claude
1, Allée André Gide
F-45100 Orleans la Source (FR)**
Inventeur : **Sinay, Pierre
5, rue Jacques Monod
F-45100 Orleans la Source (FR)**
Inventeur : **Choay, Jean
21, rue Saint-Guillaume
F-75007 Paris (FR)**
Inventeur : **Lormeau, Jean-Claude
1, place Joseph Delattre
F-76150 Maromme (FR)**
Inventeur : **Nassr, Mahmoud Alexandria University
Faculty fo Sciences Chemistry Department
Alexandria (EG)**

(74) Mandataire : **Gutmann, Ernest et al
S.C. Ernest Gutmann - Yves Plasseraud 67, boulevard Haussmann
F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

CARBOHYDRATE RESEARCH, vol. 103, no. 1, mai 1982, pages 190-194, Elsevier Scientific Publishing Company, Amsterdam NL ; N. SENO et al. : "Structure of disulfated disaccharides from chondroitin polysulfates chondroitin sulfate D and K"

CARBOHYDRATE RESEARCH, vol. 105, no. 1, juillet 1982, pages 69-85, Elsevier Scientific Publishing Company, Amsterdam, NL. A. OGAMO et al.: "Preparation and properties of fluorescent glycosaminoglycuronans labeled with 5-aminofluorescein

PURE & APPL. CHEM., vol. 50, 1978, pages 1437-1452, Pergamon Press Ltd., GB; P. SINAY: "Recent advances in glycosylation reactions"

METHODS CARBOHYDR. CHEM., vol. 8, 1980, pages 305-311, GB; P. A. MANTHORPE et al.: "Allyl ethers as protecting groups. Synthesis of 3-0-[6-0-(alpha-D-galactopyranosy 1)-beta-D-galactopyranosyl]-1,2-d i-O-stearoyl-L-glycerol, a "digalactosyl diglyceride""

TETRAHEDRON LETTERS, no. 30, 1972, pages 3055-3058, Pergamon Press, GB; J. KISS et al.: "Syntheses of heparin saccharides. Stereospecific synthesis of derivatives of 2-amino-2-deoxy-4-O(alpha-D-glucopyranuronosyl)-D-glucose"

HELVETICA CHIMICA ACTA, vol. 58, Fasc. 6, 1975, pages 204-205, P. C. WYSS et al.: "205. Synthesis of heparin saccharides IV. Synthesis of disaccharides possessing the structure of a repeating unit of heparin"

TETRAHEDRON LETTERS, no. 5, 1972, pages 431-433, Pergamon Press, GB; A. KLEMER et al.: "Synthese von Athyl-2-Amino-2-Desoxy-4-0-(beta-D-Glucuronopyranosyl)-alpha,beta-D-Glucopyranosid"

(56) Documents cités :

CARBOHYDRATE RESEARCH, vol. 87, 1980, pages 297-301, Elsevier Scientific Publishing Company, Amsterdam, NL; L. AYOTTE et al.: "Fractionation of heparin and heparan sulfate as barium salts: high-field, n.m.r.-spectral observations on heterogeneity"

THE JOURNAL OF BIOCHEMISTRY, vol. 92, no. 1, juillet 1982, pages 295-303, Pub. The Japanese Biochemical Society, M. KOSAKAI et al.: "High performance liquid chromatography of pyridylamino derivatives of sulfated oligosaccharides in the deamination products of porcine and whale heparins"

CHEMICAL ABSTRACTS, vol. 97, no. 19, 8 novembre 1982, page 512, no. 160637u, Columbus, Ohio, USA; J. J. HOPWOOD et al.: "Diagnosis of Sanfilippo type A syndrome by estimation of sulfamidase activity using a radiolabeled tetrasaccharide substrate"

**Description**

L'invention est relative à un procédé de synthèse organique d'oligosaccharides constituant ou comportant des fragments de mucopolysaccharides acides. Elle concerne également la synthèse de dérivés de ces oligosaccharides.

L'invention se rapporte, en outre, à de nouveaux oligosaccharides du genre indiqué ci-dessus et à leurs dérivés, possédant, notamment, des propriétés biologiques leur conférant, en particulier, un intérêt en tant que médicaments et/ou utilisables, par exemple, comme réactifs de laboratoire.

Elle vise également leurs applications notamment biologiques et biochimiques.

Par le terme « mucopolysaccharide acide », on désigne des dérivés également couramment appelés glycosaminoglycuronoglycanes. Il s'agit d'oligosaccharides et polysaccharides rencontrés plus spécialement dans les chaînes de dérivés biologiquement actifs tels que les dérivés du type héparine et héparane-sulfate.

Dans les produits naturels, les mucopolysaccharides en question sont essentiellement formés de motifs alternés sucre aminé-acide uronique, ou inversement. Dans ces motifs, le sucre aminé, désigné ci-après par A, présente plus spécialement une structure D-glucosamine.

L'acide uronique, qu'on appellera U, présente, plus spécialement, une structure acide D-glucuronique ou acide L-iduronique.

Les structures de base pour A répondent respectivement à la formule a et pour U aux formules b et c ci-dessous :

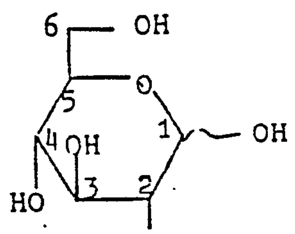

dérivé d'amine
(a) D-glucosamine

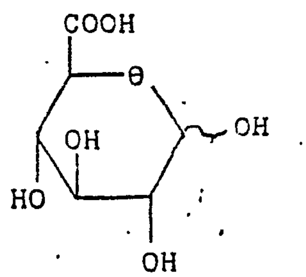

(b) acide D-glucuronique

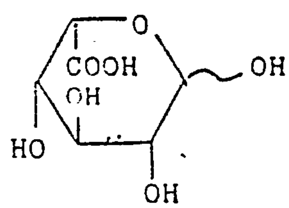

(c) acide L-iduronique

Dans les produits naturels en question, ces différents motifs sont liés entre eux de manière stéréospécifique généralement par des liaisons $1 \xrightarrow{\alpha} 4$ et $1 \xrightarrow{\beta} 4$.

On trouve ainsi, par exemple dans l'héparine, des liaisons de type $1 \xrightarrow{\alpha} 4$ (entre les motifs c et a, a et b, et a et c) et de type $1 \xrightarrow{\beta} 4$ (entre les motifs b et a).

On notera, en outre, toujours en se référant aux produits naturels, que les motifs ci-dessus comportent des substitutions spécifiques, c'est-à-dire des substitutions déterminées en des positions données. Les chaînes des produits naturels renferment, ainsi, par exemple, des motifs O-substitués acide 2-O-sulfate-L-iduronique, 3-O-sulfate-D-glucosamine, 3,6-di-O-sulfate-D-glucosamine, 6-O-sulfate-D-glucosamine et des motifs non-O-substitués, ainsi, par exemple, des motifs acide D-glucuronique, L-iduronique et D-glucosamine. En outre, les motifs a sont N-substitués en position 2 par des groupes —N-acétyle et/ou —N-sulfate.

On connaît l'importance des applications thérapeutiques des mucopolysaccharides acides ci-dessus, en particulier, pour la prévention et pour le traitement des troubles de la coagulation et de la paroi vasculaire, et en particulier des thromboses et des athéroscléroses et artérioscléroses.

On connaît, par ailleurs, les nombreux travaux de la Demanderesse pour l'obtention de fragments de haute affinité pour l'AT III et biologiquement actifs à partir de chaînes d'héparine. Les inventions mises au point sur la base de ces travaux font l'objet de différentes demandes de brevets dont la demande de brevet EP-A-27089 et la demande de brevet EP-A-64452.

On rappelle que dans la demande EP-A-27089, on décrit notamment un octasaccharide appelé ABCDEFGH possédant des propriétés antithrombotiques de grand intérêt, répondant à la structure :

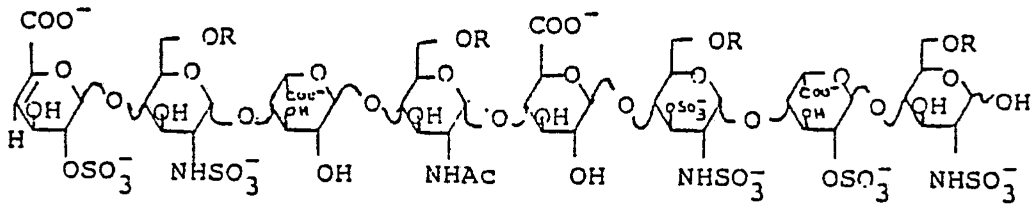

(A) (B) (C) (D) (E) (F) G H

Dans cette formule, R représente un groupe $SO_3^-$ ou un atome d'hydrogène.

Dans la demande FR ci-dessus de la Demanderesse, on décrit une composition homogène d'hexasaccharides de structure C'DEFGH, possédant également des propriétés antithrombotiques élevées. Cette structure répond à la formule :

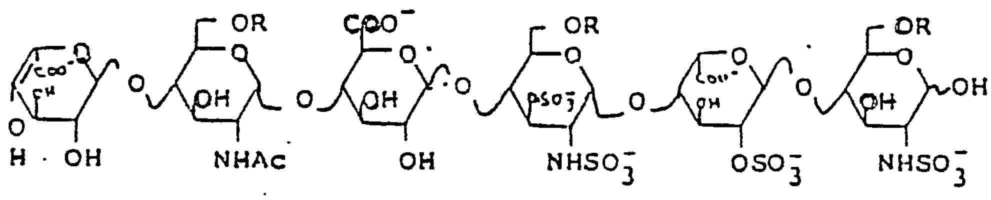

(C)' (D) (E) (F) G H

dans laquelle R représente un groupe $SO_3^-$ ou un atome d'hydrogène.

Les procédés proposés à ce jour pour obtenir ce type de produits mettent en jeu des techniques d'extraction à partir d'héparine ou de produits obtenus au cours de la préparation de l'héparine, ou encore des techniques de dépolymérisation des chaînes d'héparine sous l'action d'un agent chimique ou enzymatique, suivi de fractionnement spécifique notamment par chromatographie affine.

L'état d'avancement des travaux des inventeurs dans ce domaine les a conduits à rechercher de nouveaux moyens permettant d'accéder à ce type de produits et plus spécialement à étudier les possibilités de les obtenir par voie de synthèse.

A cet égard, il convient de mesurer le nombre de problèmes soulevés par une telle synthèse. En effet, d'une part, ces produits renferment dans leurs chaînes plusieurs types de motifs A et U. D'autre part, les liaisons entre ces motifs répondent à une stéréochimie donnée et sont de type 1, 4, dont les difficultés de réalisation particulières sont bien connues. En outre, chaque motif comporte une ou plusieurs substitutions spécifiques selon le type de produit considéré. On considérera également que les motifs glucosamine dans les produits naturels comportent deux groupes azotés différents l'un de l'autre, à savoir un groupe N-acétyle et un groupe —N-sulfate.

Il s'ensuit que de telles synthèses n'ont pratiquement jamais été envisagées jusqu'à présent dans la littérature scientifique, tout particulièrement, en ce qui concerne l'acide L-iduronique.

Tous ces éléments fixent des impératifs contraignants dont il est aisé d'apprécier les difficultés qu'ils engendrent pour l'élaboration d'un processus général et du processus de synthèse.

En recherchant des conditions de synthèse osidique appropriées à l'élaboration de ce type de composés, les inventeurs ont développé une stratégie en choisissant certains types de protection particuliers pour les produits mis en œuvre.

Les travaux effectués ont alors montré qu'avec de tels produits ainsi protégés, il était possible de réaliser un enchaînement stéréospécifique puis d'introduire, si souhaité, sur les conséquences formées, des substitutions données en des positions déterminées.

Selon un aspect présentant un intérêt dont on mesurera l'importance, le procédé mis au point présente une grande souplesse. Il est ainsi possible d'accéder, avec les avantages, notamment de spécificité et de pureté, liés à un processus de synthèse, à de nombreux dérivés d'oligosaccharides

comportant les substitutions spécifiques rencontrées avec les produits naturels, ou même des substitutions différentes et/ou encore des motifs de structure analogue avec des configurations différentes.

Grâce à ce procédé, les inventeurs ont obtenu des oligosaccharides dotés notamment de propriétés médicamenteuses de grande valeur et plus spécialement à activité antithrombotique élevée. Le procédé de l'invention permet également d'accéder à un grand nombre d'oligosaccharides particulièrement précieux, notamment comme réactifs biologiques et/ou comme composés de référence pour des études de structures.

L'invention a donc pour but de fournir un procédé d'obtention, par voie de synthèse, d'oligosaccharides et de leurs dérivés ou analogues, comportant ou correspondant à des fragments de mucopolysaccharides acides.

Elle a également pour but de fournir des moyens permettant d'établir entre des motifs de type A et U des liaisons glycosidiques selon la stéréospécificité souhaitée.

Elle vise également à fournir des moyens permettant d'introduire sur les motifs de la chaîne glycosidique des groupes fonctionnels donnés, en particulier des substituants spécifiques tels que rencontrés dans les chaînes de molécules biologiquement actives, notamment celles du type héparine et héparane-sulfate.

Elle a également pour but de fournir des moyens permettant d'obtenir des oligosaccharides tels qu'évoqués ci-dessus, mais dont les substituants et/ou la nature chimique des sucres et/ou la position et la configuration des liaisons interglycosidiques et/ou la configuration des monosaccharides et/ou l'ordre des enchaînements sont différents de ceux des produits naturels.

Selon un autre aspect, l'invention a également pour but de fournir de nouveaux oligosaccharides constituant des produits intermédiaires du procédé de synthèse en question dans lesquels tous les groupes —OH des différents motifs sont bloqués par des groupements protecteurs et les groupements précurseurs des radicaux fonctionnels éventuellement présents ; le cas échéant, ces radicaux euxmêmes sont également protégés.

Selon encore un autre aspect, l'invention vise à fournir de nouveaux oligosaccharides présentant la structure des produits naturels ci-dessus ainsi que des oligosaccharides correspondant à des fragments de ces produits.

Elle vise également à fournir de nouveaux oligosaccharides possédant les substitutions spécifiques des produits naturels.

L'invention vise, en outre, à fournir de nouveaux oligosaccharides portant des substitutions différentes de celles des substitutions spécifiques en question et/ou comportant des motifs différents par rapport aux produits naturels considérés ci-dessus.

L'invention vise également les applications biologiques de ces oligosaccharides, notamment en tant que substances actives de médicaments, réactifs de laboratoire ou produits de référence pour l'étude, en particulier, de composés comportant ce type de structure.

Le procédé de synthèse organique, selon l'invention, d'oligosaccharides comprenant des enchaînements [D-glucosamine] - [acide D-glucuronique ou acide L-iduronique], ou l'inverse, tels que rencontrés respectivement dans l'héparine ou l'héparane sulfate est caractérisé par le fait :

— qu'on met en œuvre, dans une réaction de glycosylation, deux composés terminés ou constitués respectivement par un motif A de structure D-glucosamine et un motif U de structure acide D-glucuronique ou acide L-iduronique, l'un des motifs A ou U étant un alcool dans lequel le groupe —OH de la fonction alcool occupe la position 4, l'autre motif possédant un carbone anomère activé, substitué par un groupe réactif compatible avec les autres groupements présents sur les motifs, toutes les autres positions de A et U, excepté celle dont le carbone anomère est activé et celle occupée par le groupe —OH de la fonction alcool, portant, soit des groupes amino, soit des groupes carboxyle, ou des précurseurs de ces groupes, soit encore des groupes —OH, ces groupes occupant des positions déterminées, les groupes amino et carboxyle lorsqu'ils sont présents, étant bloqués respectivement par des groupements protecteurs de fonction amino et carboxyle, les groupes —OH étant bloqués par au moins deux types de groupes protecteurs, éliminables séquentiellement en permettant l'introduction de groupes désirés en certaines positions, puis la libération de groupes —OH en d'autres positions,

— qu'on répète, si on le souhaite, l'opération de glycosylation afin d'allonger la chaîne oligosaccharidique,

— qu'on libère séquentiellement les groupements protecteurs des groupes —OH en introduisant des groupements de substitution désirés dans des positions spécifiques, puis en libérant les groupements —OH d'autres positions spécifiques ainsi que les groupes amino et carboxyle sous réserve que l'établissement de la liaison interglycosidique ne conduise pas à l'obtention d'un disaccharide à structure [2-N-sulfate ou 2-N-acétyl] - [6-O-sulfate-D-glucosamine] - [acide méthyl-D-glucuronique].

Grâce aux dispositions ci-dessus, il est ainsi possible de réaliser une liaison convalente entre des motifs de structure A et U et ce, selon la stéréochimie que présente ce type d'enchaînement dans les molécules biologiquement actives déjà considérées.

Il est même possible à l'aide de l'invention de réaliser les enchaînements souhaités selon un ordre donné et/ou possédant une stéréospécificité donnée.

Les moyens proposés selon l'invention permettent ainsi d'établir notamment une liaison de type

$1 \xrightarrow{\alpha} 4$ entre un motif D-glucosamine et soit acide D-glucuronique, soit acide L-iduronique, une liaison de type $1 \xrightarrow{\beta} 4$ entre un motif acide D-glucuronique et un motif D-glucosamine et une liaison de type $1 \xrightarrow{\alpha} 4$ entre un motif acide L-iduronique et un motif D-glucosamine.

Les intermédiaires mono ou oligosaccharides de cette synthèse sont des produits semi-ouverts ou ouverts. On appellera composé semi-ouvert à droite un composé activé ou potentiellement activable sur son carbone anomère, permettant ainsi son transfert sur l'extrémité non réductrice d'un monosaccharide ou d'un oligosaccharide. L'expression « composé semi-ouvert à gauche » désignera un monosaccharide ou un oligosaccharide possédant une seule fonction —OH libre ou potentiellement libre, permettant sa glycosylation spécifique. A titre illustratif, on indique ci-après la formule 1 d'un exemple de composé semi-ouvert à gauche et celle 2 d'un exemple de composé semi-ouvert à droite :

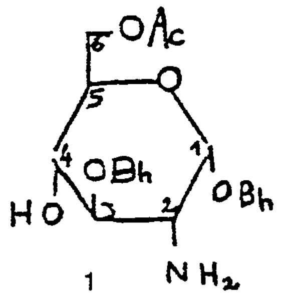

1

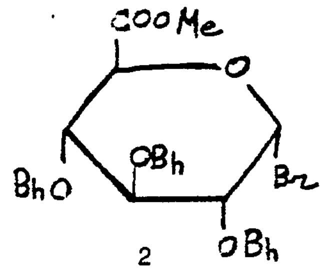

2

Il s'ensuit qu'on appellera dérivés ouverts un dérivé semi-ouvert à la fois à droite et à gauche selon la définition ci-dessus, de tels dérivés autorisant une élongation de chaîne dans les deux directions. Un dérivé de ce type répond par exemple à la formule 3 :

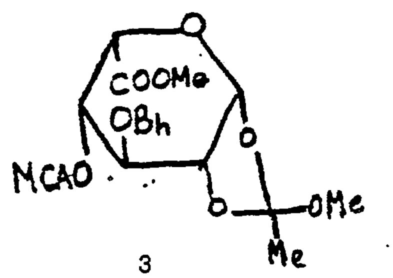

3

Quant aux dérivés fermés, il s'agit de produits dont les motifs ne peuvent pas donner lieu à élongation de chaînes en raison de la nature de leurs substituants.

Selon une disposition supplémentaire pour pouvoir ajouter des motifs à la séquence A-U ou U-A formée dans l'étape précédente, les motifs A ou U de la séquence formée doivent renfermer des groupements protecteurs temporaires, c'est-à-dire des groupements capables de bloquer sélectivement une position du motif A ou U destinée à intervenir dans une nouvelle réaction de glycosylation. Ces groupements sont éliminables en présence des autres groupements présents sur les motifs des produits de départ en recréant un alcool, ce qui permet en répétant l'étape précédente de glycosylation d'allonger le squelette glucidique.

L'invention donne donc accès à la synthèse d'oligosaccharides à enchaînements variés, qu'il s'agisse de stéréospécificité α ou β et/ou d'ordre d'enchaînement entre les motifs a et c et/ou b, l'élongation pouvant être effectuée à volonté.

Selon encore une autre disposition du procédé de l'invention, la chaîne glucidique élaborée est soumise à une ou plusieurs réactions chimiques afin d'introduire un type de groupements fonctionnels donnés ou, successivement, plusieurs types de groupements, puis de former, si on le désire, des dérivés de ces groupements fonctionnels.

Cette étape de fonctionnalisation peut être réalisée en n'éliminant que certains groupements protecteurs et/ou certains groupements précurseurs des dérivés aminés ou encore la totalité des groupements protecteurs et/ou des groupements précurseurs et en introduisant à leur place un type de substituants donné, ou, successivement, des substituants différents, puis en libérant une partie ou la totalité des groupes —OH encore bloqués, si on le désire.

Il est entendu alors que les différents groupes présents sur les motifs de la chaîne sont compatibles avec les substituants introduits à chaque étape.

La ou les réactions chimiques mises en œuvre au cours des étapes de fonctionnalisation sont réalisées de manière à ne pas altérer la structure de la chaîne et les groupes que l'on désire éventuellement maintenir et/ou ceux qui ont déjà été introduits.

Selon un mode préféré de réalisation de l'invention, pour obtenir des oligosaccharides à substitutions spécifiques comme définies ci-dessus, on utilise avec avantage des produits de départ renfermant plusieurs types de groupements protecteurs, à savoir (1) un ou plusieurs groupements semi-permanents

et (2) un ou plusieurs groupements permanents.

Par groupements semi-permanents, on entend des groupements éliminables en premier lieu après les réactions de glycosylation lorsque le squelette glucidique comporte le nombre de motifs désirés, sans enlèvement ou altération des autres groupes présents, permettant alors l'introduction de groupements fonctionnels souhaités aux positions qu'ils occupent.

Les groupements permanents sont des groupements capables de maintenir la protection des radicaux —OH durant l'introduction de groupements fonctionnels à la place des groupements semi-permanents.

Ces groupements sont choisis parmi ceux compatibles avec les groupes fonctionnels introduits après élimination des groupes semi-permanents. Il s'agit, en outre, de groupements inertes vis-à-vis des réactions effectuées pour la mise en place de ces groupes fonctionnels et qui sont éliminables sans que ces groupements fonctionnels ne soient altérés.

D'une manière avantageuse, la mise en œuvre de ces dispositions permet d'élaborer une chaîne glucidique dans laquelle les motifs A et U sont sélectivement substitués.

Pour préparer plus particulièrement des oligosaccharides renfermant les motifs A et/ou U des molécules biologiquement actives évoquées ci-dessus, on a avantageusement recours à des groupements protecteurs tels que les radicaux acyle, alcoyle éventuellement substitués ou aryle.

Les motifs des produits mis en œuvre de type A comportent, en position 2, un groupe azoté autorisant le maintien de la présence d'une fonction azotée durant les opérations mises en œuvre dans le procédé. Ce groupe azoté est avantageusement constitué par des groupes tels que $—N_3$ ou $—NHCOO—CH_2—C_6H_5$, ou tout autre groupe constituant un précurseur de fonction amine ou d'un dérivé d'amine, en particulier de $—NHSO_3^-$ ou de —NH-acyle, plus spécialement de $—NH—COCH_3$.

Quant aux fonctions carboxyle des motifs U, elles sont bloquées par des groupes inertes vis-à-vis des réactions mises en jeu pour le remplacement des groupes protecteurs et éliminables en fin de synthèse pour libérer les groupes carboxyle, éventuellement aux fins de salification. Ces groupes protecteurs de fonction carboxyle sont choisis avantageusement parmi les radicaux alcoyle, ou les radicaux aryle.

La structure du produit mis en œuvre dans la réaction de glycosylation est choisie en fonction des motifs de squelette glucidique recherché ainsi que des substitutions recherchées.

Pour former, par exemple, un disaccharide de type U-A, on utilise deux composés respectivement à structure acide uronique et sucre aminé, répondant, par ailleurs, aux définitions données ci-dessus.

En vue d'une élongation de chaîne, ces composés tels que mis en œuvre pour former le disaccharide en question, renferment, en outre, un groupement temporaire sur la position destinée à être impliquée dans la nouvelle réaction de glycosylation. Pour une élongation disaccharide U-A vers la gauche, ce groupement temporaire est présent sur le motif U et pour une élongation à droite sur le motif A.

Il est ainsi possible d'obtenir, notamment, des enchaînements $U_w A_x U_y A_z$ dans lesquels la somme des indices est comprise entre 2 et 12, ces valeurs étant incluses dans l'intervalle, w et y ne pouvant être nuls simulanément. Des enchaînements réguliers sont du type $U(AU)_n$, $(AU)_n A$, $(UA)_n$ ou encore $(AU)_n$ avec $1 \leq n \leq 6$.

Selon une variante de réalisation du procédé de l'invention, l'alternance de type A-U ou U-A rencontrée dans les structures des produits naturels peut être modifiée en utilisant, à la place de l'un ou plusieurs des motifs A ou U, un sucre constituant un analogue strutural de motif A ou U, tel qu'un sucre neutre ou un désoxy-sucre, ou encore d'autres motifs acides uroniques ou sucres aminés U ou A de configurations différentes.

Selon un mode préféré de réalisation du procédé de l'invention, on fait réagir l'alcool ci-dessus avec un dérivé réactif tel qu'un halogénure, un imidate ou un orthoester. Ces condensations sont réalisées dans des conditions anhydres.

La réaction de condensation entre l'halogénure et l'alcool est avantageusement du type Koenigs-Knorr. L'halogénure est avantageusement constitué par un bromure ou un chlorure en raison de commodités d'obtention.

On opère en milieu solvant, plus spécialement dans un solvant organique, notamment du type dichlorométhane ou dichloroéthane.

Avantageusement, on utilise un catalyseur, en général un sel d'argent ou de mercure, par exemple, le trifluorométhane sulfonate d'argent, communément appelé triflate d'argent, le carbonate d'argent, l'oxyde d'argent, le bromure mercurique ou le cyanure mercurique. On utilise également un accepteur de protons tel que la sym-collidine de même qu'un capteur pour l'eau éventuellement présente et/ou pour l'acide halogénohydrique formé, par exemple des tamis moléculaires 0,4 nm (4Å).

L'étude des conditions de réaction montre qu'il est approprié d'opérer à température ambiante ou encore à une température inférieure pouvant atteindre 0 °C ou moins, sous atmosphère d'un gaz inerte tel que l'azote ou l'argon.

Ces conditions permettent de condenser les motifs de structure a et b ou c (ou l'inverse), selon la stéréochimie souhaitée. Elles permettent également l'établissement de liaisons covalentes avec des sucres neutres ou des désoxy-sucres.

Une variante comportant l'utilisation, comme catalyseur, de dérivés mercuriques, en particulier de cyanure et/ou de bromure mercurique, s'avère appropriée pour réaliser des liaisons covalentes entre des alcools de structures variées et un précurseur L-idose du motif de structure c (acide L-iduronique). Selon

cette variante, on utilise également des tamis moléculaires 0,4 mm (4 Å). Le solvant organique est choisi selon la réactivité de l'alcool. Ainsi, on utilise avantageusement un solvant du type du nitrobenzène lorsque la condensation requiert une température supérieure à 100 °C. Pour des températures inférieures, on utilise des solvants, tels que le benzène ou le dichlorométhane. Des mélanges de solvants conviennent également pour réaliser la réaction de condensation.

Avec les motifs de type U, en particulier les motifs c, il est avantageux d'utiliser, comme groupe réactif, un orthoester. On réalise alors de préférence la réaction à une température supérieure à 100 °C.

Le milieu solvant est du type du chlorobenzène ou tout autre solvant dont le point d'ébullition dépasse 100 °C et se trouve avantageusement entre 100 et 150 °C. Pour activer la réaction, on utilise un catalyseur tel que du perchlorate de 2,6-diméthyl pyridinium.

Ce mode de réalisation de l'étape de condensation s'avère d'un grand intérêt pour établir une liaison interglycosidique entre un motif de structure c (acide L-iduronique) et un motif de structure a (D-glucosamine).

L'utilisation du groupement orthoester présente, en particulier, un double avantage.

D'une part, elle permet de conférer au carbone anomère de c la réactivité nécessaire pour la réaction de glycosylation. D'autre part, l'ouverture de ce groupe assure la mise en place en position 2 de c d'un groupe protecteur, éliminable sélectivement, en permettant l'introduction, à sa place, d'un groupe de substitution spécifique.

Ainsi, par réaction d'un groupe 1,2-O-méthoxy-éthylidène d'un motif c avec le radical —OH d'un motif a, il est possible à la fois d'établir une liaison interglycosidique entre les deux produits utilisés et de disposer en position 2 de c d'un groupe —OAc (Ac représentant un groupe acétyle) qui pourra être éliminé sélectivement aux fins d'introduction d'un groupe fonctionnel donné, par exemple —$SO_3^-$. Cette disposition permet également de laisser toute liberté pour traiter la position 4 du motif c.

Ces dispositions, particulièrement avantageuses, permettent de disposer d'un motif 2-O-sulfate L-iduronique tel qu'existant, par exemple, dans les chaînes d'héparine.

Lorsqu'on utilise comme groupe réactif un groupe imidoyle, il s'avère approprié d'opérer à basse température, plus spécialement à une température inférieure ou égale à 0 °C environ, en milieu solvant, tel que du dichlorométhane, en présence de tamis moléculaire 4 Å et d'un catalyseur tel que de l'éthérate de trifluorure de bore.

Dans l'alcool de départ, le groupe —OH libre occupe la position que l'on souhaite engager dans la liaison de glycosylation.

En choisissant l'alcool de manière appropriée, il est ainsi possible d'établir des liaisons du type 1-2, 1-3, 1-4 ou 1-6.

A partir de la séquence formée à l'issue de la réaction de condensation, on élabore une chaîne comportant le nombre de motifs désirés en répétant l'étape de glycosylation.

La fonction alcool de l'un des motifs A ou U impliqué dans la séquence glucidique déjà constituée est alors avantageusement libérée de son groupement protecteur temporaire. Le choix de ce groupement sera aisément déterminé par l'homme de l'art selon la nature des autres groupements présents sur la chaîne glucidique.

Parmi les divers groupements pouvant être utilisés, ont citera le groupe allyle qui, par traitement, par exemple d'abord avec un agent isomérisant tel que des dérivés de Pd, Rh et Ir, en particulier le chlorure de tristriphénylphosphine rhodium (I), ou encore le tertio-butoxyde de potassium, puis dans des conditions acides, en particulier avec un mélange d'oxyde mercurique et de chlorure mercurique, permet aisément de recréer un alcool à la position qu'il occupe.

De même, il est possible d'obtenir un groupe —OH par saponification à partir d'un groupe —O-acyle, en particulier —O-acétyle, ou O-chloroacétyle.

Ces radicaux peuvent être éliminés pour libérer une fonction —OH, par exemple, à l'aide de thiourée en milieu solvant, avantageusement à une température supérieure à 80 °C, de préférence de l'ordre de 100 °C.

Les dispositions qui précèdent permettent d'obtenir une chaîne glucidique à motifs alternés A-U ou U-A.

Cette alternance régulière peut être modifiée en mettant en œuvre les produits appropriés dans la réaction de glycosylation. Il est ainsi possible d'élaborer une structure irrégulière avec incorporation de motifs autres que U ou A, en particulier des sucres neutres ou encore des désoxy-sucres. Un autre type de structure irrégulière peut être obtenu en ajoutant plusieurs motifs A ou plusieurs motifs U consécutifs entre deux motifs de structure A-U ou U-A.

Il est entendu que les différentes dispositions de l'invention concernant les motifs A et U s'appliquent également aux autres motifs que peut comporter la chaîne glucidique tels que les sucres neutres ou les désoxy-sucres.

Comme déjà indiqué, les différents groupements présents sur les motifs A et U sont choisis de manière à conférer à ces derniers une réactivité suffisante pour réaliser la liaison glycosidique en question.

Les groupements protecteurs de radicaux —OH, mis à part les groupements temporaires déjà considérés, sont généralement choisis dans le groupe comprenant les radicaux acyle (notamment acétyle), alcoyle, alcoyle substitué tel que benzyle, et pour deux positions voisines, parmi les groupes

acétals ou cétals, par exemple benzylidène. Une autre forme de protection consiste à effectuer un blocage de deux groupes —OH sous forme époxyde ou de pont 1,6-anhydro.

Avantageusement, les produits utilisés dans la réaction de glycosylation renferment plusieurs types de groupements protecteurs, ce qui permet au cours de l'étape de fonctionnalisation d'introduire successivement un ou plusieurs groupements fonctionnels et de libérer un ou plusieurs radicaux —OH si on le désire.

D'une manière générale, les groupements protecteurs peuvent déjà occuper des positions déterminées sur les produits mis en œuvre dans la réaction de glycosylation.

Ils peuvent également être introduits à partir d'autres groupements une fois le squelette glucidique constitué. Cette variante comporte, par exemple, l'utilisation pour la glycosylation d'un produit A dans lequel les groupes —OH en positions 2 et 3 et en positions 1 et 6 sont bloqués sous forme anhydre, respectivement 2,3-époxyde et 1,6-anhydro. Grâce à ce blocage, on dispose durant l'élaboration du squelette glucidique d'un élément constituant potentiellement un motif A mais n'interférant pas avec les réactions mises en jeu dans la synthèse. Cette disposition présente l'avantage de laisser une large liberté pour effectuer les réactions désirées sur les groupements des autres motifs.

On remarquera, en outre, dans le cas considéré, que l'ouverture de la fonction époxyde par de l'azide de sodium permet d'introduire, en position 2, un groupe $N_3$ qui constitue donc un précurseur de fonction amine.

D'une manière préférée, pour disposer d'une chaîne glucidique permettant d'introduire sélectivement un ou plusieurs types de substituants au cours de l'étape de fonctionnalisation, en particulier les substitutions spécifiques ci-dessus, on met en œuvre des produits comportant plusieurs types de groupements protecteurs, à savoir des groupes semi-permanents et les groupes permanents définis plus haut.

Comme déjà indiqué, les substitutions des produits naturels considérés, mises à part celles des positions 2 des motifs A, sont essentiellement constituées par des groupes sulfate.

Les travaux des inventeurs pour mettre au point des conditions de sulfatation appropriées ont montré qu'il est possible et même avantageux d'effectuer une réaction de sulfatation en présence de groupements benzyle. Contrairement aux opinions admises dans ce domaine, l'élimination de groupements permanents benzyle, en présence de groupements —O-sulfate, peut être réalisée.

D'une manière préférée, les radicaux —OH des produits de départ destinés à être sulfatés sont alors protégés par des groupes acyle, en particulier acétyle, tandis que les radicaux —OH destinés à être libérés en fin de synthèse sont protégés par un groupe permanent tel que le groupe benzyle.

Selon une grande souplesse du procédé de l'invention, il est possible de soumettre l'ensemble de la chaîne glucidique élaborée à une réaction chimique donnée afin d'introduire un type de substituant déterminé.

Ce traitement peut consister par exemple en une estérification, notamment une sulfatation à l'aide d'un agent approprié, réalisée dans des conditions n'altérant pas la structure osidique. Cette sulfatation peut être réalisée de manière spécifique ou non, le cas échéant sur le glycoside totalement déprotégé.

Selon un mode préféré de réalisation de l'invention, l'étape de fonctionnalisation est cependant réalisée sélectivement de manière à introduire sur la chaîne, successivement, plusieurs types de substituants puis de libérer certains radicaux —OH.

Selon des conditions particulièrement avantageuses, permettant d'introduire des groupes sulfate sur des positions déterminées des motifs, de libérer des radicaux —OH sur d'autres positions, de former en position 2 des motifs A un dérivé d'amine et en position 6 des motifs U des dérivés d'acide, on met en œuvre des motifs répondant aux caractéristiques suivantes.

Les groupes semi-permanents de ces motifs occupent des positions destinées à être sulfatées et sont constitués par des groupes —O-acétyle.

Quant aux positions correspondant à un groupe —OH destinées à être libérées, elles sont occupées par des groupes semi-permanents constitués par des groupes benzyle.

Les positions 2 des motifs A sont substituées par des groupes tels que $N_3$ ou $NH—COO—CH_2—C_6H_5$ et les positions 6 des motifs U sont occupées par des groupes carboxyle protégés par un radical alcoyle, en particulier méthyle.

Ce jeu de conditions permet de réaliser l'étape de fonctionnalisation, par exemple comme suit :

On introduit tout d'abord sélectivement les groupes sulfate après avoir éliminé les groupes —O-acétyle de blocage. Cette réaction est réalisée de manière à ne pas affecter les groupes benzyle et les groupes azotés et carboxyle présents.

A cet égard, on effectue avantageusement une réaction de saponification à l'aide d'une base forte telle que la soude.

Cette réaction est réalisée de préférence à une température inférieure à l'ambiante et plus spécialement voisine de 0 °C.

On soumet le produit résultant de l'hydrolyse à l'action d'un agent d'alcoylation afin d'introduire, sur le groupe carboxyle, les groupes alcoyle protecteurs qui se sont trouvés éliminés lors de l'hydrolyse.

Par réaction avec un agent de sulfatation, on obtient alors l'introduction de groupes sulfate aux positions libérées par l'hydrolyse et laissées libres après l'action de l'agent d'alcoylation.

Des conditions de réaction satisfaisantes pour la conduite de la sulfatation comprennent la mise en

œuvre d'un agent de sulfatation, tel qu'un complexe triméthylamine/$SO_3$. Cette réaction est avantageusement réalisée en milieu solvant, plus spécialement dans un solvant tel que le diméthylformamide. De préférence, on opère à une température supérieure à l'ambiante, généralement voisine de 50 °C, ce qui correspond à une durée de réaction d'environ 12 heures.

Après l'introduction des groupes sulfate sur les fonctions alcool, on procède à la libération des groupes —OH bloqués par les radicaux benzyle.

L'élimination de groupes benzyle est avantageusement réalisée par hydrogénation catalytique dans des conditions compatibles avec le maintien des groupes sulfate et la transformation des groupes azotés en groupes fonctionnels amine.

On opère de préférence sous pression d'hydrogène en présence d'un catalyseur du type Pd/C.

Cette réaction est avantageusement réalisée en milieu solvant organique, en particulier alcoolique, additionné d'eau.

Pour obtenir l'hydrogénation des groupes azotés précurseurs et l'élimination des radicaux protecteurs des groupes —OH, la réaction est avantageusement réalisée sur une durée d'environ 3 à 4 jours.

Comme déjà indiqué, les groupes fonctionnels amine se présentent sous forme de dérivés de type N-acétyle ou N-sulfate dans les molécules biologiquement actives considérées.

Pour former des groupes N-acétyle, on soumet le produit résultant de la réaction d'hydrogénation à l'action d'un agent d'acétylation. A cet égard, l'anhydride acétique constitue un agent particulièrement approprié.

Pour réaliser cette réaction d'acétylation sélective sans affecter les autres substituants présents sur les motifs, il convient, notamment, d'opérer à pH basique, en particulier voisin de 8 en milieu aqueux.

On peut également souhaiter former des groupes N-sulfate, ce qui peut être réalisé à l'aide d'un agent de sulfatation du type indiqué ci-dessus. Des pH supérieurs à 9, avantageusement de l'ordre de 9-10, sont utilisés pour la sulfatation.

Après la réaction de sulfatation, l'addition d'une base forte permet de libérer les groupes carboxyle.

Les produits formés peuvent être aisément salifiés à l'aide de résines échangeuses d'un cation approprié. Dans les produits naturels, le cation en particulier est constitué par du sodium. On utilise donc avantageusement des résines échangeuses de cations sodium.

On peut également former des sels de potassium, lithium, magnésium, calcium. On utilise alors une résine échangeuse de protons, puis on neutralise l'acide formé avec la base du cation.

L'invention vise également les oligosaccharides constituant des intermédiaires dans les différentes étapes du procédé de synthèse défini ci-dessus.

Dans une famille, ces oligosaccharides renferment au moins un motif binaire A-U et U-A complètement protégé et possédant soit un groupe réactif sur le carbone anomère du motif à l'extrémité réductrice, soit un seul groupe —OH libre sur le motif à l'extrémité non réductrice, ce groupe —OH occupant la position 3, 4 ou 6 dans le cas d'un motif A et la position 2, 3 ou 4 dans le cas d'un motif U.

Dans une autre famille, les oligosaccharides sont constitués par des motifs complètement protégés tels qu'obtenus à l'issue de l'étape de glycosylation. Une autre famille encore comprend les produits dans lesquels un ou plusieurs groupes —OH sont libérés.

Ces différents oligosaccharides comportent une chaîne à base de motifs binaires de structure $(A\text{-}U)_n$ ou $(U\text{-}A)_n$ dans laquelle n est un nombre de 1 à 6.

Ces oligosaccharides correspondent à un enchaînement de type a-b, a-c.

Dans un groupe d'oligosaccharides intermédiaires de l'invention, la chaîne glycosidique est constituée par un seul type de ces enchaînements binaires.

Dans un autre groupe, plusieurs de ces types sont présents.

Des oligosaccharides correspondants comportent dans leurs chaînes des motifs a-b et a-c.

Il est entendu que l'ordre des enchaînements considéré ci-dessus dans l'un ou plusieurs des motifs binaires peut être inversé conformément à l'invention.

Selon une variante, les oligosaccharides intermédiaires définis ci-dessus renferment un ou plusieurs motifs consécutifs a ou encore b ou c.

Selon une autre variante, les oligosaccharides intermédiaires renferment un ou plusieurs motifs de sucres neutres et/ou plusieurs désoxy-sucres dans leur structure. Les différents groupements protecteurs de ces sucres répondent aux définitions données ci-dessus pour les motifs A et U.

Dans ces oligosaccharides, les motifs constitutifs sont reliés entre eux par des liaisons de type 1-2, 1-3, 1-4 ou 1-6 selon la nature de l'alcool mis en œuvre dans l'étape de glycosylation.

Les oligosaccharides possédant la structure de fragments d'héparine ou d'héparane-sulfate comportent des liaisons c1 $\xrightarrow{\alpha}$ 4a, a1 $\xrightarrow{\alpha}$ 4b, a1 $\xrightarrow{\alpha}$ 4c et b1 $\xrightarrow{\beta}$ 4a.

Un groupe d'oligosaccharides préférés renferme au moins un motif binaire possédant une structure de type b1 $\rightarrow$ 4a, c'est-à-dire [acide-D-glucuronique] 1 $\xrightarrow{\beta}$ 4 [D-glucosamine] répondant à la formule I :

(Voir Formule I p. 11)

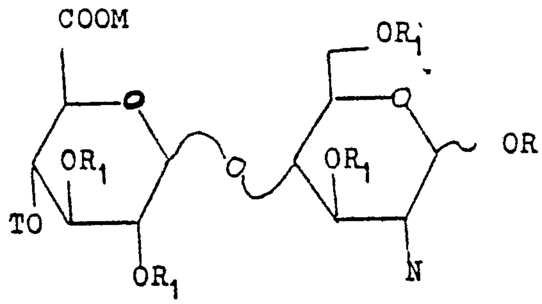

(I)

dans laquelle :

— les radicaux $R_1$, indentiques ou différents les uns des autres, éventuellement conjointement avec R, représentent un groupe protecteur, en particulier un groupement sp semi-permanent ou un groupement p permanent,

— T, un groupement temporaire t, ou un groupement permanent p, ou au atome d'hydrogène,

— N, un groupe azoté précurseur d'amine ou de dérivé d'amine,

— R, un radical aliphatique ou aromatique, notamment un radical alcoyle comportant de 1 à 4 atomes de carbone, où OR représente un groupe réactif tel qu'un halogénure ou encore R un radical alcoyle et

— M, un groupement bloquant la fonction acide, ces différents symboles présentant les significations données ci-dessus.

Dans un sous-groupe, tous les radicaux R, $R_1$ et T sont indentiques et représentant un groupement p ou sp.

Dans un autre sous-groupe, les radicaux $R_1$ sont différents les uns des autres, l'un au moins représentant un groupement de type sp, éventuellement conjointement avec R, le ou les autres radicaux $R_1$ représentant un groupe p.

On notera que les significations générales des symboles de la formule I s'appliquent également aux formules des différents groupes considérés ci-après. De même, on retrouve dans chacun de ces groupes, notamment, les deux sous-groupes évoqués ci-dessus.

Des oligosaccharides préférés répondent aux formules (II), (III) ou (IV) :

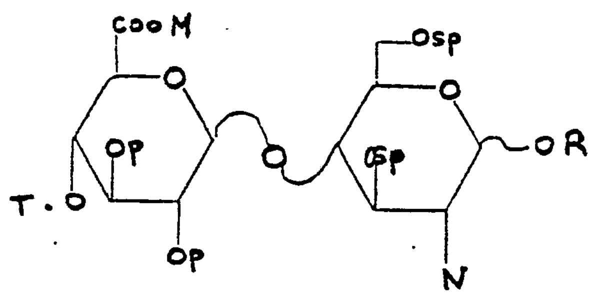

(II)

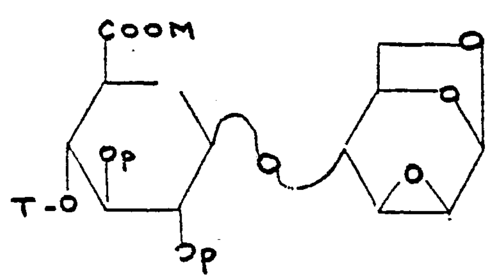

(III)

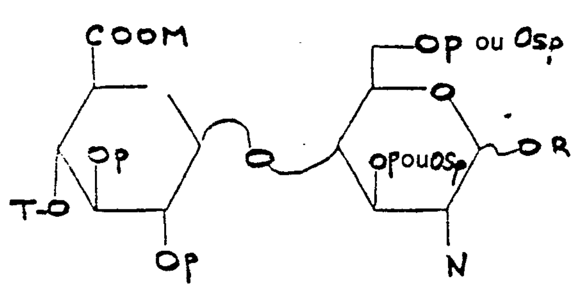

(IV)

dans lesquelles les différents symboles présentent les significations données plus haut.

De préférence, dans les formules (II) à (IV), les symboles donnés présentent indépendamment, ou en combinaison, les significations suivantes :

— M représente un atome d'hydrogène ou un radical alcoyle, en particulier méthyle,

— sp un groupe acyle, en particulier acétyle,

— p, un groupe alcoyle substitué, en particulier benzyle,

— R, un groupe acyle en α ou β, en particulier un groupe acétyle, un radical alcoyle, en particulier méthyle ou alcoyle substitué, notamment benzyle, ou —OR un halogène, en particulier un bromure, ou encore un radical imidoyle,

— N, un groupe azido,

— T, le groupe t représentant un radical acyle, en particulier acétyle, un radical acyle halogéné, en particulier, un radical monochloro ou trichloroacétyle, ou le groupe p représentant un radical alcoyle substitué en particulier le radical benzyle, le cas échéant lui-même paraméthoxy ou encore un atome d'hydrogène.

Un autre groupe préféré d'oligosaccharides renferme au moins un motif de type a1 $\xrightarrow{\alpha}$ 4b, c'est-à-dire [D-glucosamine] 1 $\longrightarrow$ 4 [acide D-glucuronique] répondant à la formule (V) :

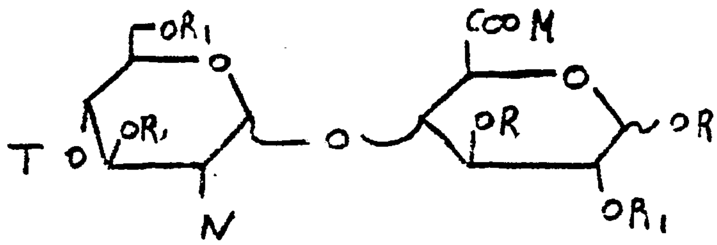

(V)

Des oligosaccharides préférés répondent aux formules (VI) ou (VII) suivantes :

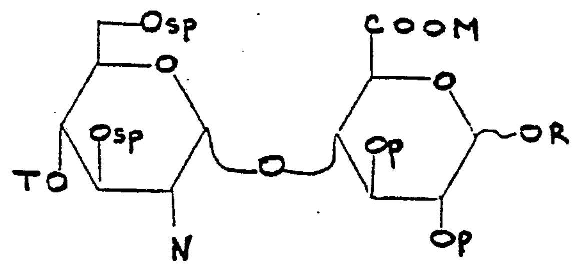

(VI)

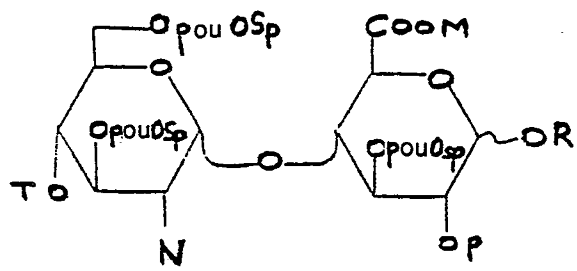

(VII)

Dans ces formules (VI) et (VII), les symboles M, N, sp, p présentent, de préférence, les significations particulières données ci-dessus en rapport avec les formules (II) à (IV), et R représente, en outre, de préférence, un groupe propényle, allyle, imidoyle, ou —H, avec N représentant alors plus spécialement un groupe —NH-acétyle.

On rappelle que l'ordre des enchaînements des motifs peut être inversé.

Dans un autre groupe préféré, les oligosaccharides renferment au moins un motif binaire de type c1 $\xrightarrow{\alpha}$ 4a, c'est-à-dire [acide-L-iduronique] 1 $\xrightarrow{\alpha}$ 4 [D-glucosamine], répondant à la fomule (VIII) :

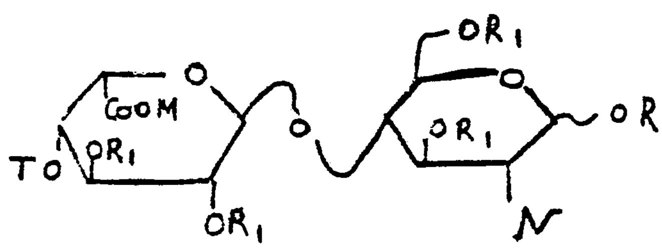

(VIII)

Des oligosaccharides préférés répondent aux formules (IX) et (X) suivantes :

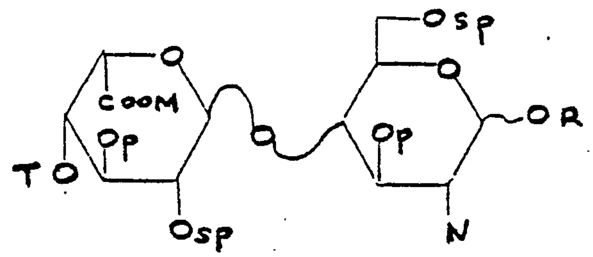

(IX)

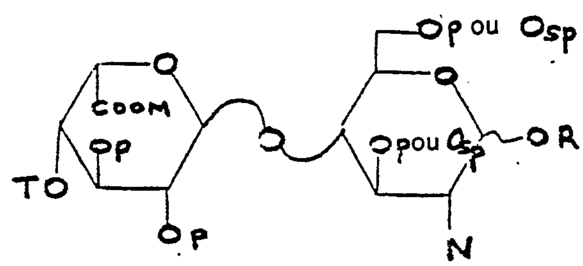

(X)

De manière préférée, les symboles figurant dans ces formules (IX) et (X) présentent les significations suivantes :

— les différents groupes sp et p peuvent être indentiques et représentent un radical acyle, en particulier acétyle, ou différents, et choisis parmi les radicaux acyle, en particulier acétyle ou benzoyle et les radicaux aryle ou alcoyle substitué,

— N représente un groupe azoté précurseur éventuellement différent de celui présent dans les composés de formules (I) à (V), en particulier un groupe NHCOO-(alcoyle substitué), notamment un groupe $—NH—COO—CH_2—C_6H_5$, ce qui permet de soumettre ces groupes azotés à des traitements différents et de former des dérivés d'amine différents en position 2 des motifs A,

— T représente le radical acétyle, acyle halogéné, en particulier, monochloro ou trichloroacétyle, p-méthoxybenzoyle, les symboles p, M et R présentant avantageusement les significations préférées données ci-dessus en rapport avec les formules (II) à (IV).

Un autre type de motif binaire d'oligosaccharides préférés présente une structure a1 $\xrightarrow{\alpha}$ 4c, c'est-à-dire [D-glucosamine], 1 $\xrightarrow{\alpha}$ 4 [acide-L-iduronique] répondant à la formule (XI) suivante :

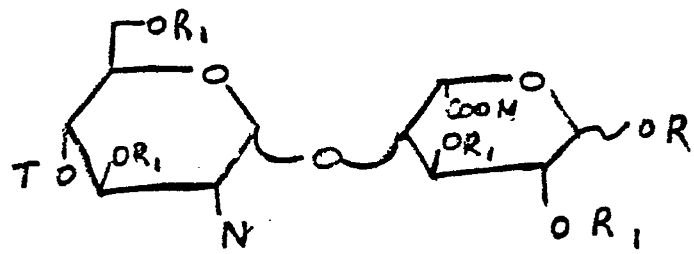

(XI)

Des oligosaccharides particuliers répondent aux formules (XII) et (XIII) :

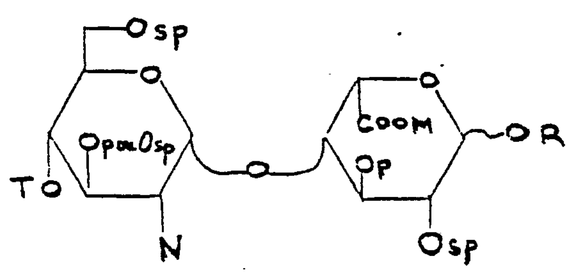

(XII)

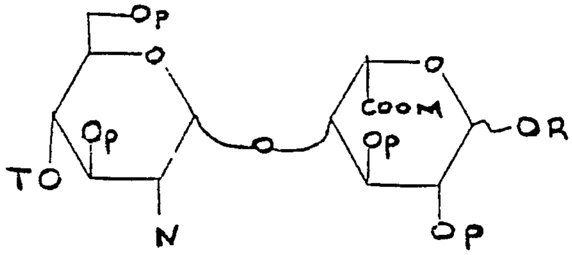

(XIII)

dans lesquelles les significations préférées correpondent à celles données ci-dessus pour les formules (II) et (IV).

Une autre famille d'oligosaccharides intermédiaires préférée entrant dans le cadre de l'invention correspond aux produits dont les groupes protecteurs ont été éliminés partiellement en cours de synthèse. En particulier, de tels produits comportent un groupe —OH à la place des groupes sp.

Des produits intermédiaires préférés correspondent à des oligosaccharides présentent la structure de la séquence complète octasaccharidique (ABCDEFGH) ou hexasaccharidique (C'DEFGH) évoquées ci-dessus.

D'une manière préférée, il s'agit de disaccharides AB, BC, CD, etc... de trisaccharides ABC, BCD..., de tétrasaccharides ABCD, BCDE... de pentasaccharides, ABCDE..., d'hexasaccharides, ABCDEF... d'heptasaccharides ABCDEFG ou BCDEFGH ou de l'octasaccharide lui-même.

Parmi ces oligosaccharides, on citera les structures BC, DE, DEF, EF, GH, FGH, EFG, EFGH, DEFGH et CDEFGH.

Des disaccharides intermédiaires préférés correspondent aux motifs binaires des formules (I) à (XIII).

Un groupe préféré de trisaccharides intermédiaires présente une structure DEF et répond à l'une des formules XIX à XXI.

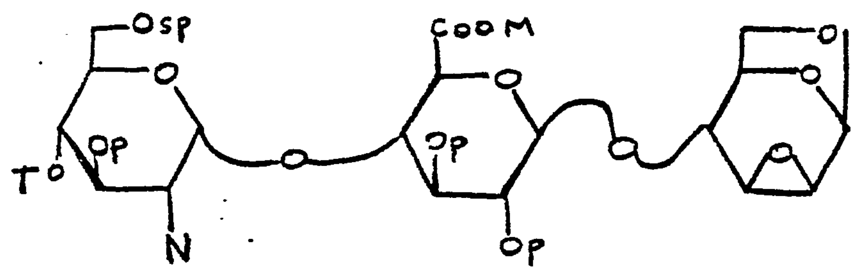

(XIX)

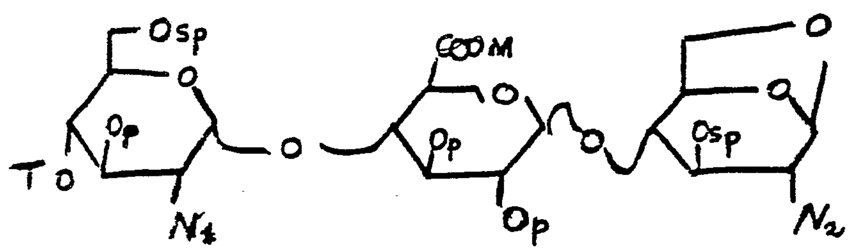

(XX)

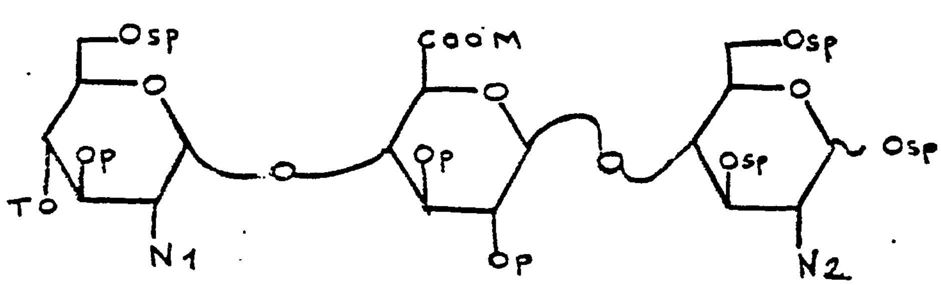

(XXI)

De préférence, $N_1$ et $N_2$, indentiques ou différents l'un de l'autre, représentent un groupe azido ou —NH-acyle, en particulier —NH-acétyle.

D'autres trisaccharides préférés possèdent une structure de type FGH de formule

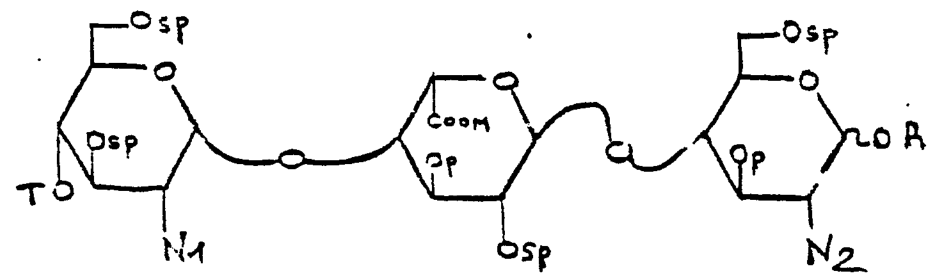

(XXII)

dans laquelle les différents symboles présentent les significations données ci-dessus, les deux substituants $N_1$ et $N_2$ des deux motifs glucosamine de structure F et H étant indentiques ou encore avantageusement différents, comme dans les produits naturels, et choisis parmi le groupe azido ou —NH—COO-acyle, en particulier —NH—COO-acétyle ou —NH—COO—$CH_2$—$C_6H_5$.

D'autres oligosaccharides intermédiaires préférés sont constitués par des tétrasaccharides. Des tétrasaccharides plus spécialement avantageux possèdent la structure EFGH et répondent à la formule suivante

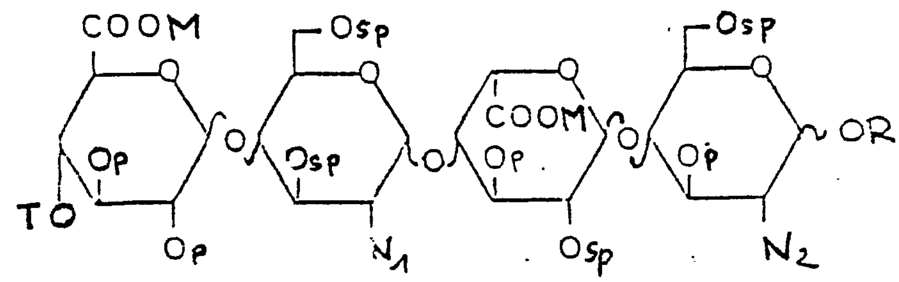

(XXIII)

dans laquelle les significations préférées des différents symboles correspondent à ceux indiqués pour la formule XXII.

Une autre famille d'oligasaccharides intermédiaires est constituée par des pentasaccharides, en particulier, par ceux de structure DEFGH de formule :

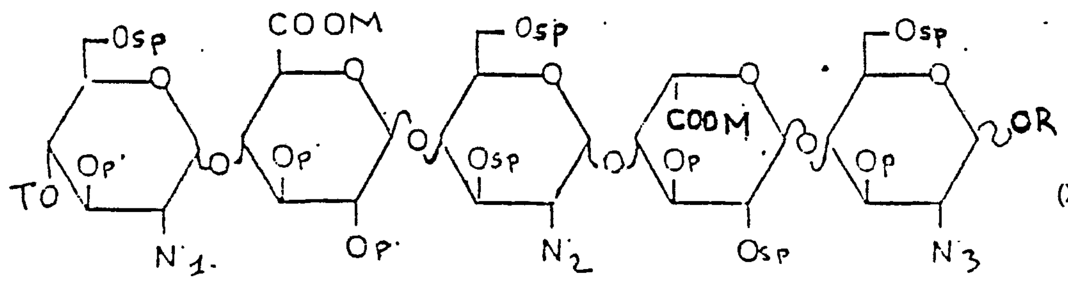

(XXIV)

dans laquelle les différents symboles présentent les significations préférées ci-dessus, $N_1$, $N_2$, $N_3$ pouvant être identiques ou différents les uns des autres et choisis parmi les significations déjà données.

Comme évoqué ci-dessus pour les motifs binaires, l'invention concerne également les oligosaccharides ci-dessus dans lesquels un, plusieurs ou, le cas échéant, la totalité des groupes —OH se trouvent libérés en cours de synthèse.

L'invention vise, en outre, en tant que produits nouveaux, les oligosaccharides répondant respectivement aux différentes définitions données ci-dessus, mais renfermant un ou plusieurs groupements fonctionnels, à l'exclusion du disaccharide [2-N-sulfate (ou 2-N-acétyl)-6-O-sulfate-D-glucosamine]-acide méthyl-D-glucuronique.

Ces groupements fonctionnels sont constitués, de préférence, par des esters, et se présentent plus spécialement sous forme d'anions minéraux.

Des esters particulièrement préférés, en raison de leur présence dans les molécules biologiquement actives de type héparine, héparane-sulfate, sont constitués par des esters sulfates.

D'autres esters avantageux correspondent à des esters phosphates.

Ces groupements fonctionnels sont portés par une ou plusieurs fonctions alcool primaire et/ou alcool secondaire et/ou amine primaire.

Une famille préférée d'oligosaccharides de l'invention renferme ainsi des motifs a comportant un anion tel que défini ci-dessus en position 6 et/ou 3.

Une famille particulièrement préférée renferme un motif a comportant un ester, en particulier un groupe sulfate, en position 6 et en position 3.

Des oligosaccharides de cette famille renferment, en position 2, un groupe fonctionnel amine primaire avantageusement substitué par un sulfate ou par un autre groupe substituant.

Dans les oligosaccharides de l'invention renfermant au moins deux motifs a les groupements fonctionnels amine en position 2 peuvent être substitués par un même groupement ou par des groupements différents.

Un groupe préféré d'oligosaccharides de la famille considérée renferme des motifs a comportant des groupes sulfate sur les fonctions alcool secondaire et spécialement alcool primaire.

Des oligosaccharides préférés de ce groupe comportent en position 2 de ces motifs un groupe $-NHSO_3^-$. D'autres oligosaccharides comportent un groupe —NH-acyle, en particulier —NH-acétyle.

D'une manière préférée, les esters ci-dessous se présentent sous forme de sels avec un cation minéral ou organique, en particulier un cation métallique, notamment un cation alcalin, ou encore un cation dérivé d'une base organique azotée, par exemple du triéthylammonium.

Les cations utilisés sont constitués par du sodium. D'autres cations sont appropriés tel que les cations potassium, magnésium ou calcium.

Dans une autre famille préférée d'oligosaccharides de l'invention, les groupes carboxyle des motifs b ou c sont libres ou se présentent de préférence sous forme de sels avec un cation organique ou minéral tel que défini ci-dessus. Ils peuvent également être protégés comme rapporté plus haut.

Des produits préférés renferment des motifs c comportant un groupe sulfate en position 2.

D'autres produits préférés présentent des sulfates sur le motif b.

Dans ces différentes familles d'oligosaccharides, les fonctions hydroxyle des cycles pyraniques sont soit libres, soit protégées par des groupements permanents de type alcoyle, en particulier par des groupes méthyle.

Des produits préférés de ces différentes familles renferment, en combinaison, des motifs A et U répondant aux caractéristiques ci-dessus.

Compte tenu de leur présence dans les molécules biologiquement actives ci-dessus et notamment dans l'octasaccharide ABCDEFGH ou l'hexasaccharide CDEFGH les oligosaccharides préférés correspondent aux produits de formules (I) à (XIII) et (XVIII) à (XXIV) ci-dessus, mais dans lesquelles les groupes sp sont remplacés par les anions. Des produits préférés correspondent aux sels des produits définis ci-dessus.

D'autres oligosaccharides préférés comportent en outre à la place des groupes N des motifs a, des groupes NH-acyle, en particulier $-NHCOCH_3$, $-NHSO_3^-$.

Des disaccharides préférés de ce type présentent une structure de type BD, DE, EF ou GH et répondent respectivement aux formules (XXV) à (XXVIII) suivantes :

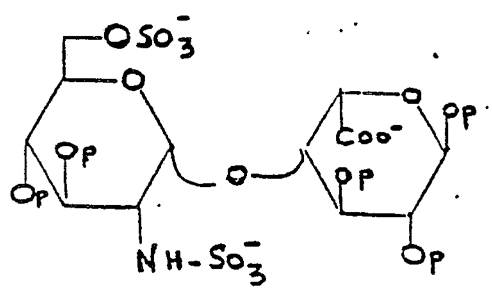

(XXV)

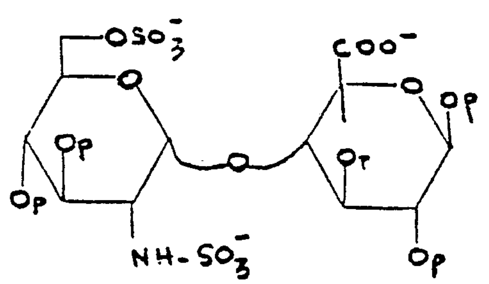

(XXVI)

ou N—H-acyle

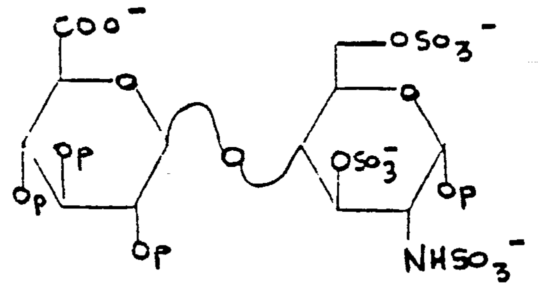

(XXVII)

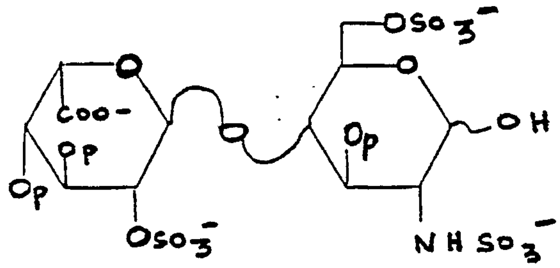

(XXVIII)

D'autres oligosaccharides préférés de l'invention renferment ou sont constitués par un enchaînement de structure DEF ou FGH respectivement de formules (XXIX) et (XXX) suivantes :

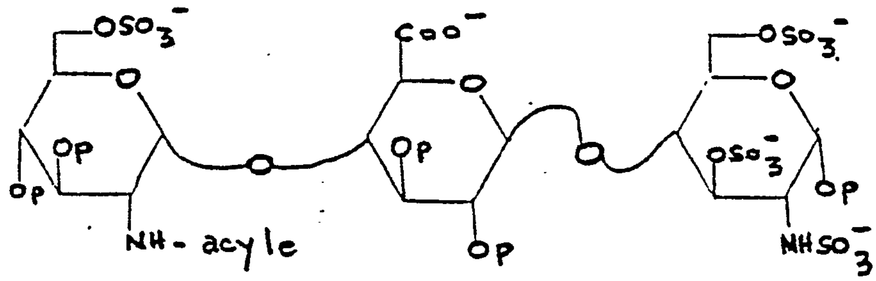

(XXIX)

ou $NHSO_3^-$

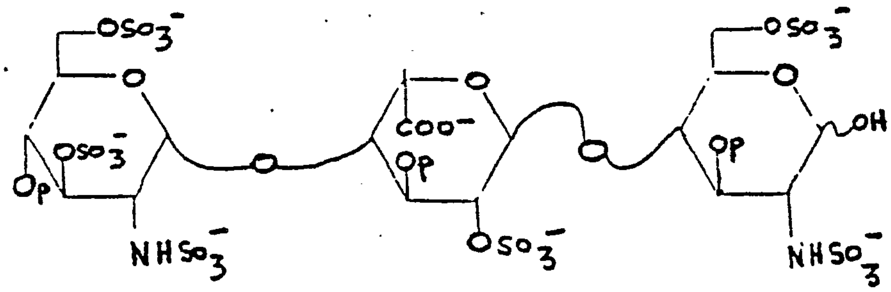

(XXX)

D'autres oligosaccharides préférés renferment ou sont constitués par des tétrasaccharides de structure EFGH répondant à la formule (XXXI) suivante :

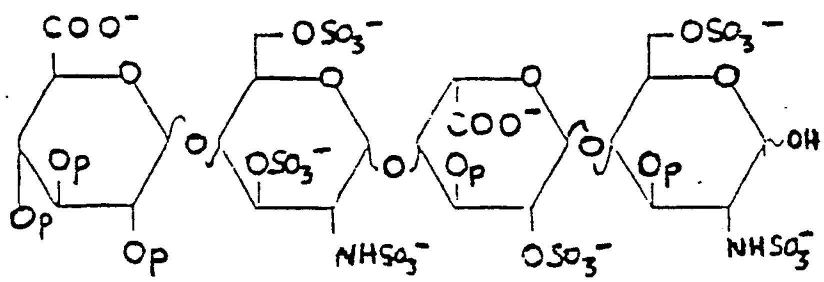

(XXXI)

D'autres oligosaccharides encore spécialement préférés renferment ou sont constitués par des pentasaccharides de formule (XXXII) suivante :

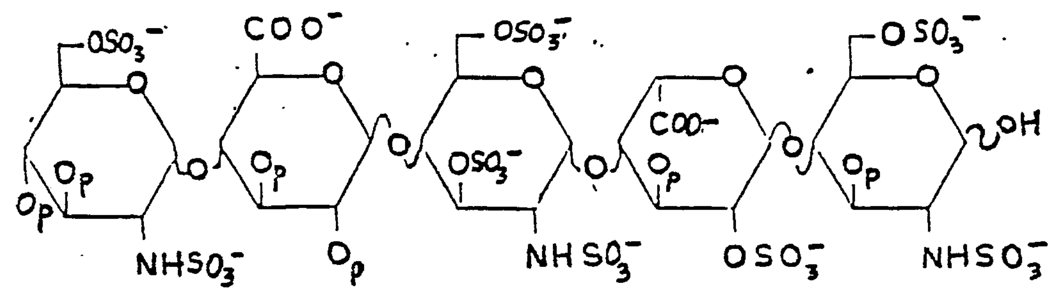

(XXXII)

ou NH-acyle.

Des oligosaccharides de l'invention particulièrement préférés comprennent ou sont constitués par des hexasaccharides de structure CDEFGH répondant à la formule (XXXIII) suivante :

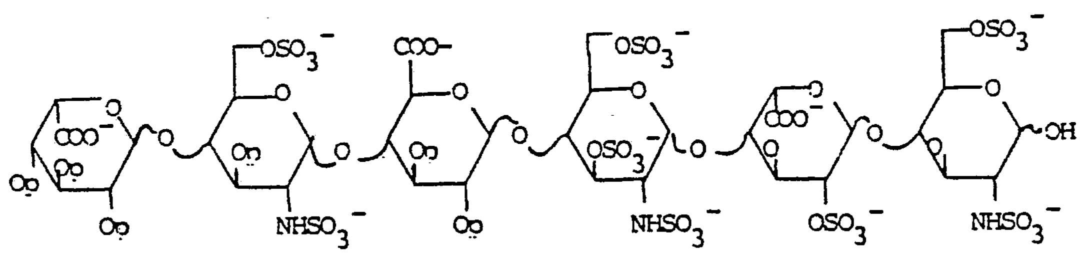

ou NH-acyle.

(XXXIII)

D'autres oligosaccharides répondent à l'une des formules (XXV) à (XXXIII) ci-dessus, mais renferment des groupes —OH libres à la place des groupes —Op. Ces produits sont alors complètement « déprotégés ».

Dans d'autres oligosaccharides encore une partie des groupes $—OSO_3^-$ peut être remplacée par des groupes —OH.

De préférence, les oligosaccharides de l'invention comportent des sels, éventuellement des sels doubles, des anions ci-dessus avec les cations déjà définis. Grâce à leur structure, les produits de l'invention constituent des intermédiaires de synthèse de grand intérêt permettant d'obtenir des fragments donnés, ou des dérivés de fragments, de molécules biologiquement actives.

Ils constituent, notamment, des composés de référence pour des études de structure.

L'étude pharmacologique d'oligosaccharides de l'invention a montré chez certains de ces composés des activités biologiques leur permettant de contrôler de manière spécifique certaines étapes de la coagulation sanguine. Des produits intéressants sont constitués, par exemple, par les trisaccharides de formule (XXIX), sulfatés et déprotégés et plus particulièrement le dérivé de l'exemple 13 bis.

D'une manière remarquable, les pentasaccharides de formule (XXXIII) sulfatés et déprotégés et tout spécialement le dérivé 50 se révèlent dotés notamment d'une haute affinité pour l'AT III et d'une très haute activité d'inhibition sélective du facteur X activé ou facteur Xa du sang.

L'invention concerne donc également leur application à la constitution de réactifs biologiques, utilisables en laboratoire, notamment comme éléments de comparaison pour l'étude d'autres substances dont on souhaite tester l'activité anticoagulante, notamment au niveau de l'inhibition du facteur Xa et du dosage de l'antithrombine III.

Le trisaccharide de formule (XXIX) à structure DEF dans laquelle le motif D comporte un groupe N-sulfate présente, par exemple, une activité anti-Xa mesurée selon le test de Yin-Wessler, de l'ordre de 7 u/mg.

Le pentasaccharide 50 de l'exemple 9 est caractérisé par des titres Yin-Wessler nettement supérieurs à ceux de l'héparine.

Plus spécialement, ce pentasaccharide est doté d'une activité anti-Xa (Yin-Wessler) égale ou supérieure à 2 000 u/mg et d'une haute affinité pour l'AT III.

Dans un test utilisant un substrat chromogène, cette activité a même été de 4 000 unités anti Wa/mg (méthode de TEIEN A.M. et LIE modifiée ; Thrombosis Research N° 10, 1977, 388-410).

Ce test consiste à utiliser le facteur Xa commercialisé par la Société SIGMA en solution à 8 u/ml dans du sérum physiologique, la concentration du substrat étant de 1,33 mM.

Pour effectuer ce test, on peut procéder de la façon suivante.

On mélange 10 µl de solution à doser et 300 µl de plasma humain dilué avec du tampon Tris maléate 0,02 M, pH 5.

On laisse incuber une minute à 37 °C.

On ajoute 100 µl du susdit facteur Xa (8 u/ml) et une minute après, on injecte la solution obtenue dans le substrat.

L'activité anticoagulante globale de ce produit est très faible, 4 u/mg dans le test APTT.

Ces propriétés leur permettent de contrôler, de manière spécifique, certaines étapes de la coagulation sanguine.

L'étude de ces produits montre qu'ils sont capables d'exercer une activité antithrombotique puissante. En outre, des dérivés selon l'invention présentent un grand intérêt pour lutter contre les troubles de la paroi vasculaire, (athéroscléroses et artérioscléroses).

De plus, ils présentent l'avantage de ne pas avoir d'effet d'activation sur l'aggrégation plaquettaire et de ne pas entraîner de thrombocytopénie. Ils présentent également l'avantage d'être pratiquement dénués d'effet sur le temps de saignement, ce qui élimine les risques d'hémorragie. Ces deux propriétés sont extrêmement importantes pour les applications médicales.

En outre, on observe notamment par voie souscutanée une pharmacocinétique allongée, ce qui procure également à ces produits un grand intérêt.

Les oligosaccharides de l'invention sont, en outre, avantageusement dépourvus de toxicité.

Ces produits sont donc particulièrement précieux pour l'élaboration de médicaments utilisables, notamment, pour la prévention et le traitement des thromboses.

L'invention est donc relative également à des préparations pharmaceutiques qui renferment lesdits oligosaccharides à activité anti-Xa élevée, plus spécialement les pentasaccharides dont question cidessus.

Elle est plus particulièrement relative à des préparations pharmaceutiques dépourvues de substances pyrogènes, contenant une quantité efficace de principes actifs en association avec des excipients pharmaceutiques.

Elle concerne également les compositions dans lesquelles le véhicule pharmaceutique est approprié pour l'administration par voie orale. Des formes d'administration de l'invention appropriées pour l'administration par voie orale peuvent être avantageusement des gélules gastrorésistantes, des comprimés ou tablettes, des pilules, ou encore présentées sous forme de liposomes.

D'autres compositions pharmaceutiques comprennent ces oligosaccharides en association avec les excipients appropriés pour l'administration par voie rectale. Des formes d'administration correspondantes sont constituées par des suppositoires.

D'autres formes d'administration de l'invention sont constituées par des aérosols ou des pommades.

L'invention concerne également des compositions pharmaceutiques injectables, stériles ou stérilisables pour l'administration tant par voie intraveineuse qu'intramusculaire ou sous-cutanée.

Ces solutions renferment avantageusement 1 000 à 100 000 u (Yin-Wessler)/ml d'oligosaccharides, de préférence de 5 000 à 50 000, par exemple de 25 000 u/ml, lorsque ces solutions sont destinées à l'injection par voie sous-cutanée. Elles peuvent contenir, par exemple, de 500 à 10 000, notamment 5 000 u/ml d'oligosaccharides lorsqu'elles sont destinées à l'injection par voie intraveineuse ou par perfusion.

Avantageusement, de telles préparations pharmaceutiques sont présentées sous la forme de seringues non récupérables, prêtes à l'emploi.

L'invention concerne également les compositions pharmaceutiques contenant lesdits oligosaccharides en association avec un autre principe actif, utilisable en particulier pour la prophylaxie et le traitement de thrombose, tel qu'un agent veinotonique comme la dihydroergotamine, un sel d'acide nicotinique ou un agent thrombolytique comme l'urokinase.

Les compositions pharmaceutiques de l'invention sont particulièrement adaptées pour le contrôle (préventif ou curatif) de certaines étapes de la coagulation du sang chez l'homme ou l'animal), notamment dans le cas où le patient est soumis à des risques d'hypercoagulabilité résultant notamment d'opérations chirurgicales, de processus athéromateux, de développement de tumeurs et de troubles de la coagulation par des activateurs bactériens ou enzymatiques, etc.

Afin d'illustrer l'invention, on indique, ci-après, un exemple de posologie utilisable chez l'homme : cette posologie comprend, par exemple, l'administration au patient de 1 000 à 25 000 u (Yin et Wessler) par voie sous-cutanée, une à trois fois par jour, selon le niveau des risques d'hypercoagulabilité ou la condition thrombotique du patient, ou de 1 000 à 25 000 u/24 heures, par voie intraveineuse, en administrations discontinues à intervalles réguliers, ou continues par perfusion, ou encore de 1 000 à 25 000 u (trois fois par semaine) par voie intramusculaire ou sous-cutanée (ces titres sont exprimés en unités Yin-Wessler). Ces doses peuvent être naturellement ajustées pour chaque patient en fonction des résultats et des analyses de sang effectuées auparavant, la nature des affectations dont il souffre et, d'une manière générale, son état de santé.

Outre les compositions pharmaceutiques renfermant les oligosaccharides, tels quels, l'invention vise également les compositions pharmaceutiques renfermant au moins un oligosaccharide tel que défini cidessus, conjugué, par liaison covalente, à un support soluble ou à un support insoluble, avantageusement au moyen de sucre terminal réducteur.

Des conjugués fixés à des supports solubles préférés sont constitués par des oligosaccharides

conjugués à l'AT III.

Un conjugué de ce type comportant le pentasaccharide 49 est tout spécialement préféré. De tels produits constituent des médicaments particulièrement intéressants dans la prévention de thromboses, en cas de déficits en AT III.

D'autres conjugués préférés avec supports solubles sont formés d'un oligosaccharides fixé à un véhicule tel qu'une protéine, notamment la polylysine, ou le sérum albumine bovine.

Ces produits sont utilisables comme immunogènes eux-mêmes sources d'anticorps circulants produits in vivo ou d'anticorps monoclonaux, clonés in vitro selon les techniques appropriées.

Dans d'autres conjugués préférés, les oligosaccharides de l'invention sont conjugués à des supports insolubles. On utilisera avantageusement les supports classiques.

Ces conjugués sont utilisables comme immunoabsorbants, par exemple pour une purification de haute spécificité de l'AT III et pour son dosage ou pour l'élaboration, par fixation sur des polymères biocompatibles, de nouveaux polymères hémocompatibles athrombotiques.

L'invention vise également l'application des oligosaccharides considérés en médecine nucléaire, en tant que produits radiopharmaceutiques. Ces produits sont alors marqués par des traceurs choisis parmi ceux couramment utilisés dans ce domaine, et notamment à l'aide de technétium 99 m.

A cet effet, on transforme le technétium 99 m obtenu à partir de générateurs du commerce, sous forme de pertechnétate de sodium de valence 7 non réactif, en technétium réduit de valence 4 qui serait de forme la plus réactive du technétium. Cette transformation est effectuée grâce à un système réducteur réalisé à partir de sels d'étain (chlorure stanneux), de sels de fer (sulfate ferreux), de sels de titane (trichlorure de titane) ou autres sels.

La plupart du temps, cette simple réduction du technétium suffit, dans des conditions de pH données, à réaliser la fixation du technétium sur la molécule considérée.

On peut utiliser les produits de l'invention, qui constituent en quelque sorte un support, à des doses de l'ordre de 10 à 200 u Yin-Wessler.

Pour l'élaboration de ces réactifs radiopharmaceutiques, on peut opérer conformément à la méthode de P.V. Kulkarni et al. dans The Journal of Nuclear Medecine 21, N° 2, p. 117-121.

Les produits ainsi marqués sont avantageusement utilisés dans des tests in vivo pour la détection et le diagnostic d'extension des thromboses et des états thrombotiques.

Les oligosaccharides de l'invention peuvent être également utilisés pour la détermination de la spécificité des nombreuses enzymes impliquées dans le métabolisme des glycosaminoglucuronoglycanes.

D'autres caractéristiques avantageuses de l'invention apparaîtront dans les exemples qui suivent et en se reportant aux figures 1 à 32 illustrant les produits mis en œuvre dans les synthèses décrites.

Dans ces figures, les références numériques des formules sont utilisées également dans les exemples pour désigner les mêmes produits.

Les abréviations utilisées dans ces formules présentent les significations suivantes :

Ac : un groupe acétyle ; Me : méthyle ; Bn : benzyle ;

Bz : benzoyle ; MCAO : monochloroacétyle ; Tr : trityle ;

but. : butyle et S un groupe $SO_3^-$.

Exemple 1

Synthèse du dérivé 13

à savoir du (prop-1'-ényl 2,

3-di-O-benzyl-α-D-glucopyranoside)

uronate de méthyle de formule

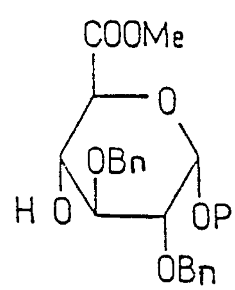

On effectue cette synthèse à partir du glucose selon les étapes a) à m) suivantes :

a) préparation du dérivé allyle ;

b) blocage des positions 4 et 6 du dérivé allyle par un groupe benzylidène ;

c) introduction de groupes benzyle en positions 2 et 3 ;

d) déblocage des positions 4 et 6 par l'élimination du groupe benzylidène ;

e) introduction d'un groupe trityle en position 6, suivie d'une réaction d'acétylation de la position 4 ;

f) élimination du groupe trityle en position 6 ;

g) oxydation du groupe alcool primaire en position 6 ;

h) méthylation du groupe carboxyle en position 6 ;
i) introduction du groupe propényle en position 1 ;
j) élimination du groupe acétyle en position 4.

Ces étapes sont réalisées comme suit (voir les figures 1 et 2) :

a) préparation de l'allyl α-D-glucopyranoside (composé 1).

Une solution d'acide chlorhydrique gazeux (18 g) dans de l'alcool allylique (600 ml) est chauffée à 70 °C. On ajoute alors du glucose anhydre (300 g) et on maintient à cette température pendant 3 heures. La réaction peut être suivie en chromatographie sur couche mince (c. c. m.) dans le solvant méthanol/chloroforme (1/4, v/v). La solution brune obtenue après 3 heures est concentrée à sec, sous vide, neutralisée par une solution concentrée d'ammoniaque (50 ml) puis concentrée à nouveau à sec. Au résidu obtenu, on ajoute de l'acétone (500 ml), on porte à ébullition et on maintient ainsi jusqu'à dissolution totale. Après refroidissement, le liquide est décanté. Le résidu est de nouveau soumis au même traitement jusqu'à ce que l'analyse en c. c. m. de l'extrait montre un épuisement du résidu en dérivé 1 ou bien une trop forte contamination de l'extrait par des impuretés.

Une partie de la première fraction extraite (12 g) est chromatographiée sur silice. On récupère le dérivé 1 qui peut être cristallisé dans un mélange acétone/éther (6,5 g ; pf 95-99 °C). Le reste du produit peut être purifié selon le même processus.

b) blocage des positions 4 et 6 du dérivé allyle conduisant à l'allyl 4, 6-O-benzylidène-α-D-glucopyranoside (composé 2).

Le composé 1 (37 g) est dissous dans du diméthylformamide (200 ml). Du diméthoxytoluène (41 g) est alors ajouté suivi d'acide paratoluène sulfonique hydraté (130 mg).

Après 2 heures de chauffage (bain-marie) sous vide et reflux, la réaction est terminée (c. c. m. méthanol/chloroforme, 2/25, v/v). Le solvant est évaporé. Le sirop est dissous dans du méthanol (le minimum), cette solution est versée goutte à goutte dans une solution aqueuse de bicarbonate de sodium (6,3 g dans 320 ml d'eau). Le précipité obtenu est recristallisé dans l'éthanol (21 g ; p. f 120-121 °C). Les eaux-mères livrent encore du produit 2. Rendement total (37 g ; 71,4 %).

c) introduction du groupe benzyle conduisant à l'allyl 2, 3-di-O-benzyl-4, 6-O-benzylidène-α-D-glucopyranoside (composé 3).

Le composé 2 (45 g) est dissous dans du DMF anhydre (500 ml). De l'hydrure de sodium (28 g d'une dispersion à 50 % dans l'huile) est ajouté.

Après 30 minutes, le mélange est refroidi à 0 °C et on ajoute alors, goutte à goutte, du bromure de benzyle (52 ml). La réaction est suivie en c. c. m. (éther/hexane, 1/1, v/v). On ajoute ensuite lentement du méthanol (150 ml), évapore à sec et reprend par du chloroforme. La phase chloroformique, est lavée avec de l'eau, séchée sur sulfate de sodium. Après évaporation du solvant, le résidu est cristallisé dans un mélange éther/hexane (36,5 g : PF 83-84 °C).

Ce produit est légèrement contaminé par une impureté migrant plus haut en c. c. m. (éther/hexane ; 1/1 ; v/v).

d) élimination du groupe benzylidène conduisant à l'allyl 2, 3-di-O-benzyl-α-D-glucopyranoside (composé 4).

A une solution du composé 3 (56 g) dans le méthanol (1 l) on ajoute de l'eau (450 ml) puis de l'acide paratoluène sulfonique hydraté (17 g).

Après 2 heures à 80 °C, on laisse refroidir le mélange, on évapore le solvant et on reprend le résidu par du chloroforme (1 l). La solution chloroformique est lavée avec de l'eau jusqu'à pH neutre, puis séchée sur sulfate de sodium.

On obtient ainsi un sirop jaune pâle (48 g) qui est engagé dans l'étape suivante (synthèse du composé 5).

e) introduction d'un groupe trityle en position 6 suivie d'une réaction d'acétylation de la position 4 conduisant successivement à l'allyl 2, 3-di-O-benzyl-6-O-trityl-α-D-glucopyranoside (composé 5) et de son analogue 4-O-acétylé (composé 6a).

Le dérivé 4 obtenu (48 g) est dissous dans de la pyridine (250 ml) et du chlorure de trityle (38,5 g) est ajouté. Après 1 heure à 100 °C, la réaction est terminée (c. c. m. éther/hexane, 1/1, v/v). A la solution précédente, on ajoute de l'anhydrique acétique (200 ml). Après une nuit, la réaction est complète (c. c. m., éther/hexane, 1/2, v/v). On évapore à sec, reprend le résidu par du chloroforme (500 ml), lave la phase chloroformique avec une solution de sulfate acide de potassium à 10 % et avec de l'eau et sèche sur sulfate de sodium.

Le chloroforme est évaporé. On obtient ainsi le composé 6 a qui est engagé tel quel dans la réaction de préparation du composé 7 a.

f) élimination du groupe trityle conduisant à l'allyl 4-O-acétyl —2, 3-di-O-benzyl-α-D-glucopyranoside (composé 7 a).

Le dérivé 6a obtenu est dissous dans du chloroforme (500 ml). A cette solution, refroidie à 0 °C, on ajoute goutte à goutte sous agitation, une solution de trifluorure de bore dans le méthanol (20 %, 120 ml). La réaction est suivie c. c. m. (toluène/acétone, 10/2, v/v).

Le mélange réactionnel est transvasé dans une ampoule à décanter. La phase chloroformique est lavée par de l'eau (2 fois 100 ml) par une solution saturée de bicarbonate de sodium, puis avec de l'eau jusqu'à pH neutre. Après séchage, et évaporation, le résidu obtenu est introduit sur une colonne de gel de silice (500 g) équilibrée dans le toluène. Après élution de la plupart des impuretés par le toluène pur, le produit est élué par un mélange toluène/acétone (10/2, v/v). On obtient ainsi 48 g du composé 7a qui sera engagé directement dans la synthèse du composé 8a.

Une partie du composé 7a a été obtenue pure : $[\alpha]_D^{20} = +11°$ (chloroforme). Ses spectres I.R. et R.M.N., de même que l'analyse élémentaire, confirment la structure.

g) oxydation du groupe alcool primaire en position 6 conduisant à l'acide (allyl-4 O-acétyl-2, 3-di-O-benzyl-α-D-glucopyranoside) uronique (composé 8a).

Une solution du composé 7a (48 g) dans l'acétone (800 ml) est refroidie à — 5 °C. On ajoute ensuite goutte à goutte, une solution de trioxyde de chrome (30 g) dans l'acide sulfurique (3,5 M ; 125 ml). On laisse le mélange revenir à la température ambiante. La réaction est contrôlée en c. c. m. (méthanol/chloroforme, 1/10, v/v). A la fin de la réaction, le mélange réactionnel est versé dans de l'eau (500 ml). Le produit est extrait par le chloroforme ( 3 fois 250 ml). La phase chloroformique est lavée à l'eau jusqu'à pH neutre, séchée sur sulfate de sodium et concentrée à sec.

Le sirop obtenu (83 g) est utilisé tel quel pour la préparation du composé 9a.

h) méthylation du groupe carboxyle en position 6 conduisant à l'(allyl-4-O-acétyl-2, 3-di-O-benzyl-α-D-glucopyranoside) uronate de méthyle (composé 9a).

Le sirop obtenu à l'étape de préparation du composé 8a est dissous dans de l'éther (300 ml). Une solution éthérée de diazométhane est alors ajoutée jusqu'à disparition du composé 8a (c. c. m. éther/hexane), 1/1, v/v. Après acidification par l'acide acétique, les solvants sont évaporés.

Le résidu obtenu (53 g) est dissous dans l'éthanol à chaud. Le dérivé 9a cristallise au refroidissement. Après recristallisation, on obtient ce composé 9a pur (18,4 g) – pf 85-86 °C – $[\alpha]_D{}^20 = +12°$ (1,2 chloroforme).

Ce produit est caractérisé par ses spectres I.R., R.M.N. et par son analyse élémentaire.

A partir du filtrat de cristallisation, on obtient encore 7,6 g du composé 9a.

Le rendement global en 9a à partir du composé 2 est de 38 %.

i) introduction du groupe propényle en position 1 conduisant au (prop-1' —ényl 4-O-acétyl-2, 3-di-O-benzyl-α-D-glucopyranoside) uronate de méthyle (composé 10a).

Le dérivé 9a (4 g) est dissous dans un mélange d'éthanol (119 ml) de benzène (51 ml) et d'eau (17 ml). On ajoute ensuite du diazabicyclo octane (170 mg) et on porte à reflux. On ajoute à la solution bouillante du chlorure de tris (triphénylphosphine)-rhodium (I) (550 mg). L'ébullition est maintenue pendant 4 heures (c. c. m., éther/hexane, 1/1, v/v).

A la fin de la réaction, la solution est filtrée et les solvants sont éliminés. Le résidu est chromatographié sur gel de silice (150 g) dans un mélange acétate d'éthyle/chloroforme (1,50, v/v). On obtient le composé 10a (3,25 g ; 81 %) qui cristallise dans l'éthanol. $[\alpha]_D^{20} = +12°$ (1, chloroforme). PF 90 °C. La structure est confirmée par l'analyse élémentaire et les spectres R.M.N. et I.R.

j) élimination du groupe acétyle en position 4 conduisant au (prop-1'-ényl 2, 3-di-O-benzyl-α-D-glucopyranoside) uronate de méthyle (composé 13).

Le dérivé 10a (350 mg) est dissous dans le méthanol (5 ml). Du méthanolate de sodium (0,2 ml, 2 M) est ajouté. Après 1 h à température ambiante, la réaction est arrêtée par addition de résine Dowex® 50 – $H^+$. Après filtration, le produit 13 est obtenu, contaminé par un peu de produit résultat de l'α – ß – élimination.

Selon une variante de l'étape e), au lieu d'effectuer une réaction d'acétylation, on réalise une réaction de benzoylation, ce qui conduit à l'allyl-4-O-benzoyl, 2, 3-di-O-benzyl-4-O-benzoyl, 6-O-trityl-α-D-glucopyranoside (composé 6b), dont on élimine ensuite le groupe trityle, ce qui permet d'obtenir l'allyl-4-O-benzoyl-2, 3-di-O-benzyl-α-D-glucopyranoside (composé 7b).

Ces réactions sont réalisées comme suit :

— préparation des composés 6b et 7b

A la solution pyridinique du composé 5 on ajoute alors du chlorure de benzoyle (1,5 équivalents) et la réaction est suivie en c. c. m. (acétate d'éthyle/benzène, 1/20, v/v). L'excès de chlorure de benzoyle est détruit par addition d'un excès de méthanol. Après évaporation à sec, le résidu, repris par du chloroforme, est lavé avec une solution de $KHSO_4$ à 10 %, avec de l'eau, séché et concentré à sec. Le sirop obtenu est engagé tel quel dans la synthèse du composé 7b. Ce sirop (105 g, obtenu à partir de 30 g de composé 3) est dissous dans du chloroforme (300 ml). De l'acide paratoluène sulfonique (76 g de monohydrate dans 100 ml de méthanol) est ajouté. Après une nuit, la réaction est terminée (c. c. m., acétate d'éthyle/chloroforme, 1/20, v/v). La phase chloroformique est lavée avec de l'eau jusqu'à pH neutre, séchée et concentrée à sec. Le sirop obtenu (98 g) est chromatographié sur une colonne de gel de silice (1,2 kg), éluée avec du chloroforme (0,6 l) puis avec un mélange acétate d'éthyle/chloroforme (1/20, v/v). On obtient ainsi le dérivé 7b pur (30 g) qu'on engage tel quel dans l'étape de préparation du composé 8b.

A partir du composé 7b, il est possible d'oxyder le groupe —$CH_2OH$ en position 6 puis d'introduire un groupe méthyle sur le groupe carboxyle obtenu, en formant successivement, l'acide (allyl 4-O-benzoyl-2, 3-di-O-benzyl-α-D-glycopyranoside) uronique (composé 8b) et l'ester méthylique correspondant (composé 9b).

Ces dérivés sont préparés en procédant comme suit :

préparation du composé 8b et de l'ester 9b :

Le composé 7b (27 g) est traité comme décrit pour 7a dans la préparation de 8a. Le sirop obtenu en fin de traitement contient le composé 8b qui est méthylé par le diazométhane comme décrit pour le composé 8a.

Le résidu obtenu à l'issue de la méthylation est purifié sur gel de silice (200 g ; éther 1/hexane 1). On obtient ainsi le composé 9b (21 g ; 77,5 %). Ses spectres I.R. et R.M.N confirment sa structure.

A partir du dérivé 9b, on prépare le dérivé propényle correspondant 10b, en opérant comme pour 10a.

Le dérivé 13 est alors obtenu à partir de 10b selon la réaction donnée pour 10a.

Selon une autre variante, on prépare l'acide (allyl 2, 3-di-O-benzyl-α-D-glucopyranoside) uronique et l'(allyl 2, 3-di-O-benzyl-α-D-glucopyranoside) uronate de méthyle (composés 11 et 12).

Le composé 8b (1,9 g) est dissous dans du méthanol (40 ml). On ajoute alors de la soude (5 N) en quantité suffisante pour avoir une concentration de 1 M en soude. La réaction est suivie en c. c. m. (méthanol/chloroforme, 1/4, v/v). Quant elle est terminée, on ajoute de l'eau (100 ml). On lave avec de l'éther, on acidifie et on extrait le produit à l'éther.

La phase éthérée acide est lavée avec de l'eau jusqu'à pH neutre. Le dérivé 11 n'est pas isolé. Il est méthylé par une solution éthérée de diazométhane, donnant ainsi le composé 12 (900 mg ; 56 %) qui est alors purifié sur une colonne de gel de silice (éther/hexane), 1/1, v/v). $[\alpha]_D^{20} = + 35,2°$ (1,3 chloroforme). Ses spectres I.R. et R.M.N. et son analyse élémentaire confirment sa structure.

De la même manière, le dérivé 11 et donc 12 peuvent être obtenus à partir de 9a ou 9b.

Le composé 13 peut être obtenu à partir du composé 12 en opérant comme suit :

Le dérivé 12 est traité par le complexe au rhodium comme décrit pour 9a. Le composé 13 est obtenu avec un rendement de 90 %. Il est caractérisé par ses spectres I.R. et R.M.N. De plus, traité par de l'anhydride acétique (1 ml pour 180 mg de 9a) il donne le composé 10a.

Selon encore une autre variante, le dérivé 13 peut être obtenu à partir de 10a ou 10b en opérant comme décrit pour l'obtention du 17 à partir de 9a ou 9b.

Exemple 2
Synthèse du disaccharide 20
ou 1 bromo-3, 6-di-O-acétyl-2-azido-4-O
[(2, 3-di-O-benzyl-4-O-chloroacétyl-β-D-glucopyranosyl)
uronate de méthyle] —D-glucopyranose de formule :

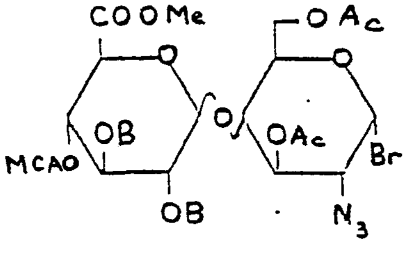

Cette synthèse comporte les étapes suivantes (voir les figures 3 et 4) :

A) la préparation à partir du dérivé 13 de l'exemple 1 du monosaccharide 16 ou (1-bromo-2, 3-di-O-benzyl-4-O-chloroacétyl-α-D-glucopyranosyde) uronate de méthyle ;

B) la condensation du composé 16 avec le monosaccharide 17 conduisant au disaccharide 18 ;

C) l'acétolyse du composé 18 conduisant au disaccharide 19 et,

D) la bromuration donnant le disaccharide 20.

A) préparation du monosaccharide 17.

Cette synthèse est effectuée à partir du monosaccharide 13 ou (prop-1'-ényl 2, 3-di-O-benzyl-α-D-glucopyranoside) uronate de méthyle, selon les trois étapes suivantes :

1. chloroacétylation du composé 13 ;
2. déblocage du carbone anomère ;
3. bromuration du carbone anomère.

1. chloroacétylation du composé 13 conduisant au composé 14, a savoir le (prop-1'-ényl 2, 3-di-O-benzyl-4-O-chloroacétyle-α-D-glucopyranoside) uronate de méthyle.

On dissout 2,8 g du composé 13 dans 30 ml de pyridine (6,56 mmol). Après refroidissement à 0 °C, on ajoute goutte à goutte 10 ml d'une solution de 2 ml de chlorure de chloroacétyle dans 20 ml de dichlorométhane. Après 30 minutes, on évapore à sec, on reprend le résidu par 200 ml de chloroforme, on lave avec une solution à 10 % de KH $SO_4$, puis avec de l'eau, on sèche et on concentre. Le sirop obtenu est chromatographié sur gel de silice (200 g ; éluant AcO-Et/hexane ; 1/3 ; v/v). On obtient ainsi 2,7 g de composé 14 pur sous forme de sirop (rendement : 80 %). $[\alpha]_D^{20} = + 2°$ (c = 1,5 ; chloroforme).

L'analyse élémentaire et le spectre de R.M.N. confirment la structure attendue.

2. déblocage du carbone anomère conduisant au composé 15 ou (2,3-di-O-benzyl-4-O-chloroacétyl-D-glucopyranoside) uronate de méthyle.

On dissout 2,7 g (5,3 mmol) du dérivé 14 dans 80 ml d'un mélange acétone/eau (5/1 ; v/v). De l'oxyde mercurique (3,1 g) est ajouté suivi d'une solution de chlorure mercurique (3,9 g) dans l'acétone (27 ml). Après 5 minutes, les sels sont éliminés par filtration. Après concentration à sec, le résidu est repris par du chloroforme.

La phase chloroformique est lavée avec une solution de KI à 10 % puis avec de l'eau. Après évaporation, le produit est cristallisé dans un mélange acétate d'éthyle/hexane. On obtient 2 g d'un solide de pf 105-107 °C ; $[\alpha]_D^{20} = — 4,7°$ (c. 1, chloroforme). L'analyse élémentaire et l'étude en RMN confirment la structure (rendement 80 %).

3. bromuration du carbone anomère conduisant au composé 16 ou (1-bromo-2, 3-di-O-benzyl-4-O-chloroacétyl-α-D-glucopyranoside) uronate de méthyle.

On dissout 2 g (4,30 mol) du composé 15 dans 50 ml de dichlorométhane. On ajoute 4,8 ml (34,4 mmol) de sym-collidine à 0 °C, suivie de bromure de bromométhylène diméthyl ammonium (17 mmol) préparé selon Hepburn D.R. et Hudson H.R. J. Chem. Soc. Perkin I (1976) 754-757. Après 4 heures de réaction, le mélange est dilué par 100 ml de dichlorométhane, puis versé dans de l'eau glacée. Après lavage avec de l'eau glacée, le solvant est évaporé. Après chromatographie sur gel de silice (20 g ; éluant hexane/acétate d'éthyle, 2/1 ; v/v) on obtient 2,06 g de composé 16 sous forme d'un sirop (rendement : 90 %). $[\alpha]_D^{20} = + 82,5°$ (c = 1,5 ; chloroforme). L'analyse élémentaire et l'étude en R.M.N. confirment la structure.

B) préparation du disaccharide 18 ou 3-O-acétyl-1,6-anhydro-2-azido-4-O-[(2,3-di-O-benzyl-4-O-chloro-acétyl-β-D-glucopyranosyl) uronate de méthyle] β-D-glucopyranose.

Cette synthèse est basée sur la condensation des monosaccharides 16 et 17 de 870 mg (3,8 mmol).

A une solution de 870 mg (3,8 mmol) de composé 17, dans le dichlorométhane, on ajoute 1 g de driérite 0,5 g de tamis moléculaires 4 Å, en poudre, et 0,525 g de carbonate d'argent fraîchement préparé. Après 2 heures d'agitation, on ajoute goutte à goutte à 0 °C 670 mg (1,3 mmol) du composé 16. Après 6 jours, les solides sont éliminés par filtration. Le sirop obtenu après concentration est chromatographié sur gel de silice (50 g ; éluant : chloroforme/acétate d'éthyle ; 4/1, v/v). On obtient le disaccharide 18 sous forme de mousse (421 mg ; 50 %). $[\alpha]_D^{20} = —17°$ (c = 1, chloroforme). L'analyse élémentaire confirme la structure. L'étude en R.M.N. confirme la configuration de la liaison interglycosidique.

C) préparation des disaccharides de structure 19 par acétolyse du disaccharide 18.

On prépare les disaccharides 19 en soumettant le disaccharide 18 à une réaction d'acétolyse comme

suit ; 300 mg du composé 18 sont dissous dans un mélange de 4 ml d'anhydride acétique et de 0,5 ml d'acide trifluoracétique fraîchement distillé. Le mélange réactionnel est soumis à agitation durant 10 h à 18 °C, puis évaporé à sec et co-évaporé avec du toluène. Le résidu est chromatographié sur une colonne de gel de silice (15 g). Par élution avec un mélange dichlorométhane acétate d'éthyle (19 : 1, v/v), on récupère 282 mg d'un mélange d'acétates anomères de structure 19 sous forme d'un sirop incolore (rendement, 86 %). Le rapport des formes α aux formes β, déterminé par analyse par R.M.N., est de 4/1.

Le spectre R.M.N. confirme la structure attendue.

D) préparation du disaccharide 20 par bromuration des disaccharides 19.

On soumet le mélange d'acétates de structure 19 à l'action de $TiBr_4$ : On soumet à agitation une solution de 140 mg du mélange d'acétates 19 dans un mélange de 3 ml de dichlorométhane et de 0,3 ml d'acétate d'éthyle à 17-18 °C, sous atmosphère d'argon sec, en présence de 140 mg de TiBr (≃ 2 équivalents) pendant 20 heures. Après refroidissement à 0 °C et dilution avec 30 ml de dichlorométhane, le mélange est lavé avec de l'eau glacée, puis avec une solution aqueuse à 5 % de bromure de potassium et ensuite avec de l'eau et séché sur du sulfate de sodium, filtré et évaporé. Le résidu est chromatographié sur une colonne de gel de silice (10 g). Par élution avec un mélange dichlorométhane — acétate d'éthyle (19 : 1, v/v), on récupère, par ordre d'élution :

— le bromure 20 (74 mg ; rendement 50 %) sous forme d'un sirop incolore, instable (immédiatement engagé dans la réaction suivante) ;

Le spectre R.M.N. confirme la structure attendue.

— une fraction (28 mg ; rendement 20 %) correspondant au produit de départ qui n'a pas réagi,

— une fraction migrant peu correspondant à des produits de O-débenzylation partielle.

## Exemple 3
## Synthèse du monosaccharide 22 ou benzyl 6-O-acétyl-3-O-benzyl-2-benzyloxycarbonylamino-2-désoxy-α-D-glucopyranoside de formule

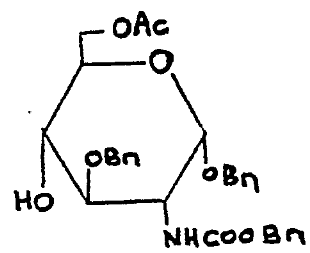

Ce dérivé est préparé à partir du benzyl-3-O-benzyl-2-benzyloxy-carbonylamino-2-désoxy-α-D-glucopyranoside (dérivé 21) en procédant comme suit (voir figure 5) :

Une suspension du composé 21 (987 mg, 2 mmol, ce composé est préparé selon P.C. Wyss and J. Kiss, Helv. Chim. Acta, 58 (1975) 1833- 1847 dans le 1,2-dichloroéthane anhydre (15 ml) est agitée à reflux pendant 30 h en présence de N-acétyl-imidazole (2,5 mmol, fraîchement préparé). Après refroidissement et dilution avec du chloroforme (50 ml), la phase organique est lavée avec une solution glacée d'acide chlorhydrique M, avec de l'eau, avec une solution aqueuse saturée d'hydrogénocarbonate de sodium, avec de l'eau, séchée (sulfate de sodium), filtrée et évaporée. Le résidu est chromatographié sur une colonne de gel de silice (50 g). L'élution par le mélange dichlorométhane acétone (15 : 1, v/v) donne le dérivé 22 sous forme d'un sirop cristallisant dans un mélange acétate d'éthyle-hexane (759 mg, 71 %), PF : 114-115 °C ; $[\alpha]_D$ = + 88° (c = 1, chloroforme).

## Exemple 4
## Synthèse du monosaccharide 33 de formule

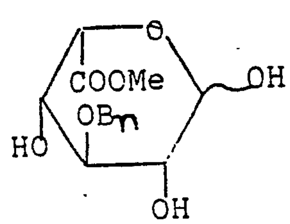

On effectue cette synthèse à partir du composé 23 selon les étapes suivantes (voir figure 6) :

1. introduction d'un groupe benzoyle en position 5,
2. méthylation de la fonction carboxyle en position 6,
3. isomérisation du groupe OH en position 5,
4. formation du cycle pyranique.

1. réaction de benzoylation

63 g de 3-O-benzyl-1, 2-O-isopropylidène-α-D-glucofuranoside (composé 23) sont dissous dans 500 ml de pyridine anhydre. On ajoute 85 g de chlorure de trityle et on chauffe à 80 °C pendant une heure. On obtient ainsi le composé 24.

Pouvoir rotatoire : $[\alpha]_D^{20} = -34,7°$, chloroforme.

La structure de ce composé a été confirmée par ses spectres I.R. et R.M.N., son analyse élémentaire est correcte.

Le mélange est ensuite refroidi à 0 °C et on ajoute 45 ml de chlorure de benzoyle. Après une nuit, on détruit l'excès des réactifs par addition de 300 ml de méthanol. Le mélange obtenu, évaporé à sec, est repris par du chloroforme. La phase chloroformique est lavée à l'eau, séchée sur sulfate de sodium et concentrée. On obtient ainsi le composé 25.

Le sirop obtenu est dissous dans 400 ml de chloroforme. Après addition de 100 ml d'une solution d'acide paratoluène-sulfonique 5 M dans le méthanol, la solution est laissée à 4 °C pendant une nuit. On obtient, après lavage à l'eau de la phase organique, 215 g d'un mélange. Le composé est obtenu par chromatographie de ce mélange sur gel de silice dans le solvant éther-hexane 2/1 (v/v). On obtient ainsi 36 g du composé 26.

Pouvoir rotatoire : $[\alpha]_D^{20} = 65,3°$, chloroforme.

La structure du composé 26 a été confirmée par ses spectres I.R. et R.M.N.

2. méthylation de la fonction carboxyle en position 6

Le composé 26 (1,88 g) est dissous dans l'acétone (20 ml). On ajoute, goutte à goutte à — 50 °C 3,5 ml d'une solution de $CrO_3$ (13 g) dans $H_2SO_4$, 3,5 M (29 ml). On laisse la température remonter et on laisse une heure dans ces conditions. Le mélange réactionnel est alors versé dans la glace et le produit est extrait au chloroforme. Après lavage à l'eau et séchage, on évapore à sec. On obtient le composé 27.

Le mélange obtenu est dissous dans du méthanol (20 ml), on ajoute ensuite 10 ml de soude 1 N et on laisse une nuit à température ambiante. Le mélange réactionnel est alors passé au travers d'une colonne (25 ml) de résine Dowex® 50 sous forme $H^+$ au préalable rincée avec du méthanol. Le produit est obtenu par concentration de l'éluat. On obtient ainsi le composé 28.

Ce composé est dissous dans l'éther et méthylé de façon classique par le diazométhane. On obtient, après évaporation, le composé 29 (1,08 g ; 70,4 %).

Pouvoir rotatoire $[\alpha]_D^{20} = -27°$, chloroforme.

L'analyse élémentaire trouvée du composé 29 est correcte. Sa structure est de plus confirmée par ses spectres I.R. et R.M.N.

3. Isomérisation du groupe —OH en position 5

A une solution d'anhydride triflique (0,8 ml) dans le dichlorométhane (16 ml), refroidie à — 20 °C, on ajoute goutte à goutte une solution de pyridine (0,8 ml) dans le dichlorométhane (8 ml). On ajoute ensuite, à — 10 °C, goutte à goutte, 800 mg de composé 29 dissous dans du dichlorométhane (8 ml). Après une heure à — 50 °C, le mélange réactionnel est versé dans un mélange d'eau et de glace (8 ml) contenant 160 mg de bicarbonate de sodium. On agite jusqu'à séparation des deux phases organique et aqueuse. La phase organique est lavée par HCl 3 %, $H_2O$, NaCl saturé, séchée et concentrée. On obtient ainsi le composé 30.

Le sirop est repris par du DMF (10 ml). On ajoute du trifluoroacétate de sodium (1,6 g) et chauffe à 80 °C pendant 3 heures. On obtient ainsi le composé 31.

Après évaporation, reprise par du dichlorométhane, lavage à l'eau et séchage, le résidu est repris par du méthanol puis le solvant est évaporé après une heure. Après chromatographie sur colonne dans le solvant éther-hexane 2/1, on obtient le composé 32 (450 mg : 56,2 %).

Pouvoir rotatoire : $[\alpha]_D^{20} = -33°$ chloroforme.

La structure du composé 32 est confirmée par ses spectres IR et RMN. L'analyse élémentaire trouvée est correcte.

4. formation du cycle pyranique

Cette synthèse est effectuée à partir du composé 32. Le composé 32 (200 mg) est dissous dans un mélange acide trifluoroacétique/eau 9/1). Après 15 minutes, les solvants sont évaporés. Le résidu est cristallisé dans de l'acétate d'éthyle/hexane. On obtient ainsi 110 mg de composé 33.

Les caractéristiques de ce dérivé sont les suivantes :

spectre IR : dans $CHCl_3$, $\gamma$ en $cm^{-1}$ : 3 450 (OH), 3 080, 3 060, 3 030 ($CH_2$ : benzyle et 1 740 ($COOCH_3$)

spectre RMN : δ en ppm par rapport au TMS : 3,75 (s, 3H⁺, COOMe) 4,98 (1 H⁺), 7,30 (s, 5H⁺, $C_6H_5$) pouvoir rotatoire : $[\alpha]_D^{20} = +13°$, méthanol,

| - analyse élémentaire pour | calculé | trouvé |
|---|---|---|
| $C_{14}H_{18}O_7$ | | |
| C........................ | 56,37 | 56,17 |
| H........................ | 6,08 | 5,85 |
| - P.F..................... | 125-126°C. | |

Exemple 5
Synthèse du dérivé 38 ou
3-O-benzyl-4-O-chloroacétyl-1
2-O-tert. butoxyéthylidène-β-L-méthyl
idopyranuronate de formule

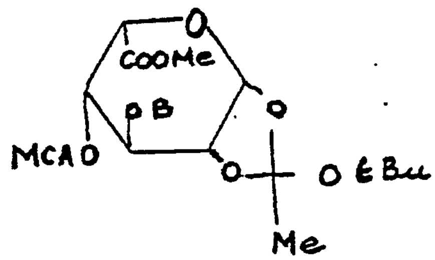

Cette synthèse (voir figure 7) est effectuée à partir du dérivé 33 à structure acide iduronique en soumettant α) le dérivé 33 à une réaction d'acétylation, β) le mélange d'acétates anomères 34 et 35 obtenus, à l'action d'un agent de bromuration afin d'introduire un atome de brome sur le carbone anomère, γ) en formant un orthoester en positions 1, 2 et δ) en effectuant une monochloroacétylation en 4 de l'orthoester.

α) réaction d'acétylation conduisant aux 1, 2, 4-tri-O-acétyl-3-O-benzyl-α, β-L-méthyl idopyranuronates (dérivés 34 et 35).

Une solution du composé 33 (3 g) dans un mélange de pyridine anhydre (20 ml) et d'anhydride acétique (10 ml) est agité à 0 °C, à l'abri de l'humidité, pendant 5 h. Le mélange réactionnel est évaporé à sec, évaporé avec du toluène (4 × 20 ml), et séché sous vide. Le résidu est chromatographié sur une colonne de gel de silice (150 g).

L'élution par le mélange toluène : acétate d'éthyle (4 : 1 v/v) donne, par ordre d'élution :

— une fraction de tête composée de dérivés furanniques,

— le composé 34, (anomère α), sirop, (170 mg, 4 %), $[\alpha]_D = -43°$ ; (c : 1, chloroforme), R.M.N. ($CDCl_3$) : δ : 6,23 (s, 1 H, H-1).

— le composé 35 (anomère β), cristallisant dans un mélange éther-hexane, 2,688 g, 63 %), P.F. : 112-113 °C, $[\alpha]_D = +9°$ (c : 1, chloroforme) R.M.N. ($CDCl_3$) : δ : 6,08 (d, 1 H, H-1, $J_{1,2}$ : 1,5 Hz).

Les anomères α et β 34 et 35 ne sont pas séparés lorsqu'on procède à la succession des synthèses décrites.

Leur mélange est utilisé directement sous forme de sirop pour les réactions ultérieures.

β) réaction de bromuration conduisant au composé 36 ou bromure de 2,4-di-O-acétyl-3-O-benzyl-α-L-méthyl idopyranuronyle.

Un mélange d'acétates 34 et 35 (212 mg ; 0,5 mmol) est dissous dans du dichlorométhane anhydre (5 ml) et de l'acétate d'éthyle anhydre (0,5 ml). Du tétrabromure de titane (250 mg, 0,7 mmol) est ajouté en une seule fois, et le mélange réactionnel est agité 24 h à la température ambiante à l'abri de l'humidité. Après refroidissement à 0 °C et dilution avec du dichlorométhane, la phase organique est lavée avec de l'eau glacée (3 fois), séchée (sulfate de sodium), filtrée et évaporée pour donner le dérivé 36 sous forme d'un sirop légèrement coloré (217 mg, 96 %), R.M.N. ($CDCl_3$) : δ : 6,41 (s, 1 H, H-1). Ce composé, très instable est immédiatement engagé dans la réaction suivante.

γ) préparation de l'orthoester ou 4-O-acétyl-3-O-benzyl-1,2-O-tert-butoxyéthylidène-β-L-méthyl idopyranuronate.

Une solution de bromure 36 (fraîchement préparée à partir de 2,122 g, 5 mmol, de mélange d'acétates 34 et 35 dans du dichlorométhane anhydre (20 ml) est agitée à la température ambiante sous atmosphère d'argon sec. De la sym-collidine (2,65 ml, 20 mmol) et du tert-butanol anhydre (3 ml ; 30 mmol) sont successivement ajoutés, et le mélange réactionnel est agité 15 h dans ces conditions. Après dilution avec du dichlorométhane (50 ml) la phase organique est lavée avec une solution aqueuse saturée d'hydrogénocarbonate de sodium, avec de l'eau, séché sur sulfate de sodium, filtré et évaporé. Le résidu est chromatographié sur colonne de gel de silice (120 g). L'élution par le mélange hexane : acétate d'éthyle (2 : 1, v/v, contenant 0,5 % de triéthylamine) donne le composé 37 sous forme d'un sirop pur (1,542 g, 70 % à partir de 34 et 35) [α] = — 23° (c : 1, chloroforme), R.M.N. ($CDCl_3$) : δ : 5,48 (d, 1 H, H-1, $J_{1,2}$ : 2,5 Hz).

δ) monochloroacétylation de l'orthoester 37 :

Une solution de l'orthoester 37 (220 mg, 0,5 mmol) dans le méthanol anhydre (10 ml) est refroidie à — 20 °C sous agitation et atmosphère d'argon sec. Du carbonate de potassium anhydre (40 mg) est ajouté et le mélange réactionnel est agité pendant 5 h dans ces conditions. Les solides sont essorés, le filtrat est évaporé et le résidu est repris dans du chloroforme (50 ml). La phase organique est lavée rapidement avec de l'eau glacée (3 fois), séchée (sulfate de sodium), filtrée et évaporée. Le résidu est immédiatement dissous dans de la pyridine anhydre (4 ml) et du dichlorométhane anhydre (2 ml). Après refroidissement à — 20 °C sous atmosphère d'argon sec, une solution de chlorure de chloroacétyle (0,1 ml, 1,24 mmol, fraîchement distillé) dans le dichlorométhane anhydre (1 ml) est ajoutée goutte à goutte. Le mélange réactionnel est agité dans ces conditions pendant 30 mn, puis versé dans un mélange eau-glace (100 ml). Après agitation pendant 15 mn, le mélange est extrait avec du chloroforme (3 × 20 ml). Les phases organiques sont lavées avec de l'eau glacée, avec une solution aqueuse à 2 % d'hydrogénocarbonate de sodium, avec de l'eau, séchées (sulfate de sodium), filtrées et évaporées. Le résidu est chromatographié rapidement sur une colonne de gel de silice (12 g). L'élution par le mélange hexane : acétate d'éthyle (5 : 2, v/v, contenant 0,2 % de triéthylamine) donne, par ordre d'élution :

un composé insaturé 39 (15 mg, 8 %),

l'orthoester 38 sirop (145 mg, 61 % à partir de 12), $[\alpha]_D$ = + 19° (c : 1, chloroforme), R.M.N. ($CDCl_3$) : δ : 5,45 (d, 1 H, H-1, $J_{1,2}$ : 2,5 Hz), 5,24 (d. de d., 1 H, H-4, $J_{3,4}$ : 2,5 Hz, $J_{4,5}$ : 1,5 Hz), 4,00 (s, 2 H ; $Cl-CH_2-COO-$).

Exemple 6

Synthèse du disaccharide 41 ou
benzyl 6-O-acétyl-3-O-benzyl-2-
benzyloxycarbonylamino-2-désoxy-4-O-
(2-O-acétyl-3-O-benzyl-α-L-méthyl idopyranuronyl)
-α-D-glucopyranoside

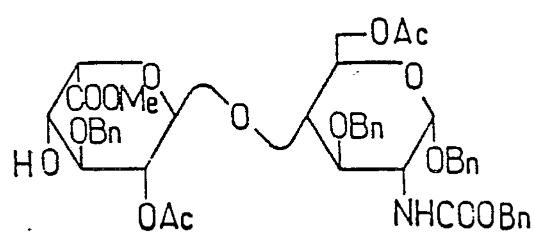

(41)

On prépare tout d'abord selon l'étape α) le disaccharide 40 par condensation des monosaccharides 38 et 22, puis on élimine le groupe monochloroacétyle en position 4 dans l'étape β, ce qui conduit au disaccharide 41 recherché (voir figure 8) :

étape α : préparation du disaccharide 40 ou benzyl 6-O-acétyl-3-O-benzyl-2-benzyloxycarbonylamino-2-désoxy-4-O (2-O-acétyl-3-O-benzyl-4-O-chloroacétyl-α-L-méthyl idopyranuronyl)-α-D-glucopyranoside.

Une solution de l'orthoester 38 (284 mg, 6,6 mmol) et de l'alcool 22 (214 mg, 0,4 mmol) dans du chlorobenzène anhydre (12 ml) est chauffée à 140 °C sous agitation et léger courant d'argon sec. Après distillation lente de 10 ml de solvant, une solution de perchlorate de 2,6-diméthylpyridinium (0,006 mmol, fraîchement préparé) dans du chlorobenzène (4 ml) est ajoutée goutte à goutte en 30 min avec distillation simultanée de solvant (4 ml). Le mélange réactionnel est agité 1 h, avec addition de solvant frais (10 ml) et distillation simultanée de telle sorte que le volume réactionnel reste constant et égal à 4 ml. Après refroidissement et dilution avec du chloroforme, la phase organique est lavée avec une solution saturée

d'hydrogénocarbonate de sodium, avec de l'eau séchée sur sulfate de sodium, filtrée et évaporée. Le résidu est chromatographié sur une colonne de gel de silice (40 g). L'élution par le mélange hexane : acétate d'éthyle (4 : 3, v/v) donne, par ordre d'élution :

— le produit 22, 120 mg, 56 %)

— le disaccharide 40, cristallisé dans un mélange éther-hexane (112 mg, 30 %, P.F. : 144-145 °C, $[\alpha]_D^{20}$ = + 35° (c : 1, chloroforme), R.M.N. ($CDCl_3$) : conforme à la structure attendue.

étape β : élimination du groupe monochloroacétyle.

Un mélange du disaccharide 40 (56 mg, 0,06 mmol) et de thiourée (7 mg, 0,1 mmol) dans de la pyridine (2,5 ml) et de l'éthanol absolu (0,5 ml) est agité à 100 °C pendant 30 mn. Après refroidissement et évaporation à sec, le résidu est repris par un mélange eau-chloroforme (1 : 1, v/v 40 ml). La phase organique est lavée avec de l'eau, séchée (sulfate de sodium), filtrée et évaporée. Le résidu est chromatographié sur une colonne de gel de silice (2 g). L'élution par le mélange acétate d'éthyle : hexane (2 : 1, v/v) donne le disaccharide 41, cristallisé dans l'éther (46 mg, 30 %), P.F. : 146-147 °C $[\alpha]_D$ = 44° (c : 1, chloroforme), R.M.N. ($CDCl_3$) : conforme à la structure attendue.

Exemple 7
Synthèse du tétrasaccharide 43 de formule

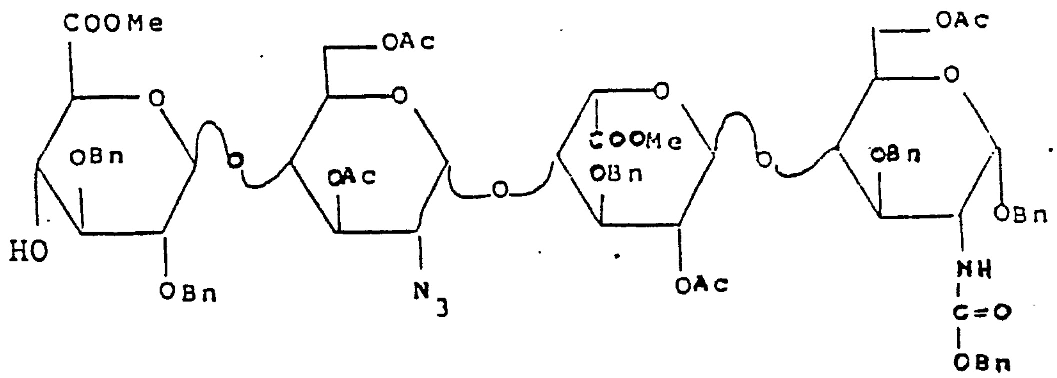
. (43)

On prépare le tétrasaccharide 43 en effectuant :

— dans l'étape a) la condensation des disaccharides 20 et 41 dont la synthèse est décrite dans les exemples 2 et 6 et en soumettant au cours de l'étape b) le tétrasaccharide 42 formé à une réaction sélective de -O-démonochloroacétylation en position 4 (voir figure 9) :

a) Réaction de condensation

Un mélange de 64 mg (80 μmol) du bromure 20 fraîchement préparé, de 51 mg (60 μmol) du composé 41 et de 80 mg de tamis moléculaire 0,4 nm (4 Å) en poudre dans 1,5 ml de dichloro-éthane anhydre est soumis à une agitation durant une demi-heure à la température ambiante, sous atmosphère d'argon sec, puis est refroidi à — 20 °C. On ajoute successivement 20 μl (150 μmol) de sym-collidine et 31 mg (120 μmol) de triflate d'argent. Le mélange réactionnel est soumis 1 h à agitation à — 20 °C, puis on laisse la température remonter vers la température ambiante pendant 15 h. Après dilution avec 50 ml de dichlorométhane, les solides sont essorés et le filtrat est lavé avec une solution aqueuse glacée d'acide chlorhydrique 1 M puis avec de l'eau (deux fois). On le sèche ensuite sur du sulfate de sodium, on le filtre puis on évapore.

Le résidu est chromatographié sur une colonne de gel de silice (8 g, gel 230-400 mesh). L'élution par le mélange hexane-acétate d'éthyle (4 : 3, v/v) permet de récupérer 37 mg de tétrasaccharide 42 (rendement 39 %) sous forme d'un verre incolore. $[\alpha]_D^{20}$ = + 56° (c = 0,6 ; $CHCl_3$) ; le spectre de R.M.N. confirme la structure attendue.

Par élution de la colonne avec le mélange acétate d'éthyle-hexane (2 : 1, v/v), on récupère 23 mg du produit de départ 41 (rendement 44 %).

b) Réaction de —O-déchloroacétylation

Une solution de 36 mg (23 μmol) du tétrasaccharide 42 dans 1,25 ml d'un mélange de pyridine et 0,25 ml d'éthanol absolu est chauffée à 100 °C en présence de 7 mg (100 μmol) de thiourée pendant 20 min. Après refroidissement et évaporation à sec, le résidu solide est repris avec 20 ml d'eau et extrait avec du chloroforme (5 fois 5 ml). Les phases organiques sont lavées avec une solution aqueuse à 10 % d'hydrogénosulfate de sodium, avec de l'eau, séchées sur du sulfate de sodium, filtrées et évaporées. Le

résidu est chromatographié sur une colonne de gel de silice (3 g). Par élution avec un mélange acétate d'éthyle-hexane (3 : 2, v/v), on obtient 27 mg du dérivé 43 (rendement 80%) sous forme d'un verre incolore $[\alpha]_D^{20} = +61°$ (c = 0,8 ; chloroforme) ; le spectre de R.M.N. confirme la structure attendue (voir figure 31).

Exemple 8 (voir figure 10)

Synthèse du pentasaccharide 45 de formule

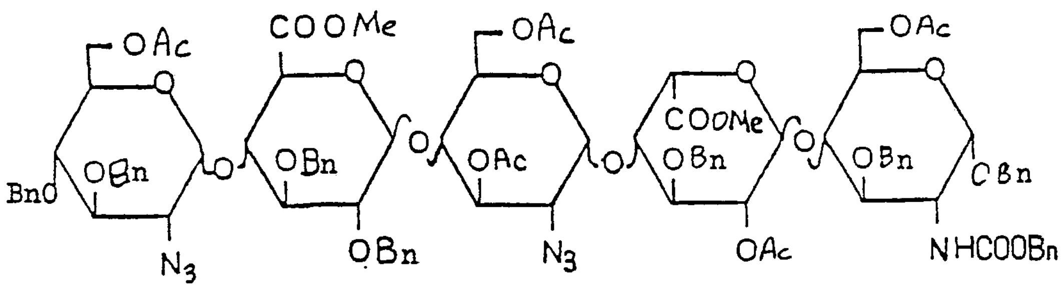

On effectue une réaction de condensation entre le tétrasaccharide 43 et le monosaccharide 44, ce qui conduit au pentasaccharide 45.

Un mélange de 27 mg (54 µmol) de bromure 44 préparé selon H. Paulsen und W. Stenzel, Chem. Ber., 111 (1978) 2334-2347, de 26 mg (18 µmol) du tétrasaccharide 43 et de 50 mg de tamis moléculaire 0,4 nm (4 Å) en poudre dans 0,8 ml de dichloroéthane est soumis à agitation durant 1/2 h à la température ambiante sous atmosphère d'argon sec, puis refroidi à — 20 °C. 16 µl (120 µmol) de sym-collidine et 26 mg (100 µmol) de triflate d'argent sont ajoutés successivement et le mélange réactionnel est soumis à agitation pendant 18 h en laissant la température remonter lentement vers la température ambiante.

Après dilution avec 50 ml de dichlorométhane, les solides sont essorés et le filtrat est lavé avec une solution aqueuse glacée d'acide chlorhydrique 1 M puis avec de l'eau (2 fois). On le sèche ensuite sur du sulfate de sodium, on le filtre, puis on évapore.

Le résidu est chromatographié sur une colonne de gel de silice (5 g, gel 230-400 mesh). Par élution avec un mélange hexane-acétate d'éthyle (4 : 3, v/v), on récupère 30 mg de pentasaccharide 45 sous forme d'un verre incolore (rendement 90 %) $[\alpha]_D^{20} = +67°$ (c 1 : chloroforme). Le spectre de R.M.N. confirme la structure attendue. On trouve en particulier pour les protons anomères des unités de glucosamine des déplacements (δ, T.M.S.) de 5,36 et 5,52 ppm pour les protons appartenant à H, F et D respectivement.

Exemple 9

Préparation du pentasaccharide 50 (voir figures 10 et 11)

On a recours aux étapes suivantes :

a) d'élimination des groupes acétyle (pentasaccharide 46).

b) de sulfatation des groupes —OH ainsi libérés (pentasaccharide 47).

c) d'hydrogénation pour libérer les groupes —OH protégés par des groupes benzyle et pour transformer le groupe —$H_3$ en groupe —$NH_2$ (pentasaccharide 48).

d) de sulfatation des groupes $NH_2$ (pentasaccharide 49, puis saponification des groupes —COOMe en position 6 (pentasaccharide 50).

Ces étapes sont réalisées comme suit :

a) élimination des groupes acétyle du dérivé 45.

Une solution de 28 mg du pentasaccharide 45 dans un mélange de 2,5 ml de 1,2-diméthoxyéthane et de 0,8 ml de méthanol est refroidie à 0 °C sous agitation. On ajoute alors 1 ml d'une solution 1 M de soude, goutte à goutte, en 10 minutes. Le mélange réactionnel est soumis à agitation 1 heure à 0 °C, puis 12 heures à la température ambiante. Après refroidissement à 0°C, on ajoute 3 ml d'acide chlorhydrique 1 M et le mélange laiteux est immédiatement extrait avec du chloroforme (5 fois 5 ml). Les phases organiques sont lavées avec de l'eau, séchées sur du sulfate de sodium, filtrées et évaporées. Le résidu est repris dans 2 ml de méthanol et traité par une solution éthérée de diazométhane (excès jusqu'à persistance de la coloration jaune) pendant une demi-heure.

Après évaporation à sec, le résidu est chromatographié sur une colonne de gel de silice (2 g, gel 230-400 mesh). L'élution par le mélange dichlorométhane-méthanol (15 : 1, v/v) permet de récupérer 18 mg du

pentasaccharide 46 (rendement 72 %) sous forme d'un verre incolore. $[\alpha]_D^{20} = +57°$ (c = 1 ; chloroforme) ;

La structure attendue est confirmée par le spectre de R.M.N.

b) sulfatation des groupes —OH.

A une solution du composé 46 (22 mg) dans le diméthylformamide (0,5 ml), on ajoute du complexe triméthylamine/$SO_3$ (22 mg, 2,5 éq/OH). Le mélange réactionnel est chauffé à 50 °C durant environ 14 h. On ajoute alors à nouveau du complexe triméthylamine/$SO_3$ (10 mg) et laisse la réaction évoluer durant 24 heures. On ajoute au mélange réactionnel du méthanol (0,5 ml) et du chloroforme (0,5 ml). La solution est introduite au sommet d'une colonne de Sephadex® $LH_{20}$, équilibrée dans un mélange $CHCl_3/CH_3OH$ (1/1 ; v/v). Les fractions contenant le produit sulfaté sont regroupées et le solvant est évaporé. On obtient ainsi un verre (30 mg).

Ce verre est ensuite chromatographié sur gel de silice (10 g) dans un solvant constitué de 3 parties du mélange acétate d'éthylène/pyridine acide acétique/eau, (6/2/0, 6/1, v/v/v/v) et de 2 parties du mélange acétate d'éthyle/pyridine/acide acétique/eau, (5/5/1/3, v/v/v/v) parties du mélange acétate d'éthyle/pyridine/acide acétique/eau, 5/5/1/3, v/v/v/v.

Les fractions contenant le produit désiré sont rassemblées et concentrées. Après évaporation des solvants, le résidu obtenu est dissous dans du méthanol additionné d'eau, puis passé au travers d'une colonne de Dowex® 50 W × 4, $Na^+$, équilibrée dans un mélange méthanol/eau (50/50, v/v). On obtient ainsi le sel de sodium (composé 47).

c) hydrogénation.

Le produit obtenu ci-dessus est dissous dans du méthanol (3,7 ml) additionné d'eau (0,3 ml).

A cette solution, on ajoute le catalyseur (Pd/C, 5 %, 40 mg) et on agite sous atmosphère d'hygrogène pendant 5 jours. Après élimination du catalyseur par filtration, l'analyse du spectre U.V. de la solution obtenue montre la disparition totale de l'absorption due aux groupes benzyle. Le solvant est alors évaporé, laissant un résidu, à savoir le composé 48.

d) sulfatation des groupes —$NH_2$, puis saponification des groupes carboxyle.

Le composé 48 est dissous dans l'eau (4 ml). Le pH est ensuite ajusté à 9,5 puis on ajoute à la solution du complexe triméthylamine/$SO_3$ (54 mg). Le pH est maintenu à 9,5 pendant toute la durée de la réaction par addition de soude 0, 1 N.

Après une nuit, on procède à une nouvelle addition d'agent de sulfatation (27 mg). Une dernière addition est effectuée après 24 h.

Après 48 h, on ajoute de la soude (3 M, 34 ml) au composé 49 formé, puis la solution est soumise à agitation pendant 3 heures à température ambiante de manière à hydrolyser les méthylesters des motifs de type acide uronique.

Le mélange réactionnel est ensuite neutralisé puis concentré jusqu'à un volume d'environ 2 ml.

La solution ainsi obtenue est déposée au sommet d'une colonne de Séphadex® G 25 (100 ml) éluée avec de l'eau. Les fractions collectées sont analysées par absorption U.V. (206 nm) et en polarométrie (265 nm). Les fractions présentant une activité optique sont regroupées, le solvant est éliminé et le résidu repris par environ 2 ml d'eau et lyophilisé.

On obtient ainsi le dérivé 50 sous forme de poudre blanche (5,6 mg, 25 % par rapport au produit 45).

L'étude en R.M.N. confirme la structure attendue. On trouve en particulier pour les protons anomères des unités de glucosamine, des déplacements (δ, T.M.S.) de 5,36, 5,45 et 5,52 ppm pour les protons appartenant à H, F et D respectivement.

## Exemple 10
### Synthèse du disaccharide 51, à savoir du 1 prop-1'-ène 2,3-di-O-benzyl-4-O [2-azido-3,4-di-O-benzyl-6-O-acétyl-α-D-glucopyranoside] uronate de méthyle de formule

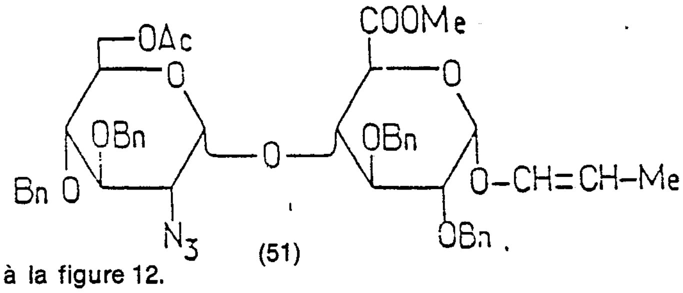

(51)

On se reportera à la figure 12.

A une solution de monosaccharide 13 (0,215 g ; 0,5 mmol) dans le dichlorométhane (3 ml), on ajoute le monosaccharide 44 (0,49 g ; 1 mmol) dans du dichlorométhane (3 ml) puis des tamis 0,4 nm (4 Å) en poudre. On refroidit le mélange à 0 °C, puis on ajoute de la sym-collidine (0,16 ml), et du triflate d'argent (0,3 g). Après 1 heure, le mélange est dilué avec du dichlorométhane (50 ml). Les solides sont essorés, puis la solution est lavée avec une solution à 5 % de bicarbonate de sodium, avec de l'eau, puis du sulfate acide de potassium à 10 % et à nouveau avec de l'eau. On obtient ainsi, après évaporation 591 mg de résidu. Après purification sur silice dans un mélange toluène/acétone 30/1 (v/v), on récupère 211 mg de disaccharide 51 pur.

Ce produit est caractérisé par son analyse élémentaire.

Exemple 11

Synthèse du disaccharide 54, à savoir du (1-trichloroacétimidyle-2,3-di-O-benzyl -4-O-[2-acétylamido-2-désoxy-3,4-di-O-benzyl -6-O-acétyl-α-D-glucopyranoside] uronate de méthyle de formule

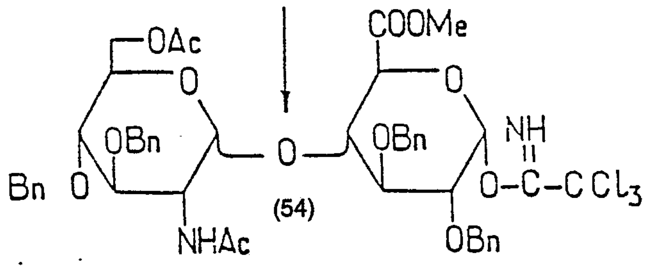
(54)

On se reportera à la figure 12 pour le schéma de synthèse.

A une solution du disaccharide 51 (180 mg) dans 6 ml d'un mélange acétone/eau (5/1 ; v/v), on ajoute successivement de l'oxyde mercurique (232 mg) puis goutte à goutte une solution de chlorure mercurique dans un mélange acétone/eau (292 mg/2 ml).

Après filtration, évaporation, reprise par du chloroforme et lavage par une solution d'iodure de potassium à 10% et à l'eau, on obtient le disaccharide 52 (140 mg).

On dissout 100 mg du disaccharide 52 dans 1,6 ml de méthanol. A cette solution, on ajoute du formiate d'ammonium (160 mg) et du catalyseur Pd/C 10 % (100 mg). Après cinq minutes, on élimine le catalyseur et on ajoute de l'anhydride acétique (10 gouttes). Après évaporation, le produit obtenu est purifié sur silice dans un mélange toluène/acétone (4/1 ; v/v). On obtient ainsi 61 mg de disaccharide 53.

Le disaccharide 53 est caractérisé par son Rf sur plaque de silice (Merck, référence 5719) dans deux solvants différents : chloroforme/acétate d'éthyle, 3/2, v/v Rf = 0,40 et toluène/acétone, 4/1, v/v ; Rf = 0,20.

Le dérivé 53 (60 mg) est dissous dans du dichlorométhane (1,5 ml). On ajoute alors du trichloroacétonitrile (75 µl) et de l'hydrure de sodium (1,5 mg). Après 15 minutes, le dérivé 53 a disparu au profit du dérivé 54. Après filtration et évaporation, on obtient 5 (67 mg). Le dérivé 54 est caractérisé par son Rf sur plaque de silice (Merck, référence 5719) chloroforme/acétate d'éthyle, 2/1, v/v ; Rf = 0,59 (0,37 pour le composé 53).

Exemple 12

Synthèse du dérivé 57, à savoir du 1,6-anhydro-2,3-époxy-4-O-acétyl [2,3-di-O-benzyl-uronate de méthyle]-α-D-glucopyranoside de formule :

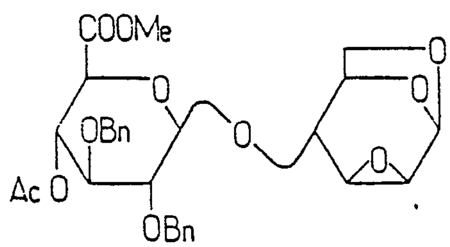
(57)

On effectue cette synthèse à partir des dérivés 55 et 56 (voir figure 13).

a) Préparation du (bromo 2,3-di-O-benzyl-4-O-acétyl-D-glucopyranoside) uronate de méthyle- (composé 55).

Synthèse du composé 1d

A une solution de 1a (32 g ; 85,5 mmol) dans la pyridine (250 ml), on ajoute du chlorure de trityle (28,6 g ; 1,2 eq) puis on chauffe à 80 °C. Une nouvelle addition de chlorure de trityle (4.6 g ; 0,2 eq) est faite après 3 heures de réaction. Lorsque la formation de 1b est complète (c. c. m. silice ; méthanol/chloroforme, 1/20, v/v) on refroidit la solution jusqu'à 0 °C, puis on ajoute du chlorure de benzoyle (15 ml ; 1,5 eq). Après une nuit, 1c est formé quantitativement. Du méthanol (150 ml) est alors ajouté goutte à goutte au mélange réactionnel qui est ensuite concentré à sec. Le résidu obtenu est repris dans du méthanol (500 ml) contenant de l'acide paratoluène-sulfonique (95 g). Après 2 heures de réaction, le mélange réactionnel est transvasé dans une ampoule à décanter contenant de l'eau glacée (2 l). Le produit 1d est extrait au chloroforme puis engagé tel que dans l'étape suivante.

Une partie de ce produit a été purifiée. L'analyse du spectre I.R. confirme la structure. C'est une gomme incolore. $[\alpha]_D^{20}$ — 61° (chloroforme).

Synthèse du composé 1j

Le sirop obtenu à l'étape précédente (95 g) est dissous dans l'acétone (1 l), puis à la solution, refroidie à 0 °C, on ajoute goutte à goutte, une solution d'oxyde de chrome (52 g) dans l'acide sulfurique 3,5 M (220 ml). Après 2 heures de réaction, le mélange réactionnel est versé dans de l'eau glacée (1 l). Le produit 1e est extrait du chloroforme (5 × 200 ml). La phase chloroformique est lavée jusqu'à pH neutre, séchée et concentrée à sec.

Au résidu obtenu ci-dessus, dissous dans le méthanol (650 ml), on ajoute goutte à goutte de la soude en solution dans l'eau (20 g dans 50 ml), puis on chauffe le mélange à 50 °C. Après une nuit, la solution obtenue est concentrée partiellement, puis versée dans l'eau (1,5 l). La phase aqueuse est ensuite lavée avec de l'éther, puis, après acidification par l'acide chlorhydrique, le produit 1f est extrait à l'éther. La phase éthérée est séchée sur sulfate de sodium, puis concentrée à sec, livrant une masse jaune (50 g) qui contient 1e.

Ce résidu (50 g) est dissous dans un mélange d'acide acétique et d'acide trifluoroacétique (15/1, v/v, 615 ml). A cette solution, agitée à 100 °C, on ajoute de l'eau (160 ml). Après une nuit on évapore à sec et élimine les traces d'acide acétique, évaporation de toluène. Le résidu formé en partie de 1f non hydrolysé et de 1j est dissous dans l'éther (400 ml).

A cette solution, on ajoute à 0 °C, une solution éthérée de diazométhane jusqu'à complète méthylation (c. c. m. silice, éther/hexane, 2/1, v/v). L'excès de diazométhane est alors détruit par l'acide acétique puis le mélange réactionnel est concentré à sec.

Le résidu est purifié sur une colonne de gel de silice (200 g) éluée d'abord par du chloroforme pur, puis par un mélange chloroforme/éther, 3/1, v/v. On obtient ainsi 1k (8,6 g ; 22,2 mmol, 26 % par rapport à 1a).

Le dérivé 1K est cristallin p.f. 122-123 °C. L'analyse élémentaire et le spectre de R.M.N. confirment sa structure.

Synthèse du composé 1l

A une solution de 1k (3,9 g ; 10 mmol) dans la pyridine (50 ml), on ajoute de l'anhydride acétique (4 ml, 42 mmol). Après 2 heures, le mélange réactionnel est évaporé à sec. On obtient ainsi 1l (4,62 g ; 98 %.

Synthèse du composé 55

A une solution de 1l (1,4 g) dans le dichlorométhane 30 ml et l'acétate d'éthyle (3 ml), on ajoute du tétrabromure de titane (1,5 g). La solution est agitée toute la nuit à température ambiante. Après dilution par du dichlorométhane, le mélange réactionnel est versé dans l'eau glacée. La phase organique est lavée avec du bicarbonate à 5 % dans l'eau, séchée et concentrée. Le résidu est chromatographié sur silice (50 g, éther/hexane, 1/1, v/v).

On obtient ainsi le composé 55 (920 mg, 62 %) ; c'est un sirop incolore $[\alpha]_D^{20}$ = + 97,5° (c = 1, chloroforme). L'analyse élémentaire et le spectre de R.M.N. confirment la structure.

b) Une solution du dérivé 56 (432 mg, 3 mmol) dans le dichlorométhane (10 ml) est agitée à 0 °C en présence de tamis moléculaire 0,4 nm (4 Å) (0,5 g), de driérite (1 g) et de carbonate d'argent fraîchement préparé (0,42 g). Après refroidissement à 0 °C, on ajoute, goutte à goutte, une solution du composé 55 (490 mg, 1 mmol) dans le dichlorométhane (6 ml). La réaction dure deux heures, le mélange réactionnel est ensuite filtré. Après évaporation à sec et chromatographie sur gel de silice du résidu, (solvant : acétate d'éthyle/chloroforme, 1/6, v/v), on obtient le dérivé 57 (285 mg ; 51 %).

La structure du dérivé 57 est confirmée par son analyse élémentaire et son spectre de R.M.N. Pouvoir rotatoire : $[\alpha]_D^{20}$ = 39° ; chloroforme ; P.F. = 156-159 °C.

Exemple 13

Synthèse du dérivé 59, de formule :

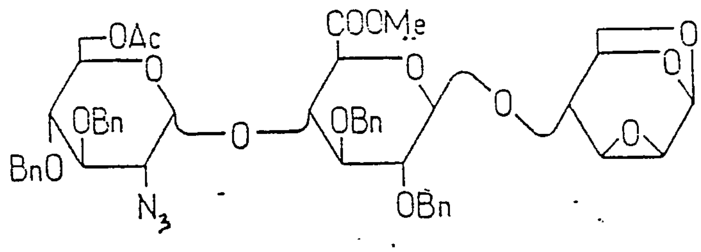

Le trisaccharide 59 est préparé par réaction du disaccharide 58 (obtenu par élimination du groupe acétyle en position 4 du composé 57 de l'exemple 12), avec le monosaccharide 44 en opérant comme suit (voir figure 13) :

— réaction de désacétylation du composé 57 :

A une solution du disaccharide 57 (260 mg) dans le méthanol (25 ml), on ajoute, à 0 °C, une solution de soude 1 N (25 ml). Après une heure, le mélange est acidifié par addition d'acide chlorhydrique 1 N (30 ml). Le produit est extrait au chloroforme. Après évaporation, le résidu est cristallisé dans un mélange acétate d'éthyle/hexane. On obtient 167 mg (rendement 70 %) du dérivé 58.

Pouvoir rotatoire : $[\alpha]_D^{20}$ = — 31° ; chloroforme, P.F. = 169-170 °C. L'analyse trouvée est correcte. La structure du dérivé 58 est de plus confirmée par son spectre R.M.N.

— condensation du disaccharide 58 avec le monosaccharide 44 :

A une solution des composés 44 (300 mg) et 58 (155 mg) dans le dichlorométhane (5 ml), on ajoute successivement du tamis de 0,4 nm (4 Å) en poudre (500 mg), puis de la collidine (100 µl) et du triflate d'argent. Après 15 minutes, la solution est diluée avec du dichlorométhane (50 ml), filtrée, lavée avec successivement de l'eau, une solution à 10 % de sulfate acide de potassium et de l'eau. Après séchage et concentration, le résidu est chromatographié sur gel de silice dans un mélange acétate d'éthyle/chloroforme (1/10, v/v). On obtient ainsi le dérivé 59 sous forme de mousse blanche.

Ce dérivé 59 est caractérisé par son analyse élémentaire, son spectre de R.M.N. et son pouvoir rotatoire ($[\alpha]_D^{20}$ = + 25° ; chloroforme).

Exemple 13 A :
Synthèse du trisaccharide de formule

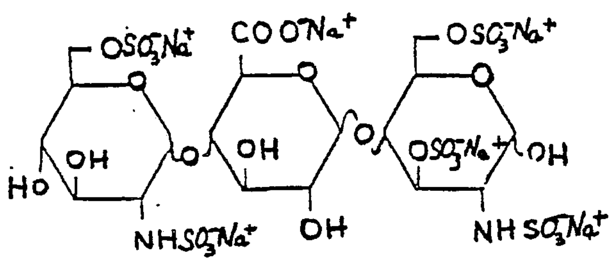

On fait réagir le monosaccharide 20 avec du méthanol dans les conditions décrites pour la synthèse du tétrasaccharide EFGH ci-dessus. On obtient ainsi un β-méthylglycoside. Le groupe MCA est éliminé de manière classique puis le disaccharide est soumis à l'action du monosaccharide 44 dans les conditions décrites ci-dessus pour l'élaboration du pentasaccharide.

Le trisaccharide obtenu est ensuite soumis aux réactions classiques aux fins de déprotection et de fonctionnalisation. La structure est confirmée par le spectre R.M.N.

Exemple 14

Synthèse du trisaccharide 62 de formule :

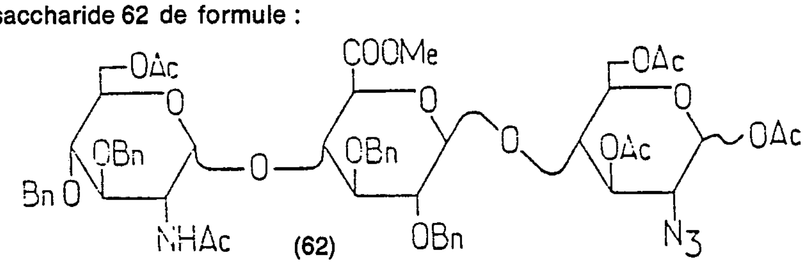

(62)

Ce trisaccharide 62 est préparé selon les étapes suivantes (voir figure 14) :

a) transformation du groupe $N_3$ en position 2 du motif glucosamine en groupe —NHAc.

b) ouverture du pont époxy 2,3 du motif à l'extrémité réductrice.

c) ouverture du pont anhydro-1,6 de ce même motif.

a) Passage de —$N_3$ à —NHAc :

A une solution du dérivé 59 (10 mg) dans un mélange DMF/éthanol (1/1 ; 1 ml), on ajoute du catalyseur Pd/$CaCO_3$ à 5 % (5 mg). La suspension est agitée sous une pression d'hydrogène de 1 atmosphère pendant 96 heures.

Après filtration du catalyseur et évaporation, le résidu est dissous dans du méthanol puis acétylé par addition d'une goutte d'anhydride acétique. On obtient quantitativement le dérivé 60.

Le dérivé 60 est caractérisé par son spectre R.M.N., son analyse élémentaire, son pouvoir rotatoire : $[\alpha]_D^{20}$ = + 35,5° ; chloroforme. P.F. : 147-149 °C.

b) Ouverture du pont époxy :

Le dérivé est tout d'abord saponifié comme indiqué pour la synthèse du dérivé 58, et ce afin d'éliminer le groupement acyle en position 6 du motif intermédiaire non réducteur et le groupement méthyl-ester en position du motif intermédiaire.

Après extraction, le résidu est dissous dans du DMF et chauffé à 120 °C, en présence d'azide de sodium, pendant 48 heures. Après évaporation, extraction par le chloroforme, lavage par HCl, 0,1 N, par l'eau, séchage et évaporation du solvant, on obtient un résidu qui est traité par du diazométhane, puis acétylé (pyridine/anhydride acétique), donnant ainsi le composé 61.

c) Ouverture du pont anhydro :

Le composé 61 est acétolysé dans les conditions habituelles (anhydride acétique, acide sulfurique) à — 20 °C. Après traitement du mélange réactionnel, on obtient le dérivé 62.

## Exemple 15

Synthèse du dérivé 63 de formule :

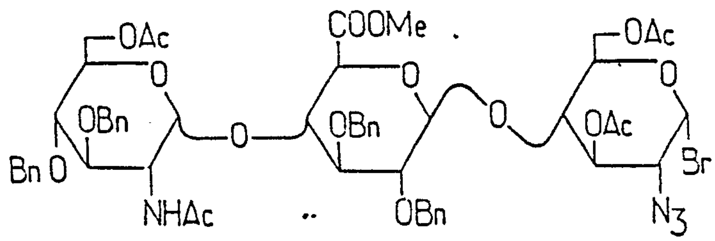

(63)

Le traitement du dérivé 62 obtenu dans l'exemple 14 par le tétrabromure de titane dans une solution de dichlorométhane et d'acétate d'éthyle conduit à l'halogène 63 dont la structure est confirmée par son spectre R.M.N. Son analyse élémentaire est correcte (voir figure 14).

## Exemple 16

Synthèse du monosaccharide 68 ou méthyl 2-acétamido-3-6-di-O-benzyl-2-désoxy-α-D-glucopyranoside de formule :

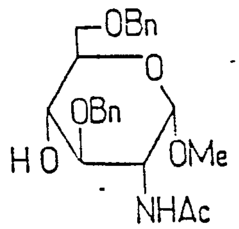

(68)

Cette synthèse est effectuée selon les 4 étapes suivantes à partir du monosaccharide 64 préparé selon la technique de A. Neuberger, Journal of the Chemical Society 1941, pages 50-51 :

1. benzylation du groupe —OH en position 3,
2. élimination du radical benzylidène aux fins de libération des groupes —OH en positions 4 et 6,
3. tosylation du groupe —OH en position 6,

4. déplacement du groupe —OTs en position 6 par un benzylate (voir figure 15).

Etape 1 : réaction de benzylation

A une solution du composé 64 (6,5 g, 20,10 mmol) dans le diméthylformamide (120 ml), on ajoute de l'hydroxyde de baryum octa-hydrate (3,6 g) et de l'oxyde de baryum (16 g). Après 10 minutes d'agitation à température ambiante, du bromure de benzyle (4,5 ml) est ajouté goutte à goutte. La réaction se poursuit pendant toute une nuit. Après dilution par du chloroforme (100 ml) le mélange réactionnel est filtré sur Célite®. Le filtrat est concentré à sec, on obtient ainsi un résidu blanc dont l'analyse en chromatographie sur couche mince indique qu'il contient un seul produit, à savoir le dérivé 65, qui sera engagé tel quel, dans l'étape suivante.

Etape 2 : élimination du groupe benzylidène

Le résidu obtenu ci-dessus est dissous dans un mélange de méthanol (370 ml) et d'eau (130 ml). A cette solution, on ajoute de l'acide paratoluène sulfonique monohydrate (3 g), puis on porte le mélange à reflux pendant une heure. Après refroidissement, la majeure partie du méthanol est évaporée, puis de l'eau (250 ml) est ajoutée. Après lavage par une faible quantité de chloroforme (100 ml), la phase aqueuse est soumise au traitement suivant :

1. précipitation des sels de baryum avec de l'acide sulfurique ;
2. filtration du sulfate de baryum formé ;
3. élimination de l'excès d'acide à l'aide d'une résine IRA 45 ($OH^-$).

Après élimination de la résine et concentration, on obtient un résidu légèrement jaune (5,7 g), à savoir le dérivé 66. Ce dérivé est engagé tel quel dans la préparation du composé 67.

Etape 3 : réaction de tosylation

Ce dérivé 66 est dissous dans un mélange de dichlorométhane (150 ml) et de DMF (10 ml). A cette solution, on ajoute du chlorure de tosyle (5,6 g, 30 mM), puis de la diméthylaminopyridine (121 mg) et enfin de la triéthylamine (5 ml). La réaction évolue à l'abri de l'humidité et sous courant d'azote sec.

Après 18 heures de réaction, on ajoute de l'eau glacée puis on abandonne le mélange sous agitation pendant 14 heures environ.

Le mélange réactionnel est ensuite dilué avec du dichlorométhane puis la phase dichlorométhane est lavée successivement avec de l'acide chlorhydrique 2 M, du bicarbonate de sodium saturé, puis avec de l'eau jusqu'à pH neutre. Après séchage sur sulfate de sodium et filtration, le solvant est évaporé. Le résidu obtenu est purifié sur une colonne de gel de silice (200 g) dilué avec un mélange acétate d'éthyle-hexane (4/1, v/v).

Les fractions contenant le dérivé 67 pur sont regroupées.

Après élimination des solvants, on obtient un résidu solide (4,6 g) qui est engagé directement dans la synthèse du composé 68.

Etape 4 : réaction de benzylation

Le dérivé 67 obtenu ci-dessus est dissous dans du diméthylformamide anhydre (50 ml). A cette solution, on ajoute une solution molaire de benzylate de sodium dans l'alcool benzylique (30 ml). Le mélange est ensuite chauffé à 90 °C pendant une heure. Après refroidissement à température ambiante, le mélange est ensuite concentré à sec. Il est ensuite repris par du chloroforme (400 ml), la phase chloroformique est lavée avec de l'eau, du chlorure de sodium saturé, séchée, puis concentrée à sec.

Le résidu est chromatographié sur une colonne de gel de silice (200 g, chloroforme/acétate d'éthyle, 1/1, v/v).

On obtient ainsi le dérivé 68 (2,3 g). Le rendement par rapport au composé 64 est de 27,6 %.

Le composé 68 est cristallin, P.F. 149-150 °C, $(\alpha)_{20}{}^{D} = 87°$ (c = 1, chloroforme). L'analyse du spectre infrarouge et l'analyse élémentaire confirment la structure attendue pour le produit 68.

## Exemple 17

Synthèse du disaccharide 73 (voir figure 15).

Cette synthèse comporte :

(1) La condensation des dérivés 68 et 69 conduisant au disaccharide 70.

(2) L'élimination des groupes benzyle conduisant au dérivé 71.

(3) La sulfatation des groupes —OH du dérivé 71, conduisant au dérivé 72, suivie de la salification des groupes anioniques et de l'élimination des groupes acétyle.

1. Synthèse du disaccharide 70

Cette synthèse est effectuée à partir des monosaccharides 68 et 69.

L'halogénure est préparé selon la technique de G.N. Bollenback et al., Journal of the American Chemical Society, 77 (1955), p. 3312.

A une solution de monosaccharide 68 (450 mg, 1,1 mmol), dans le dichloroéthane (30 ml), on ajoute du bromure mercurique (400 mg, 1,1 mmol). Après distillation d'environ 10 ml de dichloroéthane, on ajoute au mélange réactionnel des tamis moléculaires en poudre 0,4 nm (4 Å).

L'halogénure 69 (1,1 g, 2,75 mmol) dans du dichloroéthane (10 ml) est alors ajouté. Après distillation de 10 ml de dichloroéthane, le mélange réactionnel est abandonné à reflux pendant environ 14 heures à une température de 90-100 °C. Après refroidissement, le mélange réactionnel est dilué par du dichlorométhane (100 ml), puis les solides sont éliminés par filtration sur filtres plissés. La phase organique est lavée avec une solution de iodure de potassium à 10 % (2 × 25 ml), puis avec une solution de bicarbonate de sodium à 5 % (2 × 25 ml) et enfin avec de l'eau jusqu'à pH neutre. Après séchage sur sulfate de sodium, filtration et concentration, le résidu est purifié sur une colonne de gel de silice (150 g), dilué, successivement, avec trois mélanges acétone-éther (1/5 puis 1/4, puis 1/2, v/v).

On obtient ainsi le disaccharide 70 pur (390 mg) sous forme de cristaux. P.F. = 189-190 °C ; $(\alpha)_{20}^{D}$ = + 60° (c = 0,4 chloroforme). Le spectre infrarouge, de même que le spectre R.M.N. et l'analyse élémentaire, confirment la structure attendue.

2. Synthèse du disaccharide 71

A une solution du dérivé 70 (100 mg) dans le méthanol (20 ml), on ajoute du catalyseur (Pd/C, 5 %, 100 mg) et on agite la suspension ainsi obtenue sous courant d'hydrogène pendant 3 jours.

Le catalyseur est ensuite éliminé par filtration. Après évaporation, on obtient un résidu (73 mg, 97 %) constitué par le disaccharide 71. Le spectre de R.M.N. confirme la structure attendue pour ce composé.

On notera que le disaccharide 71 est le précurseur du motif de base de l'héparane-sulfate. Il suffit pour le déprotéger de le soumettre à une réaction de saponification, comme rapportée ci-après pour l'obtention du dérivé 73 à partir du dérivé 72.

3. Synthèse du disaccharide 73

A une solution du composé 71 (70 mg) dans le diméthylformamide (2 ml), on ajoute l'agent de sulfatation (complexe triméthylamine-trioxyde de soufre) (75 mg). Après une nuit, on procède à une nouvelle addition de complexe (35 mg). Après 6 heures, la réaction est terminée, le mélange est évaporé à sec, repris par du chloroforme, neutralisé avec de la triéthylamine et évaporé.

Une chromatographie sur colonne de gel de silice (20 g, méthanol/chloroforme, 1/2, v/v) permet d'isoler le dérivé 72 sulfaté pur qui se présente sous forme de poudre blanche. Ce dérivé est engagé directement dans la synthèse du disaccharide déprotégé 73.

A une solution du dérivé 72 (71 mg) dans le méthanol (9 ml), on ajoute de l'eau (4 ml) puis, goutte à goutte, une solution de soude 1 M (1 ml). Après 4 heures d'agitation à température ambiante, le mélange réactionnel est passé sur une colonne d'Amberlite® I.R. 120 $H^+$. La solution ainsi obtenue est neutralisée puis les sels sont éliminés par passage sur une colonne de Sephadex® G25 diluée avec de l'eau. Les fractions contenant le disaccharide sulfaté sont regroupées.

Après lyophilisation, on obtient le dérivé 73 sous forme d'une poudre blanche (46 mg) $(\alpha)_{20}^{D}$ = 34,5° (c = 1, eau).

L'analyse conductimétrique indique pour ce dérivé un rapport sulfate/carboxyle égal à 2. L'analyse élémentaire, de même que l'analyse en R.M.N. du carbone 13, confirment la structure attendue pour ce produit.

## Exemple 18
### Synthèse des composés 75, 76 et 77 à structure D-glucosamine (voir figure 16)

Composé 75 : Méthyl 3-O-benzyl-4,6-O-benzylidène-2-benzyloxy-carbonylamino-2-désoxy-α-D-glucopyranoside.

Une solution de composé 74 (préparé selon ZU YONG KYI, Sci. Sinica (Peking), 5 (1956) 461-467, CA 52 (1958) 3 694) (415 mg, 1 mmol) dans le N, N-diméthylformamide anhydre (10 ml) est agitée à la température ambiante à l'abri de l'humidité pendant 5 h en présence de baryte anhydre (613 mg), d'hydroxyde de barium octahydraté (158 mg) et de bromure de benzyle (0,15 ml). Le mélange réactionnel est alors dilué avec du chloroforme (50 ml), la phase organique est lavée avec de l'acide acétique à 50 % glacé, avec de l'eau, séchée (sulfate de sodium), filtrée et évaporée. Le résidu solide est recristallisé dans l'éthanol (461 mg, 91 %) ; P.F. : 202-203 °C ; $[\alpha]_D$ = + 46° (c : 1, chloroforme).

Composé 76 : Méthyl-3-O-benzyl-2-benzyloxycarbonylamino-2-désoxy-α-D-glucopyranoside.

Une suspension du composé 75 (300 mg) dans de l'acide acétique à 60 % (10 ml) est agitée à 100 °C pendant 30 min. La solution est alors refroidie, évaporée à sec, évaporée avec de l'eau (4 × 10 ml), le résidu solide est séché sous vide et recristallisé dans le 2-propanol pour donner le composé 76 (220 mg, 89 %), P.F. : 151-152 °C, $[\alpha]_D$ = + 94° (c : 1, méthanol).

Composé 77 : Méthyl 6-O-benzoyl-3-O-benzyl-2-benzyloxycarbo-carbonylamino-2-désoxy-α-D-glucopyranoside.

Une solution du composé 76 (835 mg, 2 mmol) dans un mélange de pyridine anhydre (5 ml) et de dichlorométhane (12 ml) est agitée à la température ambiante à l'abri de l'humidité en présence de cyanure de benzoyle (400 mg, 3 mmol) pendant 5 h. L'excès de réactif est alors détruit par addition de méthanol (5 ml) et agitation pendant 30 min. Le mélange réactionnel est évaporé à sec, évaporé avec du toluène et séché sous vide. Le résidu solide est recristallisé dans un mélange acétate d'éthyle-hexane pour donner le composé 77 (935 mg, 90 %) P.F. : 154-155 °C, $[\alpha]_D$ = + 74° (c : 1, chloroforme).

Exemple 19
Synthèse des composés 78 et 79 à structure
acide L-iduronique (voir figure 16)

Composé 78 : 4-O-acétyl-3-O-benzyl-1,2-O-méthoxyéthylidène-β-L-méthyl idopyranuronate.

Une solution du bromure 36 obtenu selon l'exemple 5, dans l'étape B (fraîchement préparé à partir de 0,425 g, 1 mmol, de mélange d'acétates 34 et 35) dans du dichlorométhane anhydre (10 ml) est agitée à la température ambiante sous atmosphère d'argon sec. De la sym-collidine (0,66 ml, 5 μmol) et du méthanol anhydre (0,40 ml, 10 mmol) sont successivement ajoutés, et le mélange réactionnel est agité 20 h dans ces conditions. Après dilution avec du dichlorométhane (50 ml), la phase organique est lavée avec une solution saturée aqueuse d'hydrogénocarbonate de sodium, avec de l'eau, séchée (sulfate de sodium), filtrée et évaporée. Le résidu est chromatographié sur une colonne de gel de silice (20 g). L'élution par le mélange hexane : acétate d'éthyle (3 : 2, v/v, contenant 0,5 % de triéthylamine) donne le composé 78 sous forme d'un sirop pur (302 mg, 76 % à partir des acétates 34 et 35), $[\alpha]_D$ = — 21° (c : 1, chloroforme), R.M.N. ($CDCl_3$ : δ : 5,52 (d, 1 H, H-1, $J_{1,2}$ : 3 Hz).

Composé 79 : 3-O-benzyl-1,2-O-tert-butoxyéthylidène-β-L-méthyl idopyranuronate.

Une solution de l'orthoester 37 obtenu dans l'exemple 5 dans l'étape γ (484 mg, 1,1 mmol) dans le méthanol anhydre (15 ml) est refroidie à — 20 °C sous agitation et atmosphère d'argon sec. Du carbonate de potassium anhydre (60 mg) est ajouté, et le mélange réactionnel est agité 5 h dans ces conditions. Les solides sont essorés, le filtrat est évaporé et le résidu est repris dans du chloroforme (50 ml). La phase organique est lavée avec de l'eau glacée (3 fois) séchée (sulfate de sodium), filtrée et évaporée. Le résidu est chromatographié rapidement sur une colonne de gel de silice (25 g). L'élution par le mélange hexane : acétate d'éthyle (2 : 1, v/v, contenant 0,5 % de triéthylamine), donne, par ordre d'élution :

— le composé insaturé 39 (31 mg, 7 %) sirop, $[\alpha]_D$ = + 103° (c : 1, chloroforme), R.M.N. ($CDCl_3$) : δ : 6,27 (doublet dédoublé, 1 H, H-4, $J_{3,4}$ : 5 Hz, $J_{2,4}$ : 1 Hz), 5,67 (d, 1 H, H-1, $J_{1,2}$ : 4 Hz).

— une fraction principale (271 mg, 62 %), qui est cristallisée dans un mélange éther-hexane pour donner le composé 79 (123 mg, 28 %), P.F. : 68-69 °C ; $[\alpha]_D$ = — 19° (c : 1, chloroforme), R.M.N. ($CDCl_3$) : δ : 5,41 (d, 1 H, H-1, $J_{1,2}$ : 2 Hz), 2,85 (d, 1 H, CH-4, J : 12 Hz, échangé avec $D_2O$).

Au cours de la chromatographie sur silice, et lors des essais de cristallisation de 79, un composé nouveau de Rf légèrement supérieur à celui de 79 apparaît. Une chromatographie sur gel de silice des eaux-mères de cristallisation de 79 permet d'isoler quelques fractions pures de ce nouveau composé 80 (41 mg, 11 %), sirop, $[\alpha]_D$ = + 21° (c : 1, chloroforme), R.M.N. ($CDCl_3$) : δ : 5,83 (d, 1 H, H-1, $J_{1,2}$ : 4,5 Hz).

Dans le cadre de la succession des synthèses envisagées selon l'invention, afin d'éviter la formation de 80, le sirop brut de 37 n'est pas chromatographié, mais utilisé immédiatement pour la réaction suivante.

Exemple 20
Préparation des disaccharides 81,
82 et 83 (figure 17)

Composé 81 : Méthyl-6-O-benzoyl-3-O-benzyl-2-benzyloxycarbonilamino-2-désoxy-4-O-(2,4-di-O-acétyl-3-O-benzyl-α-L-méthyl idopyranuronyl)-α-D-glucopyranoside.

Une solution de l'orthoester 78 (80 mg, 0,2 mmol) obtenu dans l'exemple 19 et de l'alcool 77 (52 mg, 0,1 mM) obtenu dans l'exemple 18 dans le chlorobenzène anhydre (8 ml) est chauffée à 140 °C sous agitation et léger courant d'argon sec. Après distillation lente de 6 ml de solvant, une solution de perchlorate de 2,6 diméthylpyridinium (0,002 mmol) fraîchement préparé selon N.K. KOCHETKOV, A.F. BOCHKOV, T.A. SOKOLOVSKAIA et V.J. SNIATKOVA, Carbonhdr. Res., 16 (1971) 17-27, dans le chlorobenzène (2 ml) est ajoutée goutte à goutte en 15 min. avec distillation simultanée de solvant (2 ml). Le mélange réactionnel est alors agité pendant 1 h, dans ces conditions, avec addition de solvant frais (10 ml) et distillation simultanée de telle sorte que le volume réactionnel reste constant et égal à 2 ml. Après refroidissement et dilution avec du chloroforme, la phase organique est lavée avec une solution

saturée d'hydrogénocarbonate de sodium, avec de l'eau, séchée (sulfate de sodium), filtrée et évaporée. Le résidu est chromatographié sur une colonne de gel de silice (15 g). L'élution par le mélange hexane : acétate d'éthyle (4 : 3, v/v) donne, par ordre d'élution :

— le produit de départ 77, (20 mg, 38 %),

— une fraction homogène en chromatographie sur couche mince (54 mg). Le spectre de R.M.N. de cette fraction montre la présence de plusieurs signaux O-méthyl (δ : 3,35-3,50) dus aux méthyl glycosides provenant du réarrangement de l'orthoester 78. Cette fraction est cristallisée dans un mélange éthanol-eau, et recristallisée dans un mélange acétate d'éthyle-hexane pour donner 81 (44 mg, 50 %), P.F. : 120-121 °C, $[\alpha]_D$ = + 17° (c : 1, chloroforme), R.M.N. ($CDCl_3$) : conforme à la structure attendue.

Composé 81 : Méthyl-6-O-benzoyl-3-O-benzyl-2-benzyloxycarbonylamino-2-désoxy-4-O-(2-O-acétyl-3-O-benzyl-4-O-chloroacétyl-α-L-méthyl idopyranuronyl)-α-D-glucopyranoside.

Une solution de l'orthoester 38 (120 mg, 0,25 mmol) obtenu dans l'exemple 5 et de l'alcool 77 (66 mg, 0,125 mmol) dans du chlorobenzène anhydre (8 ml) est chauffée à 140 °C sous agitation et léger courant d'argon sec. Après distillation lente de 6 ml de solvant, une solution de perchlorate de 2,6-diméthylpyridinium (0,0025 mmol) dans du chlorobenzène est ajoutée goutte à goutte en 15 min, avec distillation simultanée de solvant (2 ml). Le mélange réactionnel est agité pendant 1 h puis traité dans les conditions décrites pour la préparation de 81. Le résidu est chromatographié sur une colonne de gel de silice (15 g). L'élution par le mélange hexane : acétate d'éthyle (7 : 4, v/v) donne, par ordre d'élution :

— le produit 77, (40 mg, 60 %),

— le disaccharide 82, cristallisé dans un mélange éther-hexane, (36 mg, 30 %), P.F. : 143-144 °C, $[\alpha]_D$ = + (c : 1, chloroforme), R.M.N. ($CDCl_3$) : conforme à la structure attendue.

Composé 83 : O-déchloroacétylation et acétylation du disaccharide 82.

Un mélange du disaccharide 82 (12 mg) et de thiourée (5 mg) dans de la pyridine (1,2 ml) et de l'éthanol absolu (0,3 ml) est agité à 100 °C pendant 30 min. Après refroidissement, le mélange réactionnel est évaporé à sec et le résidu est repris par un mélange eau-chloroforme (1 : 1, v/v, 20 ml). La phase organique est lavée avec de l'eau, séchée (sulfate de sodium), filtrée et évaporée. Le résidu est lavé sur une colonne de gel de silice (1 g). L'élution par le mélange acétate d'éthyle : hexane (1 : 1, v/v) donne le disaccharide 83 (8 mg) sous forme d'un sirop pur qui n'a pas été analysé, mais immédiatement acétylé (pyridine : anhydride acétique, 2 : 1, v/v, 1,5 ml). Après 15 h à la température ambiante, le mélange réactionnel est évaporé à sec et le résidu est appliqué sur une colonne de gel de silice (0,5 g). L'élution par le mélange acétate d'éthyle : hexane (1 : 1, v/v) donne le disaccharide 81 (7 mg), cristallisé dans un mélange éther-hexane, P.F. : 120-120,5 °C, P.F. de mélange avec 81 : 120-121 °C.

## Exemple 21

### Synthèse du trisaccharide 85 (voir figure 18)

Une solution du bromure 84 (préparé selon H. Paulsen et W. Stenzel, Chem. Ber 111 (1978) 2334-2347, 110 mg, 0,25 mmol) et de l'alcool 41 (préparé selon l'exemple 6, 113 mg, 0,13 mmol) dans du dichlorométhane anhydre (2,5 ml) est agitée à l'abri de la lumière sous atmosphère d'argon sec en présence de tamis moléculaire 0,4 nm (Å) (poudre, 100 mg) pendant 30 min. Après refroidissement à 20 °C, de la symcollidine (70 µl, 0,55 mmol) et du triflate d'argent (78 mg, 0,30 mmol) sont ajoutés successivement et l'agitation est maintenue dans ces conditions pendant 2 heures. Le mélange réactionnel est alors dilué avec du dichlorométhane (50 ml), les solides sont essorés, et le filtrat est lavé avec une solution 0,1 M d'acide chlorhydrique glacée, avec de l'eau, avec une solution aqueuse saturée d'hydrogénocarbonate de sodium, avec de l'eau, séché (sulfate de sodium), filtré et évaporé.

Le résidu est chromatographié sur une colonne de gel de silice (18 g). L'élution par le mélange hexane : acétate d'éthyle (4 : 3, v/v) donne le trisaccharide 85 sous forme d'un verre incolore qu'il n'a pas été possible de cristalliser (139 mg, 88 %) ; $[\alpha]_D$ = + 83° (cl, chloroforme) ;

Spectre R.M.N. (90 MHz, $CDCl_3$ : δ : 7,25 (m, 25 H, 5 Ph.) ;
5,44 (d. de d., 1 H, $H_{3''}$, $J_{2'',3''}$ : 10,5 Hz, $J_{3'',4''}$ : 9 Hz) ;
5,26 (d, 1 H, $H_{1''}$, $J_{1'',2''}$ : 3,5 Hz) ; 3,59 (s, 3 H, COOMe) ;
3,06 (d. de d., 1 H, $H''$, $J_{1'',2''}$ : 3,5 Hz, $J_{2'',3''}$ : 10,5 Hz) ;
2,12 2,08, 2,01 et 12,97 (4s, 12 H, 4 OAc).

## Exemple 22

### Synthèse du trisaccharide 89 (voir figure 18)

Selon les quatre étapes suivantes :

a) élimination des groupes acétyle,

b) sulfatation,

c) hydrogénation,
d) sulfatation des fonctions amino,

a) Elimination des groupes acétyle conduisant au trisaccharide 89 :

Une solution de trisaccharides 85 (122 mg) dans un mélange de 1,2-diméthoxyéthane (6 ml) et de méthanol (2 ml) est agitée à 0 °C. Une solution aqueuse 1 M de soude (2 ml) est ajoutée goutte à goutte en 10 min, et le mélange de réaction est agité 6 heures à 0 °C. De l'acide chlorhydrique 1 M est alors ajouté goutte à goutte jusqu'à pH = 0 (apparition d'un précipité blanc). Le mélange est versé dans de l'eau glacée (100 ml) et extrait avec du chloroforme (5 fois 10 ml). Les phases organiques sont lavées avec de l'eau glacée, séchées (sulfate de sodium), filtrées et évaporées. Le résidu sirupeux est dissous dans du méthanol (2 ml) et traité par une solution éthérée de diazométhane jusqu'à persistance de la coloration jaune. Après 30 min, le mélange réactionnel est évaporé à sec. Le résidu est chromatographié sur une colonne de gel de silice (10 g). L'élution par le mélange acétate d'éthyle/hexane (2 : 1, v/v) donne le trisaccharide 86 sous forme d'une mousse incolore qu'il n'a pas été possible de cristalliser (85 mg, 81 %) ; $[\alpha]_D$ = + 77° (cl, chloroforme), spectre RMN (90 MHz, $CDCl_3$) : absence de signaux OAc (vers $\delta$ = 2 ppm).
Analyse élémentaire : en accord avec la structure recherchée.

b) Sulfatation conduisant au trisaccharide 87.

A une solution du dérivé 86 (41 mg) dans le DMF (2 ml) on ajoute du complexe triméthylamine/trioxyde de soufre ($TMA/SO_3$ ; 60 mg ; 2,5 équivalents par OH).

Après une nuit à 50 °C, la réaction est complète. Du méthanol (0,5 ml) est ajouté puis la solution est déposée sur une colonne de Séphadex® LH-20 (1,5 × 25 cm) équilibrée dans un mélange chloroforme/méthanol (1 : 1 ; v/v). L'élution par le même mélange permet de séparer le produit de la réaction de l'excès de réactif et du solvant de réaction. Le résidu obtenu est chromatographié sur une colonne de gel de silice (10 g), élué par un mélange acétate d'éthyle/pyridine/acide acétique/eau (98 : 56 : 13 : 32 ; v/v/v/v). Le produit pur obtenu est dissous dans du méthanol, puis passé à travers une colonne de résine Dowex® 50 W × 4, $Na^+$ (5 ml). Après évaporation et séchage, on obtient le dérivé 87 (58 mg, 100 %). Il est homogène en c.c.m. (acétate d'éthyle/pyridine/acide acétique/eau ; 5 : 5 : 1 : 3 ; v/v/v/v et acétate d'éthyle/méthanol/acide acétique ; 7 : 3 : 0,1 : v/v/v).

$[\alpha]_D^{20}$ = + 55° (méthanol). Le spectre de RMN est compatible avec la structure recherchée.

c) Hydrogénation conduisant au trisaccharide 88.

Une solution du composé 87 (20 mg) dans un mélange de méthanol (2 ml) et d'eau (0,5 ml) est agitée pendant 96 heures sous une pression d'hydrogène de 0,2 bar, en présence de Pd/C à 5 % (20 mg). Le catalyseur est alors éliminé par filtration. L'analyse en ultraviolet confirme l'absence de noyaux aromatiques. Après évaporation, le produit est engagé dans la synthèse du trisaccharide 89.

d) Sulfatation conduisant au trisaccharide 89.

Le dérivé 88 obtenu précédemment est dissous dans l'eau (2 ml). Le pH de la solution est ajusté à 9,5 puis il est maintenu à cette valeur au moyen d'un pH-stat. On ajoute alors le complexe $TMA/SO_3$ (14 mg ; 5 éq./$NH_2$). Après une nuit, la même quantité de complexe est ajoutée. Après 48 heures, le pH est amené à 12 au moyen de soude 2M, puis il est maintenu à cette valeur pendant 2 heures. Après neutralisation par l'acide chlorhydrique, le mélange réactionnel est chromatographié sur une colonne de Séphadex® G-25, éluée avec de l'eau. Le composé 89 est détecté par une réaction colorée au carbazole, caractéristique des acides uroniques (Bitter et Muir, Anal. Biochem. 4 (1962) 330-334). Ces fractions contenant 89 sont regroupées et passées au travers d'une colonne de résine Dowex® 50 W × 4, $Na^+$ éluée avec de l'eau. Après lyophilisation, on obtient 89 (4,5 mg).

L'analyse colorimétrique des constituants glucidiques donne 2,55 moles d'acide uronique pour 5,15 moles de glucosamine (rapport 1/2).

Le spectre de RMN de ce produit confirme la structure (séquence anomérie des liaisons, substitutions par les sulfates).

(Voir exemple page 40)

Exemple 23
Synthèse du disaccharide 92 de formule :

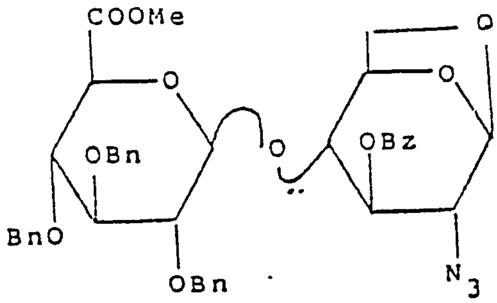

(92)

On se reportera à la figure 19.

Le produit 91 (1 g), en solution dans du dichlorométhane (50 ml), est agité en présence de driérite (6 g) et de carbonate d'argent fraîchement préparé (4,5 g), pendant 1 heure sous une atmosphère d'argon. On ajoute alors l'halogénure 90 (2,8 g) dissous dans du dichlorométhane (10 ml). Après 1 heure 1/2 on ajoute à nouveau 2,8 g d'halogénure 90. Après une nuit les solides sont éliminés par filtration et le résidu obtenu après évaporation des solvants est purifié sur colonne de silice dans le solvant acétate d'éthyle/chloroforme (1/30 ; v/v).

On obtient ainsi le produit 92 (866 mg ; rendement 42 %). Il est cristallisé dans un mélange hexane/acétate d'éthyle.

P.F. : 104-106 °C ; $[\alpha]_D^{20}$ = 0° (c = 1 ; chloroforme).

L'analyse élémentaire et le spectre RMN sont conformes à la structure recherchée.

Exemple 24
Synthèse du dérivé 94 de formule :

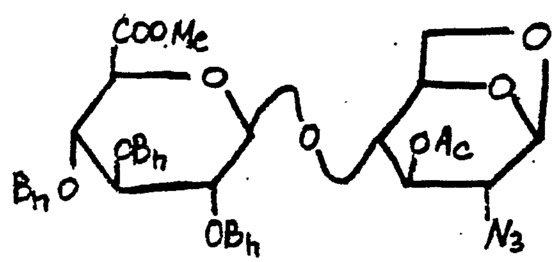

On se reportera aux figures 19 et 20.

Le dérivé 92 (1,5 g) est dissous dans un mélange de chloroforme et de méthanol (1/1 ; v/v). On ajoute ensuite 2 ml de méthanolate de sodium (2M dans méthanol). Après 20 minutes, la solution est neutralisée par addition de résine Dowex® 50 conduisant au dérivé 93 qui n'est pas isolé. Après filtration et évaporation, une méthylation classique par le diazométhane dans l'éther permet de réestérifier la fraction d'acide carboxylique éventuellement libéré. Après évaporation le résidu est traité par un mélange de pyridine (20 ml) et d'anhydride acétique (2 ml) pendant une nuit. Après évaporation, le résidu est cristallisé dans de l'acétate d'éthyle/hexane donnant le produit 94 (1,125 g ; rendement 81,6 %).

P.F. : 103-105 °C ; $[\alpha]_D^{20}$ = + 5,2° (c = 1 ; chloroforme).

L'analyse élémentaire et le spectre RMN sont conformes à la structure recherchée.

En variante, on prépare le dérivé 94 en opérant comme décrit ci-dessus mais en mettant en œuvre le dérivé 95 au lieu du dérivé 91.

Exemple 25
Synthèse du dérivé 97 (voir figure 20).

Dans une première étape, on procède à une ouverture du pont anhydre, puis dans l'étape suivante, on effectue une réaction de bromuration.

1. Ouverture du pont 1,6-anhydro.

Le composé 94 (1 g) est dissous dans l'anhydride acétique (10 ml), puis refroidi à — 20 °C sous argon. A la solution froide, on ajoute de l'acide sulfurique concentré (100 µl). Après 30 minutes, le mélange

réactionnel est dilué par du chloroforme (150 ml) puis versé sur une solution aqueuse de bicarbonate de sodium (26,5 g dans 400 ml). A la fin du dégagement gazeux la phase chloroformique est lavée deux fois avec une solution saturée de NaCl puis séchée et concentrée. Après chromatographie sur silice (50 g) dans un mélange d'acétate d'éthyle et de chloroforme 1/20 v/v, on obtient le composé 96 (995 mg ; rendement 86,7 %).

Ce composé se présente sous forme d'une mousse blanche.

Le spectre et l'analyse élémentaire confirment l'obtention de la structure recherchée.

2. Bromuration.

A du tétrabromure de titane (213 mg) on ajoute une solution du dérivé 96 (0,2 g) dans du dichlorométhane/acétate d'éthyle) (9/1 ; v/v 4 ml). Après une nuit sous agitation suivie d'une dilution par du dichlorométhane, on verse sur un mélange eau glace (50 ml), puis on lave avec deux fois 50 ml d'eau glacée. Après séchage et évaporation le sirop obtenu est chromatographié sur silice dans le solvant acétate d'éthyle/chloroforme 1/20 ; v/v. On obtient ainsi le dérivé 97 avec un rendement de 25 à 50 %.

Spectre de RMN : (ppm, $CDCl_3$) : 2,04 ; 2,11 : 2 singulets de 3 protons 2-OAc ; 3,7 : 1 singulet de 3 protons COOMe ; 6,33 : 1 doublet de 1 proton $H_1$ ; $J_{1,2}$ = 3,5 Hz.

## Exemple 26
## Synthèse du tétrasaccharide 98 (voir figure 20).

Une solution de bromure 97 (50 mg, 60 μmol) et de l'alcool 41 préparé selon l'exemple 6 (43 mg), 50 μmol) dans du dichlorométhane anhydre (1 ml) est agitée à l'abri de la lumière sous atmosphère d'argon sec en présence de tamis moléculaire (4 nm) (poudre, 100 mg) pendant 15 minutes. Après refroidissement à — 10 °C, de la sym-collidine (11 μl, 80 μmol) et du trifluorométhanesulfonate d'argent (triflate d'Ag, 18 mg, 70 μmol) sont ajoutés successivement, et l'agitation est maintenue dans ces conditions pendant 3 heures. Le mélange réactionnel est alors dilué avec du dichlorométhane (30 ml), les solides sont essorés, et le filtrat est lavé avec une solution 0,1 M d'acide chlorhydrique glacée, avec de l'eau, avec une solution aqueuse saturée d'hydrogénocarbonate de sodium, avec de l'eau, séché (sulfate de sodium), filtré et évaporé.

Le résidu est chromatographié sur une colonne de gel de silice (7 g). L'élution par le mélange hexane-acétate d'éthyle (4 : 3, v/v) donne 56 mg de tétrasaccharide 98 (rendement 70 %) sous forme d'un verre incolore qu'il n'a pas été possible de cristalliser.

Caractéristiques du spectre RMN :
(270 $MH_z$, $CDCl_3$) : S : 7,25 (m, 35 H, 7 Ph) ;
5,35 (d. de d., 1 H, $H_3''$ $J_{2'',3''}$ : $H_Z$, $J_{3'',4''}$ 9 Hz) ;
5,27 (d., 1 H, $H_1''$, $J_{1'',2''}$ : 3.5 H) ; 4.31 (d., 1 H, $H_1'''$ $J_{1'''2'''}$ : 7,5 $H_z$) ;
3,68 (s, 3 H, COOMe IDO) ; 3,59 (s, 3H, COOMe gluco) ;
3,37 (d. de d., 1H, $H_2'''$, $J_{1''',2'''}$ : 7,5 $H_z$, $J_{2'''\ 3'''}$ : 9.5 $H_z$)
3,18 (d. de d., 1 H, $H_2''$, $J_{1'',2''}$ : 3,5 $H_z$, $J_{2'',3''}$ : 11 Hz) ;
2,06 et 1,97 (2 s, 9 et 3 H, 4 OAc).
Ce spectre est reporté sur la figure 33.

## Exemple 27
## Synthèse du tétrasaccharide 99 (voir figure 21)

Une solution de tétrasaccharide 98 (28 mg) dans le méthanol anhydre (3 ml) est refroidie à — 15 °C sous atmosphère d'argon sec. Du carbonate de potassium anhydre (12 mg) est ajouté et le mélange est agité 6 heures dans ces conditions. Les solides sont alors essorés, le filtrat est évaporé et le résidu est repris avec du chloroforme (15 ml). La phase organique est lavée avec une solution aqueuse saturée de chlorure de sodium, avec de l'eau, séchée (sulfate de sodium), filtrée et évaporée.

Le résidu est chromatographié sur une colonne de gel de silice (2 g). L'élution par le mélange acétate d'éthyle/hexane (3 : 2, v/v) donne le trétrasaccharide 99 sous forme d'un verre incolore (22 mg, 85 %).

Spectre RMN (270 MHz, $CDCl_3$) : δ : 7,30 (m, 35 H, 7 Ph) ;
5,37 (d, 1H, $H_1''$, $J_{1'',\ 2''}$ : 3,5 Hz) ;
5,29 (d. de d., 1H, $H_3''$, $J_{2'',3''}$ : 10 Hz, $J_{3'',4''}$ : 8,5 Hz) ;
5,09 (d, 1H, $H_1$, $J_{1,2}$ : 3,5 Hz) ;
3,57 (s, 3 H, COOMe ido) ;
3,43 (s, 3H, COOMe gluco) ; 2,06 (s, 3H, OAc).

Ce composé 99, qui est un dérivé mono-O-acétylé (en position 3 sur le 2e motif) du tétrasaccharide 103 est un intermédiaire potentiel pour la synthèse d'un analogue de ce tétrasaccharide qui ne serait pas sulfaté sur la position 3 du deuxième motif.

Exemple 28

Synthèse du tétrasaccharide 103 (voir figure 21).

On a recours aux étapes a) à d) suivantes :

a) élimination des groupes acétyle,
b) sulfatation,
c) hydrogénation,
d) sulfatation des groupes amine.

a) Elimination des groupes acétyle conduisant au dérivé 103 :

Une solution du tétrasaccharide 98 (40 mg) dans un mélange de 1,2-diméthoxyéthane (3 ml) et de méthanol (1 ml) est refroidie à — 15 °C. Une solution aqueuse M de soude (1 ml) est ajoutée goutte à goutte en 10 mn et le mélange réactionnel est agité 5 heures à 0 °C. De l'acide chlorhydrique M est alors ajouté goutte à goutte jusqu'à pH = 0 et le mélange est versé dans de l'eau glacée (50 ml). Après extraction avec du chloroforme (5 fois 5 ml), les phases organiques sont lavées avec de l'eau, séchées (sulfate de sodium) filtrées et évaporées.

Le résidu est dissous dans du méthanol (1 ml) et traité par une solution éthérée de diazométhane jusqu'à persistance de la coloration jaune. Après 30 min, le mélange réactionnel est évaporé à sec. Le résidu est chromatographié sur une colonne de gel de silice (3 g). L'élution par le mélange acétate d'éthyle/hexane (2 : 1, v/v) donne le tétrasaccharide 100 (27 mg, 75 %) ; P.F. 126-127 °C (éthanol) ; $[\alpha]_D$ = + 55° (cl, chloroforme).

Spectre RMN (90 MHz, $CDCl_3$) : absence totale de signaux OAc (vers $\delta = 2$).

Analyse élémentaire : conforme avec la structure recherchée.

b) Sulfatation conduisant au dérivé 101.

A une solution du dérivé 100 (24 mg) dans le DMF (1 ml), on ajoute le complexe TMA/$SO_3$ (24 mg). Après une nuit à 50 °C, la réaction de sulfatation est complète.

Du méthanol (0,5 ml) est ajouté au mélange réactionnel puis celui-ci est déposé sur une colonne de Séphadex® LH-20 équilibrée en chloroforme méthanol (1 : 1, v/v). Les fractions contenant 101 sont regroupées. Après évaporation à sec, le résidu est chromatographié sur gel de silice (10 g) dans le mélange acétate d'éthyle/pyridine/acide acétique/eau (160 : 77 : 19 : 42 ; v/v/v/v). Les fractions pures sont regroupées. Après concentration à sec, le résidu est passé au travers d'une colonne Dowex® 50 W × 4, $Na^+$ éluée avec de l'eau. Le produit obtenu (30 mg) est homogène en chromatographie sur couche mince dans le solvant ci-dessus. Son spectre RMN confirme la structure. $[\alpha]_D^{20}$ = + 39° (1, méthanol).

c) Hydrogénation conduisant au dérivé 102.

Une solution du dérivé 101 (10 mg) dans un mélange de méthanol (1,8 ml) et d'eau (0,2 ml) est agitée sous une pression d'hydrogène de 0,2 bar en présence de Pd/C à 5 % (10 mg). Après 96 heures, le catalyseur est éliminé par filtration. L'analyse en ultraviolet confirme l'absence de noyaux aromatiques. Après évaporation, le dérivé 102 est utilisé tel quel pour la préparation du dérivé 103.

d) Sulfatation conduisant au dérivé 103.

Le dérivé 102 obtenu à l'étape précédente, est dissous dans l'eau (2 ml). Le pH de cette solution est ajustée à 9,5 ; il est maintenu à cette valeur pendant toute la durée de la sulfatation. Le complexe TMA/$SO_3$ (14 mg) est ajouté. Une deuxième addition est faite après 24 heures (14 mg). Après 48 heures, le pH est amené à 12, puis à 7, deux heures plus tard. Le mélange réactionnel est alors chromatographié sur une colonne de Séphadex® G-25 (50 ml). Les fractions contenant le dérivé 103 (détection par réaction colorée des acides uroniques) sont regroupées, passées au travers d'une colonne de résine Dowex® 50 W × 4 $Na^+$ puis lyophilisées.

On obtient ainsi le tétrasaccharide 103 (2 mg).

L'analyse colorimétrique des constituants 30 du dérivé 103 donne 1,84 moles de glucosamine pour 2,06 moles d'acides uroniques.

La structure du dérivé 103 (séquence, anomérie, position des groupes sulfates) est confirmée par le spectre RMN (270 MHz, TMS) : $\delta$ pour les protons anomères respectivement des 1^er^, 2^e^, 3^e^ et 4^e^ motifs, 4,72 ; 5,30 ; 5,55 ; et 5,67.

Exemple 29

Synthèse du monosaccharide 115 (voir figure 22).

Cette synthèse est effectuée selon les étapes 1 à 7 suivantes :

Etape 1 : synthèse du monosaccharide 105.

On prépare ce monosaccharide à partir du composé 104 obtenu selon la technique de N. L. Holder et B. Fraser-Reid, Canadian Journal of Chemistry, 51 (1973) page 3357. A une solution du composé 104 (1 g, 12,67 mmol) dans le dichlorométhane (20 ml), on ajoute du chlorure de tosyle (0,55 g), puis de la diméthylaminopyridine (16 mg) et enfin, de la triéthylamine (0,7 ml). Après agitation sous courant d'azote à l'abri de l'humidité, pendant environ 14 heures, la réaction est arrêtée par addition de glace et d'eau. Après dilution du mélange réactionnel avec du dichlorométhane (50 ml), la phase dichlorométhane est lavée avec de l'acide chlorhydrique 2 M, puis une solution saturée de bicarbonate de sodium, et enfin avec de l'eau jusqu'à pH neutre. Après séchage et évaporation, on obtient un résidu, à savoir le dérivé 105 (1,4 g, 97 %) qui est engagé tel quel dans la synthèse du dérivé 106.

Etape 2 : synthèse du dérivé 106.

Le monosaccharide 105 (31,8 g) et de l'iodure de sodium (39 g) sont dissous dans de l'acétonitrile (250 ml), puis la solution est portée à reflux pendant 3 heures. Après refroidissement du mélange réactionnel, le précipité blanc formé est filtré. Le filtrat est concentré, le résidu est repris par du chloroforme, puis la phase chloroformique est lavée avec de l'eau jusqu'à pH neutre, séchée sur sulfate de sodium et concentrée à sec. On obtient un sirop qui est chromatographié sur une colonne de gel de silice (200 g, éther-hexane, 1/1, v/v). On obtient ainsi le dérivé iodé 106 (24,7 g, 71,5 %). $[\alpha]_{20}^{D}$ = 24° (1, chloroforme).

Le spectre infrarouge, le spectre de RMN et l'analyse élémentaire confirment la structure de 106.

Etape 3 : synthèse du dérivé 107.

A une solution du dérivé 106 dans de la pyridine anhydre (200 ml), on ajoute de l'anhydride acétique (43 ml). Après environ 14 heures sous agitation, la réaction est terminée. Le mélange réactionnel est concentré à sec, puis le résidu est purifié sur une colonne de gel de silice, sous pression, dans un solvant acétate d'éthylehexane (1/6, v/v). Les fractions pures sont regroupées. On obtient ainsi le produit 107 (16,4 g, 70 %). Ce produit se présente sous forme d'un sirop. $[\alpha]_{20}^{D}$ = 4,5° (1,3, chloroforme). L'analyse élémentaire ainsi que l'analyse du spectre infrarouge confirment la structure.

Etape 4 : synthèse du dérivé 108.

A une solution du dérivé 107 (4 g) dans de la pyridine (100 ml), refroidie à 0 °C, on ajoute du fluorure d'argent (AgF, 6,9 g). Après deux heures et demie, le mélange réactionnel est versé dans un mélange contenant du chloroforme et de l'éther (1/4, v/v, 1 1). La suspension obtenue est passée au travers d'un filtre plissé. Le filtrat est concentré à sec. Puis le résidu est repris dans du chloroforme (500 ml). La phase chloroformique est lavée avec du sulfate acide de potassium en solution à 10 % dans l'eau, puis avec de l'eau jusqu'à pH neutre. Après séchage sur sulfate de sodium et concentration à sec, on obtient un résidu (2,7 g), qui est chromatographié sur une colonne de silice (200 g) (éluant : acétate d'éthyle-hexane, 1/4, v/v). Les fractions contenant le produit 108 sont regroupées et après évaporation des solvants, on obtient un produit cristallin (1,62 g, 54 %).

PF : 81-82 °C, $[\alpha]_{25}^{D}$ = — 20° (1, chloroforme).

L'analyse du spectre infrarouge, l'analyse élémentaire et l'analyse du spectre de résonance magnétique nucléaire confirment la structure du composé 108.

Etape 5 : synthèse du dérivé 109.

Le produit 108 (2 g) est dissous dans du méthanol (20 ml) et du chloroforme (20 ml). A cette solution, on ajoute du méthanolate de sodium (2M, 2 ml). Après 1,5 heure, la réaction de désacétylation est terminée. Le mélange réactionnel est dilué avec du chloroforme. La phase chloroformique est lavée avec de l'eau jusqu'à pH neutre, séchée, puis évaporée à sec. On obtient ainsi un résidu (1,8 g, 100 %). Il est immédiatement dissous dans du tétrahydrofuranne (50 ml), puis de l'hydrure de bore ($BH_3$, 1M) dans le tétrahydrofuranne ; (10 ml) est ensuite ajouté. Après une heure de réaction, l'excès d'hydrure de bore est détruit par addition d'éthanol. A la fin du dégagement gazeux, le mélange réactionnel est dilué par addition de tétrahydrofuranne (100 ml). De la soude 3 M (12 ml) est ensuite ajoutée, suivie d'eau oxygénée (120 volumes, 8 ml). Après 2 heures de chauffage à 50 °C, la réaction est arrêtée. La solution est versée dans du chloroforme (500 ml), puis la phase organique ainsi obtenue est lavée avec de l'eau, de l'acide chlorhydrique 2 M, puis enfin avec de l'eau jusqu'à pH neutre. On obtient ainsi une phase chloroformique très laiteuse, qui devient limpide au cours du séchage sur sulfate de sodium. Après filtration, le chloroforme est évaporé puis le résidu obtenu est chromatographié sur silice (200 g chloroforme méthanol, 30/1, v/v).

On obtient ainsi le dérivé de l'idose 109 (1,05 g, 55 %). Ce produit se présente sous forme d'un sirop. $[\alpha]_{20}^{D}$ = + 85,5° (1, chloroforme).

L'analyse élémentaire ainsi que l'analyse en RMN confirment la structure attendue.

Etape 6 : synthèse du dérivé 112.

Cette synthèse est effectuée à partir du dérivé 109 en une seule étape (les intermédiaires 110 et 111 ne sont pas isolés). A une solution du dérivé 109 (2,25 g, 6 mmol) dans le dichlorométhane (50 ml), on ajoute successivement de la diméthylaminopyridine (60 mg ; 0,24 mmol) de la triéthylamine (1,7 ml ; 12 mmol) et du chlorure de trityle (2,5 g ; 9 mmol). Après environ 14 heures, la réaction est terminée. On obtient ainsi en solution le dérivé 110 ajouté alors au mélange réactionnel de la diméthylaminopyridine (150 mg), de la triéthylamine (1,7 ml) et du chlorure de benzoyle (1,05 ml). Après 6 jours, le dichlorométhane est éliminé par passage d'un courant d'azote et remplacé par du diméthylformamide (40 ml). Le mélange réactionnel est chauffé à 70 °C pendant une nuit. On ajoute alors à nouveau du chlorure de benzoyle (1 ml) et de la triéthylamine (1,7 ml), puis on maintient le chauffage à 70 °C pendant 2 jours. Le diméthylformamide est ensuite évaporé, puis le résidu est repris par du chloroforme, la phase chloroformique est lavée avec de l'eau, avec une solution saturée de bicarbonate de sodium, puis avec une solution d'acide chlorhydrique 2 M et enfin avec de l'eau jusqu'à pH neutre. Après séchage, le chloroforme est évaporé, ce qui permet d'obtenir le composé 111.

Celui-ci est immédiatement soumis à une réaction pour éliminer le groupe trityle afin d'obtenir le dérivé 112. Le résidu contenant le dérivé 111 est dissous dans 25 ml de chloroforme et on ajoute à cette solution 10 ml d'une solution d'acide paratoluènesulfonique monohydrate dans le méthanol (1 M). Après 4 heures de réaction à température ambiante, la réaction est terminée. Le mélange réactionnel est alors dilué avec du chloroforme, lavé avec de l'eau, séché puis évaporé à sec. Le résidu obtenu est chromatographié sur gel de silice (200 g, éther-hexane, 3/1, v/v). Le dérivé 112 est ainsi obtenu à l'état pur (1,5 g ; 52 %). Ce dérivé se présente sous forme d'un sirop. $[\alpha]_{20}^{D}$ = — 8° (1, chloroforme).

L'analyse du spectre infrarouge et du spectre RMN confirment la structure du produit attendu.

Etape 7 : synthèse du composé 115.

Cette synthèse est effectuée directement à partir du dérivé 112 sans isoler les intermédiaires 113 et 114. A la solution du composé 112 (1,2 g) dans l'acétone (20 ml), on ajoute, goutte à goutte, après refroidissement à 0 °C, une solution (2,9 ml) d'oxyde de chrome ($CrO_3$ ; 1,17 g) dans l'acide sulfurique 3,5 M (5 ml). Après 30 minutes d'agitation à 0 °C, la température est ramenée à l'ambiante. La réaction évolue pendant 3 heures. Le mélange réactionnel est ensuite versé dans une ampoule à décanter contenant de l'eau glacée (100 ml). Le produit formé est extrait par du chloroforme (3 × 50 ml). La phase chloroformique est lavée avec de l'eau jusqu'à pH neutre, puis séchée sur sulfate de sodium, filtrée et concentrée à sec. Le résidu obtenu (le composé 113) est dissous dans du méthanol (130 ml). On ajoute à cette solution de la soude 3 M (17 ml) puis on laisse le mélange sous agitation pendant environ 14 heures. Après acidification par l'acide sulfurique, le composé 114 est extrait à l'éther, puis immédiatement méthylé par du diazométhane selon la méthode classique pour donner le composé 115.

Après évaporation de l'éther, le composé 115 est obtenu pur au moyen d'une chromatographie sur gel de silice (50 g ; éther-hexane ; 4/1 ; v/v). Les fractions pures contenant le dérivé 115 sont rassemblées et les solvants sont éliminés. On obtient ainsi le dérivé 115 de l'acide iduronique (587 mg, 59 % par rapport au dérivé 112). Ce produit se présente sous forme d'un sirop. $[\alpha]_{25}^{D}$ = + 98° (2,65, chloroforme).

L'analyse en RMN, l'analyse en infrarouge et l'analyse élémentaire confirment la structure attendue.

## Exemple 30

Synthèse du disaccharide 117 (voir figures 22 et 23).

Cette synthèse s'effectue à partir du monosaccharide 115 préparé comme ci-dessus et du monosaccharide 44 préparé selon la technique de H. Paulsen et W. Stenzel, Chemische Berichte 111 (1978) 2234-2247.

A une solution du composé 115 (200 mg, 0,5 mmol) dans le dichlorométhane (10 ml), on ajoute successivement le composé 44 (0,450 g) de la sym-collidine (150 µl) et du triflate d'argent (260 mg).

Le mélange réactionnel est maintenu à 0 °C sous courant d'azote et sous agitation à l'abri de l'humidité et de la lumière pendant 3 heures.

Il est ensuite dilué avec du dichlorométhane (100 ml) puis les solides sont éliminés par filtration sur filtres plissés. La solution obtenue est lavée avec une solution saturée de bicarbonate de sodium avec de l'eau et avec de l'acide sulfurique 2 M, puis à nouveau avec de l'eau jusqu'à pH neutre.

Après séchage sur sulfate de sodium et évaporation du dichlorométhane, le résidu obtenu est chromatographié sur gel de silice (50 g ; chloroforme/acétate d'éthyle ; 15/1 ; v/v).

On obtient ainsi le dérivé 117 pur (327 mg, 82 %). Le produit se présente sous forme d'un sirop. $[\alpha]_{20}^{D}$ = + 57° (1, chloroforme).

L'analyse en R.M.N. de même que l'analyse élémentaire confirment la structure et l'anomérie du disaccharide 117.

Exemple 31

Synthèse du disaccharide 122 (voir figure 23).

On met en œuvre les étapes suivantes de :
— élimination des groupes acétyle,
— sulfatation,
— hydrogénation,
— sulfatation du groupe amine primaire,
— élimination du groupe Me du radical —COOH conduisant au composé 118.

Le disaccharide 117 (260 mg) est dissous dans du méthanol (5 ml) et de la soude 1 M (1 ml) est ajoutée goutte à goutte. A la fin de la réaction, le mélange réactionnel est introduit au sommet d'une colonne de résine Dowex® 50 sous forme HT (5 ml). L'effluent est concentré à sec, repris par du méthanol, et le produit acide libre, obtenu à l'issue de la saponification du dérivé 117 est méthylé par addition de diazométhane. On obtient ainsi le dérivé 118 qui est purifié au moyen d'une colonne de gel de silice (20 g ; éther/hexane ; 8/1 ; v/v). Le rendement en composé 118 est de 92 mg. Ce produit est engagé directement dans la synthèse du dérivé 119.

— Sulfatation conduisant au disaccharide 119.

Le produit 118 obtenu ci-dessus (92 mg) est dissous dans du diméthylformamide (5 ml) puis du complexe triméthylamine/sulfure trioxyde (25 mg) est ajouté. La solution est portée à 50 °C pendant environ 14 heures. Après évaporation à sec, le résidu est repris par du chloroforme, puis la phase chloroformique est lavée avec de l'eau, séchée et concentrée à sec. Le solide obtenu est purifié sur une colonne de gel de silice (15 g ; éluant : méthanol/chloroforme ; 1/4 v/v). Après évaporation des fractions pures, on obtient le disaccharide sulfaté 119 (58 mg ; 55,6 %).

— Hydrogénation conduisant au disaccharide 120.

Le disaccharide 119 (58 mg) est dissous dans un mélange méthanol-eau (15 ml + 2 ml). On ajoute ensuite du catalyseur (Pd/C 5 % ; 60 mg) puis on soumet cette suspension à agitation sous atmosphère d'hydrogène pendant 48 heures. A ce stade, on constate la disparition totale des groupes benzyle portés par le dérivé 119, de même que la réduction du groupe azide du dérivé 119 en groupement aminé. Le catalyseur est éliminé par filtration, puis le mélange réactionnel est concentré à sec.

On obtient ainsi le disaccharide 120 qui sera traité directement pour obtenir le produit 121 puis 122.

— Sulfatation du groupe —$NH_2$ conduisant au disaccharide 122.

Le disaccharide 120 est dissous dans l'eau (6 ml). On ajoute à cette solution du complexe triméthylamine/trioxyde de soufre (25 mg), tout en maintenant le pH à 9,5 par addition de soude (0,1 N). Après 45 heures de réaction, de la soude 1 N est ajoutée pour amener le pH à 12. Puis, il est maintenu à cette valeur pendant 1 heure. La solution de 121 est ensuite neutralisée avec de l'acide chlorhydrique 1 N, puis passée au travers d'une colonne de Dowex® 50 (5 ml) sous forme Na+. L'éluat de cette colonne est introduit sur une colonne G1 × 2 (16 ml, 1,6 × 8 cm). La colonne est éluée par un gradient de chlorure de sodium de 0 à 3 M. Les fractions contenant le disaccharide 122 sous forme de sels de sodium sont rassemblées, concentrées, puis le produit est dessalé par passage sur une colonne de Sephadex® G25 (50 ml) éluée avec de l'eau. On obtient ainsi le disaccharide 122 (27 mg, 68 %). Après lyophilisation, le produit se présente sous la forme d'une poudre blanche. $[\alpha]_{20}^{D}$ = + 95,5° (1,3 ; eau).

L'analyse en R.M.N. du carbone 13 confirme la structure attendue pour le produit 122.

Exemple 32 :
Préparation du 2-O-(α-L-idopyranosyl)-D-galactose (composé 128) (voir figure 24).

Cette synthèse est effectuée selon les quatre étapes a) à d) suivantes.

a) Préparation du bromure de 2, 3, 4, 6-tétra-O-acétyl-α-L-idopyranosyle (composé 124)

Une solution de 5 g de penta-O-acétyl-α-L-idopyranose (composé 1, préparé selon P. PERCHEMLIDES, T. OSAWA, F.A. DAVIDSON et R.W. JEANLOZ, Carbohydr. Res., 3 (1967) 463), dans du dichlorométhane anhydre (100 ml) est saturée à 0 °C avec du gaz bromhydrique. Après 2 h à température ambiante, le milieu réactionnel est versé sur de la glace et extrait au chloroforme. La phase organique est lavée avec de l'eau, séchée (chlorure de calcium) et évaporée. Le résidu est cristallisé dans le mélange dichloroéthane-éther-pentane, donnant le bromure 124 (5 g, 95 %), P.F. 126-127 °C $[\alpha]_D$ = — 120° (c : 0,75, chloroforme).

b) Préparation du benzyl 3, 4, 6-tri-O-benzyl-2-O (2, 3, 4, 6-tétra-O-acétyl-α-L-idopyranosyl)-β-D-galactopyranoside (composé 126)

Une solution de 200 mg de benzyl-3, 4, 6-tri-O-benzyl-β-D-galactopyranoside (composé 125 préparé selon la méthode de J.C. JACQUINET et P. SINAY décrite dans Tetrahedron, 32 (1976) 1693) dans le dichloroéthane anhydre (10 ml) est agitée sous azote sec à 90 °C en présence de tamis moléculaire 0,4 nm (300 mg) et de bromure mercurique (80 mg), jusqu'à ce que le volume soit réduit de moitié. Une

solution du bromure 124 (300 mg) dans du dichloroéthane (10 ml) est ajoutée durant 3 h ; le volume du milieu réactionnel étant maintenu constant par distillation continue du dichloroéthane. 3 h après la fin de l'addition, le milieu réactionnel est refroidi à la température ambiante, dilué avec du chloroforme (100 ml), filtré, lavé successivement avec une solution aqueuse à 10 % d'iodure de potassium, avec une solution aqueuse dilué d'hydrogénocarbonate de sodium, avec de l'eau, séché (sulfate de sodium) et évaporé. Le résidu est purifié par chromatographie sur une colonne de gel de silice (30 g) à l'aide du mélange acétate d'éthyle-hexane (1 : 1, v/v), donnant le disaccharide 126 à l'état de sirop (290 mg, 90 %), $[\alpha]_D$ = — 57° (c : 1,1, chloroforme).

Analyse : calculé pour $C_{48}H_{54}O_{15}$ : C, 66, 20 ; H, 6,25 ; 0,27, 55. Trouvé : C : 65,98 ; H, 6,13 ; 0,27, 35 %.

c) Préparation du benzyl-3, 4, 6-tri-O-benzyl-2-O-(α-L-idopyranosyl)-β-D-galactopyranoside (composé 127)

Le disaccharide 126 (200 mg) est dissous dans du méthanol anhydre (10 ml) et une solution M de méthylate de sodium dans le méthanol anhydre (0,2 ml) est ajoutée. Au bout d'une heure, le milieu réactionnel est neutralisé à l'aide de résine Dowex® 50 ($H^+$), filtré et évaporé. Le résidu est purifié par chromatographie sur une colonne de gel de silice (10 g) à l'aide du mélange dichloroéthane-acétone (7 : 3, v/v) donnant le disaccharide 127 (154 mg, 95 %), à l'état pur, $[\alpha]_D$ = — 43° (c : 1,4, chloroforme).

Analyse : calculé pour $C_{40}H_{46}O_{11}$ : C, 68,36 ; H, 6,60 ; 0,25,04 ; trouvé : C, 68,38 ; H, 6,64 ; 0,25, 27 %.

d) Préparation du 2-O-(α-L-idopyranosyl)-D-galactose (composé 128)

Le disaccharide 127 (200 mg) est hydrogéné pendant 48 h dans de l'éthanol (10 ml) contenant de l'acide acétique (0,1 ml), en présence de palladium sur charbon à 10 % (50 ml). Le produit obtenu est purifié par chromatographie sur une colonne de gel de silice (15 g), à l'aide de mélange méthanol-chloroforme (4 :1, v/v), donnant le disaccharide libre 6 sous forme d'une poudre blanche hygroscopique (97,5 mg, 100 %), $[\alpha]_D$ = + 28° (c : 1,2, méthanol) + 15° (10 min) → + 93° (2 h) (c : 1,2 eau).

Analyse : calculé pour $C_{12}H_{22}O_{11}$ : C, 42,10 ; H, 6,48 trouvé : C, 41,71 ; H, 6,48 %.

### Exemple 33 :
### Synthèse du 1,2 : 3,4-di-O-isopropylidène-6-O (α-L-idopyranosyl)-α-D-galactopyranose (composé 131)

On prépare tout d'abord le 1,2 : 3.4-di-O-osopropylidène-6-O (2, 3, 4, 6-tétra-O acétyl-α-L-idopyranosyl)-α-D-galactopyranose (composé 130).

Une solution de 300 mg de 1,2 : 3,4-di-O-isopropylidène-α-D-galactopyranose (composé 129 préparé selon R.C. HOCKETT, H.G. FLETCHER et J.B. AMES dans J. Am. Chem. Soc., 63 (1941) 2516), dans du dichloroéthane anhydre (20 ml) est agitée sous azote sec à 90 °C en présence de tamis moléculaire 0,4 nm (500 mg) et de bromure mercurique (300 mg), jusqu'à ce que le volume soit réduit de moitié. Une solution de bromure 124 (550 mg) dans du dichloroéthane (10 ml) est ajoutée et, après 24 h, une nouvelle solution de bromure 124 (125 mg) dans du dichloroéthane (2 ml). 24 h après cette dernière addition, le milieu réactionnel est traité comme décrit plus haut pour la préparation du disaccharide 126. Une purification par chromatographie sur une colonne de gel de silice (50 g) à l'aide du mélange dichloroéthane-acétone (9 :1, v/v) conduit au disaccharide 130, qui est cristallisé dans le mélange dichloroéthane pentane (650 mg, 95 %), P.F. 160-161 °C, $[\alpha]_D$ = — 86° (c : 1, chloroforme).

Analyse : calculé pour $C_{26}H_{38}O_{15}$ : C, 52,88 ; H, 6,48 ; O, 40,64 ; trouvé : C, 52,89 ; H, 6,41 ; O, 40,63 %.

Le dérivé 130 est ensuite mis en œuvre pour la préparation du 1.2 : 3,4-di-O-isopropylidène-6-O(α-L-idopyranosyl-α-D-galactopyranose (composé 131), en procédant comme suit :

Le disaccharide 130 (300 mg) est désacétylé selon la technique décrite précédemment pour la préparation du disaccharide 127. Une purification par chromatographie sur une colonne de gel de silice (15 g) à l'aide du mélange méthanol-chloroforme (4 :1, v/v) conduit au disaccharide 131 obtenu sous forme d'une poudre amorphe et hygroscopique (204 mg, 95 %), $[\alpha]_D$ = — 63° (c : 0,7, méthanol).

Analyse : calculé pour $C_{18}H_{30}O_{11}$ : C, 51,18 ; H, 7,16 ; O, 41,66 ; trouvé : C, 50,86 (voir figures 24 et 25.

### Exemple 34 :
### Préparation du benzyl-2, 3, 4-tri-O-benzyl-6-O-(α-L-idopyranosyl)-β-D-galactopyranoside (composé 134)

On prépare tout d'abord le benzyl-2, 3, 4-tri-O-benzyl-6-O (2, 3, 4, 6-tétra-O-acétyl-α-L-idopyranosyl)-β-D-galactopyranoside (composé 133).

Une solution de 200 mg de benzyl-2, 3, 4-tri-O-benzyl-β-D-galactopyranoside (composé 132), préparé selon K. MIYAT et R.W. JEANLOZ, Carbohydr. Res., 21 (1972) 45), dans du dichloroéthane anhydre (15 ml) est agitée sous azote sec à 90 °C en présence de tamis moléculaire 0,4 nm (300 mg) et de bromure

mercurique (80 mg), jusqu'à ce que le volume soit réduit à 5 ml. Une solution de bromure 124 (160 mg) dans du dichloroéthane (10 ml) est ajoutée et le milieu réactionnel est agité à 90 °C pendant 24 h. Un traitement analogue à celui décrit précédemment pour la préparation du disaccharide 126 conduit à un résidu qui est purifié par chromatographie sur une colonne de gel de silice (30 g) à l'aide du mélange dichloroéthane-acétone (12 : 1, v/v), donnant le disaccharide 133 (227 mg ; 70 %), $[\alpha]_D$ = — 30° (c : 1, chloroforme).

Analyse : calculé pour $C_{48}H_{54}O_{15}$ : C, 66,20 ; H, 6,25 ; O, 27,55. Trouvé : C, 65,96 ; H, 6,23 ; O, 27,66 %.

Le composé 133 est ensuite mis en œuvre pour la préparation du benzyl-2, 3, 4-tri-O-benzyl-6-O-(α-L-idopyranosyl)-β-D-galactopyranoside (composé 134) en procédant comme suit :

Le disaccharide 133 (200 mg) est désacétylé selon la technique décrite précédemment pour la préparation du disaccharide 127. Une purification par chromatographie sur une colonne de gel de silice (10 g) à l'aide du mélange chloroforme-méthanol (9 :1, v/v) conduit au disaccharide 134 obtenu sous forme amorphe (147 mg, 90 %), $[\alpha]_D$ = — 88° (c : 0,8 chloroforme).

Analyse : calculé pour $C_{40}H_{46}O_{11}$ : C, 68,36 ; H, 6,60 ; O, 25,04. Trouvé : C, 68,74 ; H, 6,68 ; O, 25,37 % (voir figure 25).

Exemple 35 :
Préparation du 2-acétamido-1, 3, 6-tri-O-acétyl-
4-O-(2,3,4,6-tétra-O-acétyl-α-L-idopyranosyl)
2-désoxy-β-D-glucopyranose (composé 138) (figure 26).

La préparation de ce composé est effectuée selon les étapes a) à c) suivantes.

a) Préparation du 2-acétamido-3-O-acétyl-1,6-anhydro-2-désoxy-4, O-(2,3,4,6-tétra-O-acétyl-α-L-idopyranosyl-β-D-glucopyranose (composé 136).

Une solution de 1 g de 2-acétamido-3-O-acétyl-1,6-anhydro-2-désoxy-β-D-glucopyranose (composé 135 préparé selon F. SCHMITT et P. SINAY, Carbohydr. Res., 29 (1973) 99) dans du nitrobenzène anhydre (40 ml) est agitée pendant 2 h à 130 °C en présence de tamis moléculaire 0,4 nm en poudre (1 g), préalablement activé pendant 48 h à 250 °C. Une solution de bromure 124 (1,43 g) dans le dichloroéthane (10 ml) est ajoutée et le milieu réactionnel est maintenu à 130 °C pendant 10 h. Une nouvelle addition de bromure 124 (0,7 g) dans le dichloroéthane (5 ml) est alors effectuée et la réaction poursuivie pendant 24 h. Un traitement analogue à celui décrit pour la préparation du disaccharide 126 conduit à un composé qui est purifié par chromatographie sur une colonne de gel de silice (200 g) à l'aide du mélange acétate d'éthyle-éther (5 :1, v/v), donnant le disaccharide 136 (1,8 g, 85 %), $[\alpha]_D$ = 70,6° (c : 1, chloroforme).

Analyse : calculé pour $C_{24}H_{33}O_{14}N$ : C, 51,52 ; H, 5,94 ; N, 2,50 ; O, 40,03. Trouvé : C, 51,35 ; H, 5,89 ; N, 2,51 ; O, 40,05 %.

b) Préparation du 2-acétamido-1,6-anhydro-2-désoxy-4-O-(α-L-idopyranosyl)-β-D-glucopyranose (composé 137)

Le disaccharide 136 (500 mg) est désacétylé selon la technique décrite précédemment pour la préparation du disaccharide 127. Une purification par chromatographie sur une colonne de gel de silice (40 g) à l'aide du mélange acétate d'éthyle-méthanol (2 : 1, v/v) conduit au disaccharide 137 (300 mg, 90 %), $[\alpha]_D$ = — 65° (c : 1,6, méthanol).

Analyse : calculé pour $C_{14}H_{23}O_{10}N$, 0,5 $H_2O$ : C, 44,92 ; H, 6,46 ; N, 3,74. Trouvé : C, 44,95 ; H, 6,61 ; N, 4,27 %.

c) Préparation du 2-acétamido-1,3,6-tri-O-acétyl-4-O-(2,3,4,6-tétra-O-acétyl-α-L-idopyranosyl) 2-désoxy-β-D-glucopyranose (composé 138)

Le disaccharide 136 (150 mg) est acétolysé à température ambiante pendant 12 heures en présence d'un mélange (5 ml) anhydride acétique-acide acétique et acide sulfurique concentré (7 : 3 : 0,1, v/v/v). Le milieu réactionnel est ensuite versé dans de l'eau glacée et agité pendant 4 heures, puis extrait avec du chloroforme (100 ml). La phase chloroformique est lavée avec une solution aqueuse diluée d'hydrogéno-carbonate de sodium, avec de l'eau, séchée (sulfate de sodium) et évaporée. Le résidu est purifié par chromatographie sur une colonne de gel de silice (10 g) à l'aide du mélange acétate d'éthyle-éther (5 : 1, v/v) donnant le disaccharide 138 qui est cristallisé dans le mélange acétate d'éthyle-pentane (120 mg, 64 %), PF 120 °C, $[\alpha]_D$ = 40° (c : 1, chloroforme).

Analyse élémentaire : calculé pour $C_{28}H_{39}O_{18}N$ : C, 49,63 ; H, 5,80 ; O, 42,50 ; N, 2,07 ; trouvé : C, 49,68 ; H, 5,91 ; O, 42,16 ; N, 2,12 %.

Exemple 36 :
Synthèse du 2-acétamido-2-désoxy-4-O-(α-L-
idopyranosyl)-D-glucopyranose (composé 139) (figure 26).

Le disaccharide 138 (100 mg) est désacétylé selon la technique décrite précédemment pour la préparation du disaccharide 127. Une purification par chromatographie sur une colonne de gel de silice (5 g) à l'aide du mélange méthanol-chloroforme (3 :2, v/v) conduit au disaccharide 139 qui est cristallisé dans de l'éthanol aqueux (48 mg, 85, PF 143-145 °C, $[\alpha]_D$ = — 20° → — 31° (c : 0,8, eau-méthanol, 19 : 1, v/v), au bout de 14 h.

Analyse : calculé pour : $C_{14}H_{25}NO_{11}$, 0,5 $H_2O$ : C, 42,86 ; H, 6,68 ; N, 3,57. Trouvé : C, 42,83 ; H, 6,68 ; N, 3,59 %.

## Exemple 37 :
## Variante de préparation du composé 138 selon les étapes 1 à 6 (voir figure 27).

1. Préparation du benzyl 2-acétamido-3,6-di-O-benzyl-2-désoxy-4-O-(6-O-tosyl-β-D-glucopyranosyl)-α-D-glucopyranoside (composé 140).

Une solution du composé 139 (0,2 g) dans la pyridine (5 ml) est refroidie à 0 °C. On ajoute alors du chlorure de tosyle (0,07 g) dissous dans la pyridine (2 ml). La réaction est abandonnée à la température ambiante pendant 24 heures. Après addition de quelques gouttes d'eau, le mélange est agité pendant une demi-heure avant d'être versé sur de la glace. Après reprise par du chloroforme (0,2 l), la phase chloroformique est lavée successivement avec une solution aqueuse à 10 % de $KHSO_4$, de l'eau, une solution saturée de $NaHCO_3$ et de l'eau. Après séchage sur sulfate de sodium et concentration à sec, le résidu est chromatographié sur gel de silice (20 g) dans un mélange acétate d'éthyle/méthanol (15/l, v/v). On obtient ainsi le composé 140 pur (150 mg ; 60 %). $[\alpha]_D^{20}$ = + 74° (1,1 chloroforme).

Analyse élémentaire : Calculé pour $C_{42}H_{49}O_{13}N$ S (807, 912) C, 62,40 ; H, 6,11 ; N, 1,73 ; O, 25,74 ; S, 3,97. Trouvé : C, 62,77 ; H, 6,13 ; N, 1,73 ; O, 24,98 ; S, 3,48.

Le spectre R.M.N. confirme la structure recherchée.

2. Préparation du benzyl 2-acétamido-3,6-di-O-benzyl-2-désoxy-4-O-(2,3,4-tri-O-acétyl-6-O-tosyl-β-D-glucopyranosyl)-α-D-glucopyranoside (composé 141).

A une solution du composé 139 (200 g) dans la pyridine (5 ml), on ajoute de l'anhydride acétique (5 ml). Après une nuit à température ambiante, le mélange réactionnel est concentré à sec. Le résidu est chromatographié sur une colonne de gel de silice (25 g) dans un mélange acétate d'éthyle/hexane (3/1, v/v). On obtient ainsi le composé 141 (208 mg, 90 %) sous forme de sirop. $[\alpha]_D^{20}$ = + 70° (1, chloroforme).

Analyse élémentaire : calculé pour $C_{48}H_{55}NSO_{16}$ (934, 023) C, 61,78 ; H, 5,94 ; N, 1,5 ; O, 27,41 ; S, 3,43. Trouvé : C, 61,58 ; H, 5,91 ; N, 1,27 ; S, 3,23.

Le spectre R.M.N. confirme la structure recherchée.

3. Préparation du benzyl 2-acétamido-3,6-di-O-benzyl-2-désoxy-4-O (2,3,4-tri-O-acétyl-6-désoxy-6-iodo-β-D-glucopyranosyl-α-D-glucopyranoside (composé 142).

1. A partir du composé 141

A une solution du composé 141 (150 mg dans l'acétone (5 ml), on ajoute de l'iodure de sodium (150 mg). Le mélange est chauffé à 70 °C dans un tube scellé pendant 7 heures. Après évaporation à sec, le résidu est repris par l'eau et le chloroforme. La phase chloroformique est lavée avec de l'eau et séchée sur sulfate de sodium. Après évaporation à sec, le résidu est cristallisé dans un mélange chloroforme/pentane (102 mg, 70 %). p.f. 173-174 °C $[\alpha]_D^{20}$ = + 78,5° (1,2, chloroforme).

Analyse élémentaire : calculé pour $C_{41}H_{48}O_{13}NI$ (889, 773) C, 55,34 ; H, 5,44 ; N, 1,57 ; O, 23,38 ; I, 14,28. Trouvé : C, 54,98 ; H, 5,52 ; N, 1,45 ; O, 23,57 ; I, 14,10.

Le spectre R.M.N. correspond à la structure recherchée.

2. A partir du composé 139 via le composé 143.

Une solution du composé 139 (1 g) et de N-iodo-succinimide (1 g) dans le DMF (50 ml) est agitée à 0 °C pendant 30 minutes. De la triphénylphosphine (1,2 g) est alors ajoutée lentement en une heure. Après chauffage à 50 °C pendant une heure, du méthanol (1 ml) est ajouté puis le mélange réactionnel est concentré à sec. Le produit est extrait au chloroforme. La phase chloroformique est lavée avec de l'eau, avec une solution de thiosulfate de sodium puis de nouveau avec de l'eau. Après séchage et évaporation du chloroforme, le résidu est déposé sur une colonne de gel de silice (50 g). Le composé 143 contaminé par de la triphénylphosphine est élué par un mélange acétate d'éthyle méthanol (15/1, v/v).

Après évaporation du solvant de chromatographie et séchage, le dérivé 143 est dissous dans la pyridine (10 ml) puis acétylé par de l'anhydride acétique (10 ml). Après traitement classique, le dérivé 142 est cristallisé dans un mélange chloroforme/pentane. Le rendement par rapport au composé 139 est de 85 %. Ce composé est en tout point analogue à celui obtenu à partir du composé 141.

4. Préparation du benzyl 2-acétamido-3,6-di-O-benzyl-2-désoxy-4-O-(2,3,4-tri-O-acétyl-6-désoxy-β-D-xylo-hex-5-(énopyranosyl)-α-D-glucopyranoside-2 (composé 144).

A une solution du composé 142 (400 mg) dans la pyridine anhydre (5 ml), on ajoute du fluorure d'argent (400 mg). La suspension est agitée dans le noir pendant 48 heures. Le mélange est ensuite versé sous agitation dans l'éther (200 ml). Après filtration, la phase éthérée est lavée avec une solution à 10 % de $NaHSO_4$, puis avec une solution à 10 % de $NaHCO_3$ et enfin avec de l'eau. Après séchage et concentration à sec, le résidu est cristallisé dans un mélange chloroforme/éther (206 mg ; 60 %). p.f. 184-185 °C. $[\alpha]_D^{20}$ = + 70° (1,4, chloroforme).

Analyse élémentaire : Calculé pour $C_{41}H_{47}NO_{13}$ (761, 821) : C, 64,69 ; H, 6,22 ; N, 1,84. Trouvé : C, 64,5 ; H, 5,96 ; N, 1,79.

Le spectre R.M.N. est conforme à la structure recherchée.

5. Préparation du benzyl 2-acétamido-3,6-di-O-benzyl-2-désoxy-4-O(α-L-idopyranosyl)-α-D-glucopyranoside (composé 145).

Le composé 144 (380 g) est dissous dans du tétrahydrofuranne (8 ml) fraîchement distillé. Après refroidissement à 0 °C sous atmosphère d'azote, l'hydrure de bore ($BH_3$, 1M dans THF, 1 ml) est ajouté puis on laisse la température remonter jusqu'à la température ambiante. Après une heure de réaction, une nouvelle addition d'hydrure est effectuée (1 ml). Après 30 minutes, de l'éthanol est ajouté goutte à goutte. Lorsque le dégagement gazeux a cessé, le mélange est dilué par du THF (10 ml). De la soude (3 M, 1,2 ml) est ajoutée suivie par de l'eau oxygénée (120 vol ; 0,8 ml). Après deux heures à 50 °C, la solution est versée dans du chloroforme. La phase chloroformique est lavée avec une solution aqueuse d'acide chlorhydrique (0, 1N) puis avec de l'eau. Après séchage ($Na_2SO_4$) et concentration à sec, le résidu est chromatographié sur une colonne de gel de silice (45 g) dans un mélange acétate d'éthyle/méthanol (15/4 ; v/v). Le dérivé 145 est élué d'abord (63 mg ; 15 %) suivi du dérivé 139 (225 mg ; 54 %). Le dérivé 145 est cristallisé dans un mélange acétate d'éthyle/méthanol. p.f. 191 °C $[\alpha]_D^{20}$ = + 64,4° (1, méthanol).

Analyse élémentaire : Calculé pour $C_{35}H_{43}NO_{11},H_2O$ : C, 62,57 ; H, 6,75 ; N, 2,08. Trouvé C, 62,42 ; H, 6,55 ; N, 1,88.

6. Préparation du 2-acétamido-1,3,6-tri-O-acétyl-2-désoxy-4-O(2,3,4,6-tétra-O-acétyl-α-L-idopyranosyl)-D-glucopyranose (Composé 138).

Une solution du dérivé 145 (35 mg) dans le méthanol (10 ml) est agitée en présence de catalyseur (Pd/C, 5 % ; 25 mg) sous atmosphère d'hydrogène pendant 48 heures. Après filtration et évaporation, le résidu (17 mg) est acétylé par un mélange pyridine/anhydride acétique (2 ml/1 ml). Après traitement classique, le résidu est chromatographié sur une colonne de gel de silice (10 g) éluée par l'acétate d'éthyle. Après cristallisation, on obtient le composé 138 (14 mg ; 32 %). p.f. 191 °C. $[\alpha]_D^{20}$ = + 8° (0,6 chloroforme).

Exemple 38 :
Synthèse du trisaccharide 149 de formule

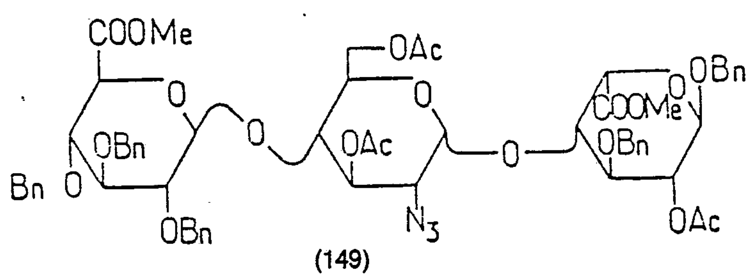

(149)

Cette synthèse est réalisée en 3 étapes (voir figure 28). Tout d'abord, on procède à la glycosylation de l'orthoester d'un dérivé d'acide L-iduronique. On élimine ensuite sélectivement le groupe monochloroacétyle, puis on fait réagir l'un des alcools formés avec un disaccharide.

1. Glycosylation de l'orthoester 38 par l'alcool benzylique.

Une solution de l'orthoester 38 (118 mg 0,25 mmol) obtenu selon l'exemple 5 et d'alcool benzylique (0,15 ml, 1,5 mmol, fraîchement distillé) dans du chlorobenzène anhydre (10 ml) est chauffée à 140 °C à l'abri de l'humidité. Après distillation lente de 8 ml de solvant, une solution de perchlorate de 2,6-diméthylpyridinium (2,5 μmol) dans le chlorobenzène (2 ml) est ajoutée goutte à goutte en 30 min avec distillation simultanée de solvant (2 ml). Le mélange réactionnel est alors agité 30 min dans ces conditions, avec addition goutte à goutte de solvant frais et distillation simultanée, de telle sorte que le

volume réactionnel reste constant et égal à environ 2 ml. Après refroidissement et dilution avec du chloroforme (50 ml), la phase organique est lavée avec une solution aqueuse à 5 % d'hydrogénocarbonate de sodium, avec de l'eau, séchée (sulfate de sodium) filtrée et évaporée.

Le résidu est chromatographié sur une colonne de gel de silice (8 g). L'élution par le mélange hexane-acétate d'éthyle (2 : 1, v/v) permet d'obtenir une fraction contenant le mélange 146 de glycosides α et β qui n'ont pas été séparés à ce stade (102 mg, 81 %), R.M.N. (90 MHz, $CDCl_3$) : δ : 7,30 (m, 10 H, 2 Ph, 3,98 (s, 2 H, Cl—$CH_2$—CO), 3,74 (s, 3 H, COOMe), 2,08 et 2,03 (2s, 3 H au total, OAc forme β et α ; β : α : ≃ 2 : 1).

2. O-démonochloroacétylation sélective.

Une solution du mélange 146 précédent (102 mg) dans de la pyridine (5 ml) et de l'éthanol absolu (1 ml) est chauffée à 100 °C pendant 20 min en présence de thiourée (25 mg). Après refroidissement, le mélange réactionnel est évaporé à sec et le résidu est repris par un mélange eau-chloroforme (1 : 1, v/v, 50 ml). La phase organique est lavée avec de l'eau, séchée (sulfate de sodium), filtrée et évaporée.

Le résidu est chromatographié sur une colonne de gel de silice (10 g). L'élution par le mélange acétate d'éthyle-hexane (4 : 3, v/v) permet d'isoler (par ordre d'élution) :

— le glycoside β 148 (26 mg, 25 %), sirop incolore, $[\alpha]_D$ + 70° (c1, chloroforme) R.M.N. (90 MHz, $CdCl_3$) : δ : 7,30 (m, 10 H, 2 Ph) ; 5,05 (m, 1 H, $H_2$) ; 4,90 (d, 1 H, $H_1$, 1,2 J = 2Hz) ; 3,78 (s, 3 H, COOMe) ; 3,12 (1 H, OH, échangé avec $D_2O$) ; 2,05 (s, 3 H, OAc).

— le glycoside α 147 (54 mg, 50 % à partir de 38) sirop incolore, $[\alpha]_D$ — 65° (c1, chloroforme) R.M.N. (90 MHz, $CdCl_3$) : δ : 7,30 (m, 10 H, 2 Ph) ; 5,05 (2 H, $H_1$ et $H_2$, constantes de couplage très faibles pour $J_{1,2} \leq 1$ Hz) ; 3,78 (s, 3 H, COOMe) ; 2,80 (1 H, OH, échangé avec $D_2O$) ; 2,06 (s, 3 H, OAc).

3. Glycosylation de l'alcool 147 à l'aide du disaccharide 97.

Une solution de l'alcool 147 (22 mg, 50 μmol), et du bromure 97 obtenu selon l'exemple 6 (57 mg, 70 μmol) dans du dichlorométhane anhydre (1,5 ml) est agitée à l'abri de la lumière et de l'humidité en présence de tamis moléculaire 0,4 nm (poudre, 50 mg). Le mélange réactionnel est refroidi à — 20 °C et de la sym-collidine (110 μl) et du triflate d'argent (26 mg, 100 μM) sont ajoutés successivement. Le mélange réactionnel est agité 2 h dans ces conditions, dilué avec du dichlorométhane (50 ml) les solides sont essorés et le filtrat est lavé avec une solution aqueuse glacée d'HCl 0,1 M, avec de l'eau, avec une solution aqueuse à 5 % d'hydrogénocarbonate de sodium, avec de l'eau, séché (sulfate de sodium), filtré et évaporé.

Le résidu est chromatographié sur une colonne de gel de silice (8 g, gel 230-400 mesh). L'élution par le mélange toluène-acétate d'éthyle (5 : 1, v/v) permet d'isoler le trisaccharide 149 sous forme d'un sirop incolore (50 mg, 86 %).

Le spectre R.M.N. (270 MHz, $CDCl_3$) est conforme avec la structure attendue. Ce spectre est représenté sur la figure 32.

Exemple 39 :
Fixation du tétrasaccharide sur BSA

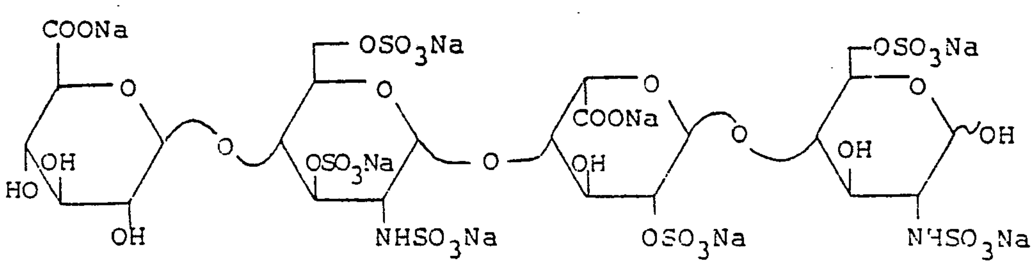

A une solution de bovine sérum albumine (BSA : 7 mg ; 0,1 μmol) et de tétrasaccharide (15 mg ; 10 μmol) dans un tampon phosphate de sodium (0,15 M ; pH 7,0 ; 2,5 ml), on ajoute du cyanoborohydrure de sodium (13 mg ; 200 μmol) et porte la solution à 37 °C pendant cinq jours. Le mélange réactionnel est ensuite chromatographié sur une colonne de Séphadex® G-50 (1 × 100 cm), éluée par l'eau, de façon à séparer les sels et le tétrasaccharide non fixé du conjugué protéine-oligosaccharide. Dans ces conditions, on obtient une fixation de 12 moles de tétrasaccharide par mole de BSA.

La même réaction peut être effectuée sur un support insoluble tel que le 2-aminoéthyl-polyacrylamide ou la 2-aminoéthyl-cellulose, ou tout autre support contenant une fonction amine primaire.

De même, en travaillant en présence d'antithrombine III (au lieu de BSA), dans les conditions définies ci-dessus, on obtient une fixation de l'oligosaccharide sur l'antithrombine III, et par là une antithrombine III activée en permanence (BJÖRK et al., FEBS Letters, 143 (1982), 96-100).

Synthèse du disaccharide 163

A une solution du composé 2 (5,6 g) et de cyanure mercurique (3,5 g) dans le dichloroéthane (40 ml), on ajoute, après distillation d'environ 30 ml de solvant, du tamis moléculaire 0,4 nm (1 g) puis le composé 1 (3,44 g : 8,82 mmol). Après une nuit sous agitation les solides sont éliminés par filtration, puis lavés avec du dichlorométhane. Celui-ci est joint à la solution puis la phase organique obtenue est lavée avec une solution saturée d'iodure de potassium, puis avec de l'eau. Après séchage et concentration à sec, le sirop obtenu (10 g) est désacétylé en présence de méthanolate de sodium (2 M, 1 ml) dans le méthanol (20 ml). Le composé 163 obtenu (2,7 g) après chromatographie sur gel de silice (50 g ; chloroforme/méthanol ; 20/1 : v/v). C'est un sirop $[\alpha]_D^{20}$ — 12° (1,1 ; chloroforme) qui est engagé tel quel dans la synthèse de 5.

Synthèse du composé 164

Le disaccharide 163 (2,7 g) est dissous dans du DMF anhydre (27 ml) puis on ajoute successivement à cette solution du chlorure de trityl (4,42 g) de la diméthylaminopyridine (135 mg) puis de la triethylamine (2,7 ml). Après deux jours à température ambiante, le mélange réactionnel est concentré sous vide, puis le résidu est chromatographié sur gel de silice (50 g ; hexane puis hexane/acétate d'éthyle ; 2/1 puis 1/1 ; v/v). On obtient ainsi 164 (2,6 g). C'est un sirop ; $[\alpha]_D^{20}$ — 16,3° (1,3 ; chloroforme).

Synthèse du disaccharide 166

Le sirop obtenu à l'issue de la préparation de 164 (2,4 g) est dissous dans du DMF (40 ml). On ajoute alors de l'hydroxyde de barium octahydrate (1,64 g), de l'oxyde de barium (7,08 g) et enfin du bromure de benzyle (2 ml). Après 4 heures de réaction, du méthanol est ajouté puis suivi de chloroforme (100 ml). Les solides sont essorés puis la phase chloroformique est concentrée à sec. Le disaccharide 165 obtenu à ce stade est directement transformé en 166. Pour cela, le résidu est repris dans du dichlorométhane (20 ml) puis une solution de $BF_3$ dans le méthanol (2 ml) est ajoutée, à 0 °C, à l'abri de l'humidité. Après 4 heures de réaction, le mélange réactionnel est dilué par du dichlorométhane puis lavé avec une solution aqueuse de bicarbonate de sodium. Après séchage et concentration, le résidu est chromatographié sur une colonne de gel de silice (100 g ; hexane/acétate d'éthyle ; 4/1 puis 1/1 ; v/v). On obtient ainsi 166 $[\alpha]_D^{20}$ — 2° (0,7, chloroforme).

Synthèse du composé 168

Le dérivé 166 est dissous dans l'acétone (20 ml). On ajoute alors, à 0 °C, une solution d'oxyde de chrome (VI) (670 mg) dans l'acide sulfurique 3,5 M (3 ml). Après 1,5 heure, de la glace et de l'eau sont ajoutées au mélange réactionnel, puis le produit oxydé est extrait au chloroforme. La phase chloroformique est lavée à l'eau, séchée, et concentrée à sec. Le résidu, dissous dans l'éther est méthylé par addition de diazométhane livrant ainsi 168 qui est purifié sur gel de silice (hexane/acétate d'éthyle ; 4/1 puis 1/1 ; v/v). C'est un sirop $[\alpha]_D^{20}$ — 8,5° (1, chloroforme). L'analyse élémentaire et le spectre I.R. confirment la structure attendue pour 168.

Remarque : 168 peut-être acétolysé et transformé en halogénure de la façon décrite dans l'exemple 25 (passage de 94 à 97).

**Revendications**

1. Procédé de synthèse organique d'oligosaccharides comprenant des enchaînements [D-glucosamine] — [acide D-glucuronique ou acide L-iduronique], ou l'inverse, tels que rencontrés respectivement dans l'héparine ou l'héparane sulfate, caractérisé par le fait

— qu'on met en œuvre, dans une réaction de glycosylation, deux composés terminés ou constitués respectivement par un motif A de structure D-glucosamine et un motif U de structure acide D-glucuronique ou acide L-iduronique, l'un des motifs A ou U étant un alcool dans lequel le groupe —OH de la fonction alcool occupe la position 4, l'autre motif possédant un carbone anomère activé, substitué par un groupe réactif compatible avec les autres groupements présents sur les motifs, toutes les autres positions de A et U, excepté celle dont le carbone anomère est activé et celle occupée par le groupe —OH de la fonction alcool, portant, soit des groupes amino, soit des groupes carboxyle, ou des précurseurs de ces groupes, soit encore des groupes —OH, ces groupes occupant des positions déterminées, les groupes amino et carboxyle lorsqu'ils sont présents, étant bloqués respectivement par des groupements protecteurs de fonction amino et carboxyle, les groupes —OH étant bloqués par au moins deux types de groupes protecteurs, éliminables séquentiellement en permettant l'introduction de groupes désirés en certaines positions, puis la libération de groupes —OH en d'autres positions,

— qu'on répète, si on le souhaite, l'opération de glycosylation afin d'allonger la chaîne oligosaccharidique,

— qu'on libère séquentiellement les groupements protecteurs des groupes —OH en introduisant des groupements de substitution désirés dans des positions spécifiques, puis en libérant les groupements —OH d'autres positions spécifiques ainsi que les groupes amino et carboxyle sous réserve que l'établissement de la liaison interglycosidique ne conduise pas à l'obtention d'un disaccharide à structure [2-N-sulfate ou 2-N-acétyl)-6-O-sulfate-D-glucosamine] - [acide méthyl-D-glucuronique].

2. Procédé selon la revendication 1, caractérisé en ce que les produits avec les motifs A et U mis en œuvre renferment plusieurs types de groupes protecteurs de radicaux —OH à savoir (1) un ou plusieurs groupes semi-permanents, c'est-à-dire des groupements éliminables en premier lieu après les réactions de glycosylation lorsque le squelette glucidique comporte le nombre de motifs désirés, sans enlèvement ou altération des autres groupes présents, et (2) un ou plusieurs groupes permanents, c'est-à-dire des groupements capables de maintenir la protection des radicaux —OH durant l'introduction de groupements fonctionnels à la place des groupements semi-permanents, ces groupements protecteurs étant choisis parmi des radicaux tels que les radicaux acyle, alcoyle, le cas échéant alcoyle substitué, ou aryle.

3. Procédé selon la revendication 1 ou 2, caractérisé en ce que les groupements protecteurs de radicaux —OH, sont choisis dans le groupe comprenant les radicaux acyle (notamment acétyle, alcoyle substitué tel que benzyle), et pour deux positions voisines, parmi les groupes acétals, ou cétals, par exemple benzylidène, une autre forme de protection consistant à effectuer un blocage de deux groupes —OH sous forme époxyde ou de pont 1-6-anhydro.

4. Procédé selon l'une quelconque des revendications précédentes, caractérisé en ce que les produits utilisés dans la réaction de glycosylation renferment plusieurs types de groupements protecteurs, les groupements protecteurs occupant déjà des positions déterminées sur les produits mis en œuvre dans la réaction de glycosylation, ou en variante étant introduits à partir d'autres groupements une fois le squelette glucidique constitué, cette variante comportant, par exemple, l'utilisation pour la glycosylation d'un produit A dans lequel les groupes —OH en position 2 et 3 et en positions 1 et 6 sont bloqués sous forme anhydre, respectivement 2,3-époxyde et 1,6-anhydre, l'ouverture de la fonction époxyde par de l'acide de sodium permettant d'introduire, en position 2, un groupe $N_3$ qui constitue donc un précurseur de fonction amine.

5. Procédé selon l'une quelconque des revendications précédentes, caractérisé en ce que les radicaux —OH des produits de départ destinés à être sulfatés sont protégés par des groupes acyle, en particulier acétyle tandis que les radicaux —OH destinés à être libérés en fin de synthèse sont protégés par un groupe permanent tel que le groupe benzyle.

6. Procédé selon l'une quelconque des revendications 1 à 5, caractérisé en ce que A comprend en position 2, un groupe azoté, avantageusement constitué par des groupes tels que $—N_3$ ou $—NHCOO—CH_2—C_6H_5$, ou tout autre groupe constituant un précurseur de fonction amine ou d'un dérivé d'amine, en particulier de $—NHSO_3^-$ ou de —NH-acyle, plus spécialement de $—NH—COCH_3$.

7. Procédé selon l'une quelconque des revendications 1 à 6, caractérisé en ce que les fonctions carboxyle des motifs U sont bloquées par des groupes inertes vis-à-vis des réactions mises en jeu pour le remplacement des groupes protecteurs et éliminables en fin de synthèse pour libérer les groupes carboxyle, éventuellement aux fins de salification, ces groupes protecteurs de fonction carboxyle étant choisis avantageusement parmi les radicaux alcoyle, ou les radicaux aryle.

8. Procédé selon la revendication 1, caractérisé en ce que les motifs A et U de la séquence formée renferment des groupements protecteurs temporaires, c'est-à-dire des groupements capables de bloquer sélectivement une position du motif A ou U destinée à intervenir dans une nouvelle réaction de glycosylation, ces groupements étant éliminables en présence des autres groupements présents sur les motifs des produits de départ en recréant un alcool.

9. Procédé selon la revendication 8, caractérisé en ce qu'aux fins d'élongation d'une unité disaccharidique U-A vers la gauche, on met en œuvre un motif U comportant un groupe temporaire et qu'aux fins d'une élongation à droite on utilise un motif A comportant ledit groupe temporaire, ce qui permet, en effectuant des réactions de glycosylation successives d'effectuer des enchaînements $U_wA_xU_yA_z$ dans lesquels la somme des indices est comprise entre 2 et 12, ces valeurs étant incluses dans l'intervalle, w et y ne pouvant être nuls simultanément, les enchaînements réguliers étant du type U $(AU)_n$, $(AU)_nA$, $(UA)_n$ ou encore $(AU)_n$ avec $1 \leq n \leq 6$.

10. Procédé selon l'une quelconque des revendications 1 à 9, caractérisé en ce que la fonction alcool de l'un des motifs A ou U impliqué dans la séquence glucidique déjà constituée est avantageusement libérée de son groupement protecteur temporaire par exemple

• à partir d'un groupe allyle par un traitement du type comportant l'utilisation d'un agent isomérisant tel que des dérivés de Pd, Rh et Ir, en particulier le chlorure de tris-triphénylphosphine rhodium (I) ou encore le tertiobutoxyde de potassium, puis dans des conditions acides, en particulier avec un mélange d'oxyde mercurique et de chlorure mercurique, ou

• par saponification à partir d'un groupe —O-acyle, en particulier —O-acétyle, ou O-chloroacétyle, ces radicaux étant éliminés pour libérer une fonction —OH, par exemple, à l'aide de thiourée en milieu solvant, avantageusement à une température supérieure à 80 °C, de préférence de l'ordre de 100 °C.

11. Procédé selon l'une quelconque des revendications 1 à 10, caractérisé en ce qu'on fait réagir ledit alcool avec un dérivé réactif tel qu'un halogène, avantageusement un chlorure ou un bromure, un imidate ou un orthoester,

— la réaction de condensation entre l'halogénure et l'alcool étant avantageusement du type Koenigs-Knorr, et étant effectuée en milieu solvant, plus spécialement dans un solvant organique, notamment du type dichlorométhane ou dichloroéthane,

avantageusement en présence d'un catalyseur, en général un sel d'argent ou de mercure, par exemple, le trifluorométhane sulfonate d'argent, communément appelé triflate d'argent, le carbonate d'argent, l'oxyde d'argent, le bromure mercurique ou le cyanure mercurique et également avec un accepteur de protons tel que la sym-collidine de même qu'un capteur pour l'eau éventuellement présente et/ou pour l'acide halogénohydrique formé, par exemple des tamis moléculaires 0,4 nm

à température ambiante ou encore à une température inférieure pouvant atteindre 0 °C ou moins, sous atmosphère d'un gaz inerte tel que l'azote ou l'argon, ou en variante, pour former des liaisons covalentes entre des alcools de structures variées et un précurseur L-iduronique, on réalise la réaction de condensation en utilisant comme catalyseur des dérivés mercuriques, en particulier de cyanure et du bromure mercurique, de tamis moléculaire, en particulier de tamis moléculaire 0,4 nm, dans un solvant organique choisi selon la réactivité de l'alcool,

— la condensation avec un orthoester tel qu'un groupe 1,2-O-méthoxy-éthylidène étant de préférence effectuée à une température supérieure à 100 °C dans un milieu solvant, du type chlorobenzène ou un solvant analogue avec un point d'ébullition supérieur à 100 °C et avantageusement entre 100 et 150 °C, en présence d'un catalyseur tel que le perchlorate de 2,6-diméthylpyridinium,

— la condensation avec un imidate étant réalisée à basse température, en particulier à une température inférieure ou égale à environ 0 °C, en milieu solvant, tel que le dichlorométhane, en présence de tamis moléculaire 0,4 nm et d'un catalyseur tel que de l'éthérate de trifluorure de bore.

12. Procédé selon la revendication 1, caractérisé en ce que la chaîne glucidique élaborée est soumise à une ou plusieurs réactions chimiques afin d'introduire un type de groupements fonctionnels donnés ou, successivement, plusieurs types de groupements, puis de former, si on le désire, des dérivés de ces groupements fonctionnels.

13. Procédé selon la revendication 12, caractérisé en ce que l'étape de fonctionnalisation est réalisée en n'éliminant que certains groupements protecteurs et/ou certains groupements précurseurs des dérivés aminés ou encore la totalité des groupements protecteurs et/ou des groupements précurseurs et en introduisant à leur place un type de substituants donné, ou, successivement, des substituants différents, puis en libérant une partie ou la totalité des groupes —OH encore bloqués, si on le désire.

14. Procédé selon la revendication 12 ou 13, caractérisé en ce que l'étape de fonctionnalisation est réalisée sélectivement de manière à introduire sur la chaîne, successivement, plusieurs types de substituants, en particulier des groupes sulfate, en des positions déterminées des motifs, pour former en position 2 des motifs A un dérivé aminé et en position 6 des motifs U, un dérivé d'acide, puis de libérer les fonctions —OH à d'autres positions, cette étape de fonctionnalisation étant réalisée en utilisant des dérivés dans lesquels les groupes semi-permanents occupent des positions destinées à être sulfatées et sont constitués par des groupes —O-acétyle, les positions correspondant à un groupe —OH destinées à être libérées sont occupées par des groupes permanents constitués par des groupes benzyle et les positions 2 des motifs A sont substituées par des groupes tels que $—N_3$ ou $—NH—COO—CH_2—C_6H_5$ et les positions 6 des motifs U sont occupées par des groupes carboxyle protégés par un radical alcoyle, en particulier méthyle.

15. Procédé selon la revendication 14, caractérisé en ce que l'étape de fonctionnalisation comprend :

— l'introduction sélective de groupes sulfate après l'élimination des groupes de blocage —O-acétyle à l'aide d'une réaction de saponification réalisée avec une base forte telle que la soude, de préférence à une température inférieure à l'ambiante et plus spécialement voisine de 0 °C, le produit résultant de l'hydrolyse étant alors soumis à l'action d'un agent d'alcoylation afin d'introduire, sur le groupe carboxyle, les groupes alcoyle protecteurs qui se sont trouvés éliminés lors de l'hydrolyse, cette alcoylation étant suivie par un traitement de sulfatation aux fins d'introduction de groupes sulfate aux positions libérées par l'hydrolyse et laissées libres après l'action de l'agent d'alcoylation, la conduite de la sulfatation comprenant la mise en œuvre d'un agent de sulfatation, tel qu'un complexe triméthylamine/$SO_3$, en milieu solvant, plus spécialement dans un solvant tel que le diméthylformamide, de préférence, à une température supérieure à l'ambiante, généralement voisine à 50 °C, ce qui correspond à une durée de réaction d'environ 12 heures,

— la libération des groupes —OH bloqués par des radicaux benzyle par

— l'élimination de groupes benzyle avantageusement réalisée par hydrogénation catalytique dans des conditions compatibles avec le maintien des groupes sulfate et la transformation des groupes azotés en groupes fonctionnels amine, de préférence sous pression d'hydrogène en présence d'un catalyseur du type Pd/C, en milieu solvant organique, en particulier alcoolique, additionné d'eau,

— la formation de groupes N-acétyle en soumettant le produit résultant de la réaction d'hydrogénation à l'action d'un agent d'acétylation tel que de l'anhydre acétique, cette réaction étant avantageusement réalisée à un pH basique, en particulier voisin de 8, en milieu aqueux, ou la formation de groupes N-sulfate à l'aide d'un agent de sulfatation du type indiqué ci-dessus, à un pH supérieur à 9, avantageusement de l'ordre de 9-10,

— la libération des groupes carboxyle par addition d'une base forte,

— la salification des groupes carboxyle en utilisant par exemple des résines échangeuses comportant le cation désiré, en particulier du sodium ou encore du potassium, du lithium, du magnésium ou du calcium.

16. Procédé selon l'une quelconque des revendications précédentes, caractérisé en ce qu'on met en œuvre, à la place de l'un ou plusieurs des motifs A ou U, un sucre constituant un analogue structural de motif A ou U, tel qu'un sucre neutre ou un désoxy-sucre.

17. Oligosaccharides constituant des intermédiaires dans le procédé selon l'une quelconque des revendications précédentes, caractérisés en ce qu'ils comprennent une chaîne à base de motifs binaires de structure $(A\text{-}U)_n$ ou $(U\text{-}A)_n$ correspondant à des enchaînements a-b ou a-c (ou l'inverse), a, b, c, répondant respectivement aux structures :

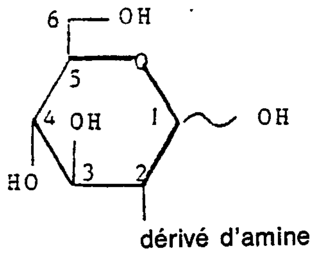

(a) D-glucosamine

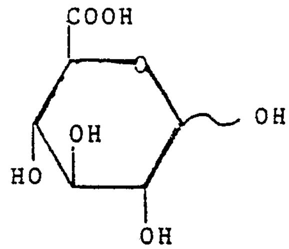

(b) acide D-glucuronique

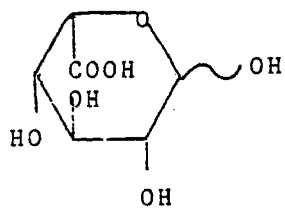

(c) acide L-iduronique

n étant un nombre de 1 à 6, ces motifs binaires étant (1) complètement protégés et possédant soit un groupe réactif sur le carbone anomère du motif à l'extrémité réductrice, soit un seul groupe —OH libre sur le motif à l'extrémité non réductrice, ce groupe —OH occupant la position 3, 4 ou 6 dans le cas d'un motif A et la position 2, 3 ou 4 dans le cas d'un motif U, ou (2) étant constitués par des motifs complètement protégés tels qu'obtenus à l'issue de l'étape de glycosylation, ou (3) comprenant les produits dans lesquels un ou plusieurs groupes —OH sont libérés, ces oligosaccharides intermédiaires comportant le cas échéant un ou plusieurs motifs consécutifs a, b, c ou encore d et/ou plusieurs motifs de sucres neutres et/ou plusieurs désoxy-sucres dans leur structure.

18. Oligosaccharides selon la revendication 17, caractérisés en ce qu'ils renferment au moins un motif possédant une structure du type b 1 $\xrightarrow{\beta}$ 4 a, répondant à la formule (I) :

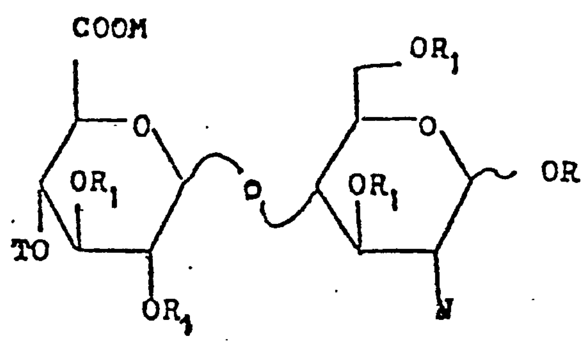

(I)

dans laquelle :

— les radicaux $R_1$, identiques ou différents les uns des autres, éventuellement conjointement avec R, représentent un groupe protecteur, en particulier un groupement sp semi-permanent ou un groupement p permanent,

— T, un groupement temporaire t, ou un groupement permanent p, ou un atome d'hydrogène,

— N, un groupe azoté précurseur d'amine ou de dérivé d'amine,

— R, un radical aliphatique ou aromatique, notamment un radical alcoyle comportant de 1 à 4

atomes de carbone, où OR représente un groupe réactif tel qu'un halogénure ou encore un radical alcoyle et

— M, un groupement bloquant la fonction acide,

et de préférence aux formules (II), (III) ou (IV)

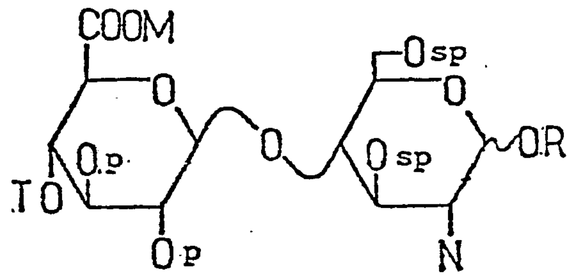

(II)

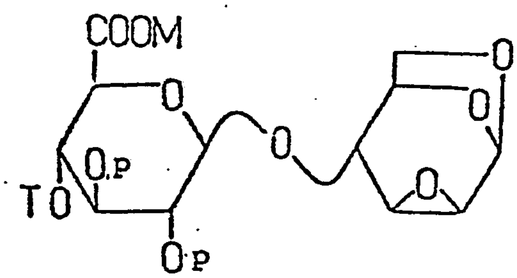

(III)

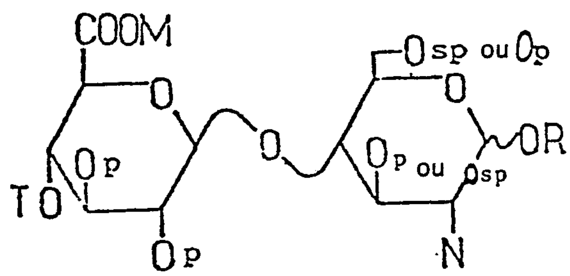

(IV)

dans lesquelles les différents symboles présentent les significations données plus haut, les symboles des formules (II) à (IV), présentant indépendamment, ou en combinaison, les significations suivantes :

— M, représente un atome d'hydrogène ou un radical alcoyle, en particulier méthyle,

— sp, un groupe acyle, en particulier acétyle,

— p, un groupe alcoyle substitué, en particulier benzyle,

— R, un groupe acyle en α ou β, en particulier un groupe acétyle, un radical alcoyle, en particulier méthyle ou alcoyle substitué, notamment benzyle, ou —OR un halogène, en particulier un bromure, ou encore un radical imidoyle,

— N, un groupe azido,

— T, le groupe temporaire t, t représentant un radical acyle, en particulier acétyle, un radical acyle halogéné, en particulier, un radical monochloro ou trichloroacétyle, ou le groupe p, p représentant un radical alcoyle substitué, en particulier le radical benzyle, le cas échéant, lui-même paraméthoxy ou encore un atome d'hydrogène.

19. Oligosaccharides selon la revendication 17, caractérisés en ce qu'ils comportent au moins un motif possédant une structure du type a 1 $\xrightarrow{\alpha}$ 4 b, répondant à la formule (V) :

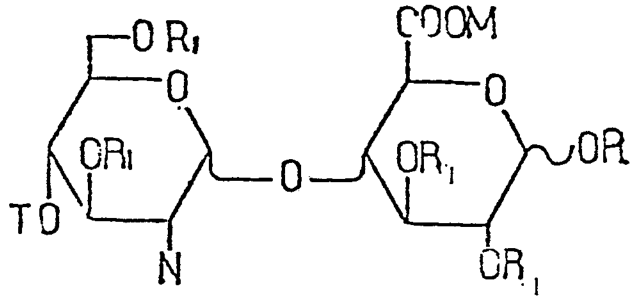

(V)

et de préférence aux formules (VI) ou (VII)

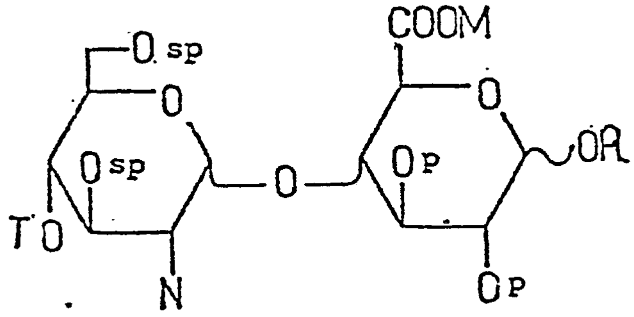

(VI)

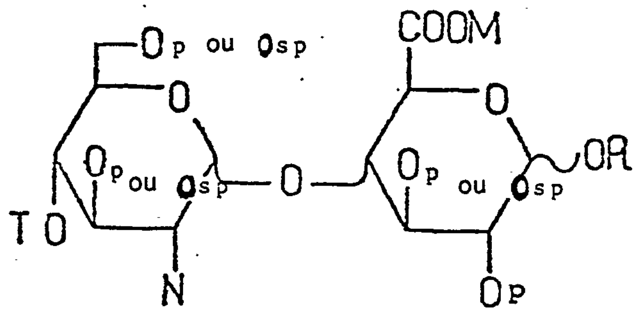

(VII)

dans lesquelles M, N, sp, p présentent, de préférence, les significations particulières données ci-dessus en rapport avec les formules (II) à (IV), et R représente, en outre, de préférence, un groupe propényle, allyle, imidoyle, ou —H, avec N représentant alors plus spécialement un groupe —NH-acétyle.

20. Oligosaccharides selon la revendication 17, caractérisés en ce qu'ils comportent au moins un motif de structure du type c 1 $\xrightarrow{\alpha}$ 4 a de formule (VIII)

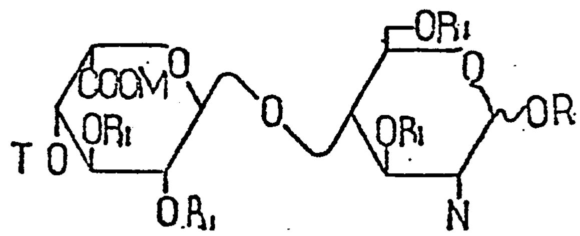

(VIII)

dans laquelle les substituants présentent les significations données ci-dessus pour la formule (I), lesdits oligosaccharides présentant de préférence les formules (IX) et (X)

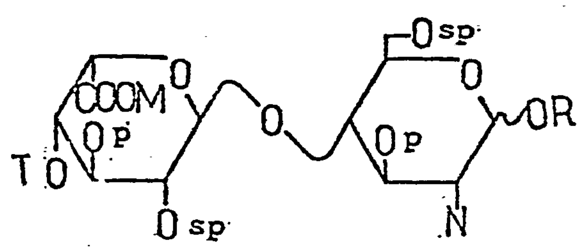

(IX)

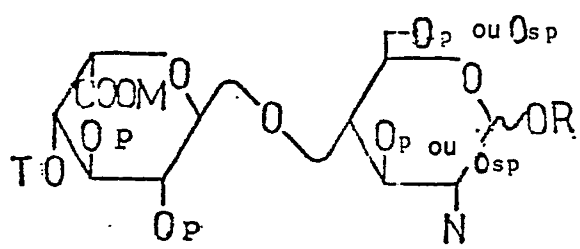

(X)

dans lesquelles :

— les différents groupes sp et p peuvent être identiques et représentent un radical acyle, en

particulier acétyle, ou différents, et choisis parmi les radicaux acyle, en particulier acétyle ou benzoyle et les radicaux aryle ou alcoyle substitué,

— N représente un groupe azoté précurseur éventuellement différent de celui présent dans les composés de formules (I) à (V), en particulier un groupe —NHCOO-(alcoyle substitué), notamment un groupe —NH—COO—$CH_2$—$C_6H_5$, ce qui permet de soumettre ces groupes azotés à des traitements différents et de former des dérivés d'amine différents en position 2 des motifs a,

— T représente le radical acétyle, acyle halogéné, en particulier, monochloro ou trichloroacétyle, p-méthoxybenzoyle, les symboles p, M et R présentant avantageusement les significations préférées données ci-dessus en rapport avec les formules (II) à (IV).

21. Oligosaccharides selon la revendication 17, caractérisés en ce qu'ils comportent au moins une structure du type a 1 $\xrightarrow{\alpha}$ 4 c de formule (XI)

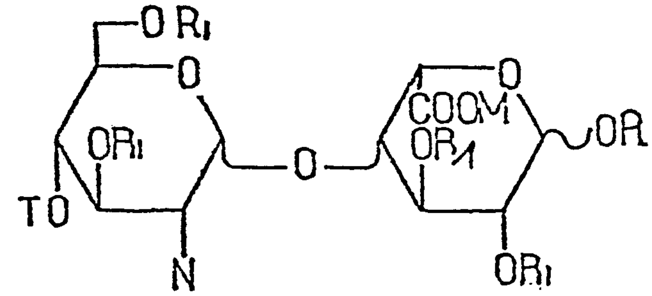

(XI)

dans laquelle les substituants présentent les significations données ci-dessus pour la formule (I), lesdits oligosaccharides répondant de préférence aux formules (XII) ou (XIII)

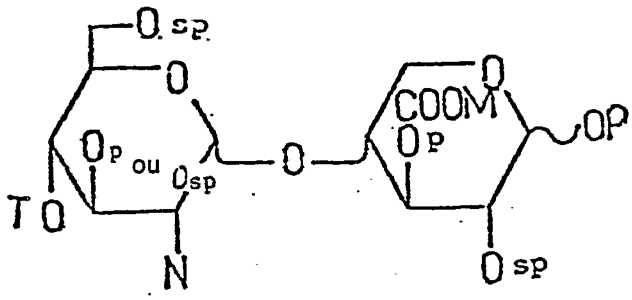

(XII)

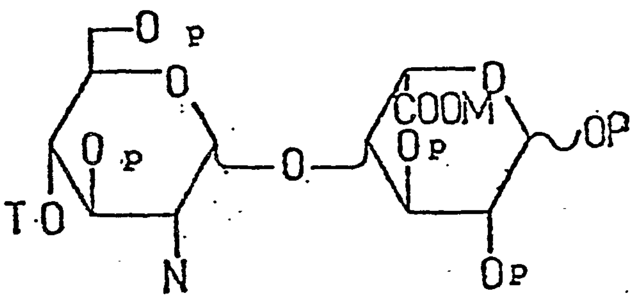

(XIII)

dans lesquelles les significations préférées correspondent à celles données ci-dessus pour les formules (II) à (IV).

22. Oligosaccharides intermédiaires selon la revendication 17, correspondant aux produits dont les groupes protecteurs ont été partiellement éliminés en cours de synthèse, ces produits comportant, en particulier, un groupe —OH à la place des groupes sp.

23. Oligosaccharides intermédiaires correspondant aux oligosaccharides renfermant des structures du type ABCDEFGH, C DEFGH, AB, BC, CD etc ... ABC, BCD ..., ABCD, BCDE ..., ABCDE ..., ABCDEF ..., ABCDEFGH or BCDEFGH, par référence à l'octosaccharide de structure :

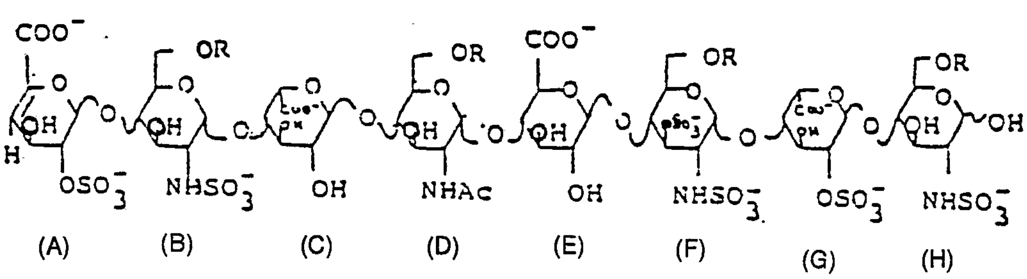

(A) (B) (C) (D) (E) (F) (G) (H)

de préférence

— une structure trisaccharidique, en particulier correspondant à l'une des formules : (XVIII) à (XXI) :

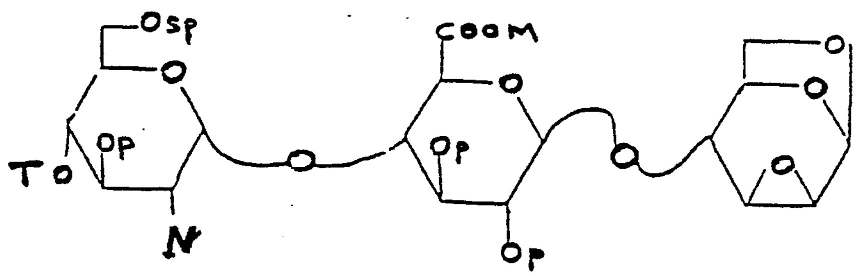

(XVIII)

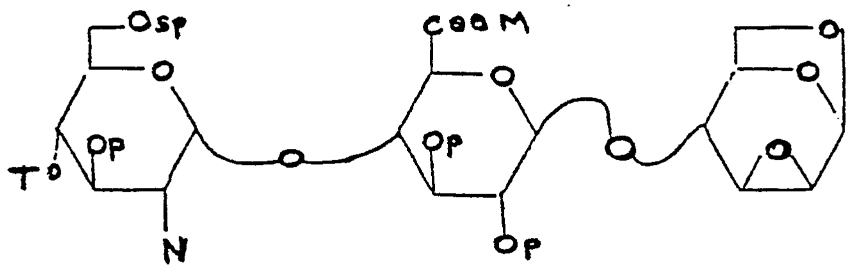

(XIX)

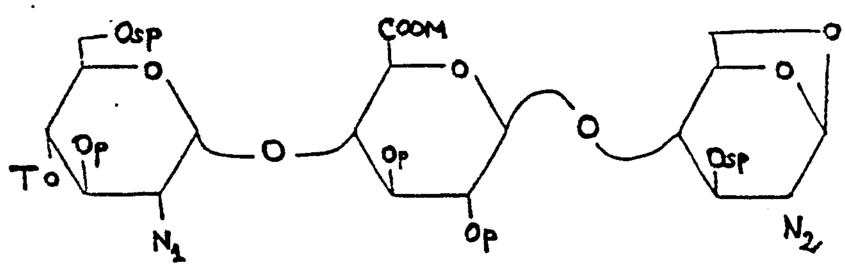

(XX)

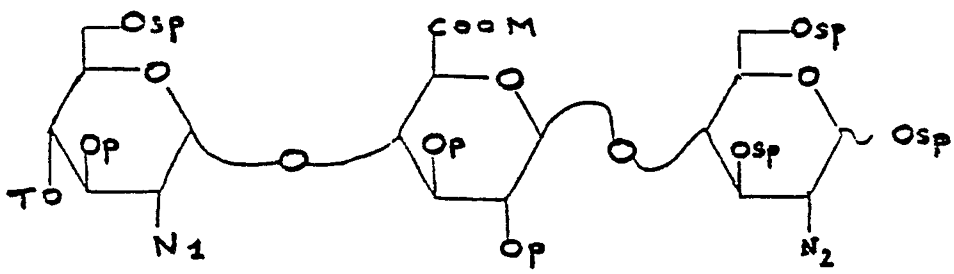

(XXI)

dans lesquelles les substituants présentent les significations ci-dessus, $N_1$ et $N_2$, étant de préférence identiques ou différents l'un de l'autre et représentant un groupe azido ou —NH-acyle, en particulier —NH-acétyle, ou —$NHSO_3^-$ ou une structure de type FGH de formule :

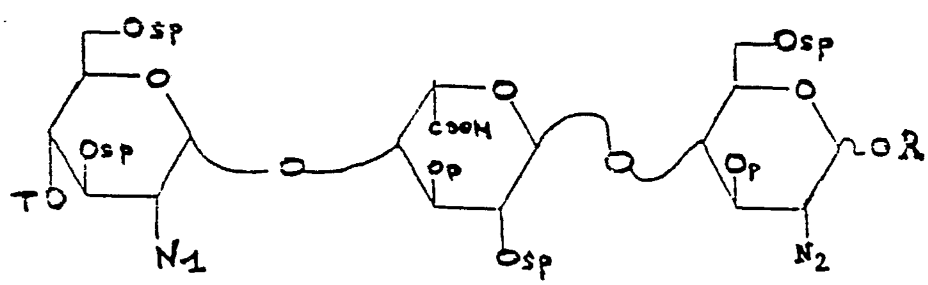

(XXII)

dans laquelle les différents symboles présentent les significations données ci-dessus, les deux

substituants $N_1$ et $N_2$ des deux motifs glucosamine de structure F et H étant identiques ou encore avantageusement différents, comme dans les produits naturels, et choisis parmi le groupe azido ou —NH-COO-acyle, en particulier —NH-COO-acétyle ou —NH—COO—$CH_2$—$C_6H_5$,

— une structure tétrasaccharidique, en particulier la structure EFGH répondant à la formule suivante :

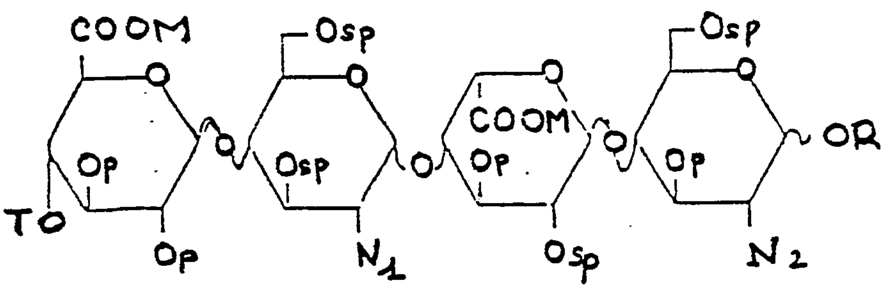

(XXIII)

dans laquelle les significations préférées des différents symboles correspondent à ceux indiqués pour la formule XXII,

— une structure pentasaccharidique, en particulier du type DEFGH, de formule

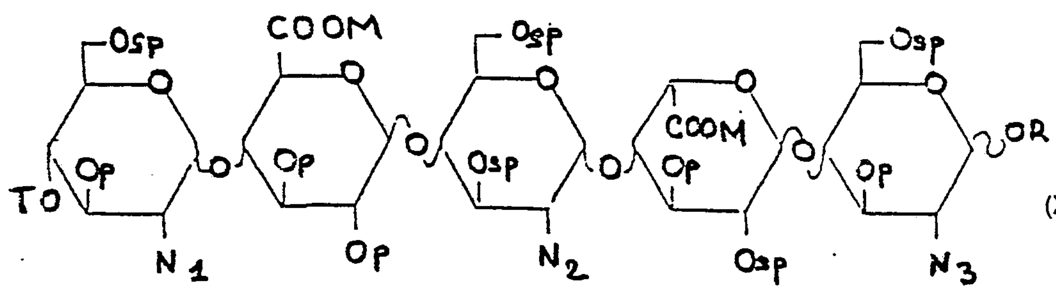

(XXIV)

dans laquelle les différents symboles présentent les significations préférées ci-dessus, $N_1$, $N_2$, $N_3$ pouvant être identiques ou différents les uns des autres et choisis parmi les significations déjà données.

24. Oligosaccharides correspondant aux produits selon l'une quelconque des revendications 17 à 23, mais dans lesquels un, plusieurs ou tous les groupes —OH sont libérés au cours du procédé de synthèse et/ou comprenant un ou plusieurs groupes fonctionnels, à l'exclusion du disaccharide [2-N-sulfate (ou 2-N-acétyl)-6-O-sulfate-D-glucosamine] - acide méthyl-D-glucuronique, ces groupements fonctionnels étant constitués, de préférence, par des esters, et se présentant plus spécialement sous forme d'anions minéraux, en particulier des esters sulfates ou des esters phosphates, ces groupes fonctionnels étant portés par une ou plusieurs fonctions alcool primaire et/ou alcool secondaire et/ou amine primaire.

25. Oligosaccharides selon la revendication 24, caractérisés en ce qu'ils comportent

— des motifs a substitués en position 6 et/ou 3 par des esters, avantageusement sous forme de sels avec un cation minéral ou organique, en particulier un cation métallique, notamment un cation alcalin, notamment du sodium, ou encore un cation dérivé d'une base organique azotée, par exemple, du triéthylammonium, lesdits motifs a comportant également de préférence en position 2 un groupe fonctionnel amino primaire, avantageusement substitué par un groupe tel qu'un sulfate ou un groupe acyle, en particulier acétyle, ces oligosaccharides comportant en outre de préférence :

— des motifs b ou c avec des groupes carboxyle libres ou sous forme de sels avec un cation minéral ou organique tel que défini ci-dessus, ou encore protégé comme indiqué ci-dessus, et plus spécialement comportant des motifs c avec un groupe sulfate en position 2 et/ou des groupes sulfate sur les motifs b, les fonctions hydroxyle des cycles pyraniques de ces motifs a, b ou c étant soit libres, soit protégées par des regroupements permanents de type alcoyle, en particulier par des groupes méthyle.

26. Oligosaccharides selon la revendication 24, correspondant aux produits de formule I à XIII et XVIII à XXIV mais dans lesquels les groupes —sp sont remplacés par des anions et de préférence renferment des groupes —NH-acyle, en particulier, —NH-acétyle, ou des groupes —$NHSO_3^-$ sur les motifs a.

27. Oligosaccharides selon la revendication 26, comprenant :

un enchaînement disaccharidique possédant une structure de type BD, DE, EF ou GH et répondant respectivement aux formules (XXV) à (XXVIII) suivantes :

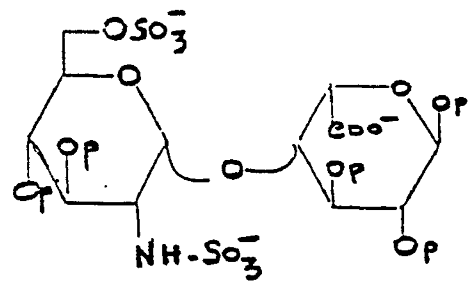

(XXV)

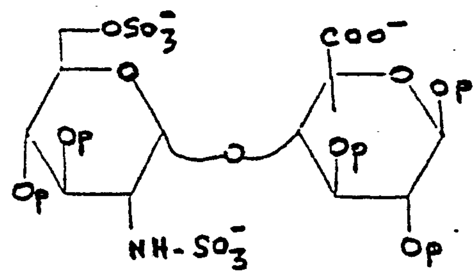

(XXVI)

ou N—H-acyle

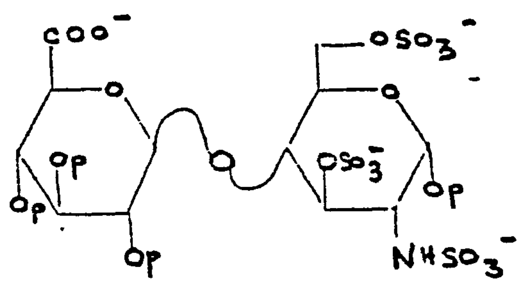

(XXVII)

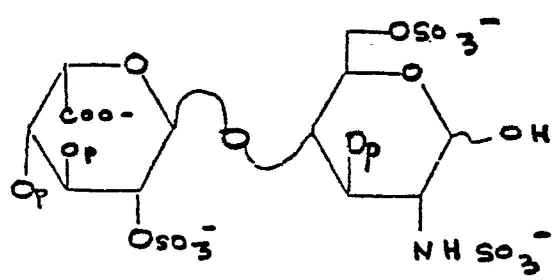

(XXVIII)

— un enchaînement trisaccharidique de structure DEF ou FGH respectivement de formules (XXIX) et (XXX) suivantes :

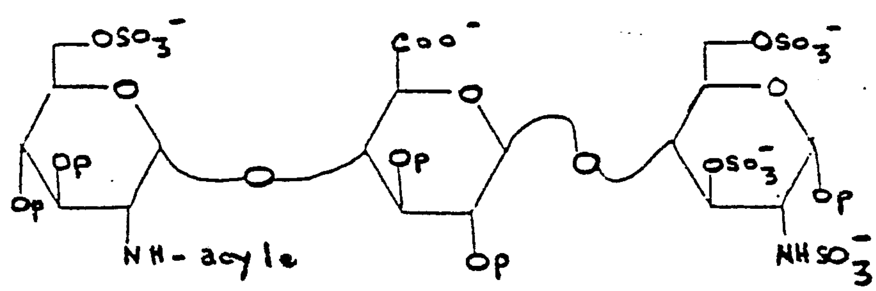

(XXIX)

ou $NHSO_3^-$

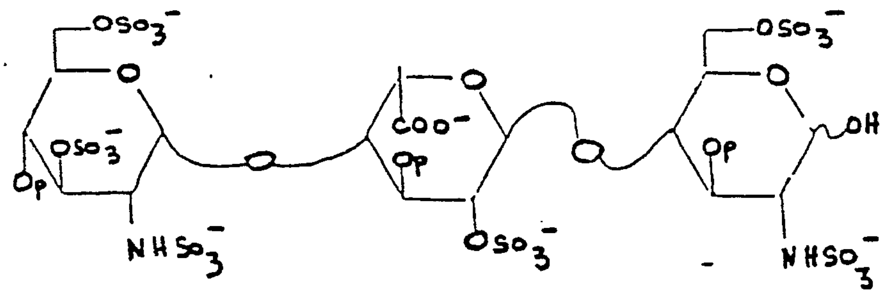

(XXX)

— un enchaînement tétrasaccharidique de structure EFGH.

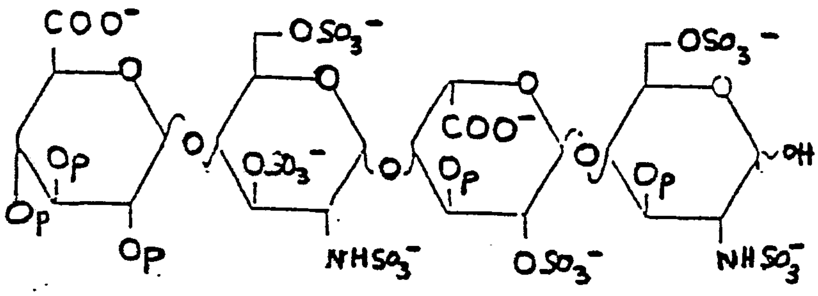

(XXXI)

— un enchaînement pentasaccharidique de structure DEFGH, de formule :

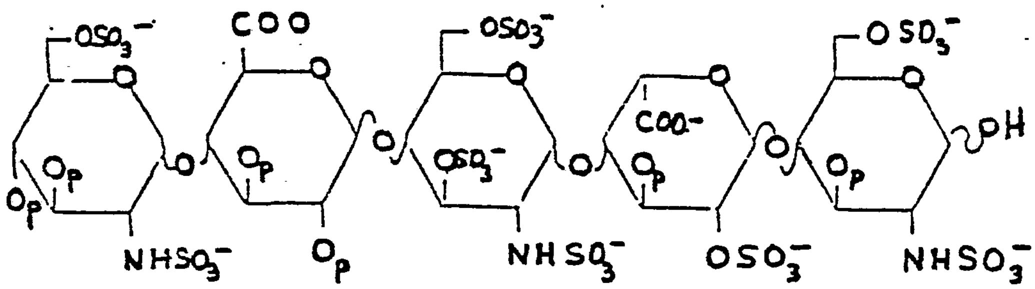

(XXXII)

ou NH-acyle

et/ou un enchaînement hexasaccharidique correspondant à la formule.

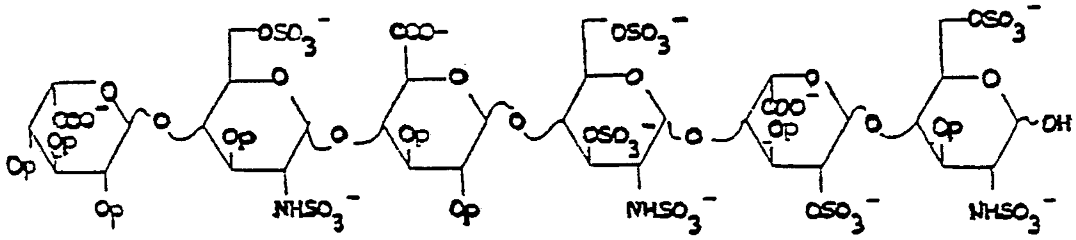

ou NH-acyle

(XXXIII)

28. Oligosaccharides selon la revendication 27, correspondant à l'un des produits de formule (XXV) à (XXXIII) mais renfermant des groupes —OH libres à la place des groupes —Op, le cas échéant, une partie des groupes sulfates étant remplacée par des groupes —OH.

29. Utilisation des oligosaccharides selon l'une quelconque des revendications 23 à 28 comme réactifs biologiques et/ou composés de référence.

30. Compositions pharmaceutiques caractérisées en ce qu'elles renferment une quantité efficace d'un oligosaccharide biologiquement actif selon la revendication 27, en association avec un véhicule inerte, en particulier un oligosaccharide de formule (XXXIII) mais sulfaté et déprotégé et plus spécialement le dérivé 50.

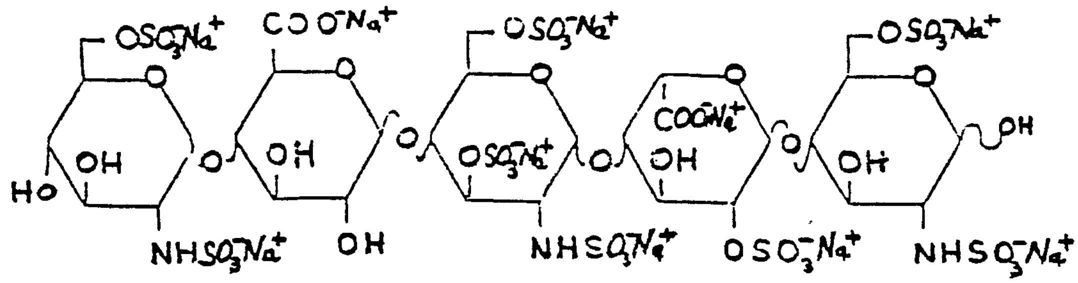

(50)

31. Compositions pharmaceutiques selon la revendication 30, caractérisées en ce qu'elles se présentent sous la forme d'une solution injectable stérile, contenant de 1 000 à 100 000 u Yin-Wessler par ml lorsque ces solutions sont destinées à l'injection par voie sous-cutanée, ou de 500 à 10 000 u Yin-Wessler par ml lorsqu'elles sont destinées à l'injection par voie intraveineuse ou à la perfusion.

**Claims**

1. A process for the organic synthesis of oligosaccharides comprising [D-glucosamine] - [D-glucuronic acid or L-iduronic acid] enchainments, or the reverse, such as encountered in heparin or heparan-sulfate, comprising :

— using in a glycosylation reaction two compounds constituted or terminated respectively by A unit of D-glucosamine structure, and U unit of D-glucuronic acid or L-iduronic acid structure, one of the units A or U being an alcohol in which the —OH group of the alcohol function occupies position 4, the other unit having an activated anomeric carbon, substituted by a reactive group compatible with the other groups present on the units ; all the positions of A and U excepted the one whose anomeric carbon is activated or the one occupied by the —OH group of the alcohol function, bearing either amino groups or carboxyl groups, or precursors of such groups, or again —OH groups, said groups occupying determined positions, the amino and carboxyl groups, when they are present, being respectively blocked by protective groups of amino and carboxyl functions, the —OH groups being blocked by at least two types of protective groups, sequencially removable while enabling the introduction of given groups at given positions, then the liberation of —OH groups to in other positions,

— repeating, if desired, the glycosylation step to elongate the oligosaccharidic chain,

— sequencially removing the protective groups of —OH groups by introducing desired substitution groups in specific positions, then making free the —OH groups in other specific positions as well as the amino and carboxyl groups, provided that the establishment of the interglycoside linkage does not lead to the obtention of a disaccharide with a [2-N-sulfate or (2-N-acetyl)-6-O-sulphate-D-glucosamine] - [methyl-D-glucuronic acid] structure.

2. A process according to claim 1, wherein the materials with A and U units, contain several types of protective groups, namely (1) one or several semi-permanent groups, i. e. groups removable in the first place after the reactions of glycosylation when the saccharide skeleton includes the number of desired units, without removal or alteration of the other groups present, and (2) one or several permanent groups, i. e. groups capable of maintaining the protection of the —OH radicals during the introduction of the functional groups in place of the semi-permanent groups, said protective groups being selected from radicals such as acyl, alkyl possibly substituted alkyl or aryl radicals.

3. A process according to claim 1 or 2, wherein the protective groups of —OH radicals are selected from the group comprising acyl radicals (particularly acetyl, substituted alkyl such as benzyl) and for two neighboured positions, from the acetals or ketals groups, for example benzylidene, another protection form consisting in blocking two —OH groups under the epoxyde form or 1-6 anhydro bridge.

4. A process according to any one of the preceding claims, wherein the products used in the glycosylation reaction contain several types of protective groups, the protective groups already occupying determined positions on the products used in the glycosylation reaction, or alternatively, being introduced from other groups once the glucidic squeleton is formed, this alternative comprising, for example, the use for the glycosylation of a product A wherein the —OH group at positions 2 and 3 and at positions 1 and 6 are blocked under anhydro form, 2,3-epoxy and 1,6-anhydro respectively, the opening of the epoxy function by sodium azide, enabling a $N_3$ group to be introduced at position 2, said group being an amino function precursor.

5. A process according to anyone of the preceding claims, wherein the —OH radicals of the starting materials intended to be sulfated are protected by acyl groups, in particular acetyl, while the —OH radicals intended to be liberated at the end of the synthesis are protected by a permanent group such as the benzyl group.

6. A process according to anyone of claims 1 to 5, wherein A comprises at the 2 position a nitrogen

group advantageously constituted by groups such as —$N_3$ or —NHCOO—$CH_2C_6H_5$, or any other group constituting a precursor of the amine function or of an amine derivative, in particular —$NHSO_3^-$ or —NH-acyl, more especially —NH—$COCH_3$.

7. A process according to anyone of claims 1 to 6, wherein the carboxyl functions of the U units, are blocked by groups inert with respect to reactions used for the replacement of the protective groups and removable at the end of the synthesis to liberate the carboxyl groups, possibly for the purposes of salt formation, these protective groups of carboxyl function being selected advantageously from among alkyl radicals or aryl radicals.

8. A process according to claim 1, wherein the A and U units of the sequence formed include temporary protective groups, wherein the groups are capable of selectively blocking a position of the A or U unit intended to take part in a novel glycosylation reaction, these groups being removable in the presence of the other groups present on the units of the starting products to recreate an alcohol.

9. A process according to claim 8, wherein for a U-A disaccharide lengthening towards the left, a U unit comprising a temporary group is used and that for lengthening to the right a A unit, comprising said temporary group is used, enabling the obtention by successively carrying out glycosylation reaction of enchainment $U_wA_xU_yA_z$ in which the sum of the indices in comprised between 2 and 12, these values being included in the range, where w and y cannot be nil simultaneously, regular enchainments being of the type U $(AU)_n$, $(AU)_nA$, $(UA)_n$ or again $(AU)_n$ with n equal to 1 $\leq$ to $\leq$ 6.

10. The process according to anyone of claims 1 to 9, wherein the alcohol function of one of the units A or U involved in the glucidic sequence already formed is advantageously liberated from its temporary protective group, for example

— from an allyl group, by a treatment such as one comprising first the use of an isomerizing agent such as Pd, Rh and Ir derivatives, in particular rhodium tris-triphenylphosphine chloride (I), or again potassium tertio-butoxide, then under acid conditions, in particular with a mixture of mercuric oxide and mercuric chloride, or

— by saponification from an —O-acyl group, in particular —O-acetyl or —O-chloracetyl, these radicals being removable to make free an —OH function, for example, by means of thiourea in a solvent medium, advantageously at a temperature higher than 80 °C, preferably of the order of 100 °C.

11. A process according to anyone of claims 1 to 10, wherein the above alcohol is reacted with a reactive derivative such as a halide, advantageously a chloride or a bromide, an imidate or an orthoester,

— the condensation reaction between the halide and the alcohol being advantageously of the Koenigs-Knorr type and carried out in a solvent medium, more especially in an organic solvent, particularly of the dichloromethane or dichloroethane type, advantageously in the presence of a catalyst generally a silver or mercury salt, for example, silver trifluoromethane sulphonate, commonly called silver triflate, silver carbonate, silver oxide, mercuric bromide or mercuric cyanide, and also with a proton acceptor such as sym-collidine and an extractor for the water possibly present and/or for the halohydric acid formed, for example 0,4 nm molecular sieves, at room temperature or again at a lower temperature which can reach 0 °C or less, in an atmosphere of an inert gas such as nitrogen or argon, or alternatively for forming covalent bonds between alcohols of various structures and an L-idose precursor of the L-iduronic acid, the condensation reaction is carried out by using as catalyst mercuric derivatives, in particular cyanide and/or mercuric bromide, molecular sieves particularly 0,4 nm molecular sieves, in an organic solvent selected according to the reactivity of the alcohol,

— the condensation with an orthoester such as a 1,2-O-methoxy-ethylidene group being preferably carried out at a temperature above 100 °C in a solvent medium of the chlorobenzene type or any other solvent whose boiling point exceeds 100 °C and is advantageously between 100 and 150 °C in the presence of a catalyst such as 2,6-dimethyl pyridinium perchlorate,

— the condensation with an imidate being carried out at low temperature, more especially at a temperature below or equal to about 0 °C, in a solvent medium, such as dichloromethane, in the presence of a 0,4 nm molecular sieve and a catalyst such as boron trifluoride etherate.

12. A process according to claim 1, wherein the developed glucidic chain is subjected to one or several chemical reactions in order to introduce a given type of functional group or, successively several types of groups, then to form, if desired, derivatives of these functional groups.

13. A process according to claim 12, wherein the functionalization step is carried out by eliminating only a part of the protective groups and/or certain precursor groups of the amino derivatives or again the whole of the protective groups and/or of the precursor groups and by introducing in their place a given type of substituent or successively different substituents, then by releasing a portion or all of the —OH groups still blocked, if desired.

14. A process according to claim 12 or 13, wherein the functionalization step is carried out selectively so as to introduce on the chain, successively, several types of substituents, particularly the sulfate group on predetermined positions of the units, to form at the 2 position of the A units an amino derivative and in the 6 position of U units, an acid derivative, and to free the —OH radicals at other positions, said functionalization step being carried out by using derivatives in which the semi-permanent groups occupying positions intended to be sulfated are constituted by —O-acetyl groups, the positions corresponding to an —OH group intended to be liberated, being occupied by permanent groups constituted by benzyl groups, the 2 positions of the A units being substituted by groups such as $N_3$ or

NH—COO—$CH_2$-$C_6H_5$ and the 6 positions of the U units being occupied by carboxyl groups protected by an alkyl radical, in particular methyl.

15. The process according to claim 14, wherein the functionalization step comprises

— the selective introduction of the sulfate groups after having eliminated the —O-acetyl blocking group by a saponification reaction carried out by means of a strong base such as soda, preferably at a temperature below ambient temperature and more especially close to 0 °C, the product resulting from the hydrolysis being then subjected to the action of an alkylation agent in order to introduce, on the carboxyl group, the protected alkyl groups which are found to be removed on hydrolysis, said alkylation being followed by a sulfation treatment to introduce sulfate groups at the positions released by hydrolysis and left free after the action of the alkylation agent, said sulfation comprising the utilization of a sulfation agent, such as a trimethylamine/$SO_3$-complex, in a solvent medium, more especially in a solvent such as dimethylformamide, preferably at a temperature higher than room temperature, generally in the vicinity of 50 °C, which corresponds to a reaction time of about 12 hours,

— the liberation of the —OH groups blocked by the benzyl radicals, by removal of the benzyl groups, by catalytic hydrogenation under conditions compatible with the maintenance of the sulfate groups and the conversion of the nitrogenous groups into amino functional groups, preferably under hydrogen pressure in the presence of a catalyst of the Pd/C type, in an organic solvent medium, in particular alcoholic, with water,

— the formation of N-acetyl group by submitting the product resulting from the hydrogenation reaction to an acetylation agent such as acetic anhydride, the reaction being advantageously carried out at a basic pH, in particular close to 8 in an aqueous medium, or of N-sulfate group by means of a sulfation agent of the above-indicated type, at a pH higher than 9, advantageously of the order of 9-10,

— the liberation of the carboxyl group by addition of a strong base,

— the salification of the carboxyl group by using for example exchange resins with the desired cation, particularly with sodium, potassium, lithium, magnesium, calcium.

16. A process according to anyone of the preceding claims, comprising the use in place of one or several A or U unit, of a structural analog of an A or U unit, such as a neutral sugar or a desoxy-sugar.

17. Oligosaccharides constituting intermediates in the process according to anyone of the preceding claims, comprising a chain based on binary units of structure $(A\text{-}U)_n$ or $(U\text{-}A)_n$ corresponding to enchainments a-b or a-c, (or the reverse),

a, b, c, respectively having the structures

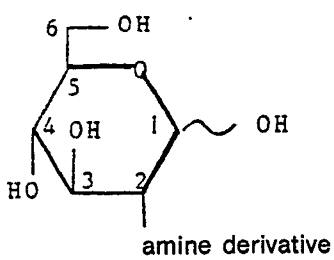

(a) D-glucosamine

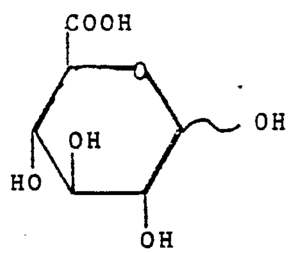

(b) D-glucuronic acid

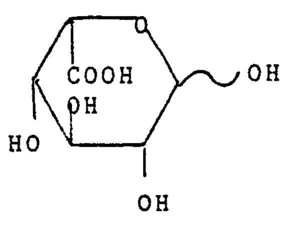

(c) L-iduronic acid

in which n is a number from 1 to 6, said binary units being (1) completely protected and possessing either a reactive group on the anomeric carbon of the unit at the reducing end ; or a single free —OH group on the unit at the non-reducing end, this —OH group occupying the 3, 4 or 6 positions in the case of an A unit and the 2, 3 or 4 positions in the case of U units, or (2) being constituted by completely protected units such as obtained at the end of the glycosylation step, or (3) comprising products in which one or several —OH groups are liberated, said intermediate oligosaccharides optionally containing one or several consecutive a, b, c or again d units and/or one or several units of neutral sugars and/or several desoxy-sugars in their structure.

18. Oligosaccharides according to claim 17 including at least one unit having a structure of the type b 1 $\xrightarrow{\beta}$ 4 a of the formula I

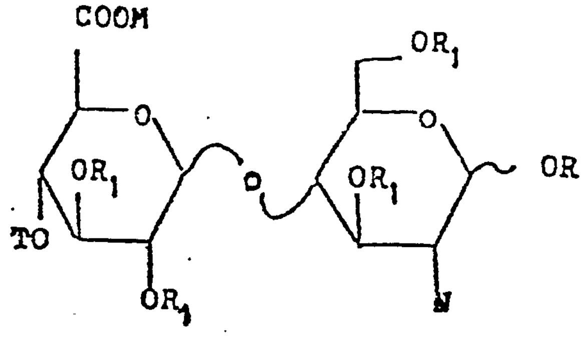

(I)

in which :

— the R radicals, identical or different from one another, optionally together whith R, represent a protective group, in particular a sp semi-permanent group or a p permanent group,

— T, is a temporary group t, or a permanent group p, or a hydrogen atom,

— N, is a nitrogenous group amine or amine derivative precursor,

— R, is an aliphatic or aromatic radical, particularly an alkyl radical comprising from 1 to 4 carbon atoms, or —OR group represents a reactive group such as a halide or again an alkyl radical and

— M, a group blocking the acid function,

and preferably of the formulae (II), (III), or (IV) :

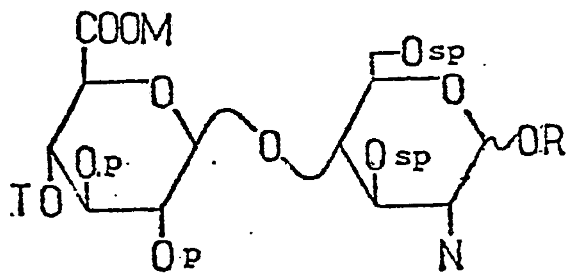

(II)

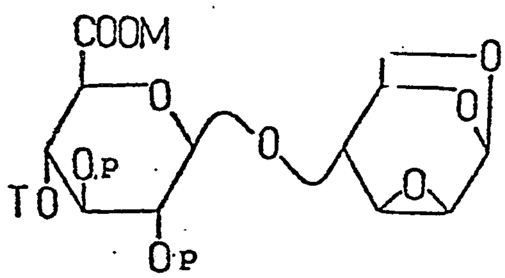

(III)

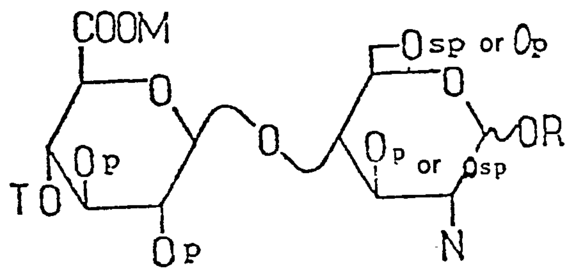

(IV)

in which the various symbols have the above-indicated meanings, the symbols given in the formulae (II) to (IV), having independently, or in combination, the following meanings :

— M represents a hydrogen atom or an alkyl radical, particularly methyl,

— sp, an acyl group, in particular acetyl,

— p, a substituted alkyl group, in particular benzyl,

— R, an acyl group at α or β, in particular an acetyl group, an alkyl radical, in particular methyl or

substituted alkyl, particularly benzyl, or —OR a halogen, in particular a bromide, or again an imidoyl radical,

— N, an azide group,

— T, the group t representing an acyl radical, in particular acetyl, a halogenated acyl radical, in particular a monochloro or trichloroacetyl radical, or the group p, p representing a substituted alkyl radical in particular the benzyl radical, as the case may be itself paramethoxy or again a hydrogen atom.

19. Oligosaccharides including at least one unit having a structure of type a 1 $\xrightarrow{\alpha}$ 4 b of the formula (V).

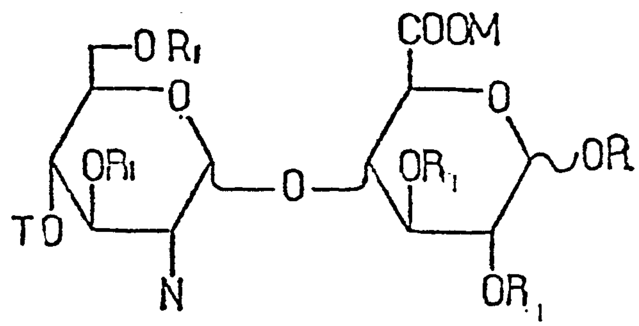

(V)

and preferably of formula (VI) or (VII)

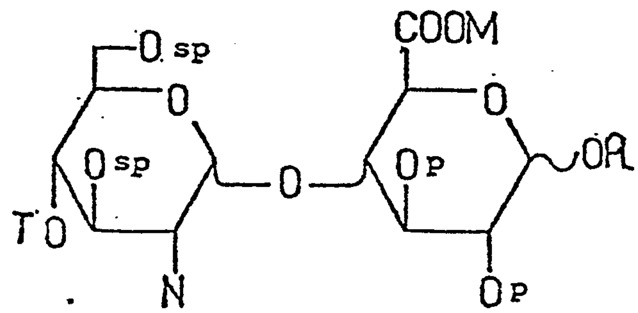

(VI)

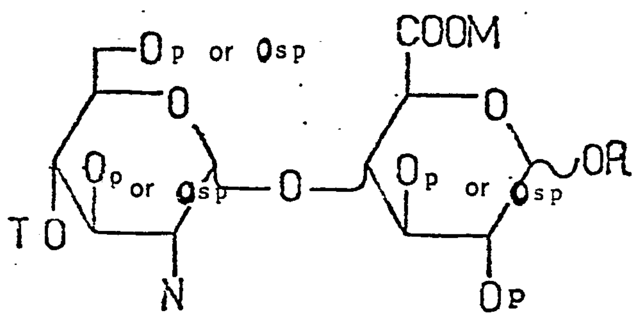

(VII)

in which M, N, sp, p, have preferably the particular meanings given above with respect to formulae (II) to (IV), R further representing a propenyl, allyl, imidoyl, or —H group, with N representing then more especially a —HN-acetyl group.

20. Oligosaccharides according to claim 17, including at least one unit having a structure of the type c 1 $\xrightarrow{\alpha}$ 4 a of the formula (VIII)

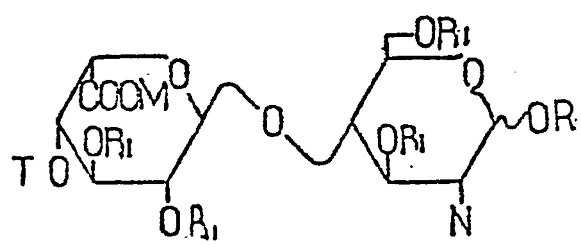

(VIII)

in which the substituents have the meanings given with respect to formula (I) and preferably of the formulae (IX) and (X)

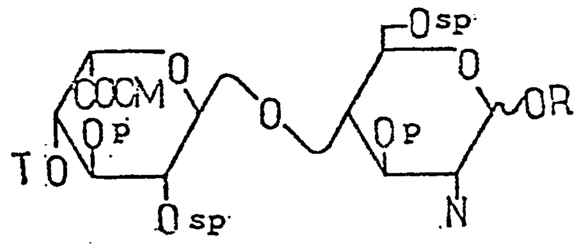

(IX)

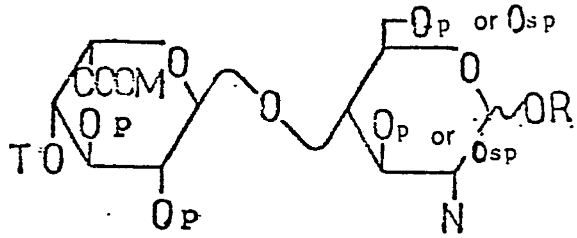

(X)

in which

— the various sp and p groups may be identical and represent an acyl radical, in particular acetyl, or different, as selected from among acyl radicals, in particular acetyl or benzoyl and aryl or substituted alkyl radicals,

— N represents a precursor nitrogen group, possibly different from that present in compounds of formulae (I) to (V), in particular a NHCOO-(substituted alkyl group), particularly a $-NH-COO-CH_2-C_6H_5$ group, which permits subjecting the nitrogenous groups to different treatments and to form different amino derivatives at 2 position of the a units,

— T represents the acetyl, halogenated acyl radical, in particular, monochloro or trichloroacetyl, p-methoxybenzoyl, the symbols p, M and R having advantageously the preferred meanings given above in respect of the formulae (II) to (IV).

21. Oligosaccharides according to claim 17 including at least one unit having a structure of the type a 1 $\xrightarrow{\alpha}$ 4 d of formula (XI)

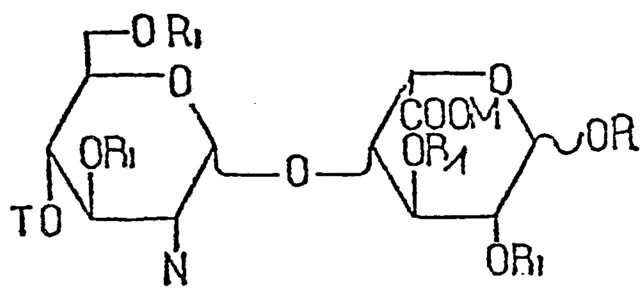

(XI)

in which the substituents have the meanings given above with respect to formula I, and preferably of formula (XII) or (XIII) :

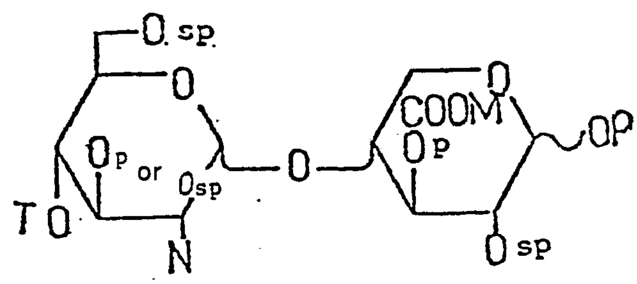

(XII)

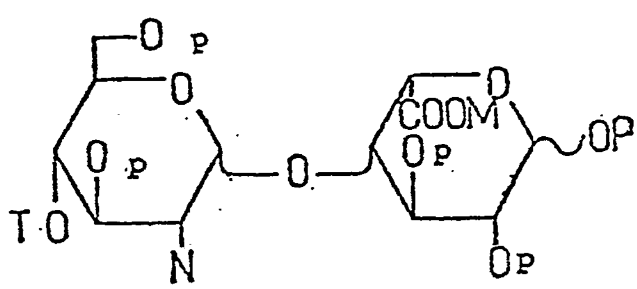

(XIII)

in which the preferred meanings correspond to those given above for formulae (II) to (IV).

22. Intermediate oligosaccharides according to claim 17, corresponding to the products from which the protective groups have been partially removed in the course of synthesis, said products including in particular —OH groups in place of the sp groups.

23. Intermediate products corresponding to oligosaccharides containing structures of ABCDEFG, CDEFGH, AB, BC, CD etc ..., ABC, BCD ..., ABCD, BCDE ..., ABCDE ..., ABCDEF, ABCDEFG or BCDEFGH, with reference to the octosaccharide of structure

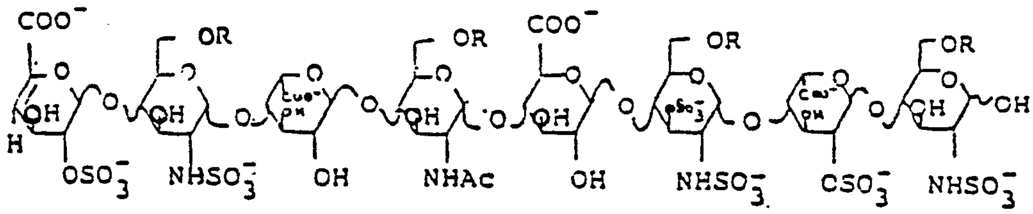

(A) (B) (C) (D) (E) (F) (G) (H)

preferably

— a trisaccharidic structure more especially corresponding to one of the formulae (XVIII) to (XXI) :

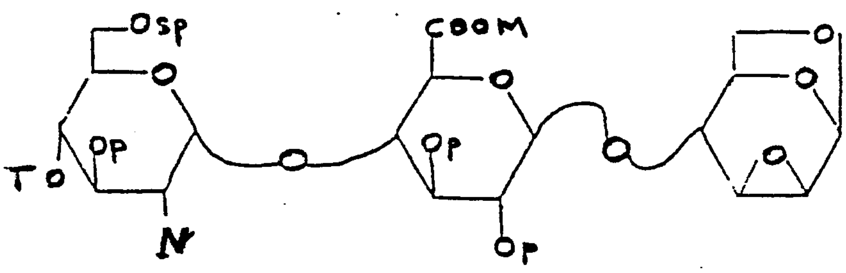

(XVIII)

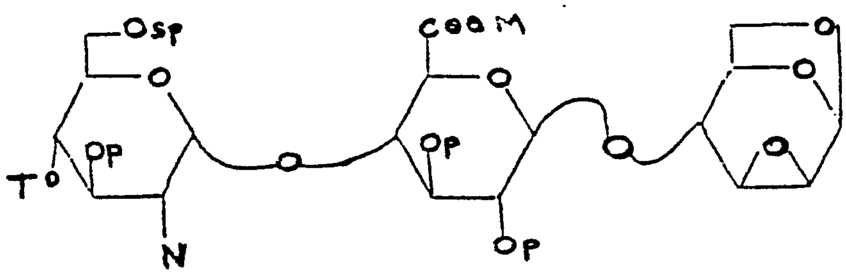

(XIX)

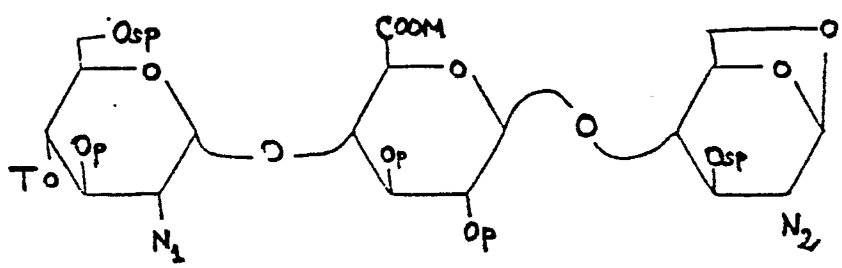

(XX)

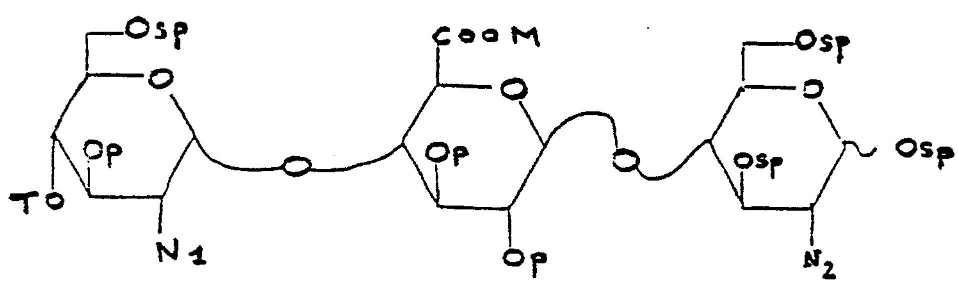

(XXI)

in which the substituents have the above mentioned meanings, $N_1$ and $N_2$ being preferably identical or different from one another and representing an azide or —NH-acyl group in particular —NH-acetyl, or $—NHSO_3^-$, or a structure of the type FGH of formula :

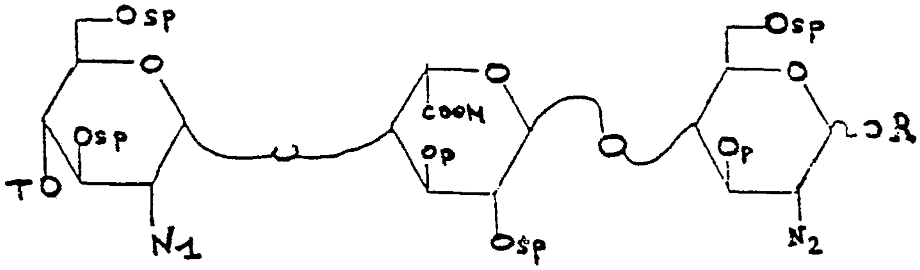

(XXII)

in which the various symbols have the above-given meanings, the two substituents $N_1$ and $N_2$ of the two glucosamine units of structure F and H being identical or again advantageously different, as in the case of natural products, and selected from among the azide or —NH-COO-acyl group, in particular —NH-COO-acetyl or $—NH—COO—CH_2—C_6H_5$,

— a tetrasaccharidic structure, particularly the structure EFGH, corresponding to the following formula

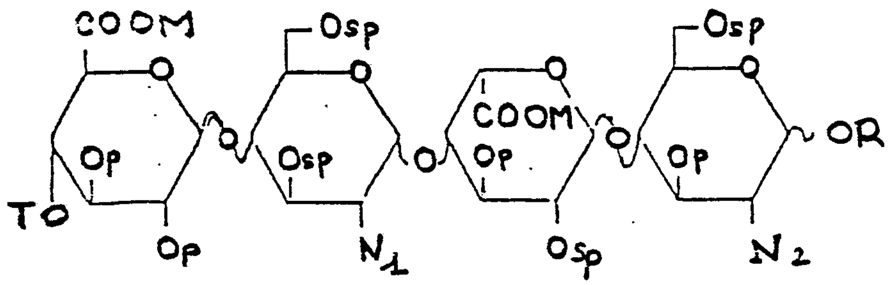

(XXIII)

in which the preferred meanings of the different symbols corresponds to those indicated for formula XXII,

— a pentasaccharidic structure, particularly of the type DEFGH of formula :

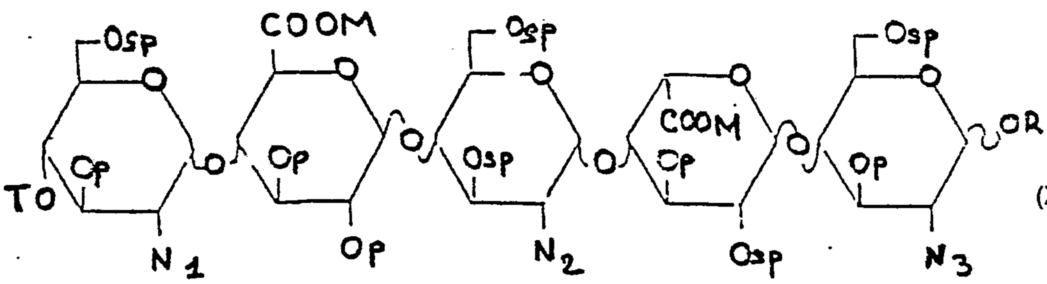

(XXIV)

in which the various symbols have the above-preferred meanings, and $N_1$, $N_2$, $N_3$ can be identical or different from one another selected from among the meanings already given.

24. Oligosaccharides corresponding to the products according to anyone of claims 17 to 23, but in which one, several or all the —OH groups are liberated in the course of synthesis and/or including one or several functional groups, with the exclusion of the disaccharide [2-N-sulfate (or 2-acetyl)-6-O-sulfate-D-glucosamine] - [methyl-D-glucuronic acid], said functional groups being constituted preferably by esters and occurring more especially in the form of inorganic anions, particularly the sulfate esters or phosphate esters, these functional groups being borne by one or several primary alcohol and/or secondary alcohol and/or primary amine functions.

25. Oligosaccharides according to claim 24 including :

— a units substituted at the 6 and/or 3 positions by esters, advantageously in the form of salt with an inorganic or organic cation, in particular a metal cation, particularly an alkali cation especially sodium, or again a cation derived from a nitrogenous organic base, for example triethylammonium, said a units having also preferably at the 2 position primary amino functional group, advantageously substituted by a group such as sulfate or an acyl group, particularly an acetyl group, and, further including preferably

— units b or c with carboxyl groups free or in the form of salt with an organic or inorganic cation such as defined above, or again protected as above mentioned, and more preferably including units c comprising a sulfate group at the 2 position and/or sulfate groups on the b units, the hydroxyl functions of the pyran rings of said units a, b or c being either free, or protected by permanent groups of the alkyl type, in particular by methyl groups.

26. Oligosaccharides according to claim 24, corresponding to the products of formulae (I) to (XVIII) and (XVIII) to (XXIV), but in which the sp groups are replaced by anions, and preferably including NH-acyl groups, in particular $—NHCOCH_3$, or $—NHSO_3$ groups on the a units.

27. Oligosaccharides according to claim 26 including

— a disaccharide chain having a structure of the type BD, DE, EF or GH and corresponding respectively to the following formulae (XXV to XXVIII) :

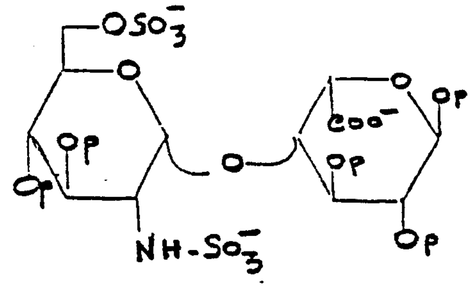

(XXV)

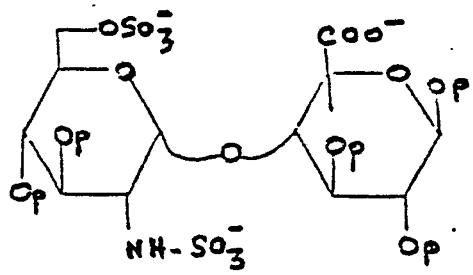

(XXVI)

or N—H-acyle

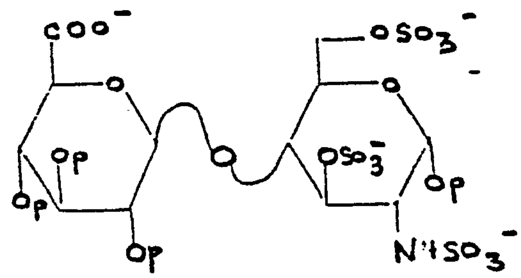

(XXVII)

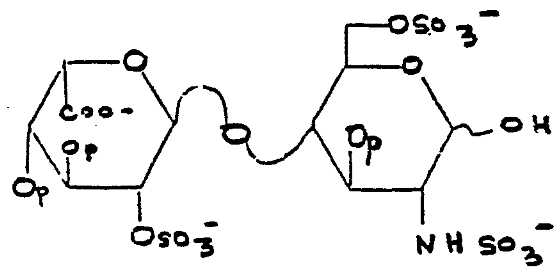

(XXVIII)

— a trisaccharide chain of the structure DEF or FGH respectively, of the following formulae (XXIX) and (XXX) :

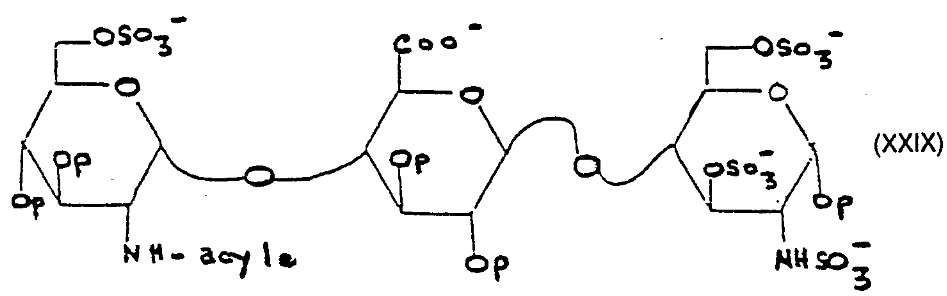

(XXIX)

or $NHSO_3^-$

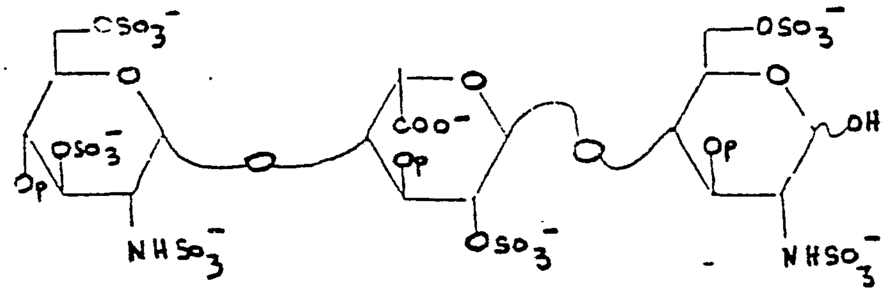

(XXX)

— a tetrasaccharide chain of structure EFGH corresponding to the following formula (XXXI) :

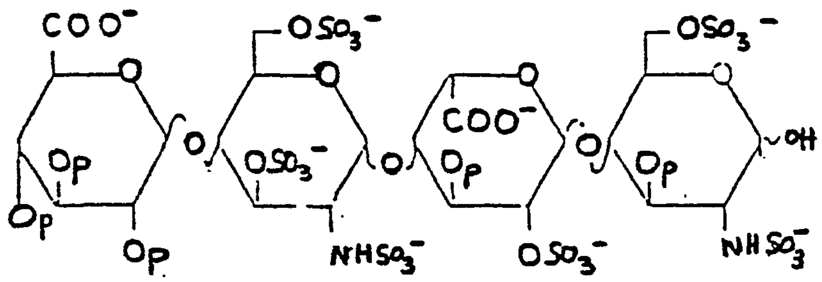

(XXXI)

— a pentasaccharide chain, containing or constituted by pentasaccharides of structure DEFGH having formula (XXXII) :

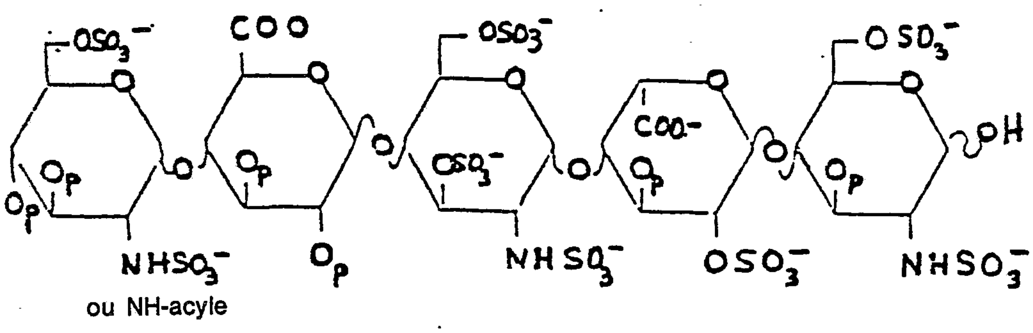

(XXXII)

ou NH-acyle

and/or an hexasaccharide chain corresponding to the following formula (XXXIII) :

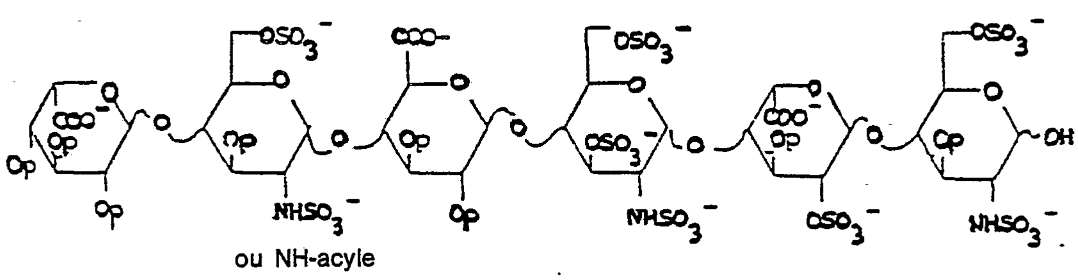

ou NH-acyle

(XXXIII)

28. Oligosaccharides according to claim 27, corresponding to one of the formulae (XXV) to (XXXIII) but containing free —OH groups in place of the —Op groups and having optionally a portion of the $—OSO_3$ groups replaced by —OH groups.

29. The use of the oligosaccharides according to anyone of claims 23 to 28, as biological reagents and/or reference compounds.

30. Pharmaceutical compositions comprising an effective amount of a biologically active oligosaccharide corresponding to an oligosaccharide according to claim 27, in association with an inert carrier, particularly the oligosaccharide of formula (XXXIII) but sulphated and deprotected, more especially derivative 50.

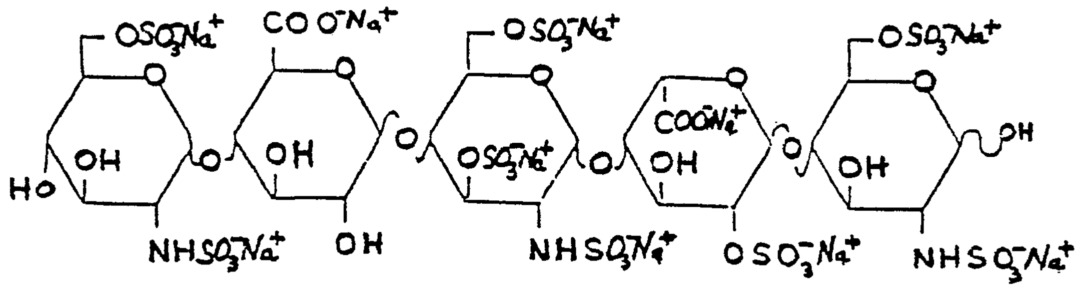

(50)

31. Pharmaceutical compositions according to claim 30, characterized by the fact they are under a sterile injectable solution form, containing 1 000 to 100 000 u Yin-Wessler per ml when said solution are intender for the injection by the sub-cutaneous route, or of 500 to 10 000 Yin-Wessler per ml when they are intended for injection by intravenous route or for the perfusion.

**Patentansprüche**

1. Verfahren zur organischen Synthese von Oligosacchariden mit [D-Glucosamin] - [D-glucuronsäure- oder L-iduronsäure-]-Ketten oder Ketten umgekehrten Aufbaus, wie man sie in Heparin bzw. Heparansulfat antrifft, dadurch gekennzeichnet, daß man

— bei einer Glykosylierungsreaktion zwei Verbindungen, die einen endständigen Rest A mit Glusosaminstruktur und einer endständigen Rest U mit D-Glucuronsäurestruktur oder L-Iduronsäurestruktur tragen bzw. aus solchen Resten aufgebaut sind, wobei einer der Reste A oder U ein Alkohol ist, bei dem die Alkohol-OH-Gruppe in der 4-Position steht und der andere Rest ein aktiviertes anomeres Kohlenstoffatom aufweist, welches durch eine mit den anderen an den Resten vorhandenen Gruppen verträgliche reaktive Gruppe substituiert ist, und sämtliche anderen Positionen von A und U mit Ausnahme jener, dessen anomeres Kohlenstoffatom aktiviert ist, und jener, die durch die Alkohol-OH-Gruppe besetzt ist, entweder Aminogruppen oder Carboxylgruppen oder Vorläufer dieser Gruppen oder auch OH-Gruppen tragen, wobei diese Gruppen bestimmte Positionen besetzen, die Aminogruppen oder Carboxylgruppen, wenn sie vorhanden sind, durch Schutzgruppen für Aminogruppen bzw. Carboxylgruppen blockiert sind, die OH-Gruppen durch mindestens zwei Arten von Schutzgruppen blockiert sind, die nacheinander abgespalten werden können und die Einführung von gewünschten Gruppen in bestimmte Positionen und dann die Freisetzung der OH-Gruppen an anderen Positionen ermöglichen, einsetzt,

— gewünschtenfalls die Glykolysierungsmaßnahme wiederholt, um die Oligosaccharidkette zu verlängern,

— die Schutzgruppen der OH-Gruppen unter Einführung der gewünschten Substituentengruppen an spezifischen Positionen und dann unter Freilegung der OH-Gruppen an anderen spezifischen Positionen sowie der Aminogruppen und Carboxylgruppen nacheinander freisetzt, mit der Maßgabe, daß die Knüpfung der Interglykosydbindung nicht zur Bildung eines Disaccharids mit [(2-N-Sulfat- oder 2-N-Acetyl-)-6-O-sulfat-D-glucosamin] - [methyl-D-glucuronsäure]-Struktur führt.

2. Verfahren nach Anspruch 1, dadurch gekennzeichnet, daß die eingesetzten Produkte mit den Resten A und U, mehrere Arten von Schutzgruppen für OH-Gruppen aufweisen, d.h. (1) eine oder mehrere semipermanente Gruppen, d.h. Gruppen, die nach den Glykosylierungsreaktionen, dann, wenn das Kohlenhydratgerüst die gewünschte Anzahl von Resten aufweist, zunächst ohne Abspaltung oder Änderung von anderen vorhandenen Gruppen abgespalten werden können, und (2) eine oder mehrere permanente Gruppen, d.h. Gruppen, die den Schutz der OH-Gruppen während der Einführung von funktionellen Gruppen an der Stelle der semi-permanenten Gruppen ermöglichen, wobei diese Schutzgruppen aus Resten, wie Acylresten, Alkylresten, gegebenenfalls substituierten Alkylresten oder Arylresten ausgewählt sind.

3. Verfahren nach Anspruch 1 oder 2, dadurch gekennzeichnet, daß die Schutzgruppen für die OH-Gruppen aus der Gruppe ausgewählt sind, die Acylreste (insbesondere den Acetylrest und substituierte Alkylreste, wie den Benzylrest), und für zwei benachbarte Positionen Acetal- und Ketal-Gruppen, beispielsweise Benzylidengruppen, umfaßt, wobei ein weiterer Form des Schutzes darin besteht, eine Blockierung der beiden OH-Gruppen in Form eines Epoxids oder der 1,6-Anhydrobrücke zu bewirken.

4. Verfahren nach einem der vorhergehenden Ansprüche, dadurch gekennzeichnet, daß die bei der Glykosylierungsreaktion verwendeten Produkte mehrere Arten von Schutzgruppen aufweisen, wobei die Schutzgruppen bereits bestimmte Positionen an den für die Glykosylierungsreaktion eingesetzten Produkten besetzen, oder gemäß einer Variante ausgehend von anderen Gruppen nach Bildung des Kohlenhydratgerüstes eingeführt werden, wobei diese Variante beispielsweise darin besteht, bei der Glykosylierung ein Produkt A zu verwenden, bei dem die OH-Gruppen in dem Positionen 2 und 3 und in

den Positionen 1 und 6 durch Wasserabspaltung blockiert sind als 2,3-Epoxid bzw. 1,6-Anhydrid, wobei die Öffnung der Epoxidgruppe durch Natriumazid die Einführung einer $N_3$-Gruppe, die einen Vorläufer der Aminogruppe darstellt, in die 2-Position ermöglicht.

5. Verfahren nach einem der vorhergehenden Ansprüche, dadurch gekennzeichnet, daß die OH-Gruppen der Ausgangsprodukte, die sulfatiert werden sollen, mit Acylgruppen, insbesondere Acylgruppen geschützt sind, während die OH-Gruppen, die am Ende der Synthese freigesetzt werden sollen, durch eine permanente Gruppe geschützt sind, wie die Benzylgruppe.

6. Verfahren nach einem der Ansprüche 1 bis 5, dadurch gekennzeichnet, daß A in der Position 2 eine stickstoffhaltige Gruppe aufweist, beispielsweise eine Gruppe der Formeln $-N_3$ oder $-NHCOO-CH_2-C_6-H_5$ oder irgendeine andere Gruppe, die einen Vorläufer für eine Aminogruppe oder ein Derivat einer Aminogruppe darstellt, insbesondere eine Gruppe der Formel $-NHSO_3^-$ oder eine —NH-Acylgruppe und insbesondere eine Gruppe der Formel $-NH-COCH_3$.

7. Verfahren nach einem der Ansprüche 1 bis 6, dadurch gekennzeichnet, daß die Carboxylgruppen der Reste U durch Gruppen blockiert sind, die gegenüber den Reaktionen inert sind, die zum Ersatz der schützenden und abspaltbaren Gruppen am Ende der Synthese zur Freisetzung der Carboxylgruppen und gegebenenfalls zur Salzbildung durchgeführt werden, wobei diese Schutzgruppen für Carboxylgruppen mit Vorteil aus Alkylresten oder Arylresten ausgewählt sind.

8. Verfahren nach Anspruch 1, dadurch gekennzeichnet, daß die Reste A und U der gebildeten Sequenz temporäre Schutzgruppen aufweisen, d.h. Gruppen, die selektiv eine Position des Restes A oder U, an der eine erneute Glykosylierungsreaktion durchgeführt werden soll, blockieren können, wobei diese Gruppen in Gegenwart von anderen an diesen Resten der Ausgangsmaterialien vorhandenen Gruppen unter Wiederbildung eines Alkohols abgespalten werden können.

9. Verfahren nach Anspruch 8, dadurch gekennzeichnet, daß man zur Verlängerung einer Disaccharideinheit U-A nach links einen Rest U einsetzt, der eine temporäre Gruppe aufweist und zur Verlängerung nach rechts einen Rest A, der die temporäre Gruppe besitzt, so daß man durch aufeinanderfolgende Glykosylierungsreaktionen Ketten der Formel $U_wA_xU_yA_z$ bilden kann, worin die Summe der Indices zwischen 2 und 12 liegt und diese Werte in dem Intervall eingeschlossen sind, wobei w und y nicht gleichzeitig Null sein können, und die normalen Ketten folgende Typen umfassen $U(AU)_n$, $(AU)_nA$, $(UA)_n$ oder auch $(AU)_n$, worin $1 \leq n \leq 6$ ist.

10. Verfahren nach einem der Ansprüche 1 bis 9, dadurch gekennzeichnet, daß die Alkoholgruppe eines der Reste A oder U, die in der bereits gebildeten Kohlenhydratsequenz vorhanden ist, mit Vorteil aus einer temporären Schutzgruppe freigesetzt wird, beispielsweise

— ausgehend von einer Allylgruppe durch eine Art von Behandlung, die die Verwendung eines Isomerisierungsmittels, wie Derivate von Pd, Rh und Ir, insbesondere Tris-triphenylphosphin-rhodium(I)-chlorid, oder auch Kalium-tert.-butoxid, und dann saure Bedingungen umfaßt, insbesondere mit einer Mischung aus Quecksilber(II)-oxid und Quecksilber(II)-chlorid oder

— durch Verseifung ausgehend von einer O-Acylgruppe, insbesondere eine O-Acetylgruppe oder einer O-Chloracetylgruppe, wobei diese Gruppen zur Bildung einer OH-Gruppe beispielsweise mit Hilfe von Thioharnstoff in einem Lösungsmittelmedium, vorzugsweise bei einer Temperatur von oberhalb 80 °C noch bevorzugter von etwa 100 °C, abgespalten werden.

11. Verfahren nach einem der Ansprüche 1 bis 10, dadurch gekennzeichnet, daß man den Alkohol mit einem reaktiven Derivat, wie einem Halogen, vorzugsweise einem Chlorid oder einem Bromid, einem Imidat oder einem Orthoester umsetzt, wobei

— die Kondensationsreaktion zwischen dem Halogenid und dem Alkohol vorzugsweise eine Koenigs-Knorr-Kondensation ist, die in einem Lösungsmittelmedium und insbesondere einem organischen Lösungsmittel, namentlich Dichlormethan oder Dichlorethan, durchgeführt wird,

vorzugsweise in Gegenwart eines Katalysators, im allgemeinen eines Silber- oder Quecksilber-Salzes, beispielsweise Silbertrifluormethansulfonat, im allgemeinen als Silbertriflat bezeichnet, Silbercarbonat, Silberoxid, Quecksilber(II)-bromid oder Quecksilber(II)-cyanid, und in Gegenwart eines Protonenakzeptors, wie sym-Collidin, und eines Akzeptors für gegebenenfalls vorhandenes Wasser und/oder die gebildete Halogenwasserstoffsäure, beispielsweise in Gegenwart von 0,4 nm-Molekularsieben,

bei Raumtemperatur oder darunter, die 0 °C oder weniger betragen kann, unter einer Inertgasatmosphäre, wie Stickstoff oder Argon.

oder gemäß einer Variante zur Bildung von kovalenten Bindungen zwischen den Alkoholen verschiedener Struktur und eines L-Iduronsäurevorläufers, durch Kondensationsreaktion unter Verwendung von Quecksilberderivaten als Katalysator, insbesondere von Quecksilber(II)-cyanid und Quecksilber(II)-bromid, eines Molekularsiebes, insbesondere eines 0,4 nm-Molekularsiebes, in einem organischen Lösungsmittel, welches in Abhängigkeit von der Reaktivität des Alkohols ausgewählt wird,

— wobei die Kondensation mit einem Orthoester, wie einer 1,2-O-Methoxy-ethyliden-Gruppe vorzugsweise bei einer Temperatur oberhalb 100 °C in einem Lösungsmittelmedium, wie Chlorbenzol oder eines analogen Lösungsmittels mit einem Siedepunkt von oberhalb 100 °C und vorzugsweise zwischen 100° und 150 °C, in Gegenwart eines Katalysators, wie 2,6-Dimethylpyridiniumperchlorat durchgeführt wird, und

— die Kondensation mit einem Imidat bei einer niedrigen Temperatur, insbesondere bei einer Temperatur unterhalb oder gleich etwa 0 °C in einem Lösungsmittelmedium, wie Dichlormethan, in

Gegenwart eines 0,4 nm-Molekularsiebes und eines Katalysators, wie Bortrifluorid-etherat durchgeführt wird.

12. Verfahren nach Anspruch 1, dadurch gekennzeichnet, daß die gebildete Kohlenhydratkette einer oder mehreren chemischen Reaktionen unterworfen wird, um einen gegebenen Typ von funktioneller Gruppe einzuführen oder nacheinander mehrere Typen von Gruppen, und gewünschtenfalls dann Derivate dieser funktionellen Gruppen zu bilden.

13. Verfahren nach Anspruch 12, dadurch gekennzeichnet, daß die Stufe der Bildung der funktionellen Gruppen in der Weise durchgeführt wird, daß nur bestimmte Schutzgruppen und/oder bestimmte Vorläufergruppen der Aminoderivate oder auch die Gesamtheit der Schutzgruppen und/oder der Vorläufergruppen abgespalten werden und an ihrer Stelle ein gegebener Typ von Substituent oder nacheinander verschiedene Substituenten eingeführt werden und schließlich gewünschtenfalls ein Teil oder die Gesamtheit der noch blockierten OH-Gruppen freigesetzt wird.

14. Verfahren nach Anspruch 12 oder 13, dadurch gekennzeichnet, daß die Stufe der Einführung der funktionellen Gruppen selektiv in der Weise erfolgt, daß nacheinander mehrere Arten von Substituenten, insbesondere Sulfatgruppen, in bestimmte Positionen der Kette eingeführt werden, um in der Position 2 der Reste A ein Aminoderivat und in der Position 6 der Reste A ein Säurederivat zu bilden, worauf die OH-Gruppen an den anderen Positionen freigesetzt werden, wobei diese Stufe der Bildung der funktionellen Gruppen in der Weise durchgeführt wird, daß Derivate verwendet werden, in denen die semi-permanenten Gruppen jene Positionen besetzen, die sulfatiert werden sollen und durch O-Acetylgruppen gebildet werden, diejenigen Positionen, die einer OH-Gruppe entsprechen und die freigesetzt werden sollen, durch permanente Gruppen besetzt sind, die durch Benzylgruppen gebildet sind, und die Positionen 2 der Reste A durch Gruppen, wie Gruppen der Formeln $-N_3$ oder $-NH-COO-CH_2-C_6H_5$, und die Positionen 6 der Reste U durch Carboxylgruppen besetzt sind, die durch eine Alkylgruppe, insbesondere eine Methylgruppe, geschützt sind.

15. Verfahren nach Anspruch 14, dadurch gekennzeichnet, daß die Stufe der Bildung der funktionellen Gruppen die folgenden Maßnahmen umfaßt

— selektive Einführung von Sulfatgruppen nach der Abspaltung der O-Acetyl-Blockierungsgruppen mit Hilfe einer Verseifungsreaktion, die mit einer starken Base, wie Natriumhydroxid, vorzugsweise bei einer Temperatur unterhalb Raumtemperatur und insbesondere in der Nähe von 0 °C durchgeführt wird, worauf das bei der Hydrolyse gebildete Produkt der Einwirkung eines Alkylierungsmittels unterworfen wird, um an der Carboxylgruppe Alkyl-Schutzgruppen einzuführen, die im Verlaufe der Hydrolyse abgespalten worden sind, wobei diese Alkylierung gefolgt wird von einer Sulfatierungsbehandlung zur Einführung von Sulfatgruppen an den durch die Hydrolyse freigesetzten Positionen und den nach der Einwirkung des Alkylierungsmittels freigelassenen Positionen, wobei die Sulfatierung die Anwendung eines Sulfatierungsmittels, wie eines Trimethylamin/$SO_3$-Komplexes, in einem Lösungsmittelmedium, insbesondere in einem Lösungsmittel, wie Dimethylformamid, vorzugsweise bei einer Temperatur oberhalb Raumtemperatur und im allgemeinen in der Nähe von 50 °C, was einer Reaktionsdauer von etwa 12 Stunden entspricht, umfaßt;

die Freisetzung der durch Benzylreste blockierten OH-Gruppen durch

— Abspaltung der Benzylgruppen vorzugsweise durch katalytische Hydrierung unter Bedingungen, die mit der Beibehaltung der Sulfatgruppen und der Umwandlung der stickstoffhaltigen Gruppen in funktionelle Aminogruppen verträglich sind, vorzugsweise unter Wasserstoffdruck in Gegenwart eines Katalysators des Typs Pd/C, in einem organischen Lösungsmittel, insbesondere einem alkoholischen, mit Wasser versetzten Lösungsmittelmedium,

— Bildung der N-Acetylgruppen durch Behandlung des bei der Hydrierungsreaktion gebildeten Produktes mit einem Acetylierungsmittel, wie Essigsäureanhydrid, wobei diese Reaktion vorzugsweise bei einem basischen pH-Wert, insbesondere in der Nähe von 8, in einem wässrigen Medium durchgeführt wird; oder Bildung von N-Sulfatgruppen mit Hilfe eines Sulfatierungsmittels des oben angegebenen Typs, bei einem pH-Wert von oberhalb 9, vorzugsweise im Bereich von 9 bis 10,

— Freisetzung der Carboxylgruppen durch Zugabe einer starken Base,

— Salzbildung der Carboxylgruppen unter Verwendung von beispielsweise Ionenaustauscherharzen, die das gewünschte Kation tragen, insbesondere von Natrium oder auch von Kalium, Lithium, Magnesium oder Calcium.

16. Verfahren nach einem der vorhergehenden Ansprüche, dadurch gekennzeichnet, daß man anstelle eines oder mehrer Reste A oder U einen Zucker einsetzt, der ein Strukturanalogon des Restes A oder U darstellt, wie einen neutralen Zucker oder einen Desoxy-Zucker.

17. Oligosaccharide, welche Zwischenprodukte bei dem Verfahren nach einem der vorhergehenden Ansprüche darstellen, dadurch gekennzeichnet, daß sie eine Kette auf der Grundlage von binären Resten der Struktur $(A\text{-}U)_n$ oder $(U\text{-}A)_n$, die der Verkettung a-b oder a-c (oder umgekehrt) entsprechen, wobei a, b und c den folgenden Strukturen entsprechen

(Siehe Schema Seite 75 f.)

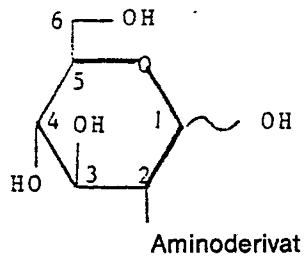

(a) D-Glucosamin

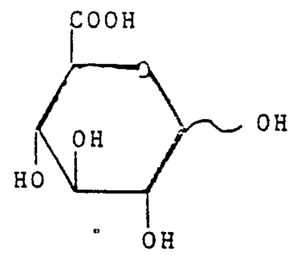

(b) D-Glucuronsäure

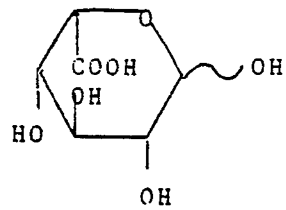

(c) L-Iduronsäure

worin n eine Zahl von 1 bis 6 darstellt, wobei diese binären Reste (1) vollständig geschützt sind und entweder eine reaktive Gruppe am anomeren Kohlenstoffatom des Restes am reduzierenden Ende oder eine einzige freie OH-Gruppe an dem Rest an dem nichtreduzierenden Ende aufweisen, wobei diese OH-Gruppe die Position 3, 4 oder 6 im Fall eines Restes A und die Position 2, 3 oder 4 im Fall eines Restes U besetzt, oder (2) durch vollständig geschützte Reste gebildet sind, wie man sie am Ende der Glykosylierungsstufe erhält, oder (3) Produkte umfassen, bei denen eine oder mehrere OH-Gruppen freigesetzt sind, wobei diese Zwischenprodukt-Oligosaccharide gewünschtenfalls einen oder mehrere folgende Reste a, b, c oder auch d und/oder mehrere Reste von neutralen Zuckern und/oder mehrere Desoxy-Zucker in ihrer Struktur aufweisen.

18. Oligosaccharide nach Anspruch 17, dadurch gekennzeichnet, daß sie mindestens einen Rest mit einer Struktur des Typs b1 $\xrightarrow{\beta}$ 4a der allgemeinen Formel (I)

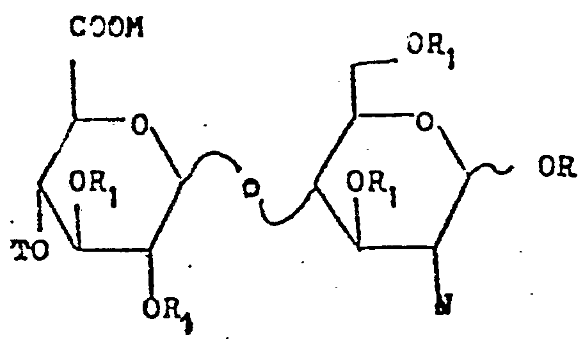

(I)

enthalten, worin

— die Reste $R_1$, die gleichartig oder verschieden sein können, gegebenenfalls zusammen mit R eine Schutzgruppe, insbesondere eine semi-permanente Gruppe sp oder eine permanente Gruppe p,

— T eine temporäre Gruppe t oder eine permanente Gruppe p oder ein Wasserstoffatom,

— N eine stickstoffhaltige Gruppe, die ein Vorläufer eines Amins oder eines Aminderivates ist,

— R einen aliphatischen oder aromatischen Rest, insbesondere einen Alkylrest mit 1 bis 4 Kohlenstoffatomen, oder OR eine reaktive Gruppe, wie ein Halogenid oder auch eine Alkylgruppe und

— M eine die Säuregruppe blockierende Gruppe bedeuten,

und vorzugsweise der Formeln (II), (III) oder (IV)

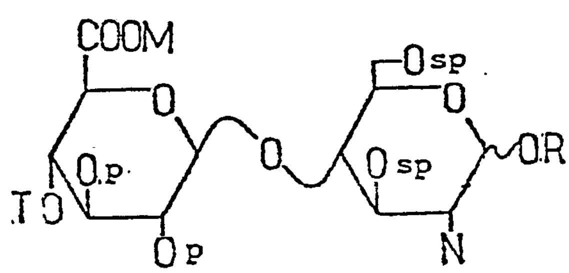

(II)

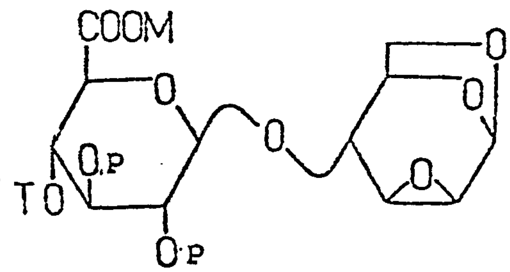

(III)

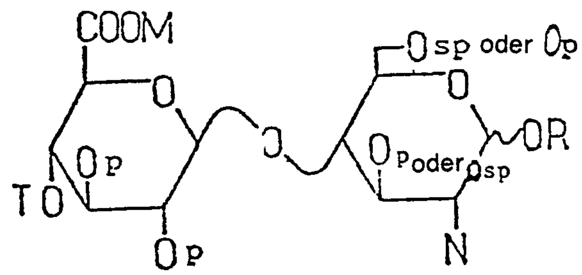

(IV)

worin die verschiedenen Symbole die oben angegebenen Bedeutungen besitzen, wobei die Symbole der Formeln (II) bis (IV) unabhängig voneinander oder in Kombination die folgenden Bedeutungen besitzen :

— M steht für ein Wasserstoffatom oder eine Alkylgruppe, insbesondere eine Methylgruppe,

— sp steht für eine Acylgruppe, insbesondere eine Acetylgruppe,

— p steht für eine substituierte Alkylgruppe, insbesondere eine Benzylgruppe,

— R steht für eine Acylgruppe in der α- oder β-Stellung, insbesondere eine Acetylgruppe, eine Alkylgruppe, insbesondere eine Methylgruppe oder eine substituierte Alkylgruppe, insbesondere eine Benzylgruppe, oder —OR bedeutet ein Halogen, insbesondere ein Bromid, oder auch einen Imidoylrest,

— N steht für eine Azidogruppe,

— T steht für die temporäre Gruppe t, worin t eine Acylgruppe, insbesondere eine Acetylgruppe, eine halogenierte Acylgruppe, insbesondere eine Monochlor- oder Trichlor-acetylgruppe bedeutet, oder die Gruppe p, worin p eine substituierte Alkylgruppe, insbesondere die Benzylgruppe, die gewünschtenfalls p-methoxysubstituiert sein kann, oder ein Wasserstoffatom.

19. Oligosaccharide nach Anspruch 17, dadurch gekennzeichnet, daß sie mindestens einen Rest aufweisen, der eine Struktur des Typs a1 $\xrightarrow{\alpha}$ 4b besitzt, entsprechend der Formel (V)

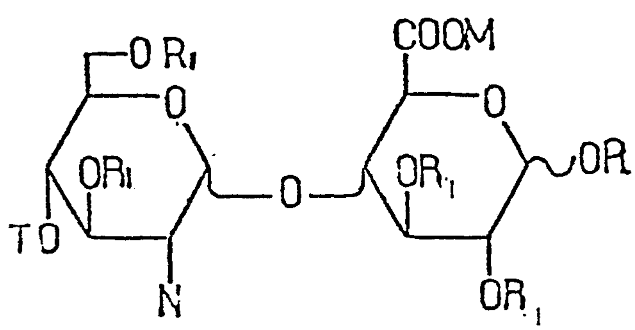

(V)

und vorzugsweise den Formeln (VI) oder (VII)

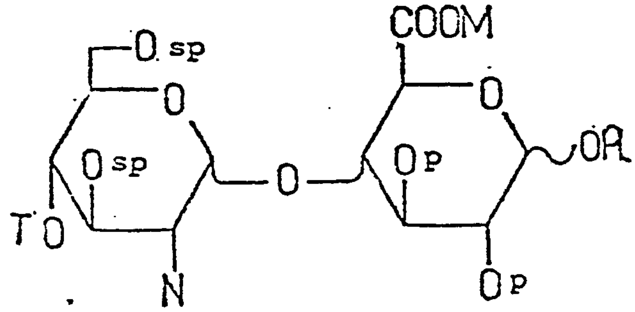

(VI)

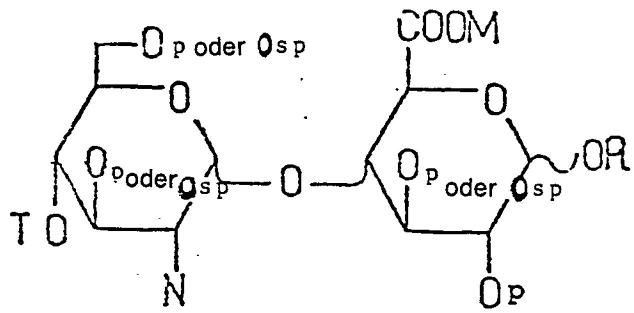

(VII)

(XI)

worin M, N, sp und p vorzugsweise die oben bezüglich der Formeln (II) bis (IV) angegebenen Bedeutungen besitzen, und R zusätzlich vorzugsweise eine Propenylgruppe, eine Allylgruppe, eine Imidoylgruppe oder ein Wasserstoffatom darstellt und N besonders bevorzugt eine NH-Acetylgruppe darstellt.

20. Oligosaccharide nach Anspruch 17, dadurch gekennzeichnet, daß sie mindestens einen Rest der Struktur des Typs c1 $\xrightarrow{\alpha}$ 4a der Formel (VIII) aufweisen

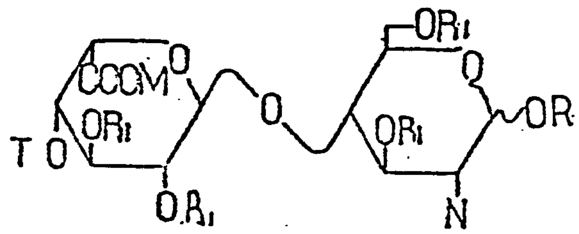

(VIII)

worin die Substituenten die oben bezüglich der Formel (I) angegebene Bedeutungen besitzen, welche Oligosaccharide vorzugsweise den Formeln (IX) und (X)

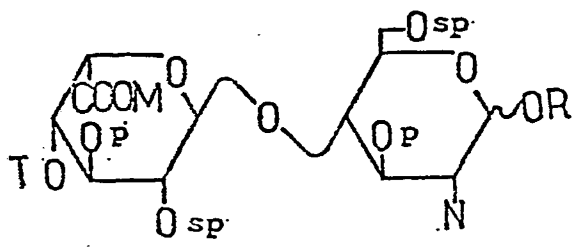

(IX)

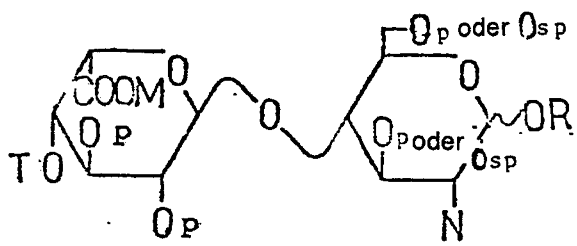

(X)

entsprechen, worin

— die verschiedenen Gruppen sp und p gleichartig sein können und einen Acylrest, insbesondere einen Acetylrest bedeuten, oder verschiedenartig sein können und unter Acylresten, insbesondere Acetyl- oder Benzoyl-Resten und Arylresten oder substituierten Alkylresten ausgewählt sind,

— N eine stickstoffhaltige Vorläufergruppe darstellt, die gegebenenfalls von derjenigen verschieden ist, die in den Verbindungen der Formeln (I) bis (V) vorhanden sind, insbesondere eine Gruppe der Formel —NHCOO-(substituiertes Alkyl), insbesondere eine Gruppe der Formel —NH—COO—$CH_2$—$C_6H_5$, was es ermöglicht, diese stickstoffhaltigen Gruppen verschiedenen Behandlungen zu unterwerfen und verschiedene Aminoderivate in der Position 2 der Reste a zu bilden,

— T bedeutet eine Acetylgruppe, eine halogenierte Acylgruppe, insbesondere eine Monochlor- oder Trichlor-acetylgruppe, eine p-Methoxybenzoylgruppe, wobei die Symbole p, M und R mit Vorteil die bevorzugten Bedeutungen besitzen, die oben unter Bezugnahme auf die Formeln (II) bis (IV) angegeben sind.

21. Oligosaccharide nach Anspruch 17, dadurch gekennzeichnet, daß sie mindestens eine Struktur des Typs a1 $\xrightarrow{\alpha}$ 4c der Formel (XI) enthalten

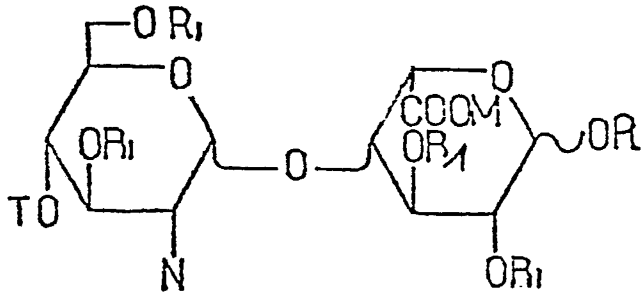

worin die Substituenten die oben bezüglich der Formel (I) angegebenen Bedeutungen besitzen, welche Oligosaccharide vorzugsweise den Formeln (XII) oder (XIII) entsprechen

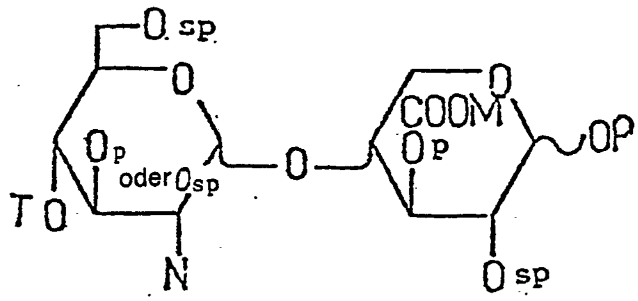

(XII)

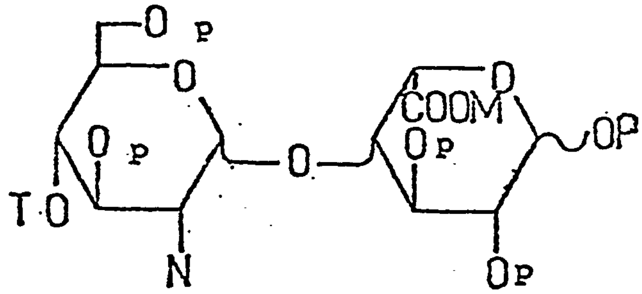

(XIII)

worin die bevorzugten Bedeutungen jenen entsprechen, die oben bezüglich der Formeln (II) bis (IV) angegeben worden sind.

22. Zwischenprodukt-Oligosaccharide nach Anspruch 17, entsprechend jenen Produkten, deren Schutzgruppen im Verlaufe der Synthese teilweise abgespalten worden sind, welche Produkte insbesondere anstelle der Gruppen sp eine OH-Gruppe aufweisen.

23. Zwischenprodukt-Oligosaccharide entsprechend Oligosacchariden, die Strukturen des Typs ABCDEFGH, CDEFGH, AB, BC, CD, usw. ... ABC, BCD, ... ABCD, BCDE ..., ABCDE ..., ABCDEF ..., ABCDEFGH oder BCDEFGH aufweisen, bezogen auf das Octosaccharid der Struktur :

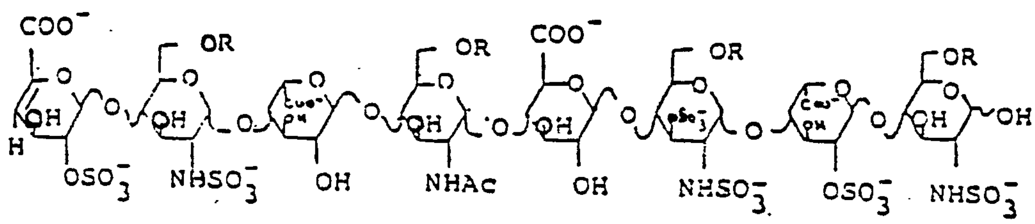

(A) (B) (C) (D) (E) (F) (G) (H)

vorzugsweise mit einer Trisaccharidstruktur, die insbesondere einer der Formeln (XVIII) bis (XXI) entsprechen :

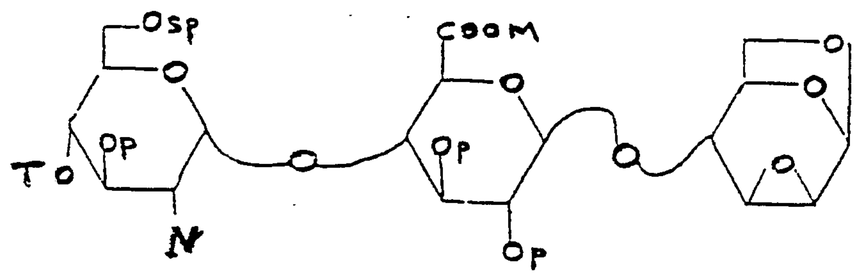

(XVIII)

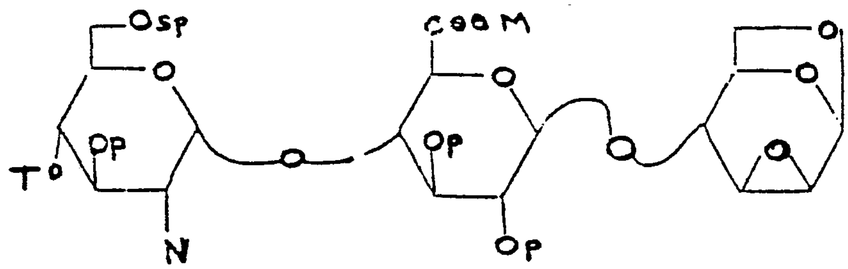

(XIX)

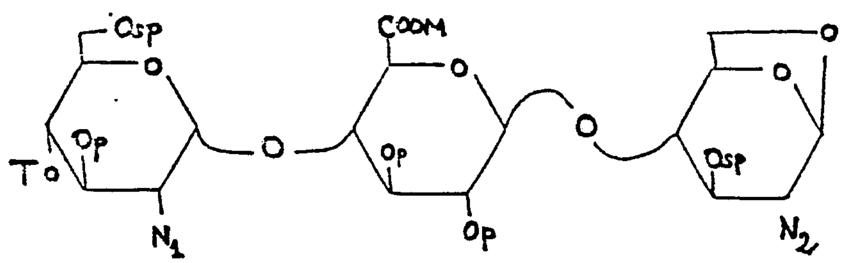

(XX)

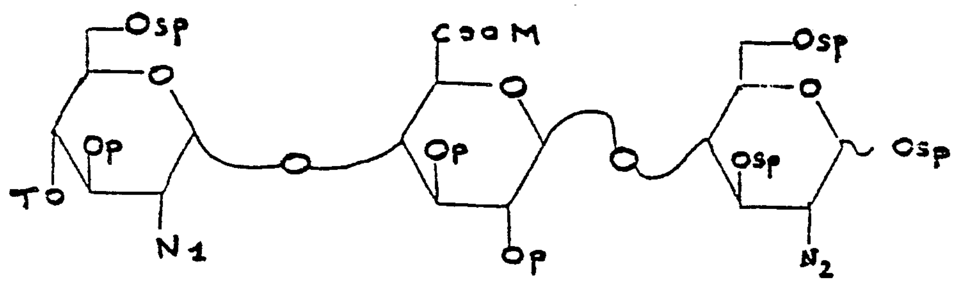

(XXI)

worin die Substituenten die oben angegebenen Bedeutungen besitzen, $N_1$ und $N_2$ vorzugsweise gleichartig oder verschieden voneinander sind und eine Azidogruppe oder —NH-Acylgruppe, insbesonder eine —NH-Acylgruppe oder eine Gruppe der Formel $—NHSO_3^-$ bedeuten, oder mit einer Struktur des Typs FGH der Formel :

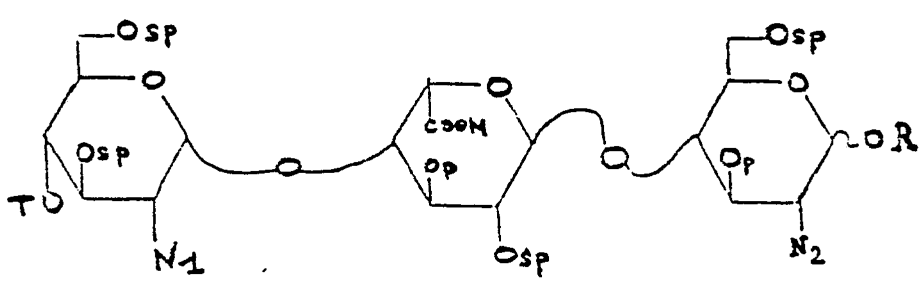

(XXII)

worin die verschiedenen Symbole die oben angegebenen Bedeutungen besitzen, die beiden Substituenten $N_1$ und $N_2$ der beiden Glucosaminreste der Struktur F und H gleichartig oder auch verschieden sind, wie bei den Naturprodukten, und aus Azido-Gruppen oder —NH-COO-Acyl-Gruppen insbesondere —NH-COO-Acetyl- oder $—NH—COO-CH_2—C_6H_5$-Gruppen ausgewählt sind,

mit einer Tetrasaccharidstruktur, insbesondere der Struktur EFGH der folgenden Formel

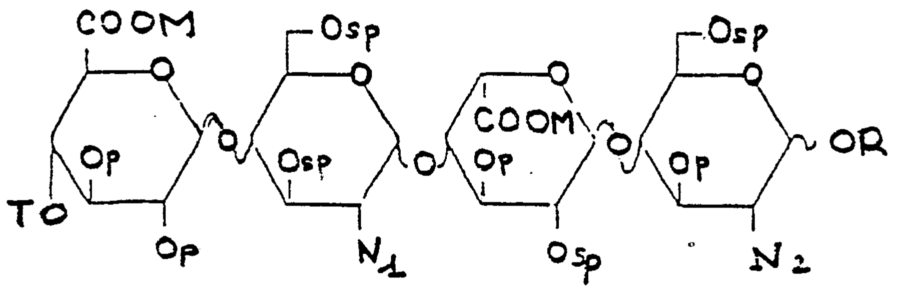

(XXIII)

worin die bevorzugten Bedeutungen der verschiedenen Symbole jenen entsprechen, die für die Formel (XXII) angegeben sind,
einer Pentasaccharidstruktur, insbesondere des Typs DEFGH der Formel

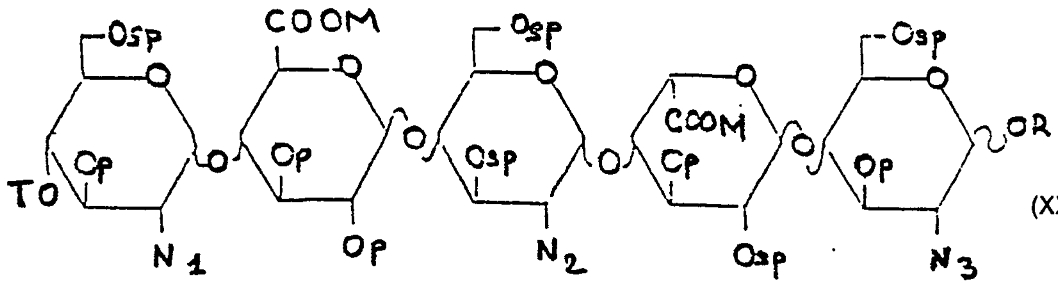

(XXIV)

worin die verschiedenen Symbole die bevorzugten angegebenen Bedeutungen besitzen, $N_1$, $N_2$, $N_3$ gleichartig oder verschieden sein können und aus den oben bereits angegebenen Bedeutungen ausgewählt sein können.

24. Oligosaccharide entsprechend den Produkten gemäß einem der Ansprüche 17 bis 23, bei denen jedoch eine, mehrere oder sämtliche OH-Gruppen im Verlaufe des Syntheseverfahrens freigesetzt worden sind, und/oder eine oder mehrere funktionelle Gruppen aufweisen, mit Ausnahme des Disaccharids [(2-N-Sulfat oder 2-N-Acetyl)-6-O-sulfat-D-glucosamin]-methyl-D-glucuronsäure, wobei diese funktionellen Gruppen vorzugsweise durch Ester gebildet sind, und die insbesondere in Form von anorganischen Anionen, insbesondere von Sulfatestern oder Phosphatestern vorliegen, wobei diese funktionellen Gruppen durch eine oder mehrere primäre Alkoholgruppen und/oder sekundäre Alkoholgruppen und/oder primäre Aminogruppen getragen werden.

25. Oligosaccharide nach Anspruch 24, dadurch gekennzeichnet, daß sie

— Reste a aufweisen, die in der Position 6 und/oder 3 durch Ester substituiert sind, vorzugsweise in Form von Salzen mit einem anorganischen oder organischen Kation, insbesondere einem Metallkation, namentlich einem Alkalikation, insbesondere von Natrium, oder auch einem von einer organischen stickstoffhaltigen Base abgeleiteten Kation, beispielsweise einem Triethylammoniumkation, welche Reste a weiterhin vorzugsweise in der Position 2 eine funktionelle primäre Aminogruppe tragen, die vorteilhafterweise durch eine Gruppe, wie ein Sulfat oder eine Acylgruppe, insbesondere eine Acetylgruppe substituiert ist, welche Oligosaccharide weiterhin vorzugsweise :

— Reste b oder c tragen, die Carboxylgruppen aufweisen, die frei sind oder in Form von Salzen mit einem anorganischen oder organischen Kation, wie es oben definiert wurde, vorliegen, oder die, wie oben angegeben geschützt sind, und die insbesondere Reste c mit einer Sulfatgruppe in der Position 2 und/oder Sulfatgruppen an den Resten b aufweisen, wobei die Hydroxylgruppen der Pyranringe dieser Reste a, b oder c entweder frei sind oder durch permanente Gruppen des Typs Alkylgruppen, insbesondere durch Methylgruppen, geschützt sind.

26. Oligosaccharide nach Anspruch 24, entsprechend den Produkten der Formeln (I) bis (XIII) und (XVIII) bis (XXIV), bei denen die Gruppen sp durch Anionen ersetzt sind die vorzugsweise an den Resten a —NH-Acyl-Gruppen, insbesondere —NH-Acetyl-Gruppen oder Gruppen der Formel $—NHSO_3^-$ aufweisen.

27. Oligosaccharide nach Anspruch 26, enthaltend

— eine Disaccharidkette mit einer Struktur des Typs BD, DE, EF oder GH entsprechend jeweils den folgenden Formeln (XXV) bis (XXVIII) :

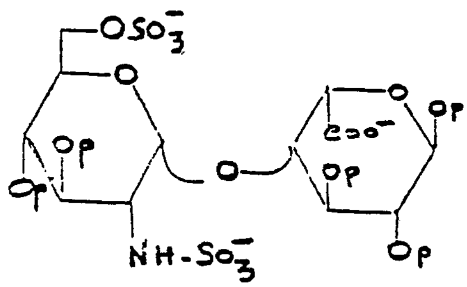

(XXV)

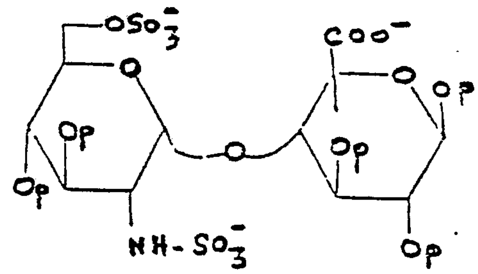

(XXVI)

oder N—H-acyle

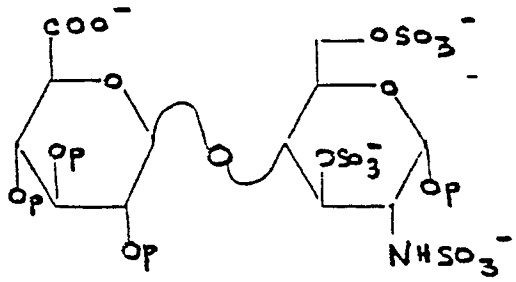

(XXVII)

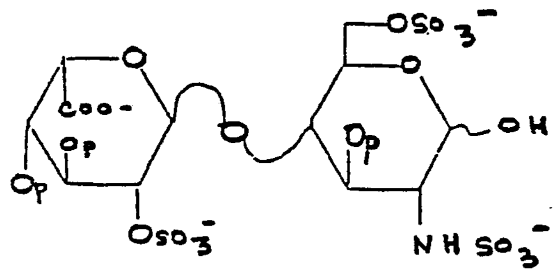

(XXVIII)

— mit einer Trisaccharidkette der Struktur DEF bzw. FGH entsprechend den folgenden Formeln (XIX) und (XXX)

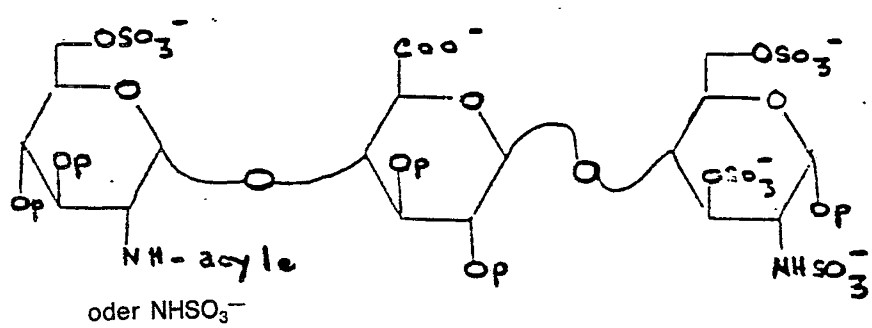

(XXIX)

oder $NHSO_3^-$

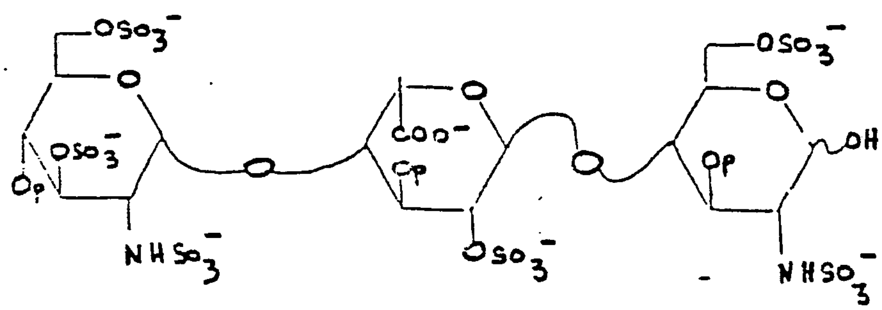

(XXX)

— eine Tetrasaccharidkette der Struktur EFGH

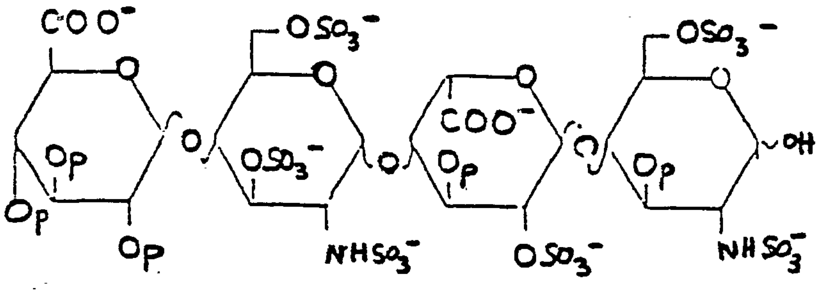

(XXXI)

— einer Pentasaccharidkette der Struktur DEFGH der Formel

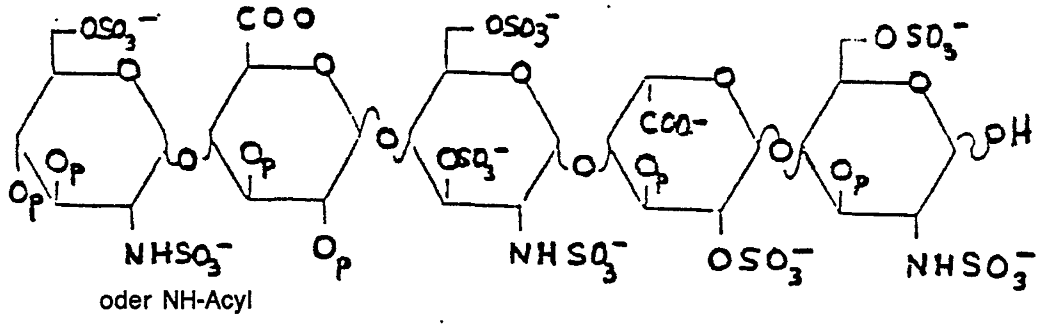

(XXXII)

oder NH-Acyl

und/oder einer Hexasaccharidkette entsprechend der Formel

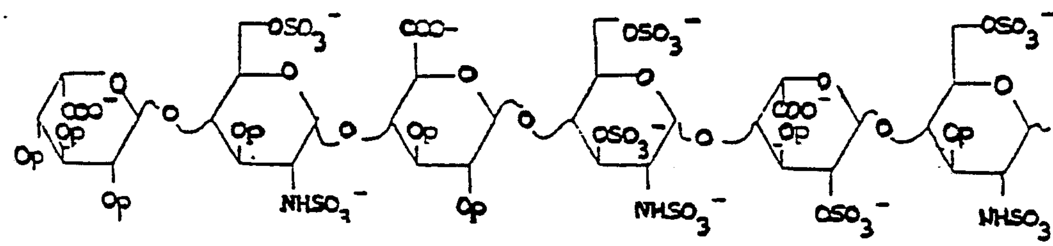

(XXXIII)

oder NH-Acyl.

28. Oligosaccharide nach Anspruch 27 entsprechend einem der Produkte der Formeln (XXV) bis (XXXIII), jedoch enthaltend freie OH-Gruppen anstelle der Gruppen —Op, wobei gegebenenfalls ein Teil der Sulfatgruppen durch OH-Gruppen ersetzt ist.

29. Verwendung der Oligosaccharide nach einem der Ansprüche 23 bis 28 als biologische Reagentien und/oder Referenzpräparate.

30. Pharmazeutische Zubereitungen, dadurch gekennzeichnet, daß sie eine wirksame Menge eines biologisch aktiven Oligosaccharids nach Anspruch 27 in Kombination nach mit einem inerten Trägermaterial, insbesondere ein Oligosaccharid der Formel (XXXIII), welches sulfatiert und von Schutzgruppen befreit ist, und insbesondere das Derivat 50

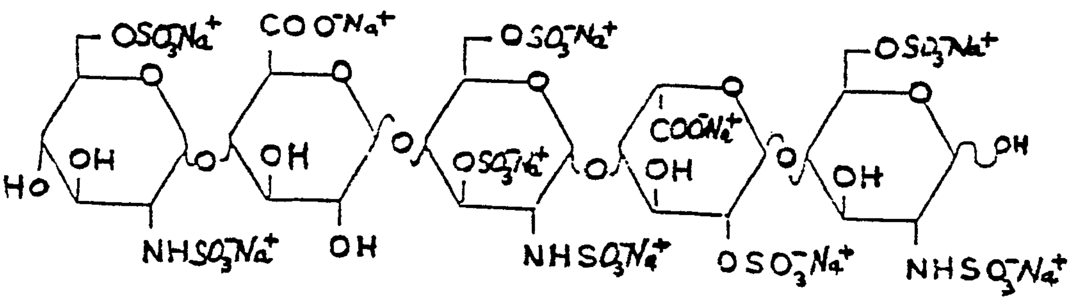

(50).

enthalten.

31. Pharmazeutische Zubereitungen nach Anspruch 30, dadurch gekennzeichnet, daß sie in Form einer sterilen injizierbaren Lösung vorliegen, die 1 000 bis 100 000 Yin-Wessler-Einheiten pro Milliliter enthalten, wenn diese Lösungen auf sub-cutanem Wege injiziert werden sollen, oder die 500 bis 10 000 Yin-Wessler-Einheiten pro Milliliter enthalten, wenn sie auf intravenösem Wege oder durch Perfusion injiziert werden sollen.

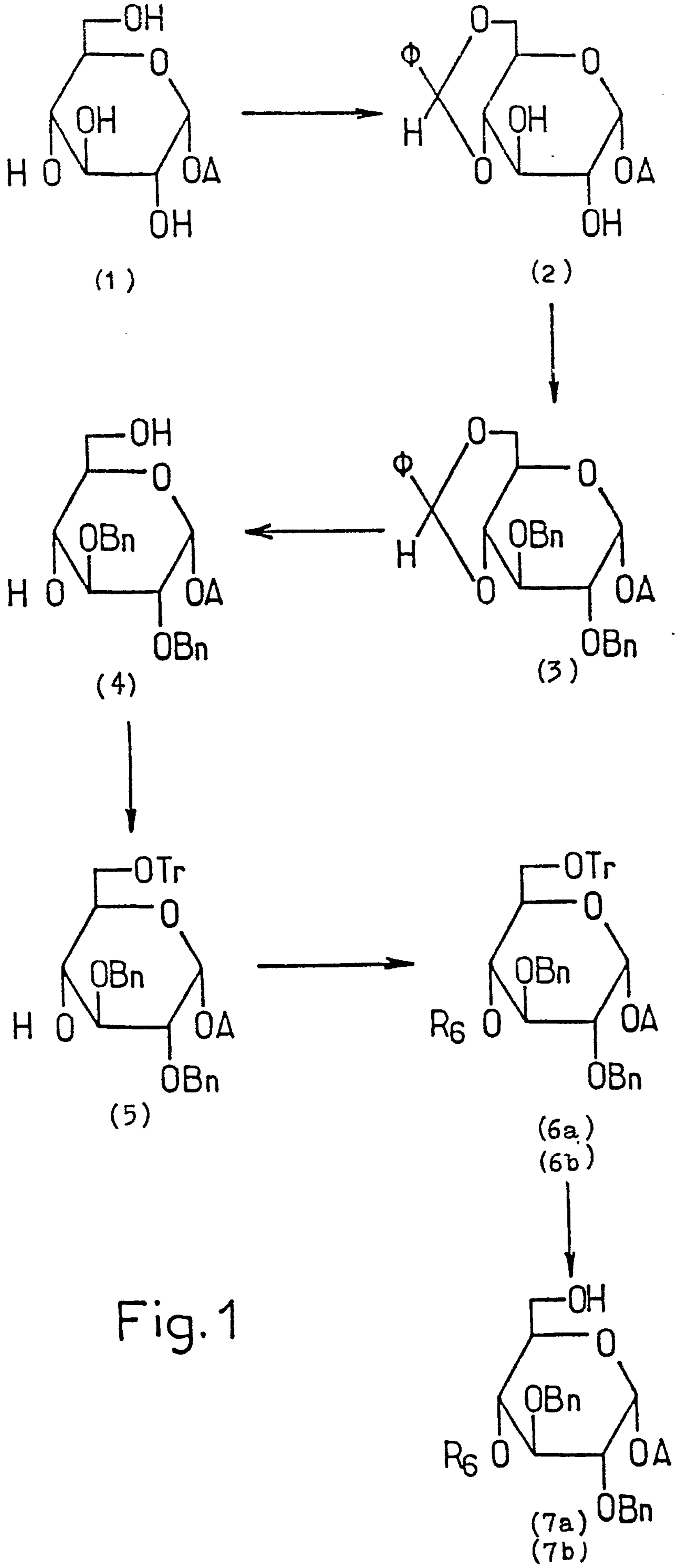

(1)

(2)

(3)

(4)

(5)

(6a)
(6b)

(7a)
(7b)

Fig. 1

Fig. 2

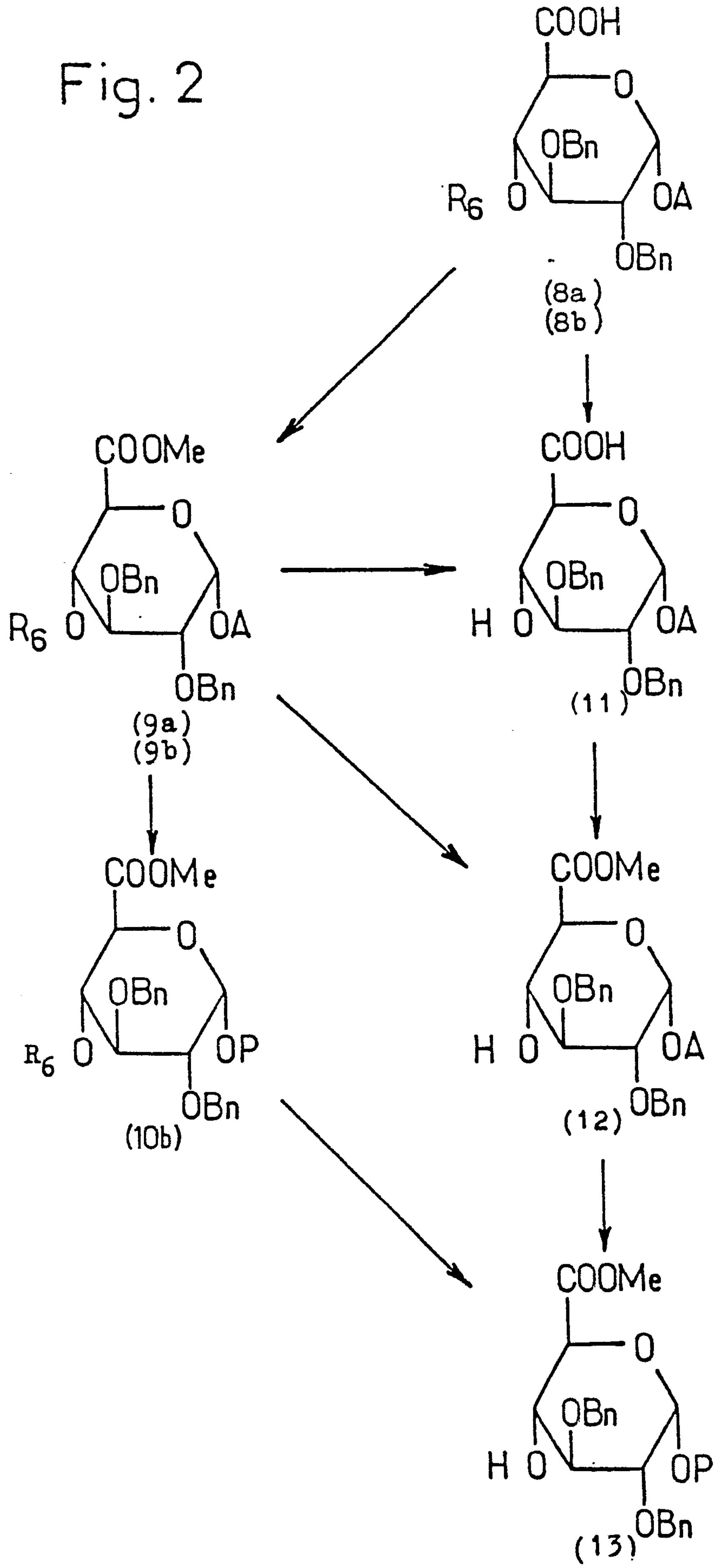

(8a)
(8b)

(9a)
(9b)

(11)

(10b)

(12)

(13)

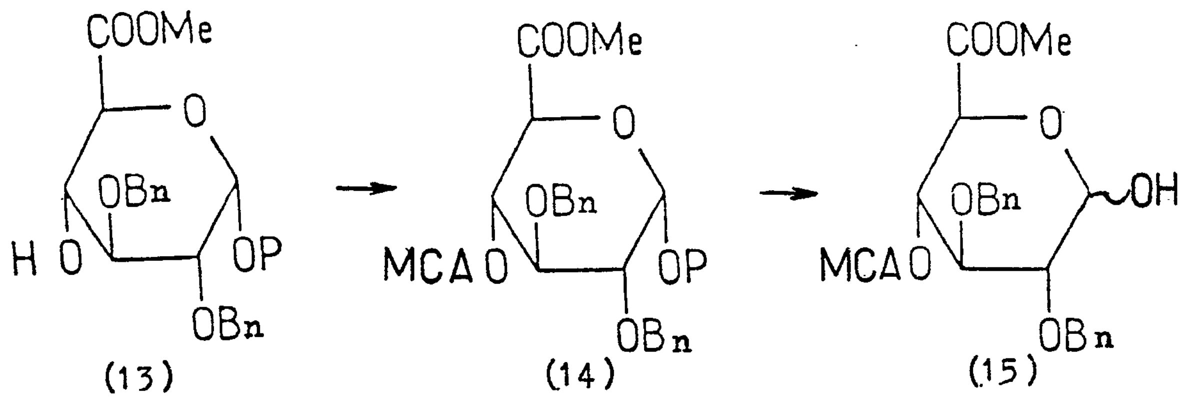

(13) (14) (15)

Fig.3.

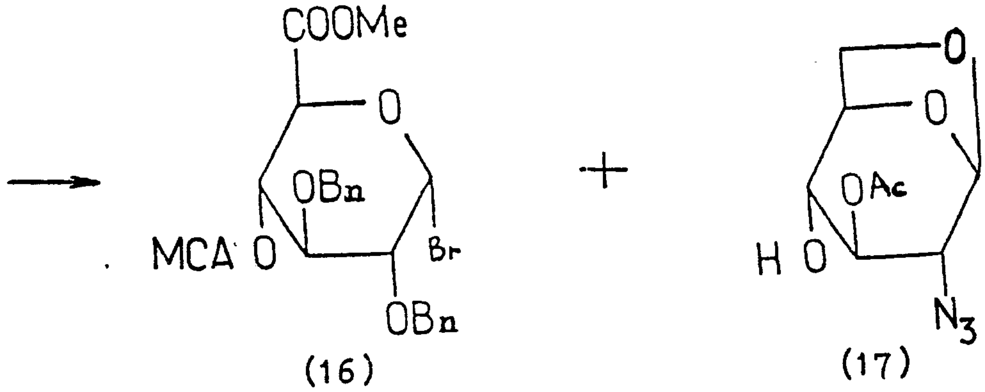

(16) (17)

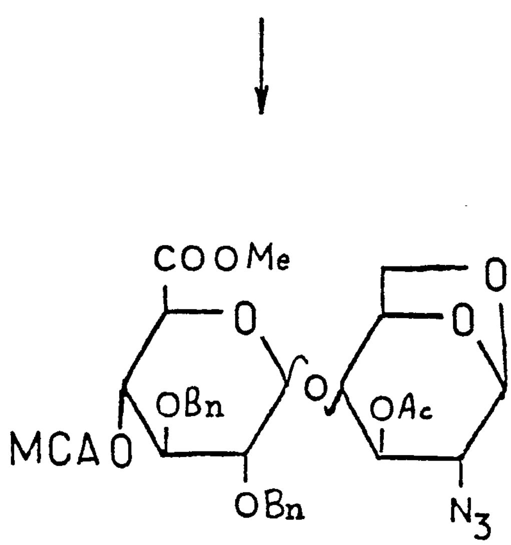

(18)

Fig.4.

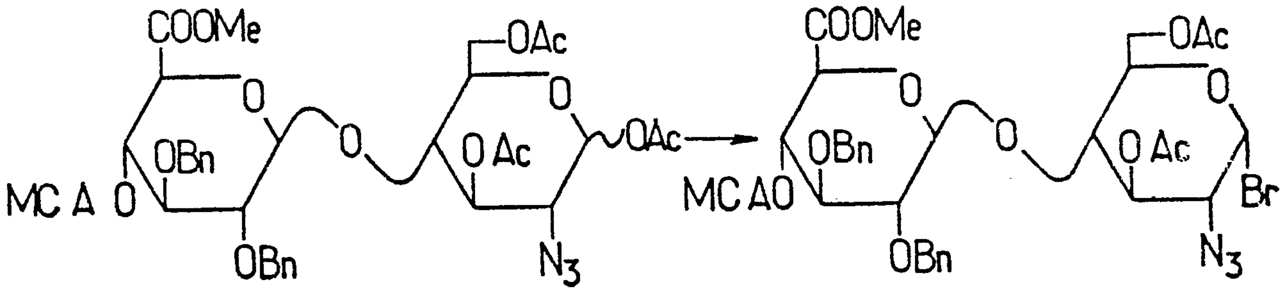

(19) (20)

Fig.5.

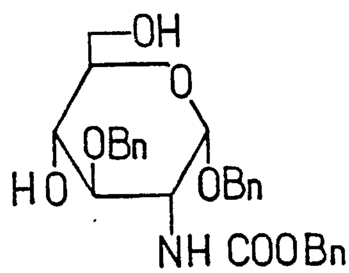

(21)

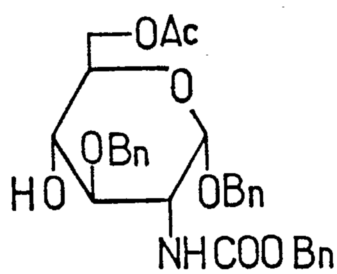

(22)

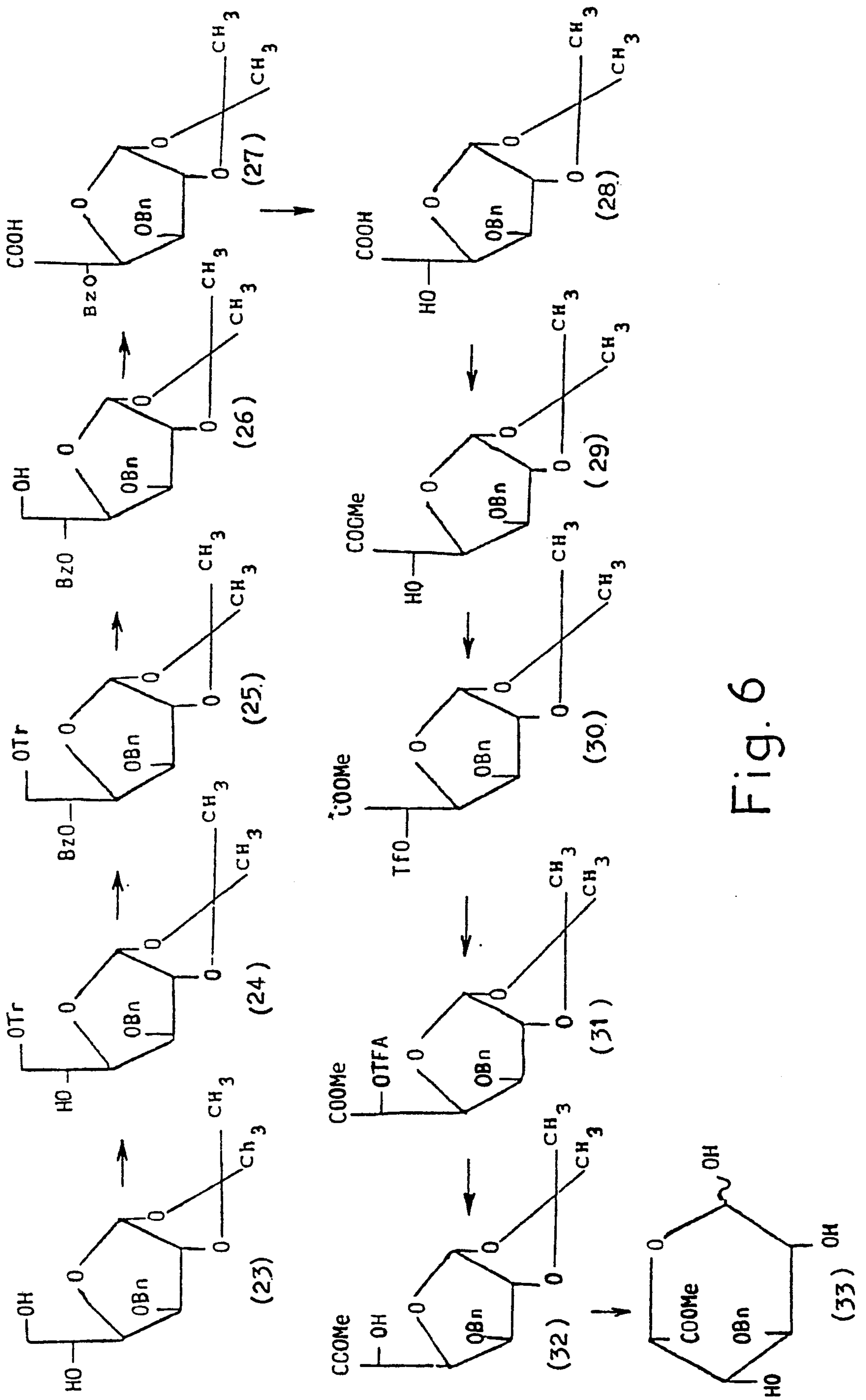

(23) (24) (25) (26) (27) (28) (29) (30) (31) (32) (33)

Fig. 6

Fig.7.

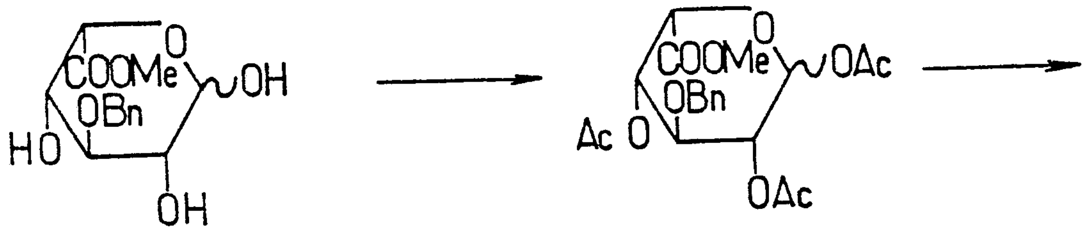

(33) (34,35)

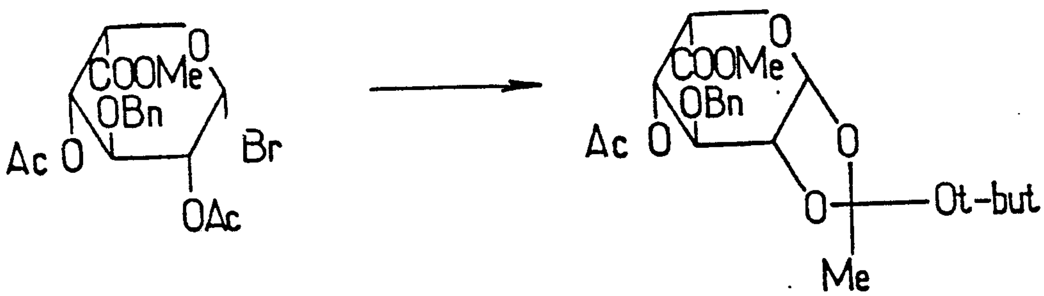

(36) (37)

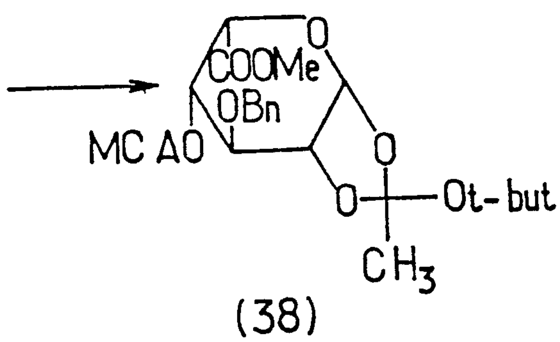

(38)

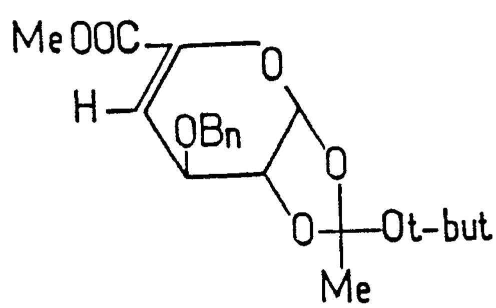

(39)

Fig.8.

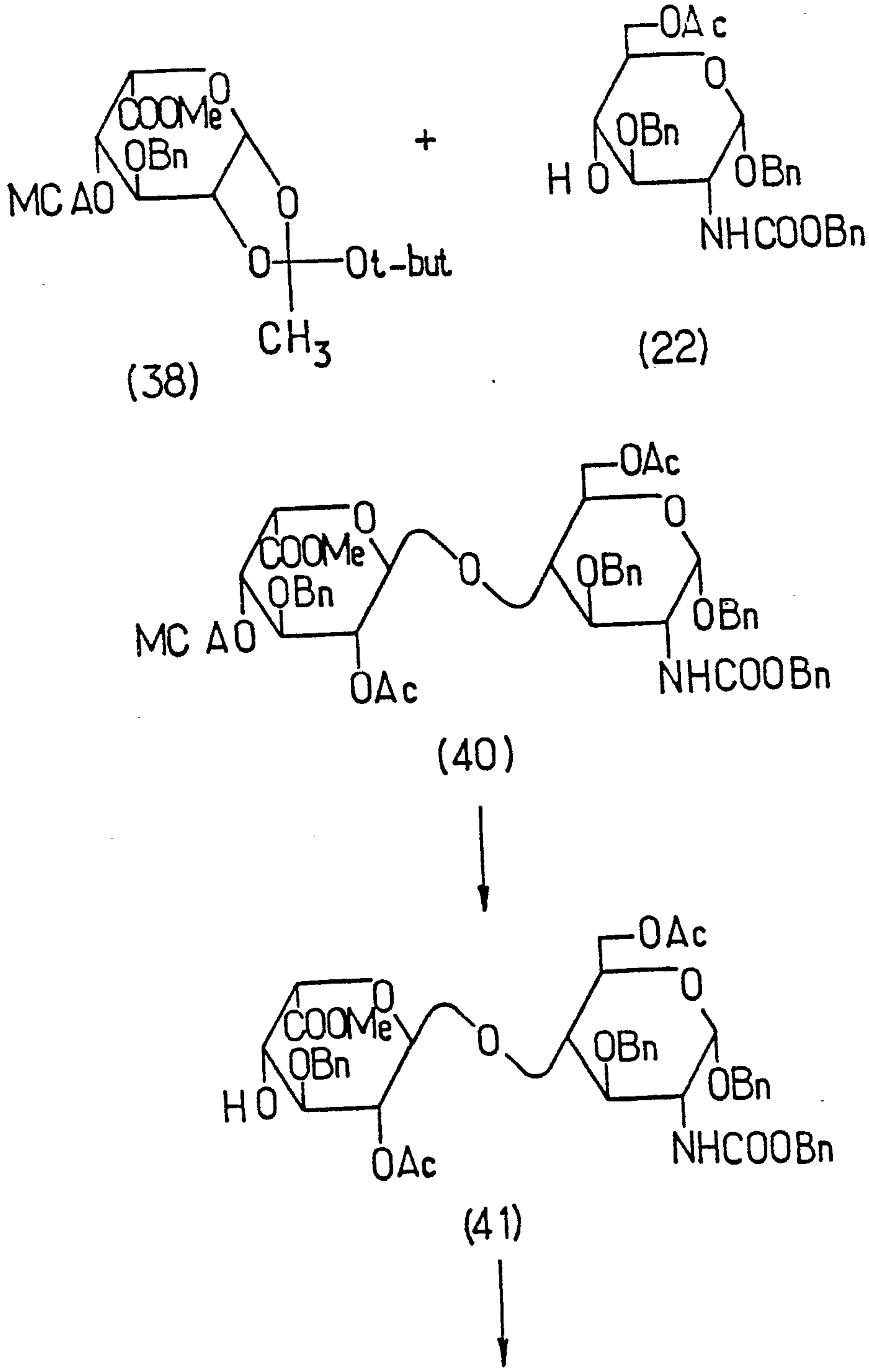

(38) + (22)

(40)

(41)

Fig. 9

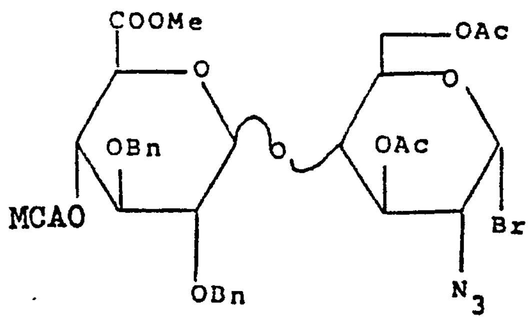

(20)

+

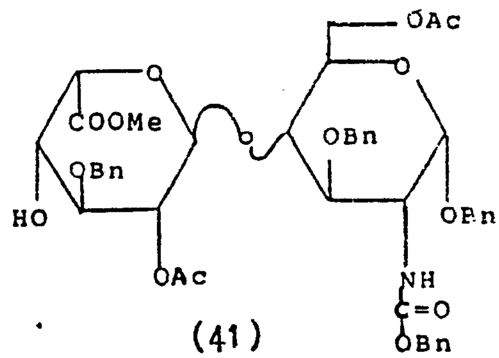

(41)

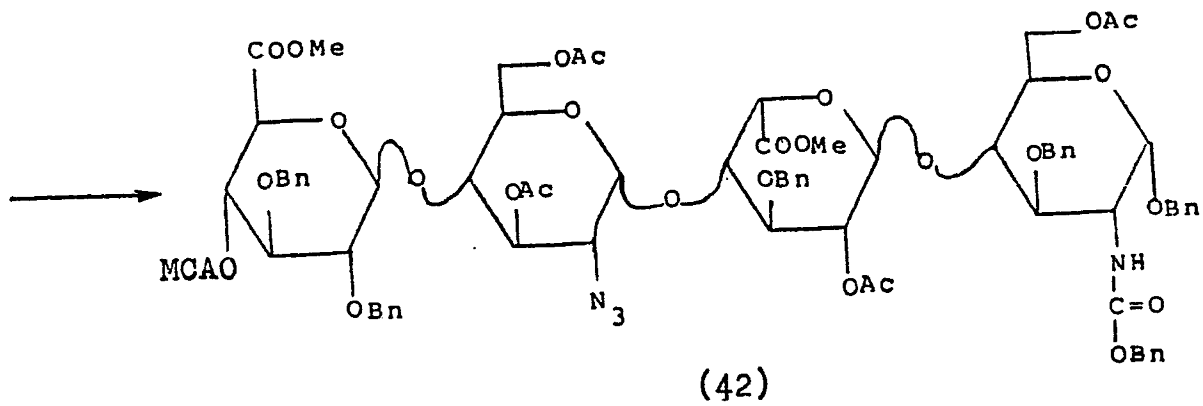

(42)

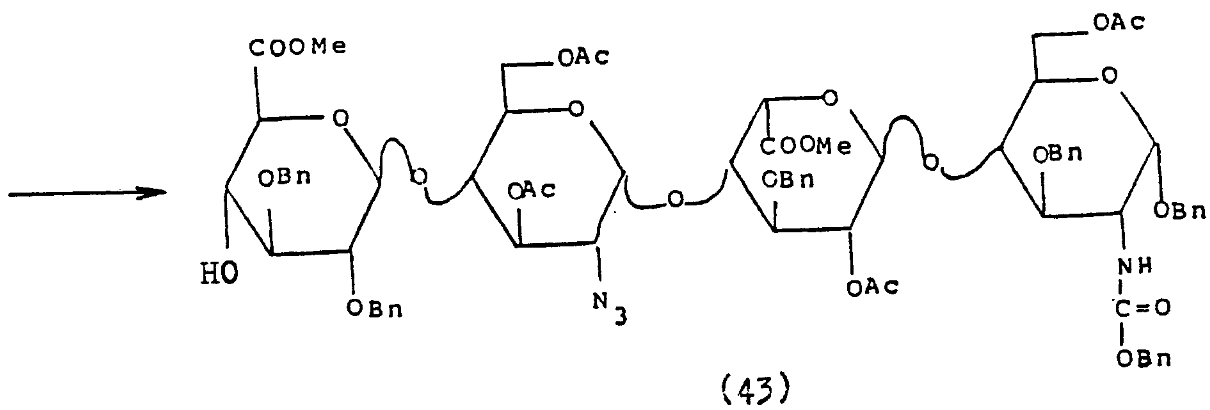

(43)

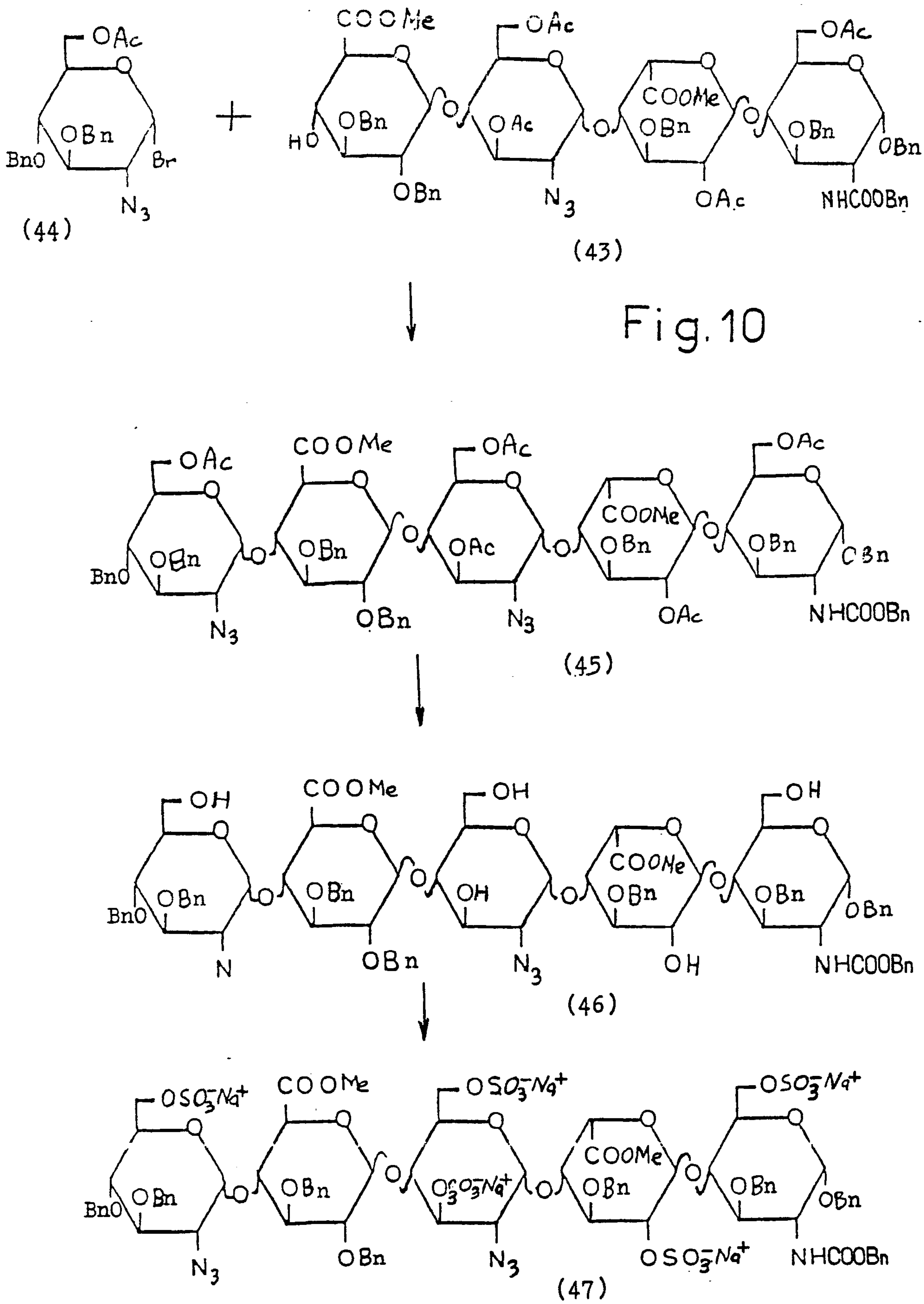

(44) (43)

Fig. 10

(45)

(46)

(47)

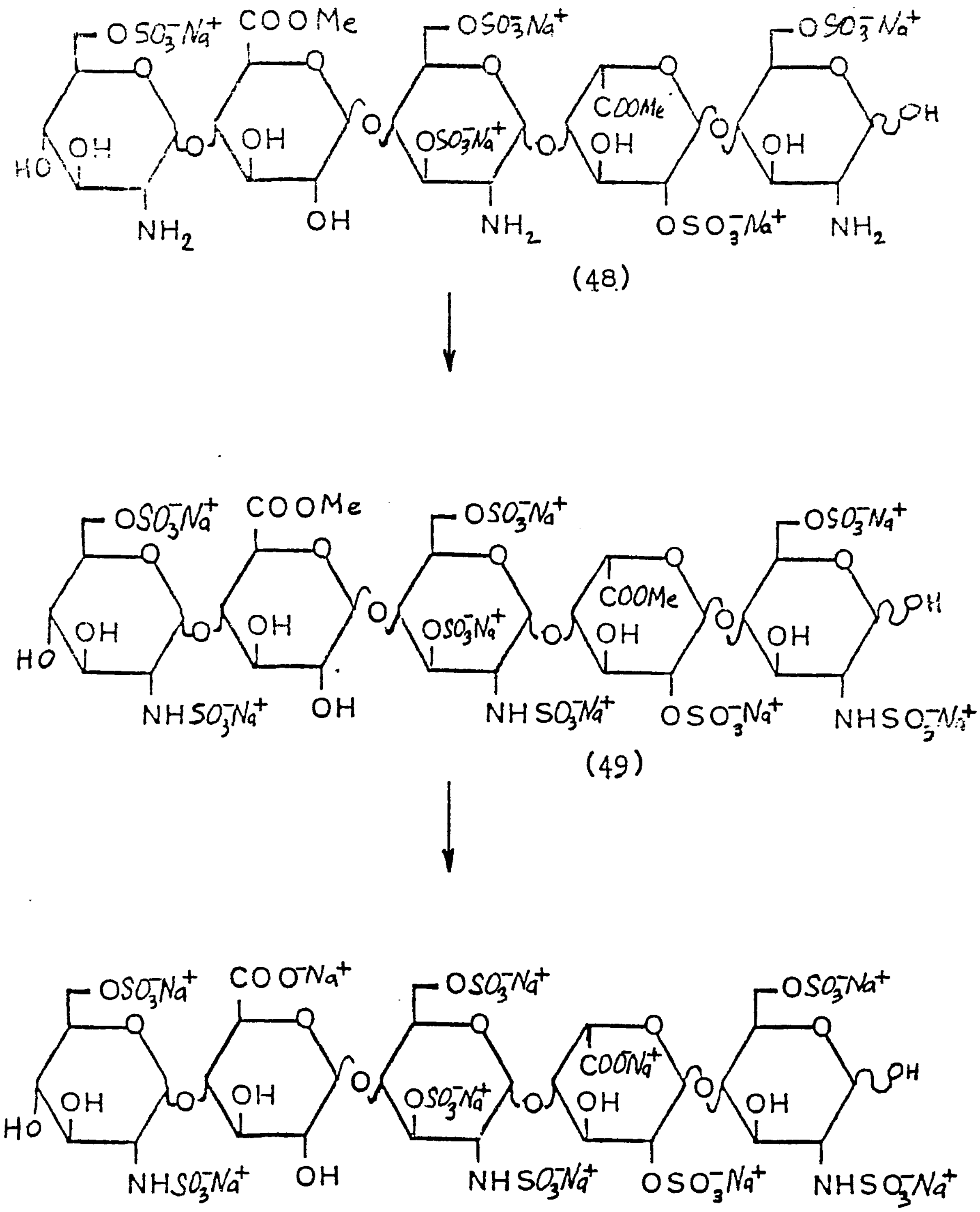

(48)

(49)

(50)

Fig.11

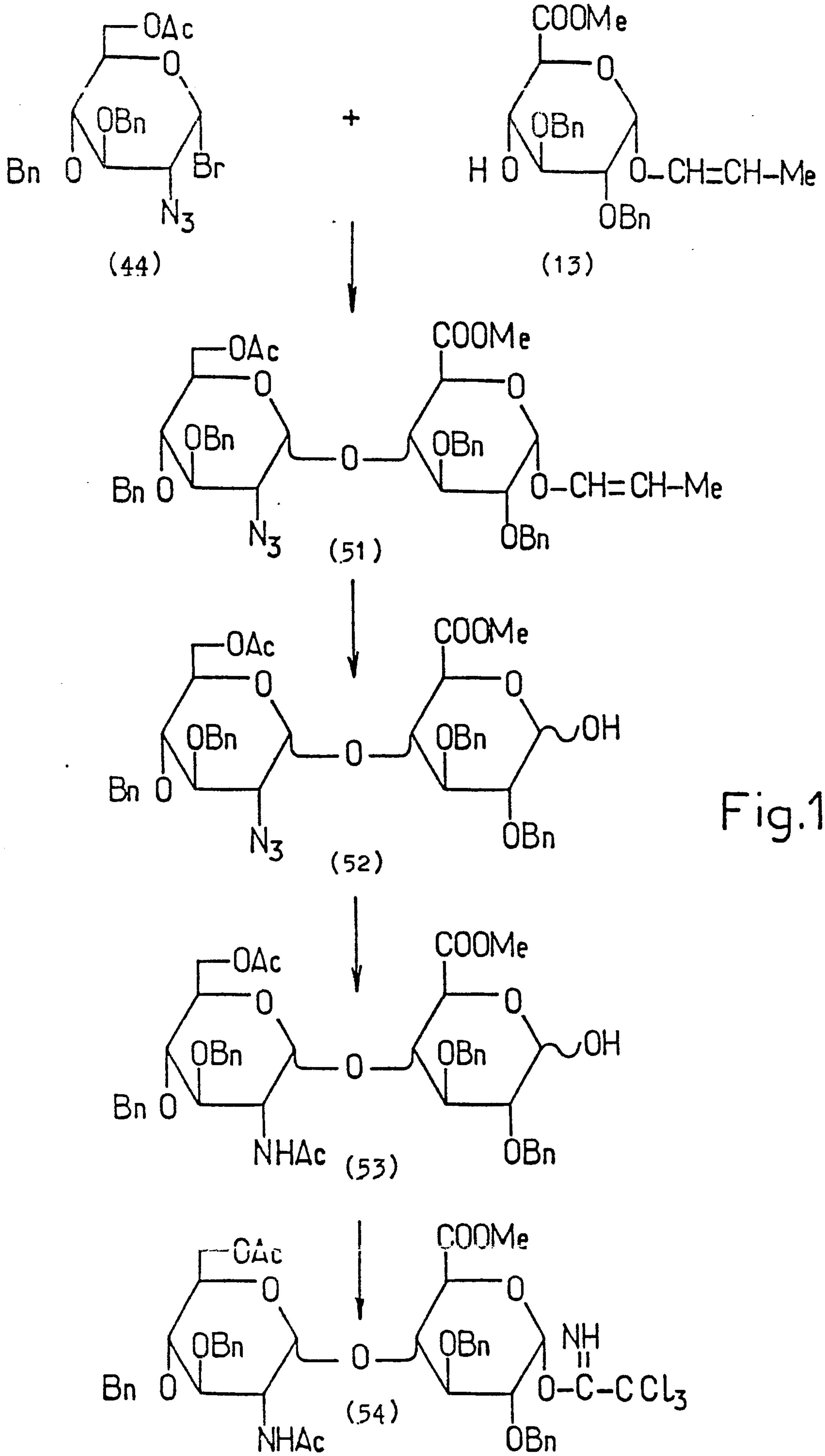

(44) + (13) → (51) → (52) → (53) → (54)

Fig.12

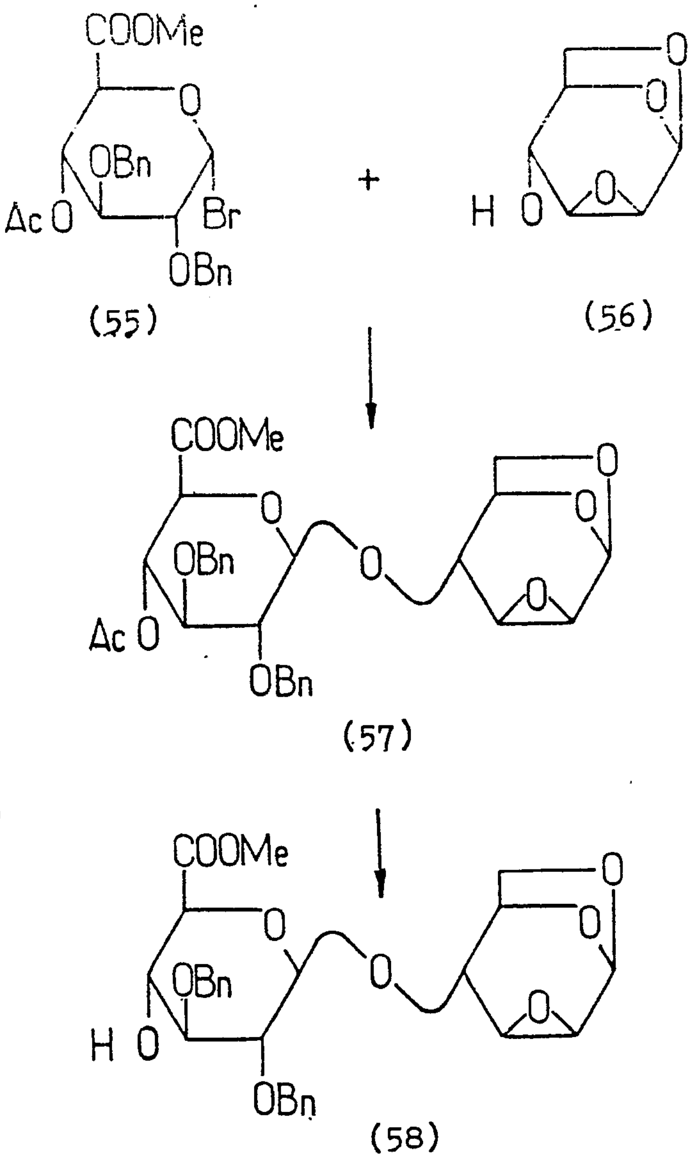

(55) (56) (57) (58)

Fig.13

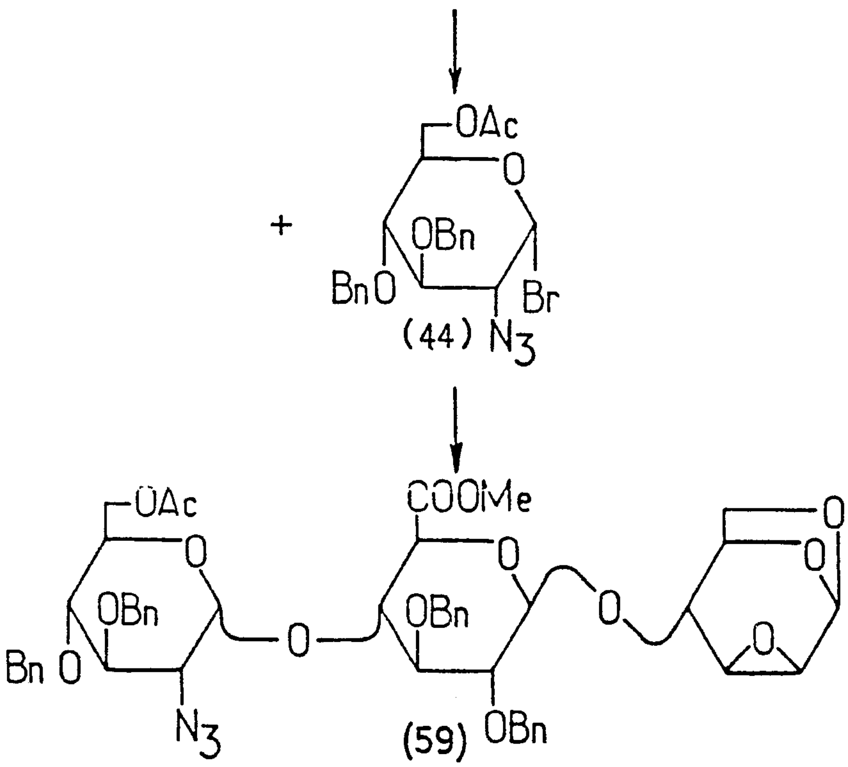

(44) (59)

Fig.13a.

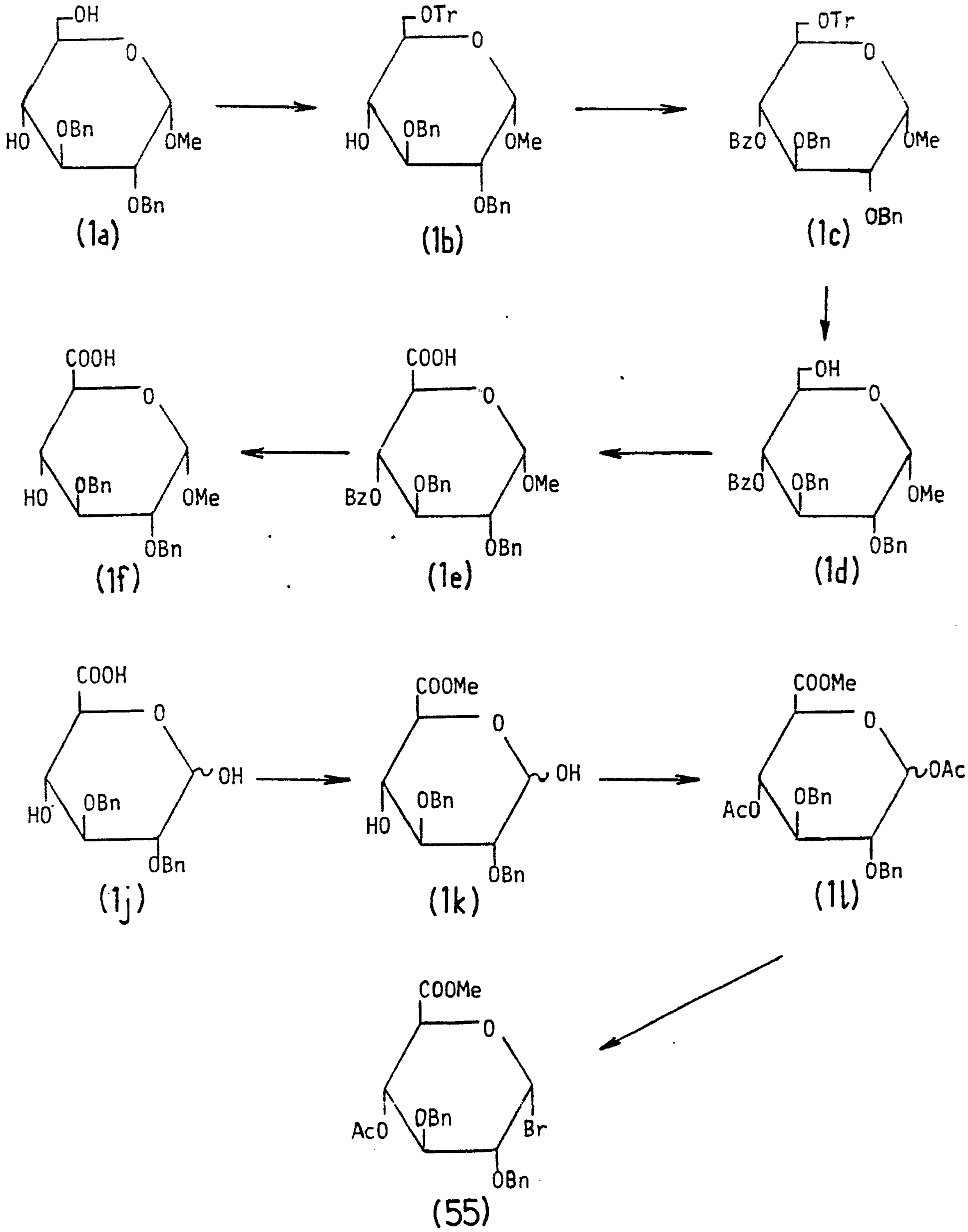

(1a) (1b) (1c) (1d) (1e) (1f) (1j) (1k) (1l) (55)

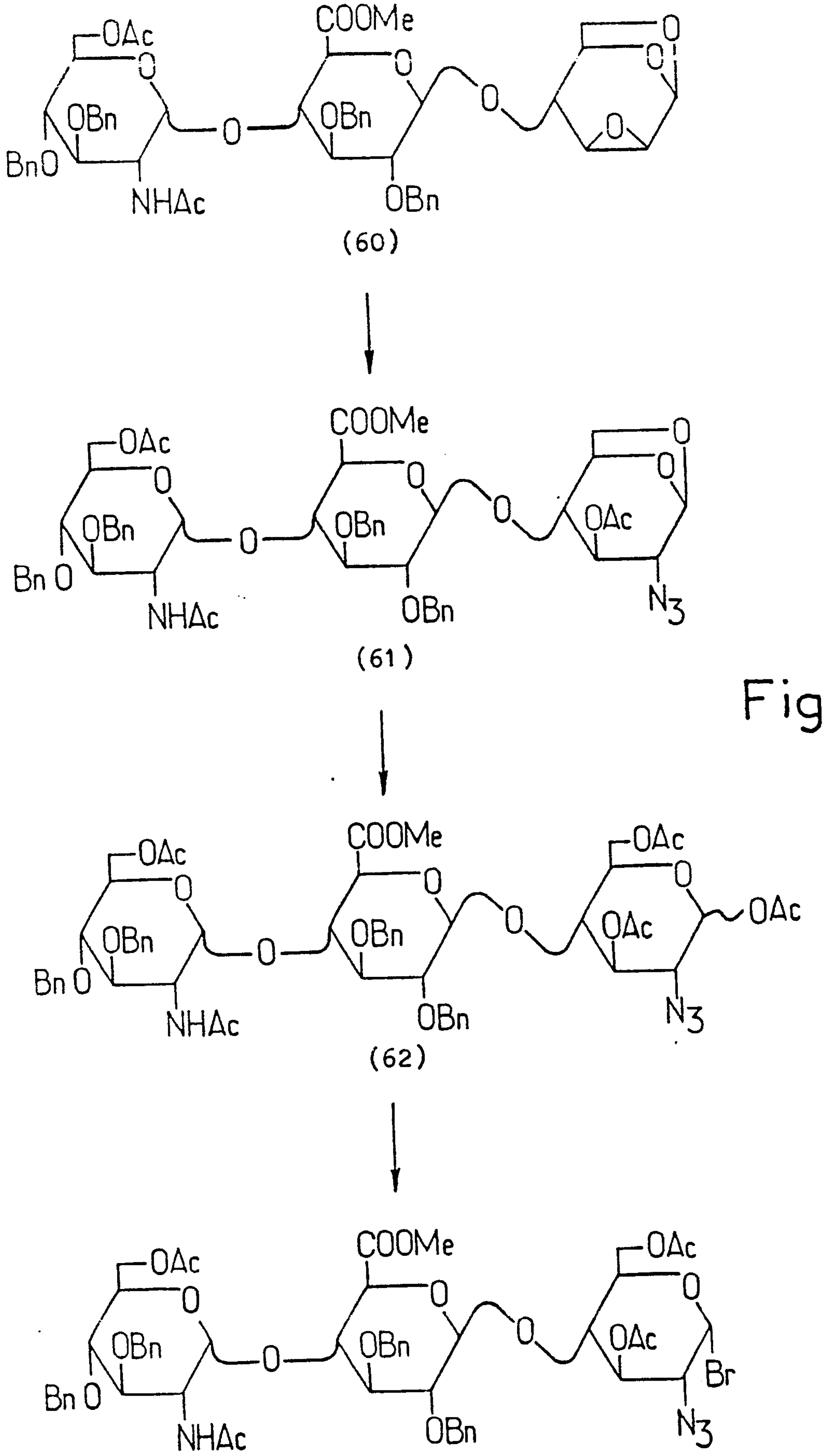

(60)

(61)

Fig.14

(62)

(63)

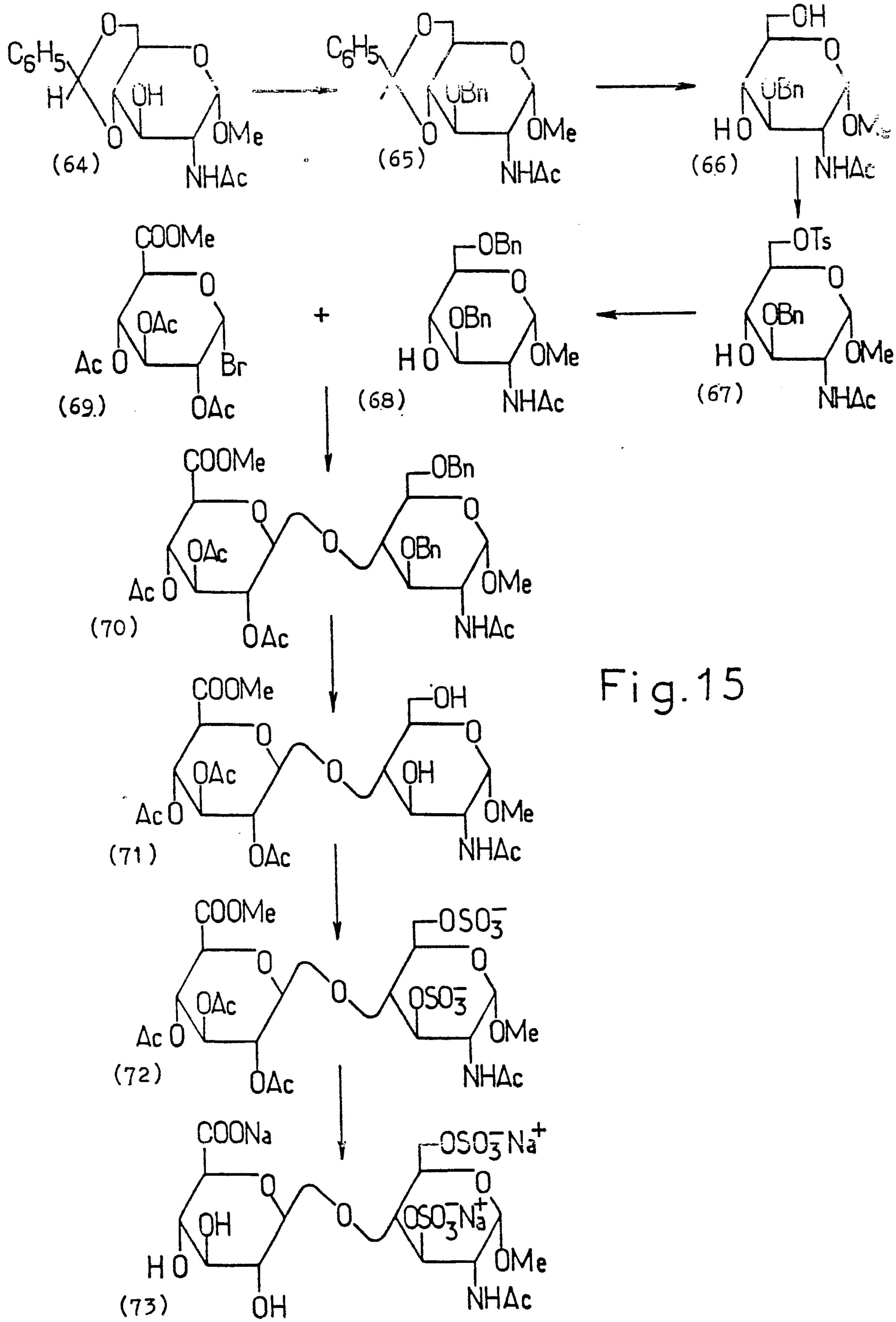

(64) (65) (66) (67) (68) (69) (70) (71) (72) (73)

Fig. 15

Fig.16.

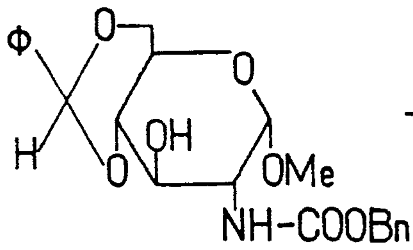

(74)

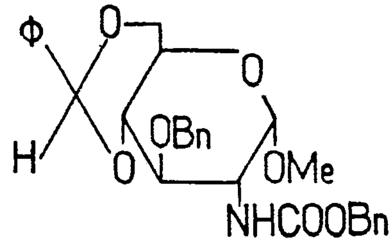

(75)

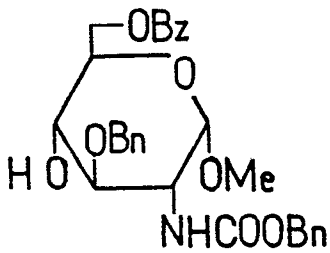

(77)

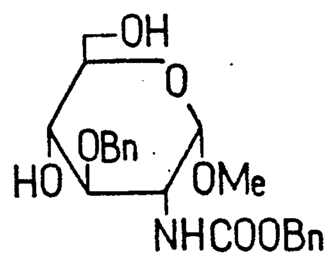

(76)

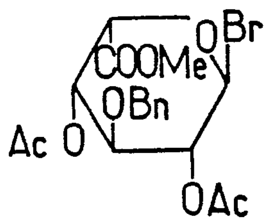

(36)

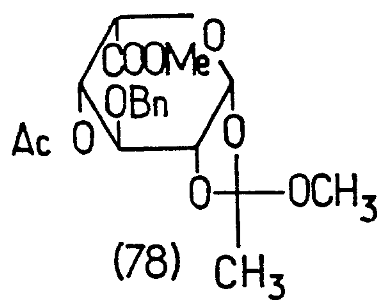

(78)

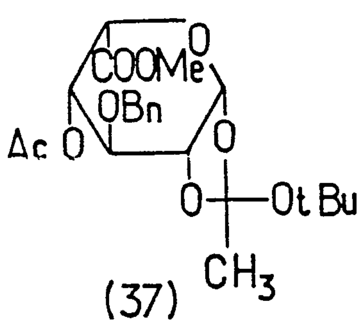

(37)

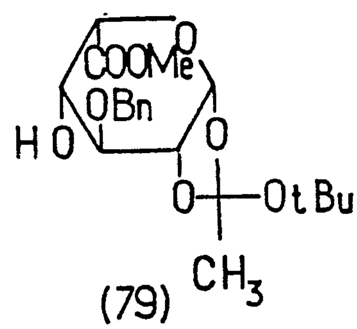

(79)

Fig.17.

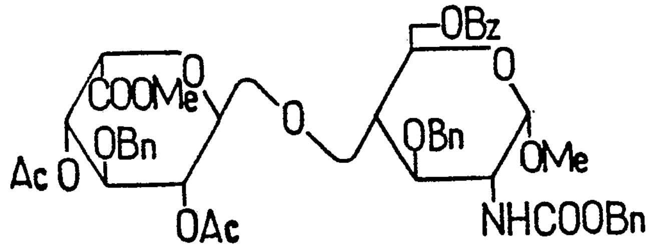

(81)

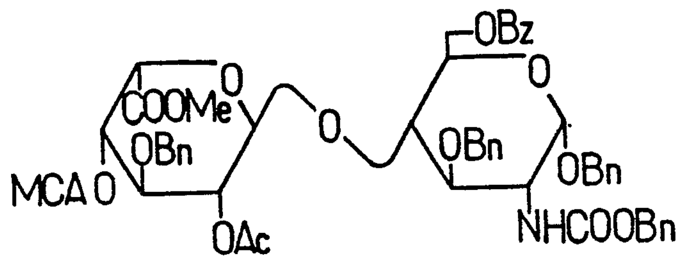

(82)

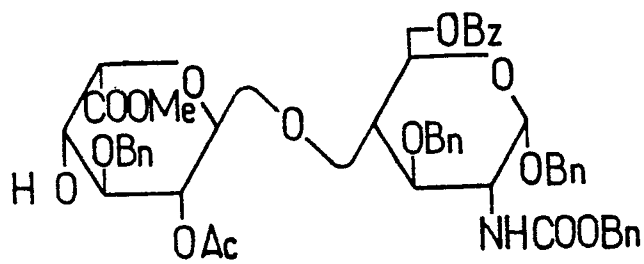

(83)

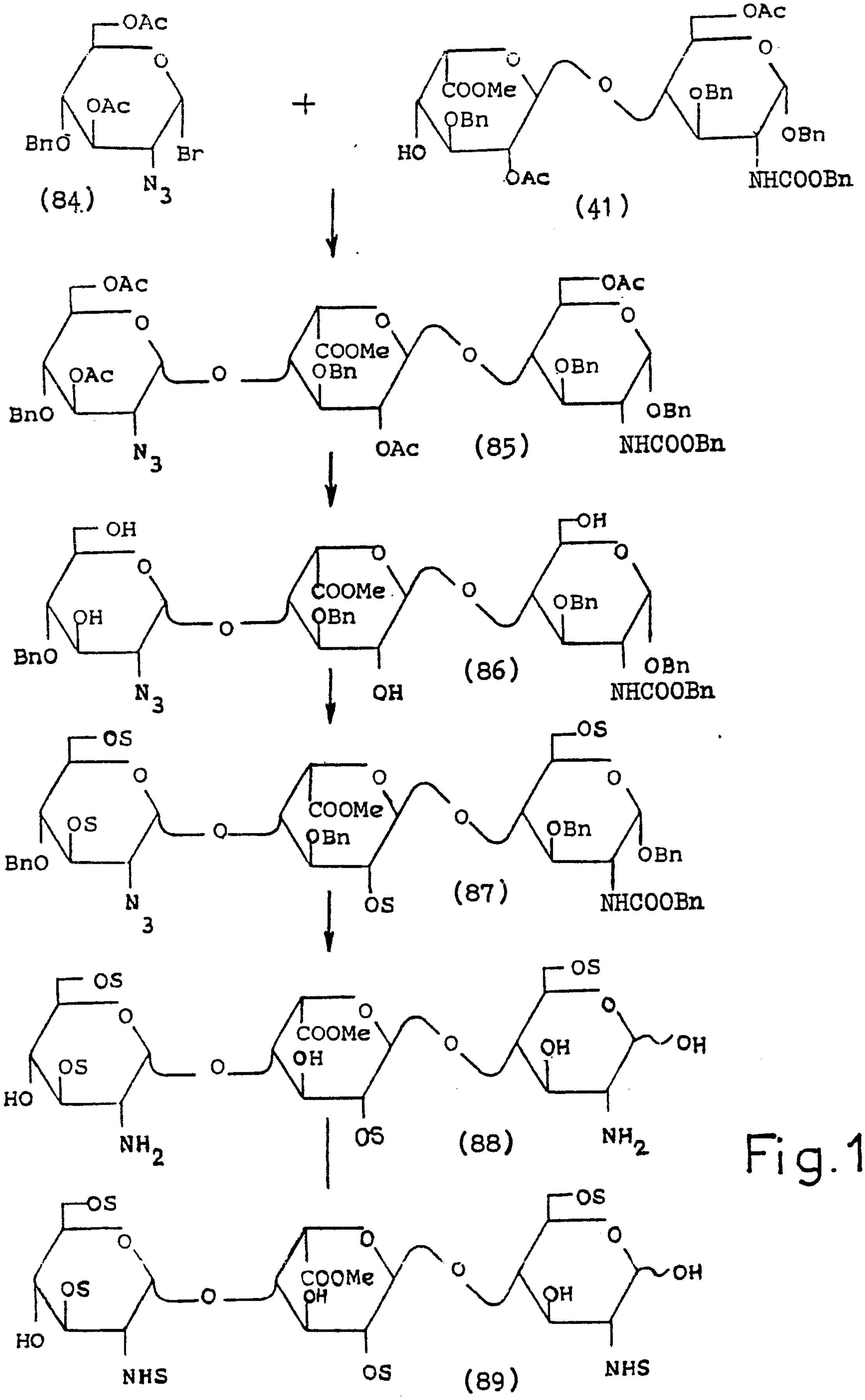

(84) (41) (85) (86) (87) (88) (89)

Fig.18

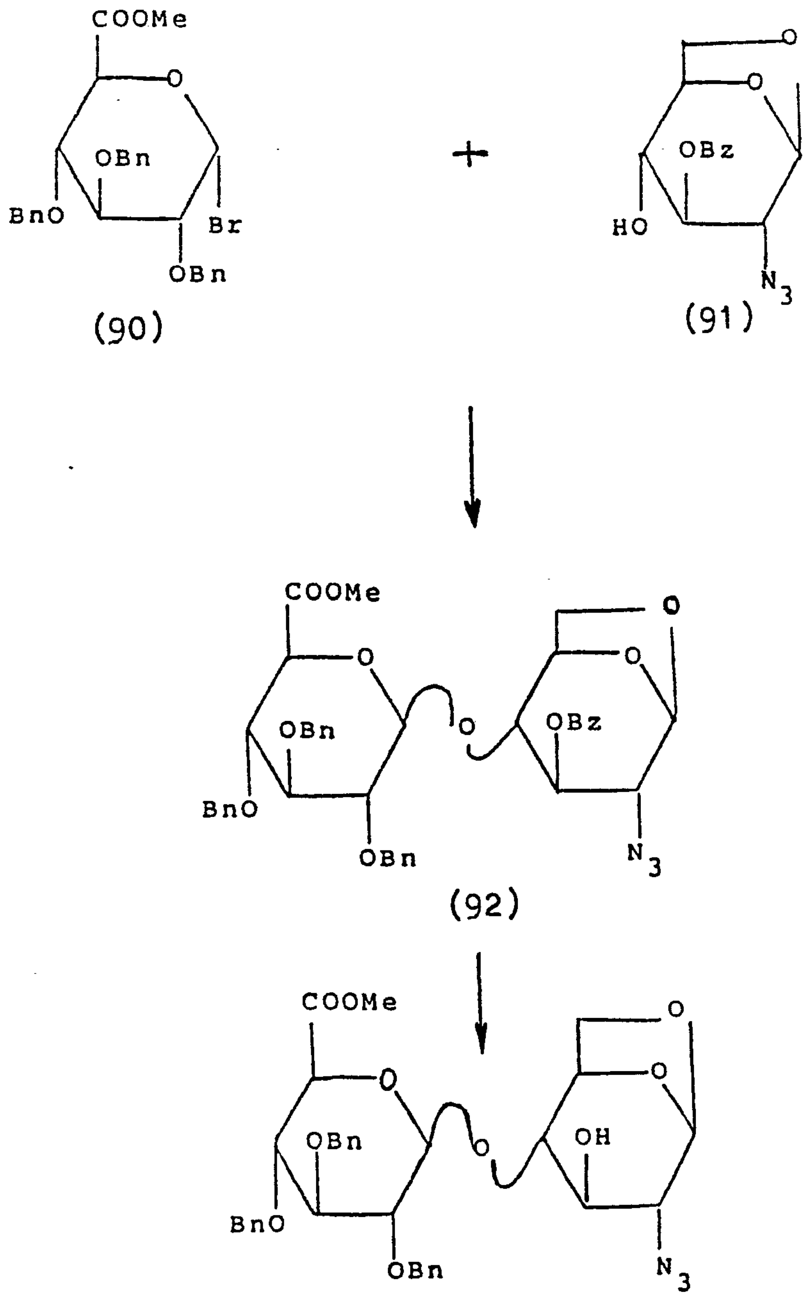

(90)

(91)

(92)

(93)

Fig. 19.

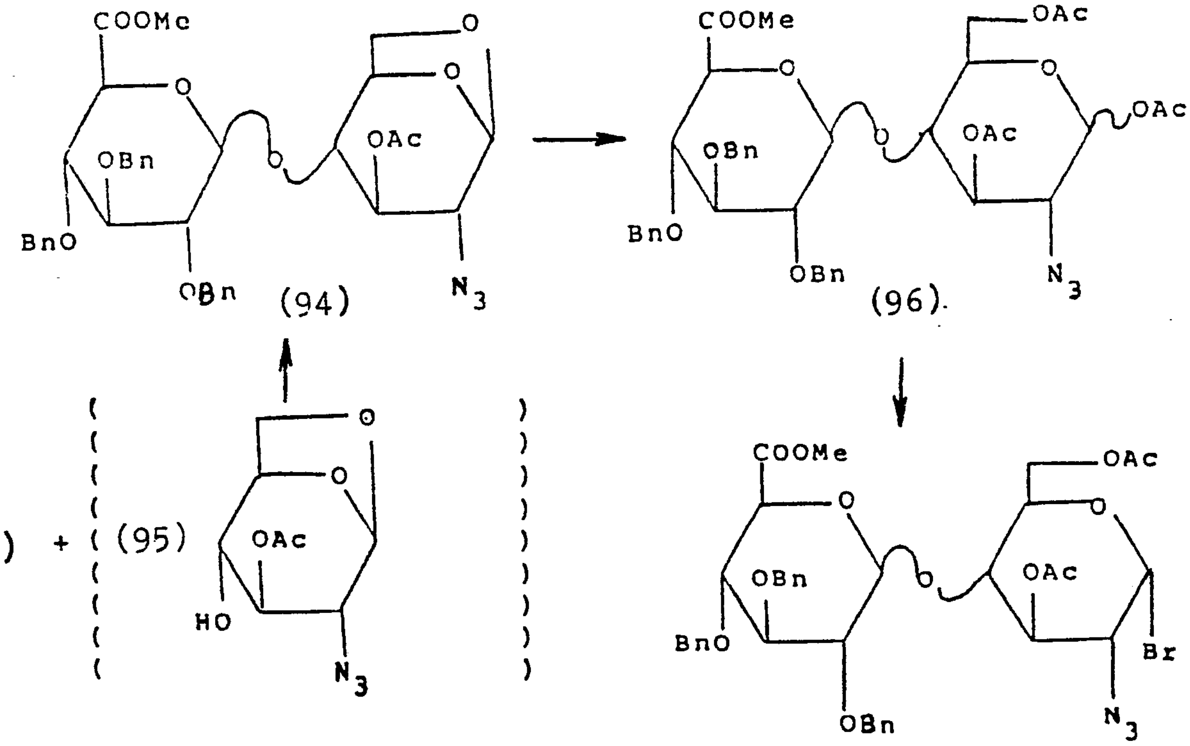

(94)

(90) + (95)

(96)

(97)

Fig. 20

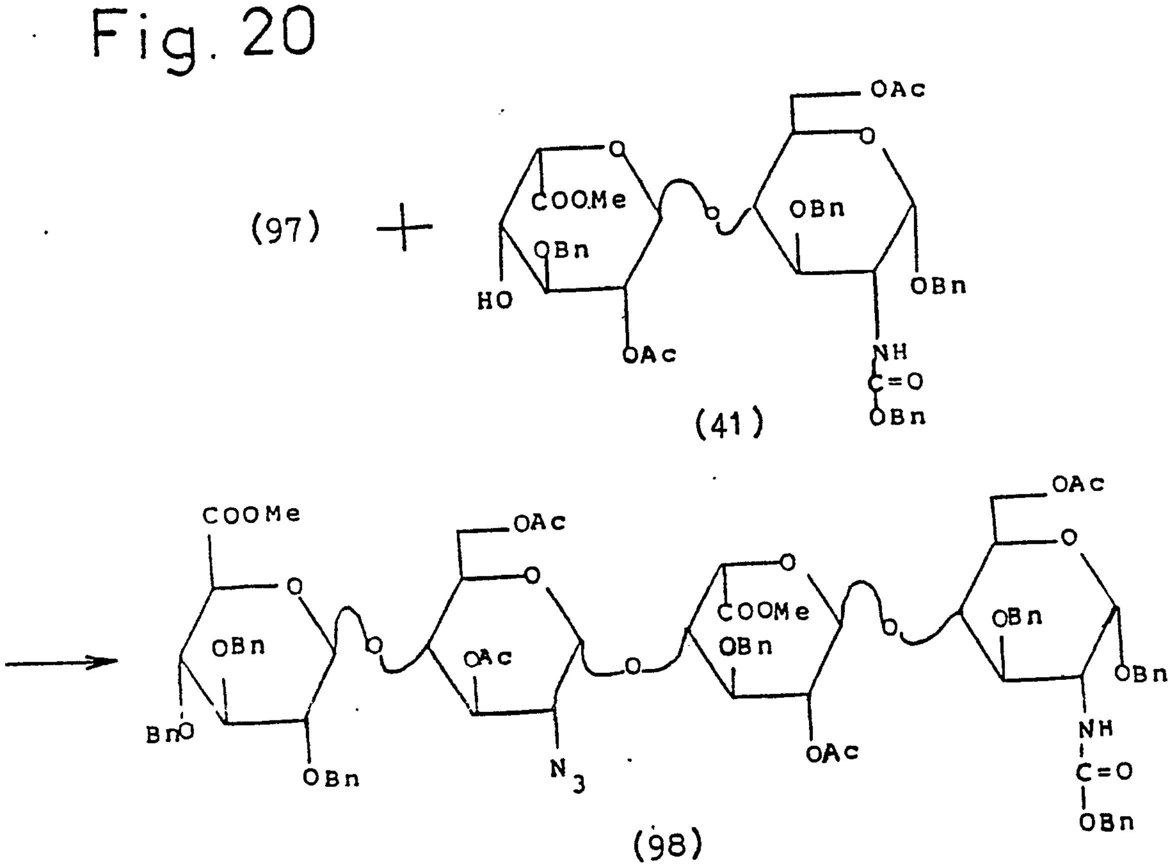

(97) +

(41)

(98)

Fig.21.

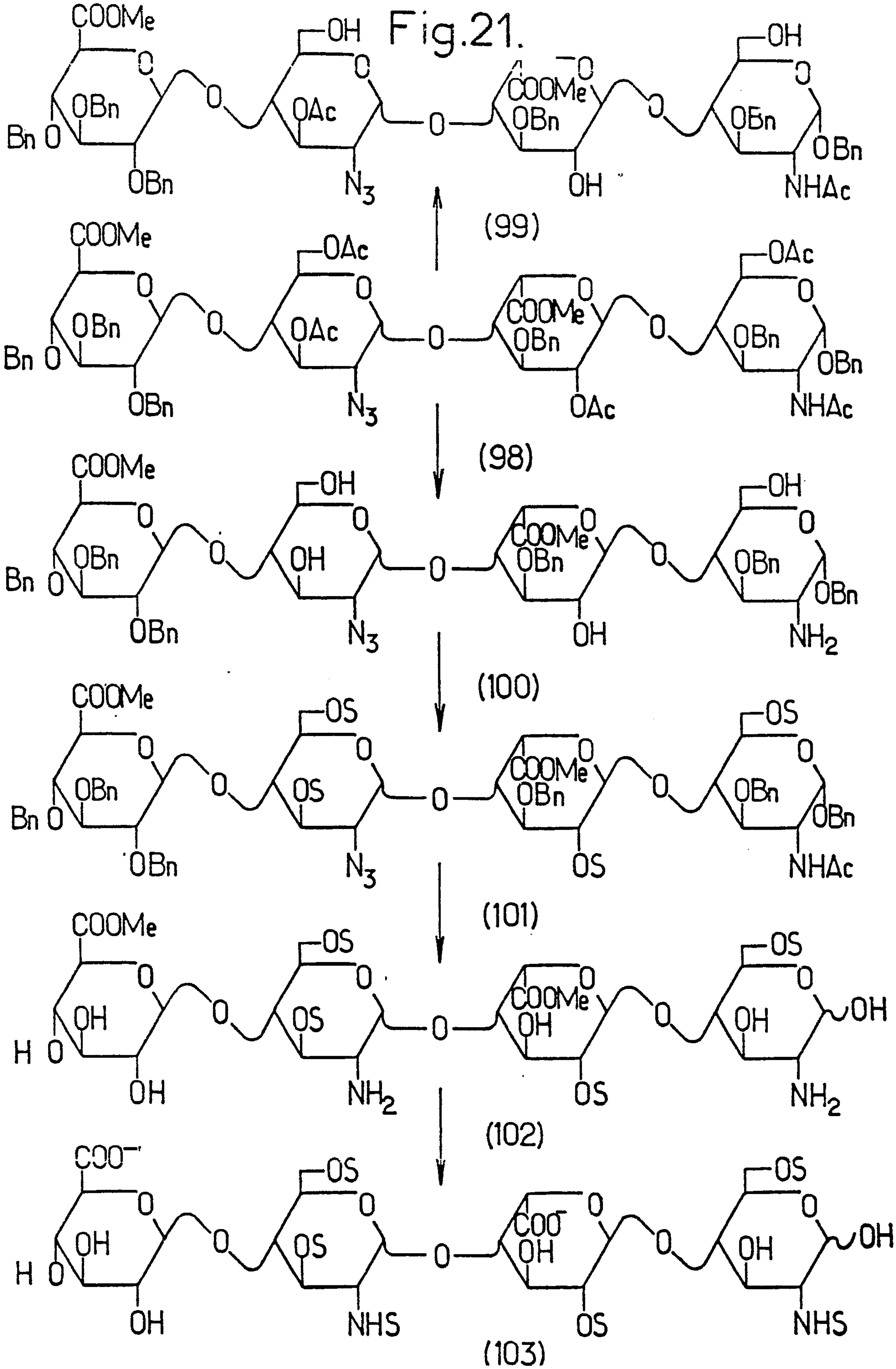

(99)

(98)

(100)

(101)

(102)

(103)

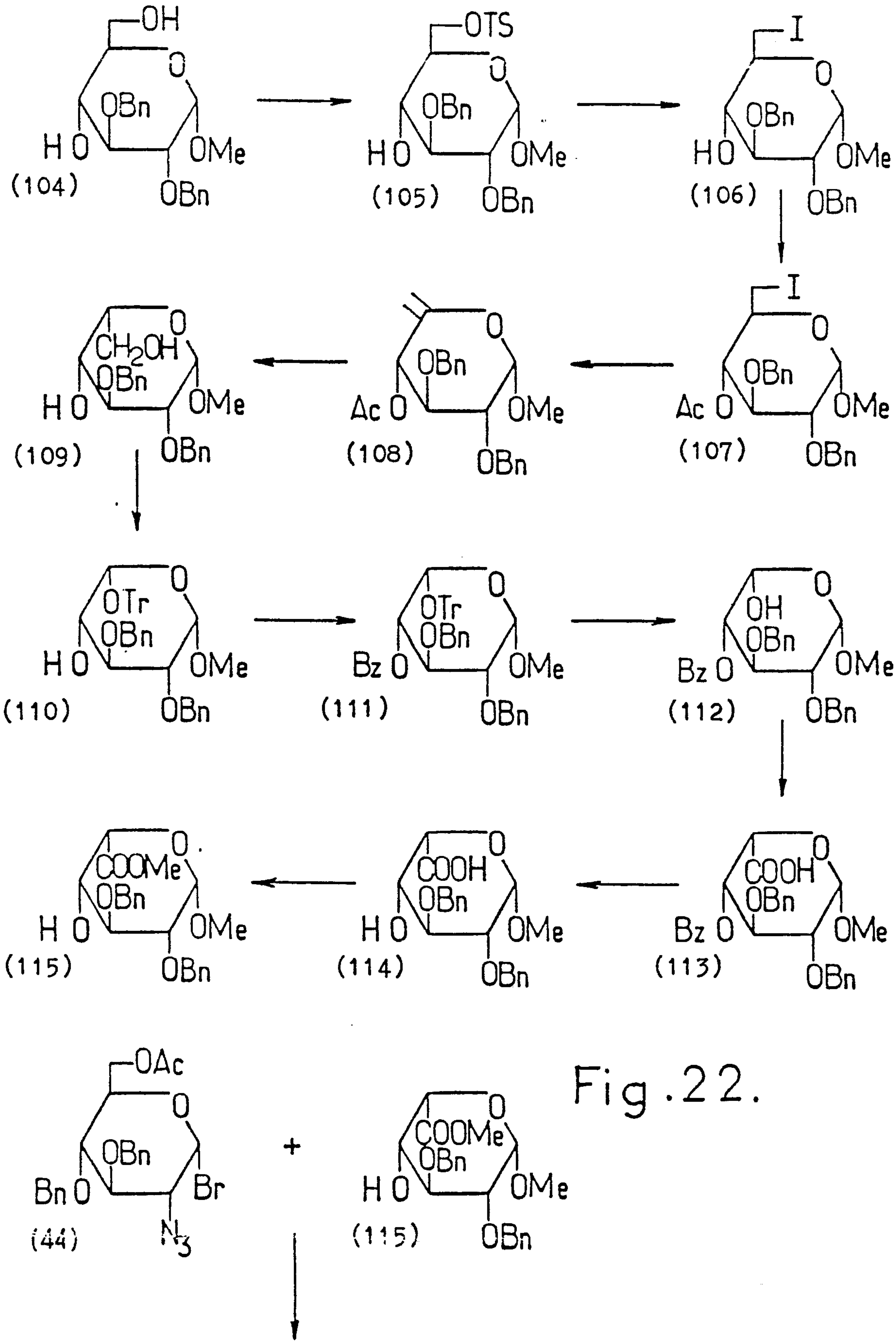

(104) (105) (106) (107) (108) (109) (110) (111) (112) (113) (114) (115)

(44) + (115)

Fig.22.

Fig. 23.

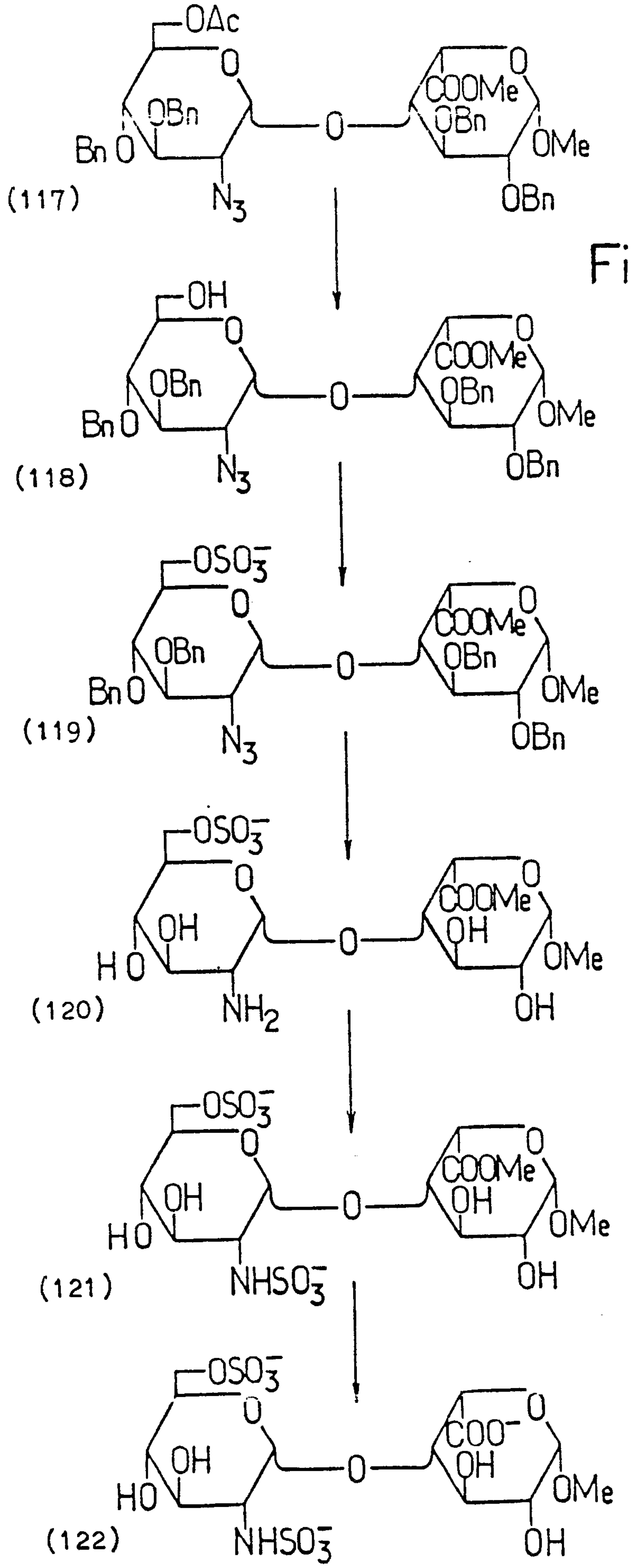

(117)

(118)

(119)

(120)

(121)

(122)

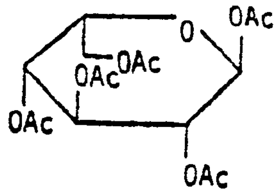

(123)

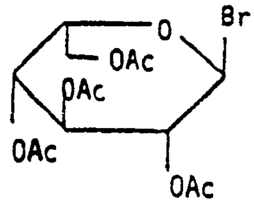

(124)

Fig. 24

(124) +

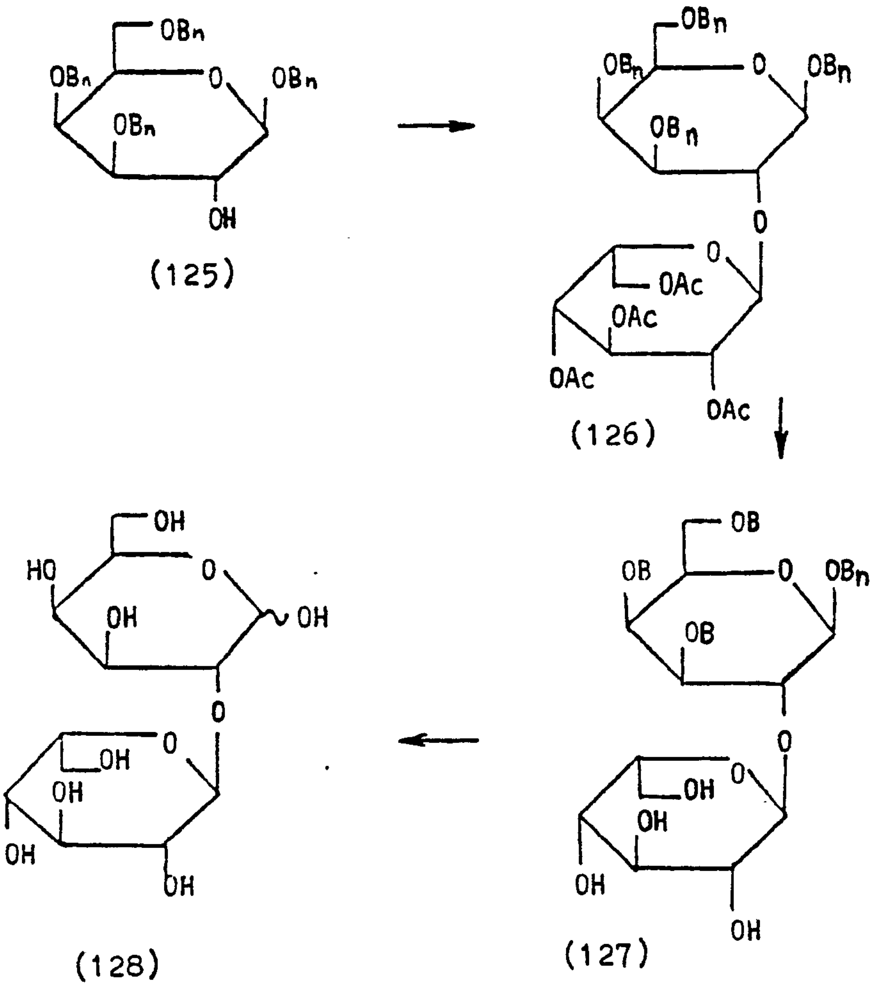

(125)

(126)

(127)

(128)

(124) +

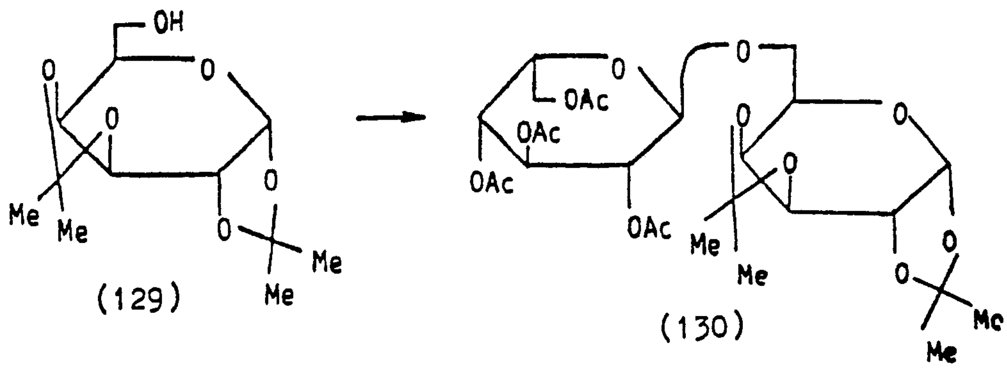

(129)

(130)

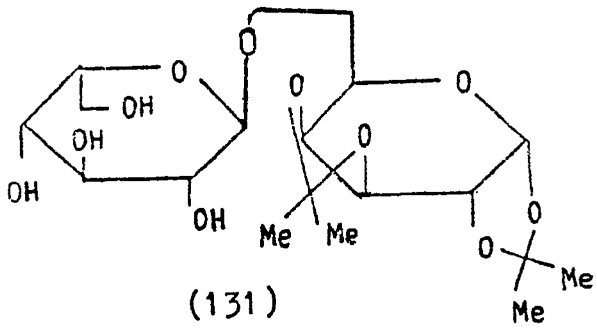

(131)

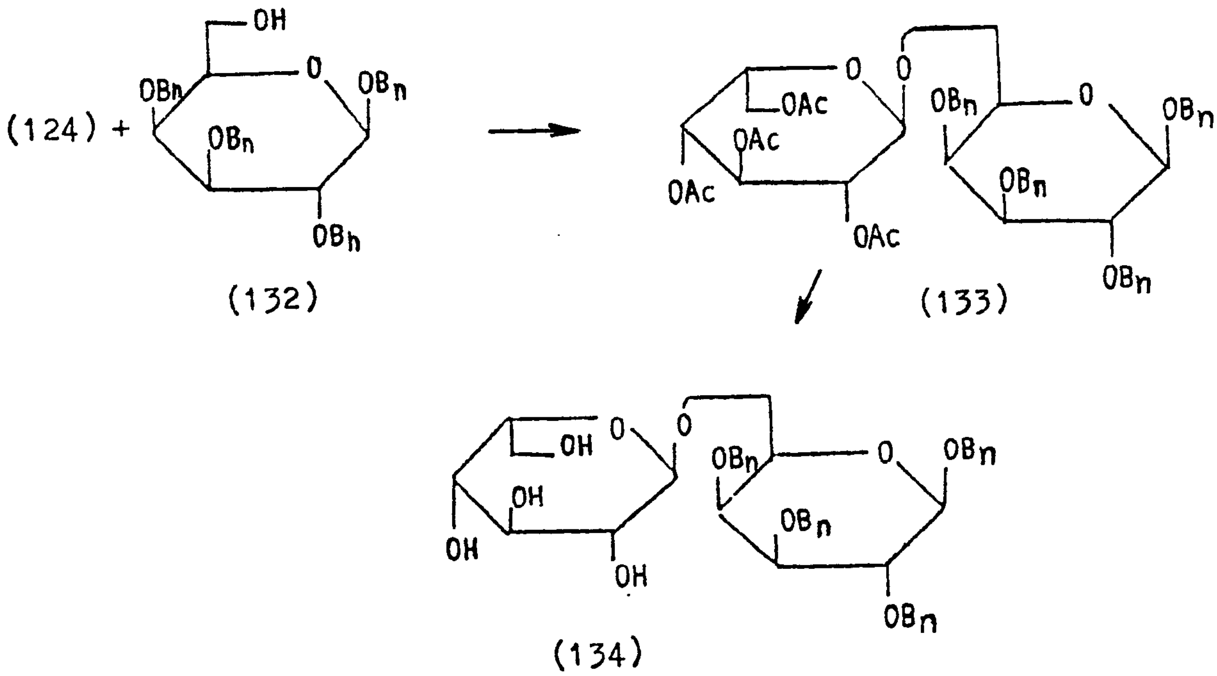

(124) +

(132)

(133)

(134)

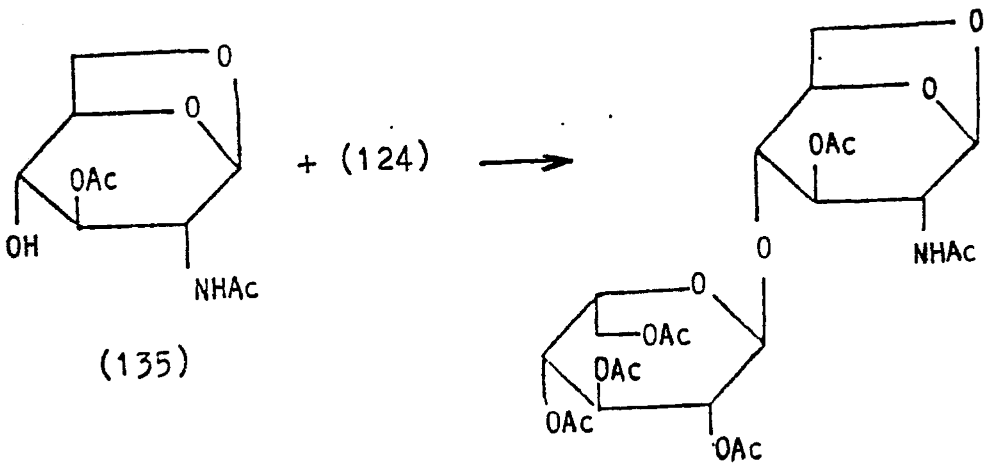

+ (124)

(135)

(136)

Fig. 25.

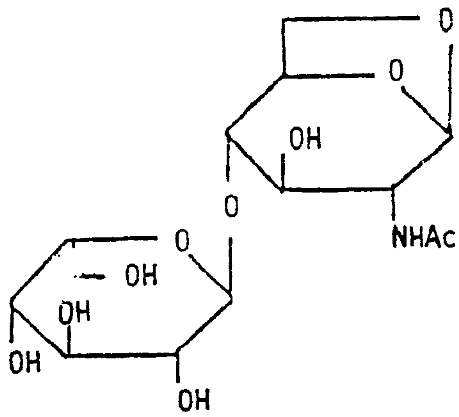

(137)

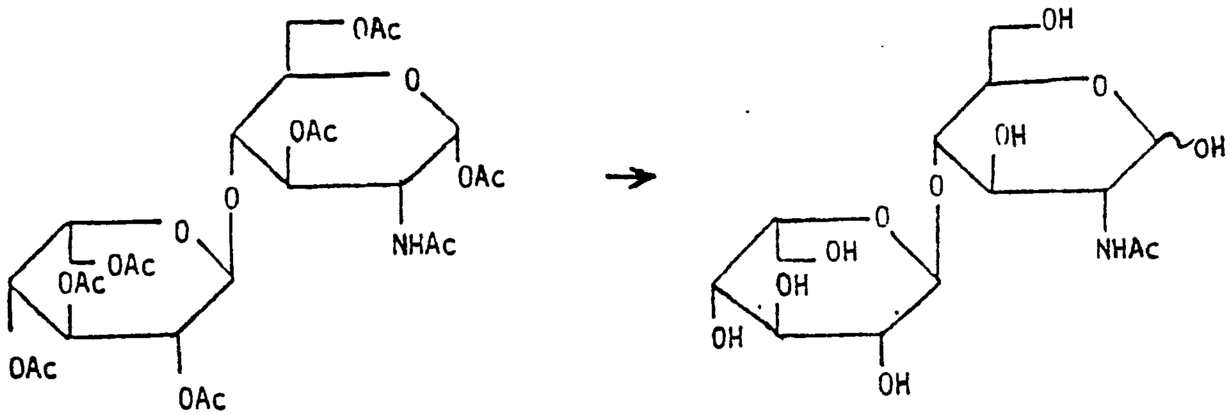

(138)

(139)

Fig. 26.

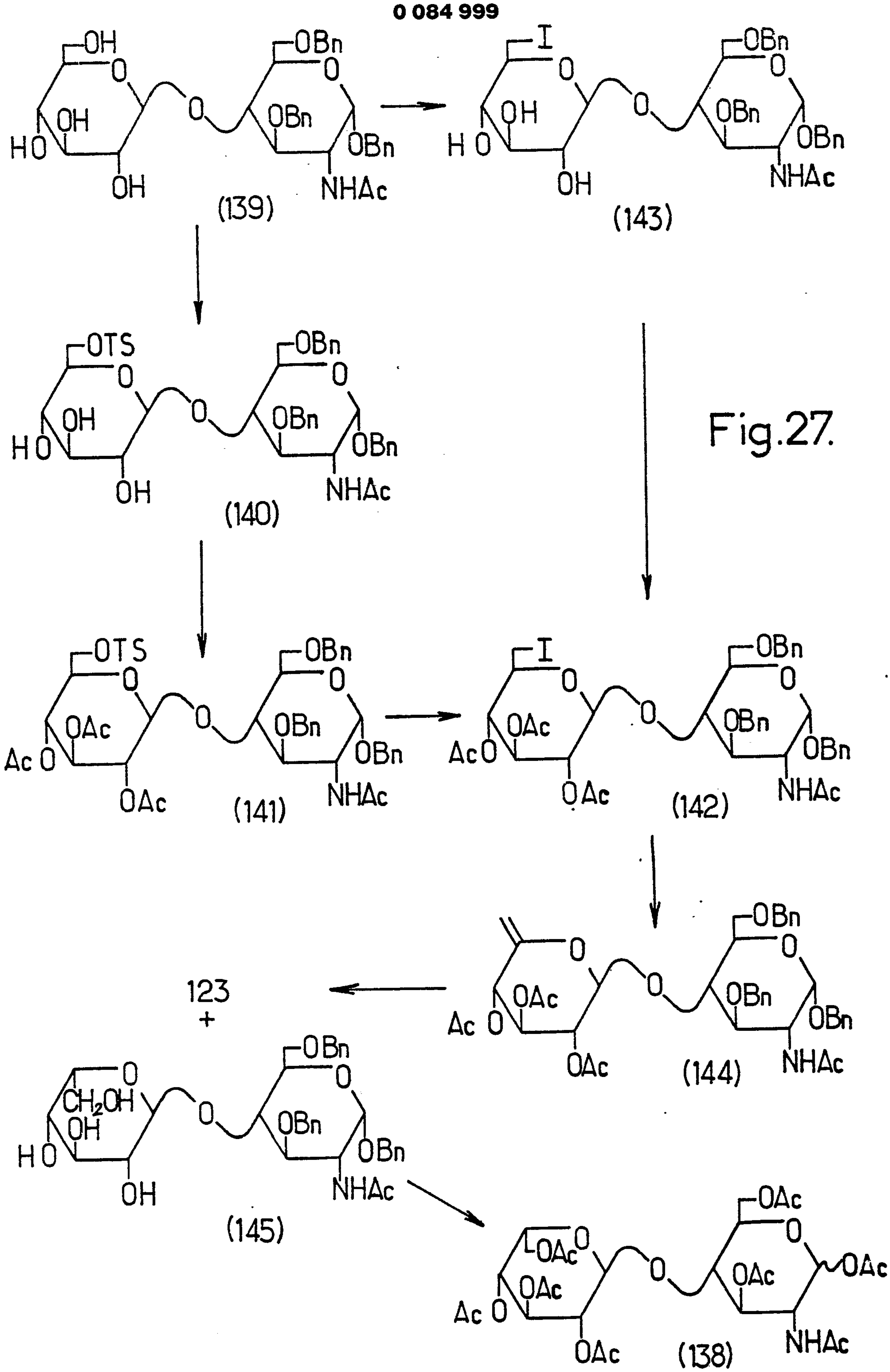

(139)

(143)

(140)

Fig.27.

(141)

(142)

(144)

123
+

(145)

(138)

Fig.28.

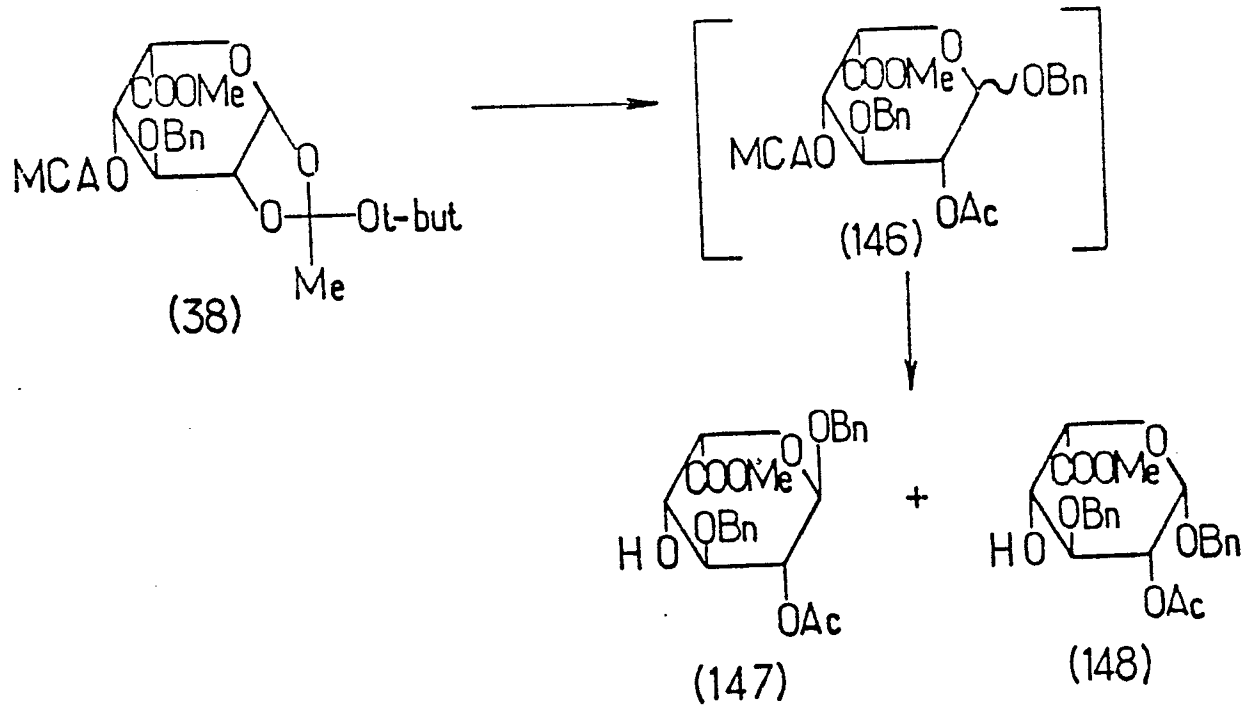

(38) (146) (147) + (148)

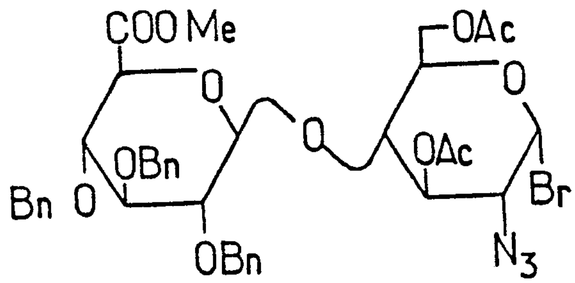

+ 147

(97)

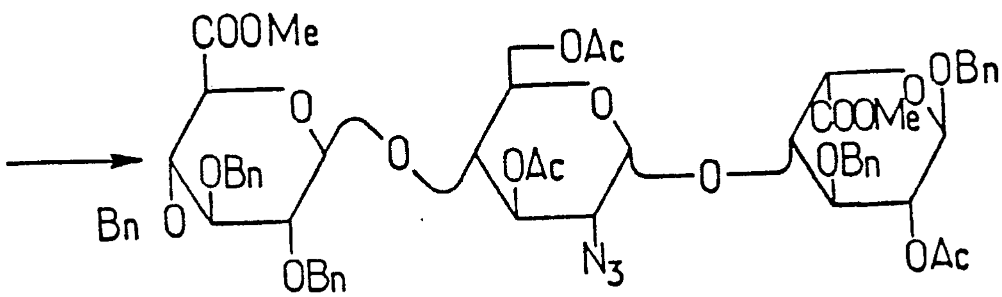

(149)

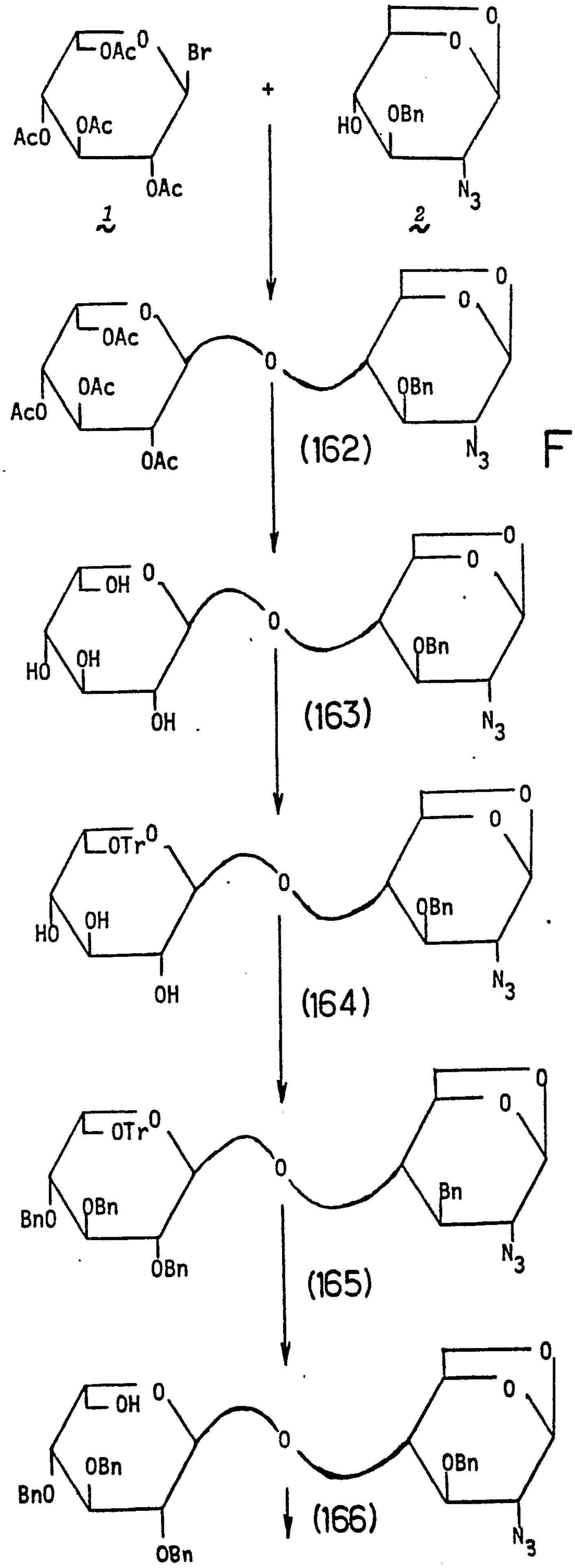


Fig. 29.

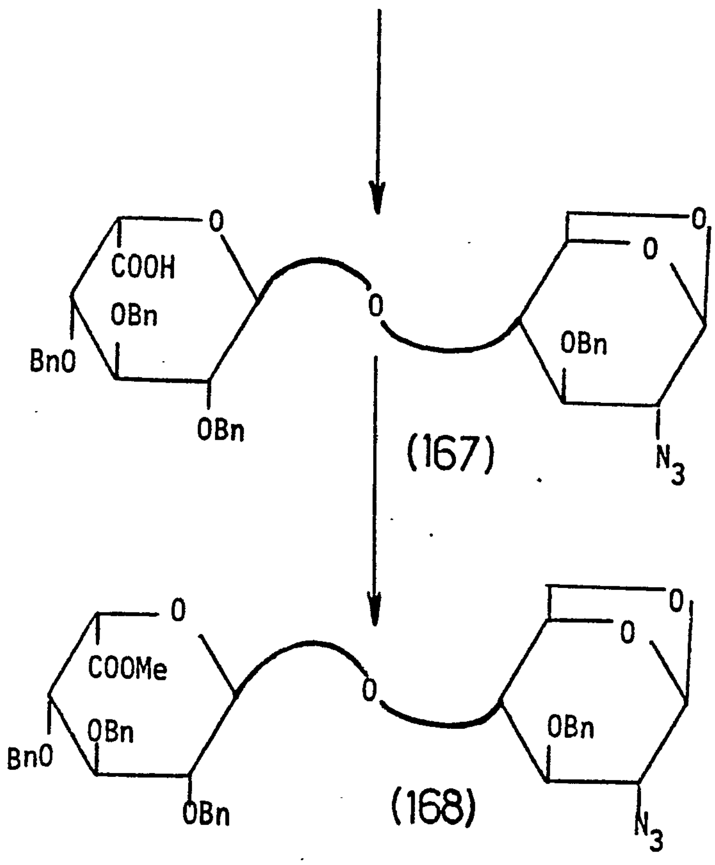

(167)

(168)

Fig.30.

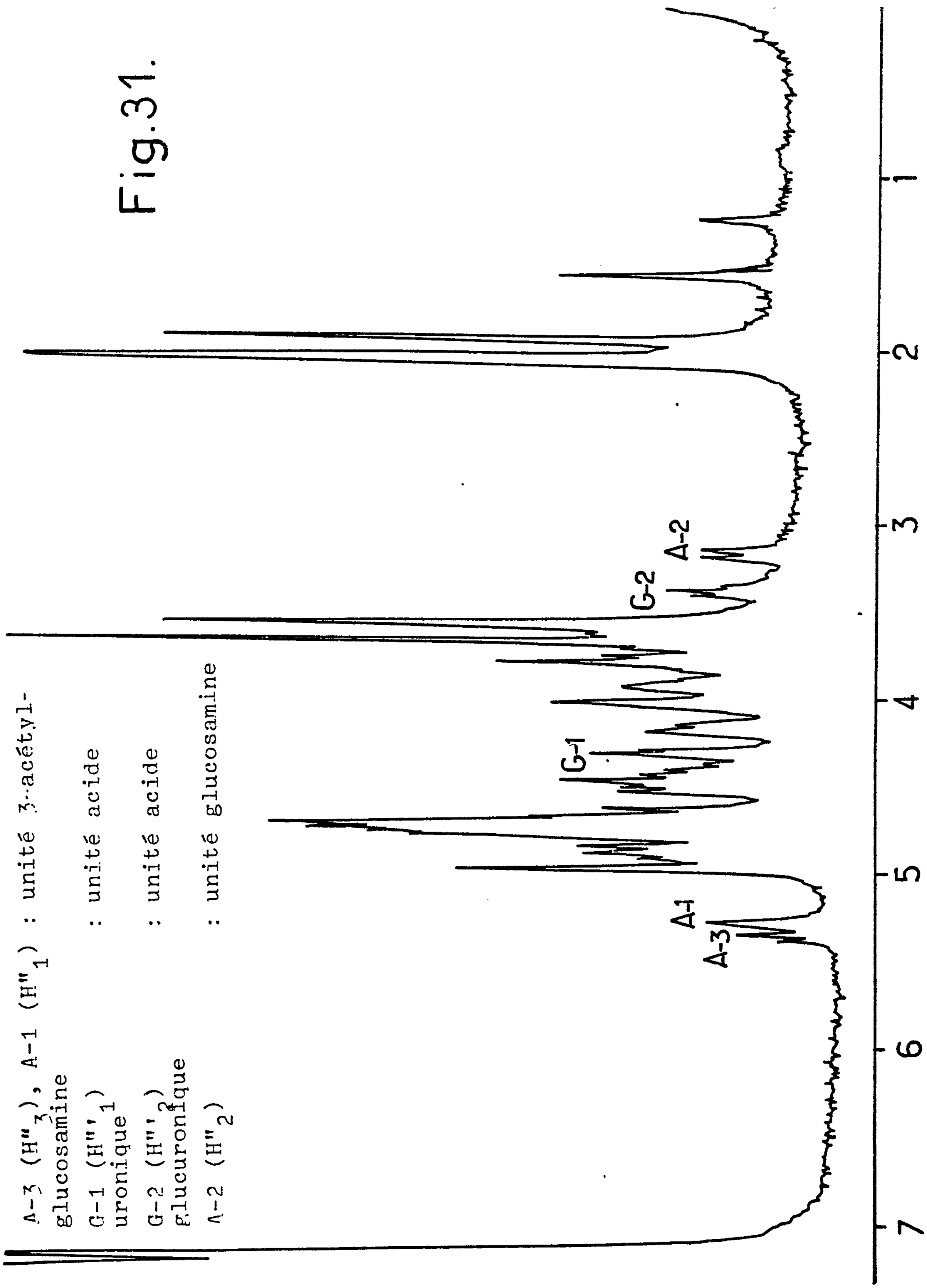
A-3 (H"3), A-1 (H"1) : unité 3-acétyl-glucosamine
G-1 (H"'1) : unité acide uronique
G-2 (H"'2) : unité acide glucuronique
A-2 (H"2) : unité glucosamine
A-3
A-1
G-1
G-2
A-2
7
6
5
4
3
2
1

Fig.31.

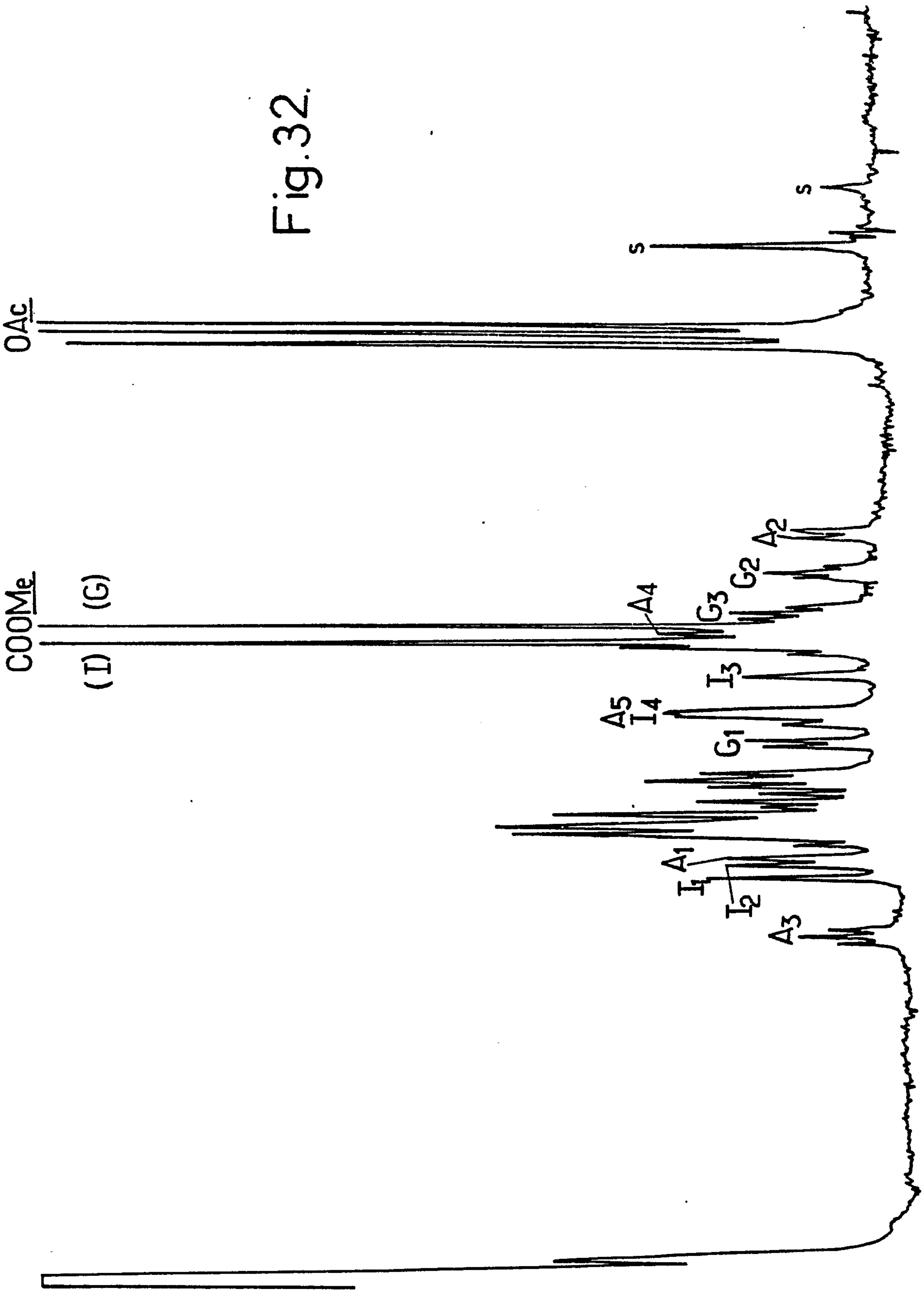


Fig. 32.